# EUROPEAN PATENT APPLICATION

(11) **EP 3 699 179 A1**
(43) Date of publication of application: **26.08.2020**
(21) Application number: 18867503.7
(22) Date of filing: 18.10.2018
(51) Int. Cl.: C07D 471/04, C07D 487/04, C07D 471/14, C07D 519/00, A61K 31/415, A61K 31/4162, A61P 35/00, A61P 35/02

(54) **PYRAZOLYL-CONTAINING TRICYCLIC DERIVATIVE, PREPARATION METHOD THEREFOR AND USE THEREOF**

(30) Priority: 19.10.2017 CN 201710979926; 10.01.2018 CN 201810023817; 30.07.2018 CN 201810882539
(71) Applicant: Jiangsu Hansoh Pharmaceutical Group Co., Ltd., Lianyungang, Jiangsu 222047 (CN); Shanghai Hansoh Biomedical Co., Ltd., Shanghai 201203 (CN)
(72) Inventor: LIU, Shiqiang, Lianyungang Jiangsu 222047 (CN); ZHOU, Yuanfeng, Lianyungang Jiangsu 222047 (CN); LIU, Yang, Lianyungang Jiangsu 222047 (CN); WU, Xuesong, Lianyungang Jiangsu 222047 (CN); LIU, Lei, Lianyungang Jiangsu 222047 (CN); BAO, Rudi, Lianyungang Jiangsu 222047 (CN)
(74) Representative: EP&C
(86) International application number: PCT/CN2018/110795
(87) International publication number: WO 2019/076336

(57) **Abstract**

The present invention relates to pyrazolyl-containing tricyclic derivative, a preparation method therefor and the use thereof. In particular, the present invention relates to a compound as shown in the general formula (I), a preparation method therefor and a pharmaceutical composition containing the compound, and the use thereof as a protease such as ERK (MAPK) inhibitor in the treatment of cancers, bone diseases, inflammatory diseases, immunological diseases, nervous system diseases, metabolic diseases, respiratory diseases and heart diseases, wherein the definition of each substituent in the general formula (I) is the same as defined in the description.

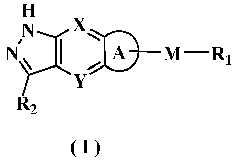

## Description

### FIELD OF THE INVENTION

The present invention belongs to the field of pharmaceutical synthesis, and specifically relates to a pyrazolyl-containing tricyclic derivative, a method for preparing the same and use thereof.

### BACKGROUND OF THE INVENTION

Members of the ERK signaling pathway, such as RAS and BRAF, often mutate in tumors. Approximately one-third of human tumors express persistently activated mutant RAS, and 8% of tumors express activated BRAF. Mutation and probability statistics related to the ERK signaling pathway in malignant tumors are shown in Table 1. According to statistics, 90% of pancreatic cancer, 50% of colorectal cancer and 30% of lung cancer have RAS mutation; 50% of melanomas, 50% of thyroid cancer, and 15% of colorectal cancer have BRAF mutation.

**Table 1. Type and probability of ERK signaling-related mutation in various cancers**

| Type of cancer | Type and probability of ERK/MAPK-related mutation |
|---|---|
| Non-small cell lung cancer | NRAS mutation (35%); |
| Pancreatic cancer | KRAS mutation (90%); |
| Colorectal cancer | KRAS mutation (45%); BRAF mutation (12%); |
| Melanoma | NRAS mutation (15%); BRAF mutation (66%) |
| Prostate cancer | KRAS mutation (90%); |
| Bladder Cancer | KRAS mutation (90%) |
| Acute myeloid leukemia | NRAS mutation (30%) |
| Ovarian cancer | BRAF mutation (30%) |
| Papillary thyroid cancer | RAS mutation (60%); BRAF (35-70%) |

Vemurafenib is the first commercially available BRAF inhibitor approved by the FDA, which is mainly used for the treatment of advanced melanoma. However, its efficacy can only be maintained for 8-9 months, and it is prone to drug resistance. Studies have demonstrated that re-activation of the ERK signaling pathway mediates the resistance to Vemurafenib in melanoma. Dabrafenib, another BRAF inhibitor, is also prone to drug resistance. In addition, Vemurafenib fails to show significant clinical activity in colorectal cancer patients with BRAF mutation, and the overall response rate is only 5%. In addition to BRAF inhibitors, MEK inhibitors currently available have also developed drug resistance to varying degrees in clinical applications. The response rate of MEK inhibitor to RAS-mutated tumor is low, and the response rate to BRAF-mutated melanoma is merely 22%. The combination of BRAF inhibitor and EGFR inhibitor was used clinically to reverse drug resistance, but the patients developed multi-drug resistance after several months.

Currently, a number of preclinical studies have demonstrated the resistance to various types of ERK upstream target inhibitors. The resistance to BRAF inhibitor and MEK inhibitor can be reversed by inhibiting ERK activity. BRAF and MEK inhibitors are studying by many pharmaceutical companies, such as Genentech, Merck, Lilly and the like. There are currently no commercially available BRAF and MEK inhibitors developed by Chinese companies. Disclosed patent applications related to selective ERK1/2 inhibitors include WO2012088314, WO2014134776, WO2014179154, WO2014137728, WO2015051314 and the like. The existing clinical candidate drugs, such as KO-947, have the problem of poor solubility, which is adverse to its druggability. The drug can exist in conventional solvents only in a suspension state, and be administrated intravenously rather than orally.

ERK inhibitor as a pharmaceutical has a good application prospect in the pharmaceutical industry. First: it has a big market demand. 90% of pancreatic cancer, 50% of colorectal cancer and 30% of lung cancer have RAS mutation; 50% of melanomas, 50% of thyroid cancer, and 15% of colorectal cancer have BRAF mutation. ERK inhibitor can be used in patients with RAS and BRAF mutations, and it is especially effective for tumors that resist BRAF and MEK inhibitors. Second: it has a clear mechanism. ERK signaling pathway is one of the main signaling pathways of cell proliferation and growth, and its mechanism is relatively clear. Third: it has a high selectivity and safety. The pilot ERK inhibitors are highly selective, and can be tens of thousands of times more selective for other kinases.

### SUMMARY OF THE INVENTION

The object of the present invention is to provide a compound of formula (I), a stereoisomer thereof or a pharmaceutically acceptable salt thereof, wherein the structure of the compound of formula (I) is as following: 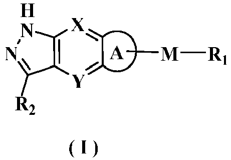 wherein:
X and Y are each independently selected from the group consisting of N and -CR₃;
M is selected from the group consisting of a bond, 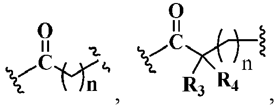 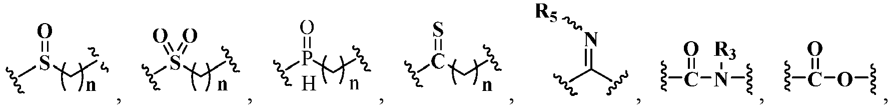 -(CH₂)ₙ- and -CR₃R₄;
ring A is selected from the group consisting of cycloalkyl, heterocyclyl, aryl and heteroaryl, wherein the cycloalkyl, heterocyclyl, aryl and heteroaryl are optionally further substituted by one or more substituents selected from the group consisting of deuterium, oxo, alkyl, deuterated alkyl, halogen, amino, nitro, hydroxy, cyano, alkoxy, hydroxyalkyl, cycloalkyl, heterocyclyl, aryl, heteroaryl, -(CH₂)ₙR₃, -(CH₂)ₙOR₃, -(CH₂)ₙSR₃, -(CH₂)ₙC(O)R₃, -(CH₂),C(O)OR₃, -(CH₂)ₙS(O)ₘR₃, -(CH₂)ₙNR₃R₄, -(CH_{z})ₙC(O)NR₃R₄, -(CH₂)ₙNR₃C(O)R₄ and -(CH₂)ₙNR₃S(O)ₘR₄; R₁ is selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl, heteroaryl, -(CH₂)ₙR₃, -(CH₂)ₙOR₃, -(CH₂)ₙSR₃, -(CH₂)ₙC(O)R₃, -(CH₂)ₙC(O)OR₃, -(CH₂)ₙS(O)ₘR₃, -(CH₂)ₙNR₃R₄, -(CH_{z})ₙC(O)NR₃R₄, -(CH₂)ₙNR₃C(O)R₄ and -(CH₂)ₙNR₃S(O)ₘR₄, wherein the alkyl, haloalkyl, cycloalkyl, heterocyclyl, aryl and heteroaryl are optionally further substituted by one or more substituents selected from the group consisting of deuterium, alkyl, haloalkyl, halogen, amino, nitro, cyano, hydroxy, alkoxy, haloalkoxy, hydroxyalkyl, cycloalkyl, heterocyclyl, aryl, heteroaryl, -(CH₂)ₙR₅, -(CH₂)ₙOR₅, -(CH₂)ₙSR₅, -(CH₂)ₙC(O)R₅, -(CH₂),C(O)OR₅, -(CH₂)ₙS(O)ₘR₅, -(CH₂)ₙNR₅R₆, -(CH₂)ₙC(O)NR₅R₆ -(CH₂)ₙNR₅C(O)R₆ and -(CH₂)ₙNR₅S(O)ₘR₆;
R₂ is selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl, heteroaryl, -(CH₂)ₙR₃, -(CH₂)ₙOR₃, -(CH₂)ₙSR₃, -(CH₂)ₙC(O)R₃, -(CH₂),C(O)OR₃, -(CH₂)ₙS(O)ₘR₃, -(CH₂)ₙNR₃R₄, -(CH₂)ₙC(O)NR₃R₄, -(CH₂)ₙNR₃C(O)R₄ and -(CH₂)ₙNR₃S(O)ₘR₄, wherein the alkyl, haloalkyl, cycloalkyl, heterocyclyl, aryl and heteroaryl are optionally further substituted by one or more substituents selected from the group consisting of deuterium, oxo, alkyl, haloalkyl, halogen, amino, nitro, cyano, hydroxy, alkoxy, haloalkoxy, hydroxyalkyl, cycloalkyl, heterocyclyl, aryl, heteroaryl, -(CH₂)ₙR₅, -(CH₂)ₙOR₅, -(CH₂)ₙSR₅, -(CH₂)ₙC(O)R₅, -(CH₂),C(O)OR₅, -(CH₂)ₙS(O)ₘR₅,-(CH₂)ₙNR₅R₆, -(CH₂)ₙC(O)NR₅R₆ -(CH₂)ₙC(O)NHR₅, -(CH₂)ₙNR₅C(O)R₆ and -(CH₂)ₙNR₅S(O)ₘR₆;
R₃ and R₄ are identical or different and are each independently selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl, heteroaryl, -(CH₂)ₙR₅, -(CH₂)ₙOR₅, -(CH₂)ₙSR₅, -(CH₂)ₙC(O)R₅, -(CH₂)ₙC(O)OR₅, -(CH₂)ₙS(O)ₘR₅, -(CH₂)ₙNR₅R₆, -(CH₂)ₙC(O)NR₅R₆ -(CH₂)ₙC(O)NHR₅, -(CH₂)ₙNR₅C(O)R₆ and -(CH₂)ₙNR₅S(O)ₘR₆, wherein the alkyl, haloalkyl, cycloalkyl, heterocyclyl, aryl and heteroaryl are optionally further substituted by one or more substituents selected from the group consisting of deuterium, alkyl, halogen, hydroxy, amino, nitro, cyano, ester group, alkoxy, hydroxyalkyl, substituted or unsubstituted cycloalkyl, substituted or unsubstituted heterocyclyl, substituted or unsubstituted aryl and substituted or unsubstituted heteroaryl;
Rs and R₆ are identical or different and are each independently selected from the group consisting of hydrogen, deuterium, alkyl, deuterated alkyl, haloalkyl, hydroxy, amino, cycloalkyl, heterocyclyl, aryl and heteroaryl, wherein the alkyl, cycloalkyl, heterocyclyl, aryl and heteroaryl are optionally further substituted by one or more substituents selected from the group consisting of deuterium, alkyl, halogen, hydroxy, amino, nitro, cyano, alkoxy, hydroxyalkyl, cycloalkyl, heterocyclyl, aryl and heteroaryl;
m is an integer of 0, 1 or 2; and
n is an integer of 0, 1, 2, 3, 4 or 5.

In a preferred embodiment of the present invention, the compound of formula (I), a stereoisomer thereof or a pharmaceutically acceptable salt thereof is a compound of formula (II), a stereoisomer thereof or a pharmaceutically acceptable salt thereof: 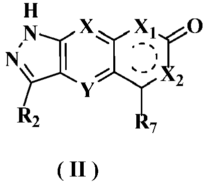 wherein:
X and Y are each independently selected from the group consisting of N and -CR₃, and preferably CH;
X₁ and X₂ are each independently selected from the group consisting of O, -NR₃ and -CR₃; represents a single bond or double bond;
R₂ is selected from the group consisting of -(CH₂)ₙR₃, -(CH₂)ₙOR₃, -(CH₂)ₙNR₃R₄, cycloalkyl, heterocyclyl, aryl and heteroaryl, wherein the cycloalkyl, heterocyclyl, aryl and heteroaryl are optionally further substituted by one or more substituents selected from the group consisting of deuterium, oxo, alkyl, deuterated alkyl, halogen, amino, nitro, hydroxy, cyano, alkoxy, hydroxyalkyl, cycloalkyl, heterocyclyl, aryl, heteroaryl, -(CH₂)ₙR₅, -(CH₂)ₙOR₅, -(CH₂)ₙSR₅, -(CH₂)ₙC(O)R₅, -(CH₂),C(O)OR₅, -(CH₂)ₙS(O)ₘR₅, -(CH₂)ₙNR₅R₆, -(CH₂)ₙC(O)NR₅R₆ -(CH₂)ₙC(O)NHR₅, -(CH₂)ₙNR₅C(O)R₆ and -(CH₂)ₙNR₅S(O)ₘR₆;
R₇ is selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl, heteroaryl, -(CH₂)ₙR₃, -(CH₂)ₙOR₃, -(CH₂)ₙSR₃, -(CH₂)ₙC(O)R₃, -(CH₂)ₙC(O)OR₃, -(CH₂)ₙS(O)ₘR₃, -(CH₂)ₙNR₃R₄, -(CH₂)ₙC(O)NR₃R₄, -(CH₂)ₙNR₃C(O)R₄ and -(CH₂)ₙNR₃S(O)ₘR₄, wherein the alkyl, haloalkyl, cycloalkyl, heterocyclyl, aryl and heteroaryl are optionally further substituted by one or more substituents selected from the group consisting of deuterium, alkyl, haloalkyl, halogen, amino, nitro, cyano, hydroxy, alkoxy, haloalkoxy, hydroxyalkyl, cycloalkyl, heterocyclyl, aryl, heteroaryl, -(CH₂)ₙR₅, -(CH₂)ₙOR₅, -(CH₂)ₙSR₅, -(CH₂)ₙC(O)R₅, -(CH₂),C(O)OR₅, -(CH₂)ₙS(O)ₘR₅,-(CH₂)ₙNR₅R₆, -(CH₂)ₙC(O)NR₅R₆, -(CH₂)ₙNR₅C(O)R₆ and -(CH₂)ₙNR₅S(O)ₘR₆;
R_{3~}R₆, m and n are as defined in formula (I).

In a preferred embodiment of the present invention, the compound of formula (I), a stereoisomer thereof or a pharmaceutically acceptable salt thereof is a compound of formula (III), a stereoisomer thereof or a pharmaceutically acceptable salt thereof: 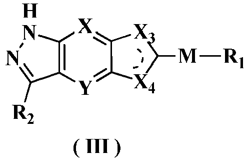 wherein:
X and Y are each independently selected from the group consisting of N and -CR₃, and preferably CH;
X₃ and X₄ are each independently selected from the group consisting of N, NR₃ and -CR₃; represents a single bond or double bond;
R₂ is selected from the group consisting of -(CH₂)ₙR₃, -(CH₂)ₙOR₃, -(CH₂)ₙNR₃R₄, cycloalkyl, heterocyclyl, aryl and heteroaryl, wherein the cycloalkyl, heterocyclyl, aryl and heteroaryl are optionally further substituted by one or more substituents selected from the group consisting of deuterium, alkyl, deuterated alkyl, halogen, amino, nitro, hydroxy, cyano, alkoxy, hydroxyalkyl, cycloalkyl, heterocyclyl, aryl, heteroaryl, -(CH₂)ₙR₅, -(CH₂)ₙOR₅, -(CH₂)ₙSR₅, -(CH₂)ₙC(O)R₅, -(CH₂),C(O)OR₅, -(CH₂)ₙS(O)ₘR₅, -(CH₂)ₙNR₅R₆, -(CH₂)ₙC(O)NR₅R₆ -(CH₂)ₙC(O)NHR₅, -(CH₂)ₙNR₅C(O)R₆ and -(CH₂)ₙNR₅S(O)ₘR₆;
M, R₁, R₃∼R₆, m and n are as defined in formula (I).

In a preferred embodiment of the present invention, the compound of formula (II), a stereoisomer thereof or a pharmaceutically acceptable salt thereof is a compound of formula (IV), a stereoisomer thereof or a pharmaceutically acceptable salt thereof: 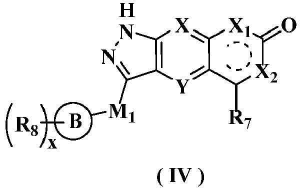 wherein:
M₁ is selected from the group consisting of a bond, O, NR₃, 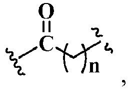 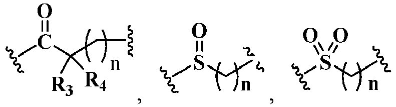 and -(CH₂)ₙ-, and preferably selected from the group consisting of a bond, O, NR₃, 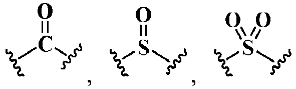 and -(CH₂)ₙ-;
ring B is selected from the group consisting of cycloalkyl, heterocyclyl, aryl and heteroaryl, wherein the cycloalkyl, heterocyclyl, aryl and heteroaryl are optionally further substituted by one or more substituents selected from the group consisting of deuterium, oxo, alkyl, deuterated alkyl, halogen, amino, nitro, hydroxy, cyano, alkoxy, hydroxyalkyl, cycloalkyl, heterocyclyl, aryl and heteroaryl;
R₈ is selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl and heteroaryl, wherein the alkyl, haloalkyl, cycloalkyl, heterocyclyl, aryl and heteroaryl are optionally further substituted by one or more substituents selected from the group consisting of deuterium, alkyl, haloalkyl, halogen, amino, nitro, cyano, hydroxy, alkoxy, haloalkoxy, hydroxyalkyl, cycloalkyl, heterocyclyl, aryl and heteroaryl;
x is an integer of 0, 1, 2, 3 or 4;
X, Y, X₁, X₂, R₃∼R₇, m, n and x are as defined in formula (II).

In a preferred embodiment of the present invention, the compound of formula (III), a stereoisomer thereof or a pharmaceutically acceptable salt thereof is a compound of formula (V), a stereoisomer thereof or a pharmaceutically acceptable salt thereof: 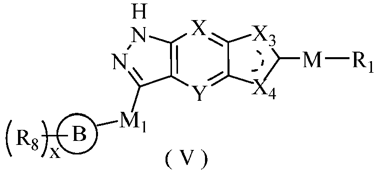 wherein:
X₃ and X₄ are each independently selected from the group consisting of N, NR₃ and -CR₃;
M₁ is selected from the group consisting of a bond, O, NR₃, 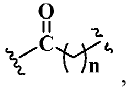 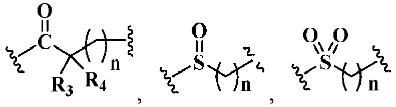 and -(CH₂)ₙ-, and preferably selected from the group consisting of a bond, O, NR₃, 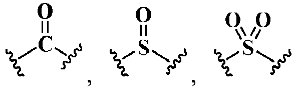 and -(CH₂)ₙ-;
ring B is selected from the group consisting of cycloalkyl, heterocyclyl, aryl and heteroaryl, wherein the cycloalkyl, heterocyclyl, aryl and heteroaryl are optionally further substituted by one or more substituents selected from the group consisting of deuterium, oxo, alkyl, deuterated alkyl, halogen, amino, nitro, hydroxy, cyano, alkoxy, hydroxyalkyl, cycloalkyl, heterocyclyl, aryl and heteroaryl;
R₈ is selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl and heteroaryl, wherein the alkyl, haloalkyl, cycloalkyl, heterocyclyl, aryl and heteroaryl are optionally further substituted by one or more substituents selected from the group consisting of deuterium, alkyl, haloalkyl, halogen, amino, nitro, cyano, hydroxy, alkoxy, haloalkoxy, hydroxyalkyl, cycloalkyl, heterocyclyl, aryl and heteroaryl;
M, X, Y, R₁, m, n and x are as defined in formula (III).

In a preferred embodiment of the present invention, the compound of formula (V), a stereoisomer thereof or a pharmaceutically acceptable salt thereof is a compound of formula (V-A), (V-B) or (V-C), a stereoisomer thereof or a pharmaceutically acceptable salt thereof: 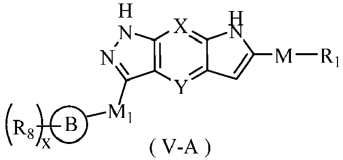 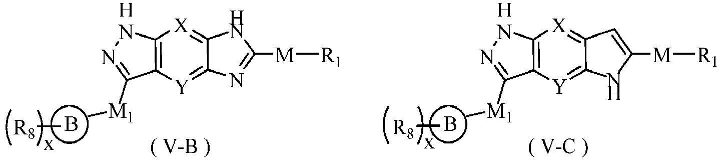 wherein:
ring B, M, M₁, X, Y, R₁, R₈ and x are as defined in formula (V).

In a preferred embodiment of the present invention, the compound of formula (V), a stereoisomer thereof or a pharmaceutically acceptable salt thereof is a compound of formula (V-D), a stereoisomer thereof or a pharmaceutically acceptable salt thereof: wherein: 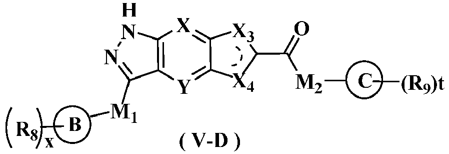
M₂ is selected from the group consisting of a bond, -(CH₂)ₙCR₃R₄-, -O(CH₂)ₙCR₃R₄-, -(CH₂)ₙNR₃-, -(CH₂)ₙNR3NR₄-, -(CH₂)ₙNHCR₃R₄- and -CR₃R₄-;
ring C is selected from the group consisting of cycloalkyl, heterocyclyl, aryl and heteroaryl, wherein the cycloalkyl, heterocyclyl, aryl and heteroaryl are optionally further substituted by one or more substituents selected from the group consisting of deuterium, oxo, alkyl, deuterated alkyl, halogen, amino, nitro, hydroxy, cyano, alkoxy, hydroxyalkyl, cycloalkyl, heterocyclyl, aryl and heteroaryl;
ring C is preferably a structure selected from the group consisting of: 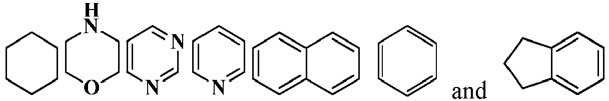
R₉ is selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl, heteroaryl, -(CH₂)ₙR₃, -(CH₂)ₙOR₃, -(CH₂)ₙSR₃, -(CH₂)ₙC(O)R₃, -(CH₂),C(O)OR₃, -(CH₂)ₙS(O)ₘR₃, -(CH₂)ₙNR₃R₄, -(CH₂)ₙC(O)NR₃R₄, -(CH₂)ₙNR₃C(O)R₄ and -(CH₂)ₙNR₃S(O)ₘR₄;
t is an integer of 0, 1, 2, 3, 4 or 5;
ring B, M₁, X, Y, R₈ and x are as defined in formula (V).

In a preferred embodiment of the present invention, the compounds of formula (IV) and formula (V), a stereoisomer thereof or a pharmaceutically acceptable salt thereof:
wherein:
ring B is a structure selected from the group consisting of:

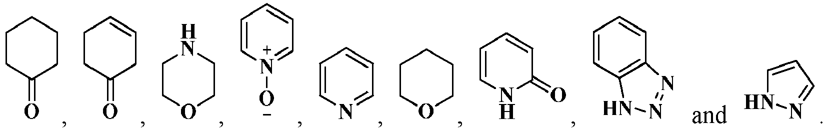

In a preferred embodiment of the present invention, the compound of formula (IV), a stereoisomer thereof or a pharmaceutically acceptable salt thereof is a compound of formula (VI), a stereoisomer thereof or a pharmaceutically acceptable salt thereof: 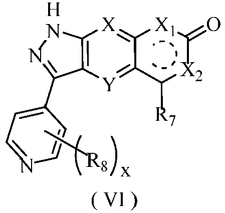 wherein:
X, Y, X₁, X₂, R₇, R₈ and x are as defined in formula (IV).

In a preferred embodiment of the present invention, the compound of formula (V) is a compound of formula (VII), a stereoisomer thereof or a pharmaceutically acceptable salt thereof: 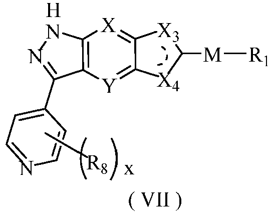 wherein:
M, X, Y, X₃, X₄, R₁, R₈ and x are as defined in formula (V).

In a preferred embodiment of the present invention, the compound of formula (I), a stereoisomer thereof or a pharmaceutically acceptable salt thereof is a compound of formula (VIII), a stereoisomer thereof or a pharmaceutically acceptable salt thereof: 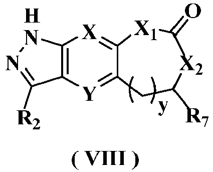 wherein:
X and Y are each independently selected from the group consisting of N and -CR₃, and preferably CH;
X₁ and X₂ are each independently selected from the group consisting of O, -NR₃ and -CR₃;
R₂ is selected from the group consisting of -(CH₂)ₙR₃, -(CH₂)ₙOR₃, -(CH₂)ₙNR₃R₄, cycloalkyl, heterocyclyl, aryl and heteroaryl, wherein the cycloalkyl, heterocyclyl, aryl and heteroaryl are optionally further substituted by one or more substituents selected from the group consisting of deuterium, alkyl, deuterated alkyl, halogen, amino, nitro, hydroxy, cyano, alkoxy, hydroxyalkyl, cycloalkyl, heterocyclyl, aryl, heteroaryl, -(CH₂)ₙR₅, -(CH₂)ₙOR₅, -(CH₂)ₙSR₅, -(CH₂)ₙC(O)R₅, -(CH₂),C(O)OR₅, -(CH₂)ₙS(O)ₘR₅, -(CH₂)ₙNR₅R₆, -(CH₂)ₙC(O)NR₅R₆ -(CH₂)ₙNR₅C(O)R₆ and -(CH₂)ₙNR₅S(O)ₘR₆;
R₇ is selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl, heteroaryl, -(CH₂)ₙR₃, -(CH₂)ₙOR₃, -(CH₂)ₙSR₃, -(CH₂)ₙC(O)R₃, -(CH₂),C(O)OR₃, -(CH₂)ₙS(O)ₘR₃, -(CH₂)ₙNR₃R₄, -(CH₂)ₙC(O)NR₃R₄, -(CH₂)ₙNR₃C(O)R₄ and -(CH₂)ₙNR₃S(O)ₘR₄, wherein the alkyl, haloalkyl, cycloalkyl, heterocyclyl, aryl and heteroaryl are optionally further substituted by one or more substituents selected from the group consisting of deuterium, alkyl, haloalkyl, halogen, amino, nitro, cyano, hydroxy, alkoxy, haloalkoxy, hydroxyalkyl, cycloalkyl, heterocyclyl, aryl, heteroaryl, -(CH₂)ₙR₅, -(CH₂)ₙOR₅, -(CH₂)ₙSR₅, -(CH₂)ₙC(O)R₅, -(CH₂),C(O)OR₅, -(CH₂)ₙS(O)ₘR₅,-(CH₂)ₙNR₅R₆, -(CH₂)ₙC(O)NR₅R₆ -(CH₂)ₙNR₅C(O)R₆ and -(CH₂)ₙNR₅S(O)ₘR₆; and
y is an integer of 0, 1, 2 or 3, and preferably y is 1;
R_{3~}R₆, m and n are as defined in formula (I).

In a preferred embodiment of the present invention, the compound of formula (I), a stereoisomer thereof or a pharmaceutically acceptable salt thereof is a compound of formula (VII-A), a stereoisomer thereof or a pharmaceutically acceptable salt thereof: 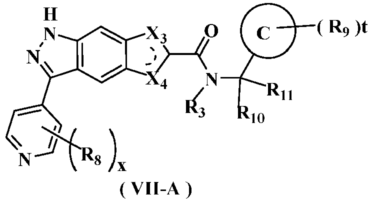 wherein:
X₃ and X₄ are each independently selected from the group consisting of N, NH and CH;
ring C is a structure selected from the group consisting of: 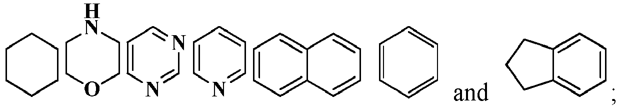
R₃ is selected from the group consisting of hydrogen, deuterium, C₁₋₈ alkyl, C₁₋₈ deuterated alkyl, C₁₋₈ haloalkyl, C₁₋₈ alkoxy and C₁₋₈ haloalkoxy;
R₈ is selected from the group consisting of hydrogen, deuterium, C₁₋₈ alkyl, C₁₋₈ deuterated alkyl, C₁₋₈ haloalkyl, C₁₋₈ alkoxy, C₁₋₈ haloalkoxy, halogen, amino, nitro, hydroxy, cyano, C₃₋₁₀ cycloalkyl, 3 to 10 membered heterocyclyl, 6 to 10 membered aryl and 5 to 12 membered heteroaryl;
R₉ is selected from the group consisting of hydrogen, deuterium, C₁₋₈ alkyl, C₁₋₈ deuterated alkyl, C₁₋₈ haloalkyl, C₁₋₈ alkoxy, C₁₋₈ haloalkoxy, halogen, amino, nitro, hydroxy, cyano, C₃₋₁₀ cycloalkyl, 3 to 10 membered heterocyclyl, 6 to 10 membered aryl and 5 to 12 membered heteroaryl;
R₁₀ and R₁₁ are identical or different and are each independently selected from the group consisting of hydrogen, deuterium, C₁₋₈ alkyl, C₁₋₈ deuterated alkyl, C₁₋₈ haloalkyl, C₁₋₈ alkoxy, C₁₋₈ haloalkoxy, halogen, amino, nitro, hydroxy, cyano, C₃₋₁₀ cycloalkyl, 3 to 10 membered heterocyclyl, 6 to 10 membered aryl, 5 to 12 membered heteroaryl, -(CH₂)ₙR₅, -(CH₂)ₙOR₅, -(CH₂)ₙSR₅, -(CH₂)ₙC(O)R₅, -(CH₂)ₙC(O)OR₅, -(CH₂)ₙS(O)ₘR₅, -(CH₂)ₙNR₅R₆, -(CH₂)ₙC(O)NR₅R₆ -(CH₂)ₙC(O)NHR₅, -(CH₂)ₙNR₅C(O)R₆ and -(CH₂)ₙNR₅S(O)ₘR₆-, wherein the C₁₋₈ alkyl, C₁₋₈ deuterated alkyl, C₁₋₈ haloalkyl, C₁₋₈ alkoxy, C₁₋₈ haloalkoxy, C₃₋₁₀ cycloalkyl, 3 to 10 membered heterocyclyl, 6 to 10 membered aryl and 5 to 12 membered heteroaryl are optionally further substituted by one or more substituents selected from the group consisting of deuterium, C₁₋₈ alkyl, halogen, hydroxy, amino, nitro, cyano, ester group, C₁₋₈ alkoxy, C₁₋₈ hydroxyalkyl, C₃₋₁₀ cycloalkyl, 3 to 10 membered heterocyclyl, 6 to 10 membered aryl and 5 to 12 membered heteroaryl;
R₅ and R₆ are identical or different and are each independently selected from the group consisting of hydrogen, deuterium, C₁₋₈ alkyl, C₁₋₈ deuterated alkyl, C₁₋₈ haloalkyl, hydroxy, amino, C₃₋₁₀ cycloalkyl, 3 to 10 membered heterocyclyl, 6 to 10 membered aryl and 5 to 12 membered heteroaryl, wherein the C₁₋₈ alkyl, C₁₋₈ deuterated alkyl, C₁₋₈ haloalkyl, C₃₋₁₀ cycloalkyl, 3 to 10 membered heterocyclyl, 6 to 10 membered aryl and 5 to 12 membered heteroaryl are optionally further substituted by one or more substituents selected from the group consisting of deuterium, C₁₋₈ alkyl, C₁₋₈ deuterated alkyl, C₁₋₈ haloalkyl, halogen, hydroxy, amino, nitro, cyano, C₁₋₈ alkoxy, C₁₋₈ hydroxyalkyl, C₃₋₁₀ cycloalkyl, 3 to 10 membered heterocyclyl, 6 to 10 membered aryl and 5 to 12 membered heteroaryl;
m is an integer of 0, 1 or 2;
n is an integer of 0, 1, 2, 3, 4 or 5;
x is an integer of 0, 1, 2, 3 or 4; and
t is an integer of 0, 1, 2, 3, 4 or 5.

In a preferred embodiment of the present invention, the compound of each formula, the stereoisomer thereof, or the pharmaceutically acceptable salt thereof is characterized in that R₁ is selected from the group consisting of hydrogen, C₁₋₈ alkyl, 5 to 10 membered aryl, 5 to 10 membered heteroaryl, -(CH₂)ₙR₃, -(CH₂)ₙOR₃, -(CH₂)ₙC(O)R₃, -(CH₂)ₙC(O)OR₃ and -(CH₂)ₙNR₃R₄, wherein the C₁₋₈ alkyl, 5 to 10 membered aryl and 5 to 10 membered heteroaryl are optionally further substituted by one or more substituents selected from the group consisting of hydrogen, C₁₋₈ alkyl, halogen, 5 to 10 membered aryl, 5 to 10 membered heteroaryl, -(CH₂)ₙR₅, -(CHR₄)ₙR₅ and -(CH₂)ₙOR₅; preferably selected from the group consisting of hydrogen, C₁₋₆ alkyl, 5 to 6 membered aryl, 5 to 6 membered heteroaryl, -(CH₂)ₙR₃, -(CH₂)ₙOR₃, -(CH₂)ₙC(O)R₃ and -(CH₂)ₙNR₃R₄; and more preferably selected from the group consisting of hydrogen, C₁₋₃ alkyl, 5 to 6 membered aryl, 5 to 6 membered heteroaryl, -(CH₂)ₙR₃, -(CH₂)ₙOR₃, -(CH₂)ₙC(O)R₃ and -(CH₂)ₙNR₃R₄.

In a preferred embodiment of the present invention, the compound of each formula, the stereoisomer thereof, or the pharmaceutically acceptable salt thereof is characterized in that R₈ is selected from the group consisting of hydrogen, cyano, C₁₋₈ alkyl, C₁₋₈ alkoxy, halogen, oxo and C₁₋₈ haloalkyl; preferably selected from the group consisting of C₁₋₆ alkyl and halogen; and more preferably selected from the group consisting of C₁₋₃ alkyl, C₁₋₃ alkoxy and halogen.

The present invention also provides an intermediate for preparing the compound of formula (VII-A), a stereoisomer thereof or a pharmaceutically acceptable salt, which is a compound of formula (IX), a stereoisomer thereof or a pharmaceutically acceptable salt thereof: 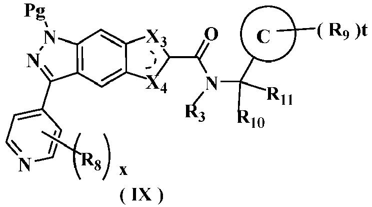 wherein:
Pg is an amino protecting group selected from the group consisting of benzyloxycarbonyl (Cbz), *tert*-butoxycarbonyl (Boc), 9-fluorenylmethoxycarbonyl (Fmoc), benzyl (Bn), *p*-methoxybenzyl (PMB), allyloxycarbonyl (Alloc), trityl (Trt) and phthaloyl (Pht), and preferably trityl (Trt);
ring C, X₃, X₄, R₃, R₈-R₁₁, t and x are as defined in formula (VII-A).

The compounds of the present invention, particularly the compound of formula (IX), especially Eamples 1, 2 and 7, have higher solubility than the ERK compounds disclosed in the prior art, and is more suitable for the development of oral drugs.

The present invention also relates to a method for preparing the compound of formula (VII-A), comprising the following step of: 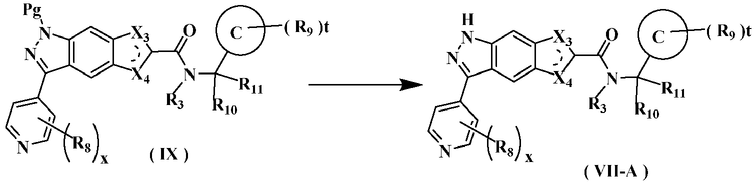 subjecting a compound of formula (IX) to a deprotection reaction under an acidic condition to obain the compound of formula (VII-A);
wherein:
ring C, Pg, X₃, X₄, R₃, R₈-R₁₁, t and x are as defined in formula (IX).

The present invention further relates to a pharmaceutical composition comprising a therapeutically effective amount of any one of the compound of formula (I), a stereoisomer thereof or a pharmaceutically acceptable salt thereof, and one or more pharmaceutically acceptable carriers, diluents or excipients.

The present invention further relates to a use of any one of the compound of formula (I), a stereoisomer thereof or a pharmaceutically acceptable salt thereof, or the pharmaceutical composition thereof in the preparation of an ERK inhibitor medicament.

The present invention further relates to a use of the compound of formula (I), a stereoisomer thereof or a pharmaceutically acceptable salt thereof, or the pharmaceutical composition thereof in the preparation of a medicament for treating a cancer, bone disease, inflammatory disease, immunological disease, nervous system disease, metabolic disease, respiratory disease and heart disease, wherein the cancer is selected from the group consisting of breast cancer, pancreatic cancer, non-small cell lung cancer (NSCLC), thyroid cancer, seminoma, melanoma, bladder cancer, liver cancer, kidney cancer, myelodysplastic syndrome (MDS), acute myeloid leukemia (AML) and colorectal cancer.

The present invention further relates to a use of the compound of formula (I), a stereoisomer thereof or a pharmaceutically acceptable salt thereof, or the pharmaceutical composition thereof in the preparation of a medicament for treating a cancer, bone disease, inflammatory disease, immunological disease, nervous system disease, metabolic disease, respiratory disease and heart disease.

The present invention also relates to a method for treating and/or preventing cancer, comprising administering a therapeutically effective amount of the compound of formula (I), a stereoisomer thereof or a pharmaceutically acceptable salt thereof, or the pharmaceutical composition thereof to a patient.

The present invention also provides a method for treating a disease condition by using the compound or the pharmaceutical composition of the present invention, wherein the disease condition includes, but is not limited to, conditions related to ERK1, ERK2, Ras, Raf and/or MEK kinase dysfunction.

The present invention also relates to a method for treating a hyperproliferative disorder in a mammal, comprising administering a therapeutically effective amount of the compound of the present invention, or a pharmaceutically acceptable salt, ester, prodrug, solvate, hydrate or derivative thereof to the mammal.

In some embodiments, the method relates to the treatment of a disease such as cancer, bone disease, inflammatory disease, immunological disease, nervous system disease, metabolic disease, respiratory disease and heart disease.

In some embodiments, the method relates to the treatment of a cancer such as acute myeloid leukemia, myelodysplastic syndrome (MDS), thymic cancer, brain cancer, lung cancer (NSCLC and SCLC), squamous cell carcinoma, seminoma, melanoma, skin cancer, eye cancer, retinoblastoma, intraocular melanoma, oral and oropharyngeal cancer, bladder cancer, stomachcancer, gastric cancer, pancreatic cancer, bladder cancer, breast cancer, cervical cancer, head cancer, neck cancer, kidney cancer, kidney cancer, liver cancer, ovarian cancer, prostate cancer, endometrial cancer, colorectal cancer, esophageal cancer, testicular cancer, gynecological cancer, thyroid cancer, CNS cancer, PNS cancer, AIDS-related cancer (such as lymphoma and Kaposi's sarcoma) or virus-induced cancer. In some embodiments, the method relates to the treatment of a non-cancerous hyperproliferative disorder such as skin disease (such as psoriasis), restenosis, or benign proliferation of the prostate (such as benign prostatic hypertrophy (BPH)). In some embodiments, the cancer is melanoma or colorectal cancer.

In some embodiments, the method relates to the treatment of a disease or condition of a subject with Ras or Raf gene mutation. In some cases, the disease is a cancer, and the mutation is a mutation in the Ras gene. For example, the disease can be melanoma in a subject with N-Ras mutation. Alternatively, the disease can be lung cancer or colon cancer in a subject with K-Ras mutation.

In some embodiments, the method relates to the treatment of a disease or condition resistant to Ras, Raf and/or MEK inhibitors. For example, the disease can be melanoma resistant to B-Raf and/or MEK inhibitors.

The treatment method provided herein comprises administering a therapeutically effective amount of the compound of the present invention to a subject. In an embodiment, the present invention provides a method for treating an inflammatory disease including autoimmunological disease in a mammal. The method comprises administering a therapeutically effective amount of the compound of the present invention, or a pharmaceutically acceptable salt, ester, prodrug, solvate, hydrate or derivative thereof to the mammal. The disease related to one or more types of ERK dysfunction includes, but is not limited to, acute disseminated encephalomyelitis (ADEM), Addison's disease, antiphospholipid antibody syndrome (APS), aplastic anemia, autoimmune hepatitis, celiac disease, Crohn's disease, diabetes (type 1), Good Pasteur's syndrome, Graves' disease, Guillain-Barre's syndrome (GBS), Hashimoto's disease, lupus erythematosus, multiple sclerosis, myasthenia gravis, visual clonus myoclonus syndrome (OMS), optic neuritis, Ord's thyroiditis, pemphigus, polyarthritis, primary biliary cirrhosis, psoriasis, rheumatoid arthritis, Lytle's syndrome, Takayasu's arteritis, temporal arteritis (also known as "giant cell arteritis"), warm autoimmune hemolytic anemia, Wegener's granulomatosis, alopecia universalis, Chagas' disease, chronic fatigue syndrome, autonomic dysfunction, endometriosis, suppurative sweat glanditis, interstitial cystitis, neuromuscular rigidity, sarcoidosis, scleroderma, ulcerative colitis, vitiligo and vulvar pain. Other diseases include bone resorption disorder and thromobsis.

In some embodiments, the method for treating an inflammatory or autoimmunological disease comprises administering a therapeutically effective amount of one or more of the compound of the present invention to a subject (such as a mammal), and the compound selectively inhibits ERK1 and/or ERK2 compared with all other kinases in the Ras/Raf/MEK/ERK pathway. Such selective inhibition of ERK1 and/or ERK2 may be advantageous for the treatment of any disease or condition described herein. For example, selective inhibition of ERK2 can inhibit inflammatory response associated with inflammatory disease and autoimmunological disease or disease associated with undesired immune response including, but not limited to, asthma, emphysema, allergies, dermatitis, rheumatoid arthritis, psoriasis, lupus erythematosus and graft versus host disease. Selective inhibition of ERK2 can further reduce inflammation or undesired immune response without reducing the ability to alleviate bacterial, viral and/or fungal infections.

### DETAILED DESCRIPTION OF THE INVENTION

Unless otherwise stated, the terms used in the specification and claims have the meanings described below.

The term "alkyl" refers to a saturated aliphatic hydrocarbon group, which is a straight or branched chain group comprising 1 to 20 carbon atoms, preferably an alkyl having 1 to 8 carbon atoms, more preferably an alkyl having 1 to 6 carbon atoms, and most preferably an alkyl having 1 to 3 carbon atoms. Non-limiting examples include methyl, ethyl, *n*-propyl, isopropyl, *n*-butyl, isobutyl, tert-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, *n*-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof. More preferably, the alkyl group is a lower alkyl having 1 to 6 carbon atoms, and non-limiting examples include methyl, ethyl, *n*-propyl, isopropyl, *n*-butyl, isobutyl, tert-butyl, *sec*-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl group can be substituted or unsubstituted. When substituted, the substituent group(s) can be substituted at any available connection point. The substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and carboxylic ester group. The alkyl of present invention is preferably selected from the group consisting of methyl, ethyl, isopropyl, *tert*-butyl, haloalkyl, deuterated alkyl, alkoxy-substituted alkyl and hydroxy-substituted alkyl.

The term "alkylene" refers to an alkyl of which a hydrogen atom is further substituted, for example, "methylene" refers to -CH₂-, "ethylene" refers to -(CH₂)₂-, "propylene" refers to -(CH₂)₃-, "butylene" refers to -(CH₂)₄- and the like. The term "alkenyl" refers to an alkyl as defined above that consists of at least two carbon atoms and at least one carbon-carbon double bond, for example, ethenyl, 1-propenyl, 2-propenyl, 1-, 2- or 3-butenyl and the like. The alkenyl group can be substituted or unsubstituted. When substituted, the substituent group(s) is preferably one or more groups independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio and heterocyclylthio.

The term "cycloalkyl" refers to a saturated or partially unsaturated monocyclic or polycyclic hydrocarbon substituent group having 3 to 20 carbon atoms, preferably 3 to 12 carbon atoms, and more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like. Polycyclic cycloalkyl includes a cycloalkyl having a spiro ring, fused ring or bridged ring. The cycloalkyl is preferably selected from the group consisting of cyclopropyl, cyclobutyl, cyclohexyl, cyclopentyl and cycloheptyl.

The term "spiro cycloalkyl" refers to a 5 to 20 membered polycyclic group with individual rings connected through one shared carbon atom (called a spiro atom), wherein the rings can contain one or more double bonds, but none of the rings has a completely conjugated π-electron system. The spiro cycloalkyl is preferably 6 to 14 membered spiro cycloalkyl, and more preferably 7 to 10 membered spiro cycloalkyl. According to the number of the spiro atoms shared between the rings, the spiro cycloalkyl can be divided into mono-spiro cycloalkyl, di-spiro cycloalkyl, or poly-spiro cycloalkyl, and the spiro cycloalkyl is preferably a mono-spiro cycloalkyl or di-spiro cycloalkyl, and more preferably 4-membered/4-membered, 4-membered/5-membered, 4-membered/6-membered, 5-membered/5-membered, or 5-membered/6-membered mono-spiro cycloalkyl. Non-limiting examples of spiro cycloalkyl include: 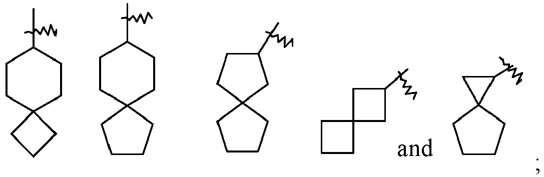 and also include spiro cycloalkyl consisted of a cycloalkyl and a heterocyclyl connected through one spiro atom, non-limiting examples thereof include:

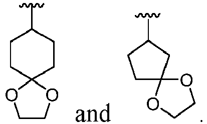

The term "fused cycloalkyl" refers to a 5 to 20 membered all-carbon polycyclic group, wherein each ring in the system shares an adjacent pair of carbon atoms with another ring, wherein one or more rings can contain one or more double bonds, but none of the rings has a completely conjugated π-electron system. The fused cycloalkyl is preferably 6 to 14 membered fused cycloalkyl, and more preferably 7 to 10 membered fused cycloalkyl. According to the number of membered rings, the fused cycloalkyl can be divided into bicyclic, tricyclic, tetracyclic or polycyclic fused cycloalkyl, and the fused cycloalkyl is preferably bicyclic or tricyclic fused cycloalkyl, and more preferably 5-membered/5-membered, or 5-membered/6-membered bicyclic fused cycloalkyl. Non-limiting examples of fused cycloalkyl include:

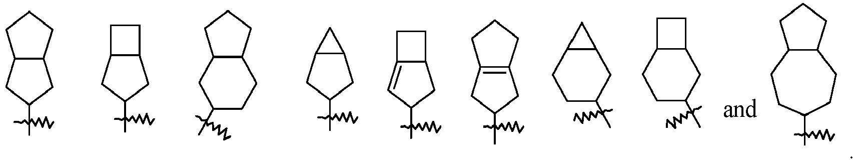

The term "bridged cycloalkyl" refers to a 5 to 20 membered all-carbon polycyclic group, wherein every two rings in the system share two disconnected carbon atoms, wherein the rings can have one or more double bonds, but none of the rings has a completely conjugated π-electron system. The bridged cycloalkyl is preferably 6 to 14 membered bridged cycloalkyl, and more preferably 7 to 10 membered bridged cycloalkyl. According to the number of membered rings, the bridged cycloalkyl can be divided into bicyclic, tricyclic, tetracyclic or polycyclic bridged cycloalkyl, and the bridged cycloalkyl is preferably bicyclic, tricyclic or tetracyclic bridged cycloalkyl, and more preferably bicyclic or tricyclic bridged cycloalkyl. Non-limiting examples of bridged cycloalkyl include:

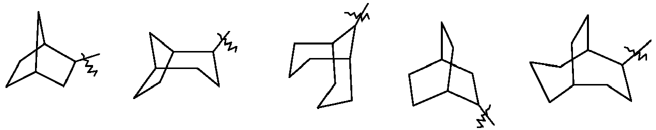

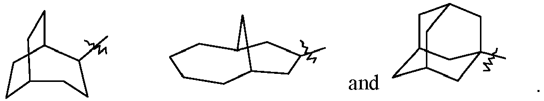

The cycloalkyl ring can be fused to the ring of aryl, heteroaryl or heterocyclyl, wherein the ring bound to the parent structure is cycloalkyl. Non-limiting examples include indanyl, tetrahydronaphthyl, benzocycloheptyl and the like. The cycloalkyl may be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and carboxylic ester group.

The term "heterocyclyl" refers to a 3 to 20 membered saturated or partially unsaturated monocyclic or polycyclic hydrocarbon group, wherein one or more ring atoms are heteroatoms selected from the group consisting of N, O and S(O)ₘ (wherein m is an integer of 0 to 2), but excluding -O-O-, -O-S- or -S-S- in the ring, with the remaining ring atoms being carbon atoms. Preferably, the heterocyclyl has 3 to 12 ring atoms wherein 1 to 4 atoms are heteroatoms; more preferably, 3 to 8 ring atoms; and most preferably 3 to 8 ring atoms. Non-limiting examples of monocyclic heterocyclyl include pyrrolidinyl, imidazolidinyl, tetrahydrofuranyl, tetrahydrothienyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, and preferably tetrahydrofuranyl, pyrazolidinyl, morpholinyl, piperazinyl and pyranyl. Polycyclic heterocyclyl includes a heterocyclyl having a spiro ring, fused ring or bridged ring. The heterocyclyl having a spiro ring, fused ring or bridged ring is optionally bonded to other group via a single bond, or further bonded to other cycloalkyl, heterocyclyl, aryl and heteroaryl via any two or more atoms on the ring.

The term "spiro heterocyclyl" refers to a 5 to 20 membered polycyclic heterocyclyl group with individual rings connected through one shared atom (called a spiro atom), wherein one or more ring atoms are heteroatoms selected from the group consisting of N, O and S(O)ₘ (wherein m is an integer of 0 to 2), with the remaining ring atoms being carbon atoms, where the rings can contain one or more double bonds, but none of the rings has a completely conjugated π-electron system. The spiro heterocyclyl is preferably 6 to 14 membered spiro heterocyclyl, and more preferably 7 to 10 membered spiro heterocyclyl. According to the number of the spiro atoms shared between the rings, the spiro heterocyclyl can be divided into mono-spiro heterocyclyl, di-spiro heterocyclyl, or poly-spiro heterocyclyl, and the spiro heterocyclyl is preferably mono-spiro heterocyclyl or di-spiro heterocyclyl, and more preferably 4-membered/4-membered, 4-membered/5-membered, 4-membered/6-membered, 5-membered/5-membered, or 5-membered/6-membered mono-spiro heterocyclyl. Non-limiting examples of spiro heterocyclyl include:

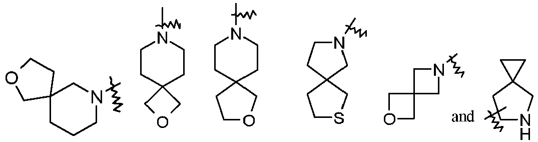

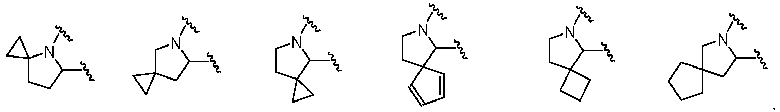

The term "fused heterocyclyl" refers to a 5 to 20 membered polycyclic heterocyclyl group, wherein each ring in the system shares an adjacent pair of atoms with another ring, wherein one or more rings can contain one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The fused heterocyclyl is preferably 6 to 14 membered fused heterocyclyl, and more preferably 7 to 10 membered fused heterocyclyl. According to the number of membered rings, the fused heterocyclyl can be divided into bicyclic, tricyclic, tetracyclic or polycyclic fused heterocyclyl, and preferably bicyclic or tricyclic fused heterocyclyl, and more preferably 5-membered/5-membered or 5-membered/6-membered bicyclic fused heterocyclyl. Non-limiting examples of fused heterocyclyl include:

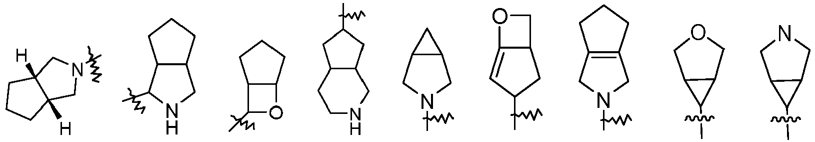

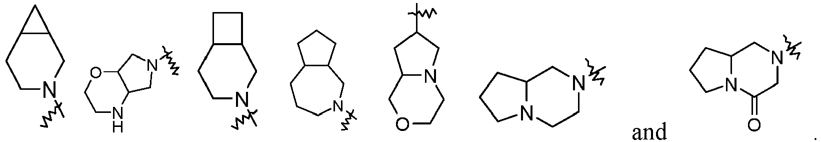

The term "bridged heterocyclyl" refers to a 5 to 14 membered polycyclic heterocyclyl group, wherein every two rings in the system share two disconnected atoms, wherein the rings can have one or more double bonds, but none of the rings has a completely conjugated π-electron system, and wherein one or more ring atoms are heteroatoms selected from the group consisting of N, O and S(O)ₘ (wherein m is an integer of 0 to 2), with the remaining ring atoms being carbon atoms. The bridged heterocyclyl is preferably 6 to 14 membered bridged heterocyclyl, and more preferably 7 to 10 membered bridged heterocyclyl. According to the number of membered rings, the bridged heterocyclyl can be divided into bicyclic, tricyclic, tetracyclic or polycyclic bridged heterocyclyl, and the bridged heterocyclyl is preferably bicyclic, tricyclic or tetracyclic bridged heterocyclyl, and more preferably bicyclic or tricyclic bridged heterocyclyl. Non-limiting examples of bridged heterocyclyl include:

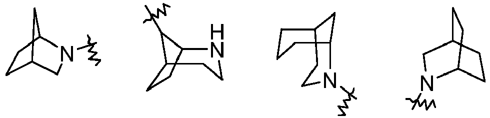

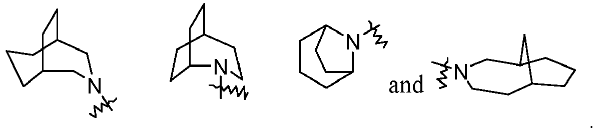

The heterocyclyl ring can be fused to the ring of aryl, heteroaryl or cycloalkyl, wherein the ring bound to the parent structure is heterocyclyl. Non-limiting examples thereof include: 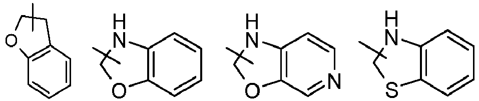 and the like.

The heterocyclyl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, oxo, carboxy and carboxylic ester group.

The term "aryl" refers to a 6 to 14 membered all-carbon monocyclic ring or polycyclic fused ring (i.e. each ring in the system shares an adjacent pair of carbon atoms with another ring in the system) having a conjugated π-electron system, preferably 6 to 10 membered aryl, for example, phenyl and naphthyl. The aryl is more preferably phenyl. The aryl ring can be fused to the ring of heteroaryl, heterocyclyl or cycloalkyl, wherein the ring bound to the parent structure is aryl ring. Non-limiting examples thereof include:

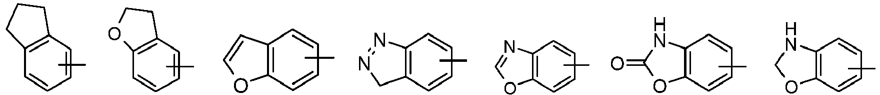

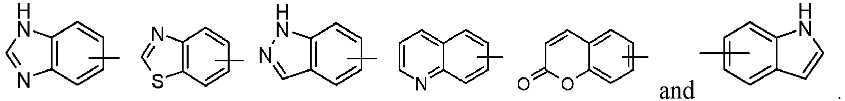

The aryl can be substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and carboxylic ester group.

The term "heteroaryl" refers to a 5 to 14 membered heteroaromatic system having 1 to 4 heteroatoms selected from the group consisting of O, S and N. The heteroaryl is preferably 5 to 12 membered heteroaryl, more preferably 5 or 10 membered heteroaryl, and most preferably 5 or 6 membered heteroaryl; for example, imidazolyl, furyl, thienyl, thiazolyl, pyrazolyl, oxazolyl, pyrrolyl, triazolyl, tetrazolyl, pyridyl, pyrimidinyl, thiadiazolyl, pyrazinyl and the like, preferably pyridyl, imidazolyl, pyrimidinyl, pyrazolyl and pyrrolyl, and more preferably pyridyl. The heteroaryl ring can be fused to the ring of aryl, heterocyclyl or cycloalkyl, wherein the ring bound to the parent structure is heteroaryl ring. Non-limiting examples thereof include:

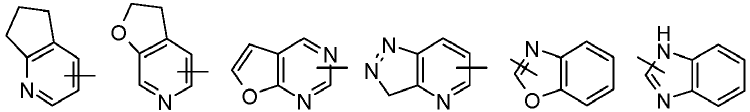

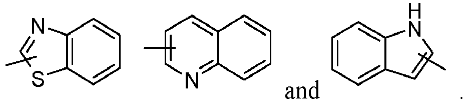

The heteroaryl can be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and carboxylic ester group.

The term "alkoxy" refers to an -O-(alkyl) or an -O-(unsubstituted cycloalkyl) group, wherein the alkyl is as defined above. Non-limiting examples of alkoxy include methoxy, ethoxy, propoxy, butoxy, cyclopropyloxy, cyclobutyloxy, cyclopentyloxy, cyclohexyloxy. The alkoxy may be optionally substituted or unsubstituted. When substituted, the substituent group(s) is preferably one or more group(s) independently selected from the group consisting of alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and carboxylic ester group.

"Haloalkyl" refers to an alkyl group substituted by one or more halogens, wherein the alkyl is as defined above.

"Haloalkoxy" refers to an alkoxy group substituted by one or more halogens, wherein the alkoxy is as defined above.

"Hydroxyalkyl" refers to an alkyl group substituted by hydroxy(s), wherein the alkyl is as defined above.

"Alkenyl" refers to a chain alkenyl, also known as alkene group. The alkenyl can be further substituted by other related group, for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and carboxylic ester group.

"Alkynyl" refers to (CH≡C-). The alkynyl can be further substituted by other related group, for example alkyl, alkenyl, alkynyl, alkoxy, alkylthio, alkylamino, halogen, thiol, hydroxy, nitro, cyano, cycloalkyl, heterocyclyl, aryl, heteroaryl, cycloalkoxy, heterocycloalkoxy, cycloalkylthio, heterocyclylthio, carboxy and carboxylic ester group.

"Hydroxy" refers to an -OH group.

"Halogen" refers to fluorine, chlorine, bromine or iodine.

"Amino" refers to a -NH₂ group.

"Cyano" refers to a -CN group.

"Nitro" refers to a -NO₂ group.

"Carboxy" refers to a -C(O)OH group.

"THF" refers to tetrahydrofuran.

"EtOAc" refers to ethyl acetate.

"MeOH" refers to methanol.

"DMF" refers to N,N-dimethylformamide.

"DIPEA" refers to diisopropylethylamine.

"TFA" refers to trifluoroacetic acid.

"MeCN" refers to acetonitrile.

"DMA" refers to N,N-dimethylacetamide.

"Et₂O" refers to diethyl ether.

"DCE" refers to 1,2-dichloroethane.

"DIPEA" refers to N,N-diisopropylethylamine.

"NBS" refers to N-bromosuccinimide.

"NIS" refers to N-iodosuccinimide.

"Cbz-Cl" refers to benzyl chloroformate.

"Pd₂(dba)₃" refers to tris(dibenzylideneacetone)dipalladium.

"Dppf' refers to 1,1'-bisdiphenylphosphinoferrocene.

"HATU" refers to 2-(7-azabenzotriazol-1-yl)-N,N,N',N'-tetramethyluronium hexafluorophosphate.

"KHMDS" refers to potassium hexamethyldisilazide.

"LiHMDS" refers to lithium bis(trimethylsilyl)amide.

"MeLi" refers to methyl lithium.

"n-BuLi" refers to *n*-butyl lithium.

"NaBH(OAc)₃" refers to sodium triacetoxyborohydride.

Different expressions such as "X is selected from the group consisting of A, B or C", "X is selected from the group consisting of A, B and C", "X is A, B or C", "X is A, B and C" and the like, express the same meaning, that is, X can be any one or more of A, B and C.

The hydrogen atom of the present invention can be substituted by its isotope deuterium. Any of the hydrogen atoms in the compounds of the examples of the present invention can also be substituted by deuterium.

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without paying excessive efforts. For example, the combination of amino or hydroxy with free hydrogen and carbon atoms with unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to exert biological activity.

A "pharmaceutically acceptable salt" refers to a salt of the compound of the present invention, which is safe and effective in mammals and has the desired biological activity.

The present invention will be further described with reference to the following examples, but the examples should not be considered as limiting the scope of the present invention.

### EXAMPLES

The structures of the compounds of the present invention are identified by nuclear magnetic resonance (NMR) and/or liquid chromatography-mass spectrometry (LC-MS). NMR shifts (δ) are given in parts per million (ppm). NMR is determined by a Bruker AVANCE-400 machine. The solvents for determination are deuterated-dimethyl sulfoxide (DMSO-*d₆*), deuterated-methanol (CD₃OD) and deuterated-chloroform (CDCl₃), and the internal standard is tetramethylsilane (TMS).

Liquid chromatography-mass spectrometry (LC-MS) is determined on an Agilent 1200 Infinity Series mass spectrometer. High performance liquid chromatography (HPLC) is determined on an Agilent 1200DAD high pressure liquid chromatograph (Sunfire C18 150×4.6 mm chromatographic column), and a Waters 2695-2996 high pressure liquid chromatograph (Gimini C18 150×4.6 mm chromatographic column).

Yantai Huanghai HSGF254 or Qingdao GF254 silica gel plate is used as the thin-layer silica gel chromatography (TLC) plate. The dimension of the silica gel plate used in TLC is 0.15 mm to 0.2 mm, and the dimension of the silica gel plate used in product purification is 0.4 mm to 0.5 mm. Yantai Huanghai 200 to 300 mesh silica gel is generally used as a carrier for column chromatography.

The raw materials used in the examples of the present invention are known and commercially available, or can be synthesized by adopting or according to known methods in the art.

Unless otherwise stated, all reactions of the present invention are carried out under continuous magnetic stirring under a dry nitrogen or argon atmosphere, the solvent is dry, and the reaction temperature is in degrees celsius.

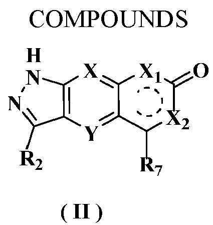

| Compound No. | R₂ | X | Y | X₁ | X₂ | R₇ |
|---|---|---|---|---|---|---|
| 1 | 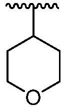 | CH | CH | NH | O | 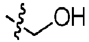 |
| 2 | 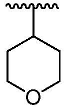 | CH | CH | NH | O | 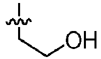 |
| 3 | 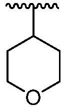 | CH | CH | NH | O | 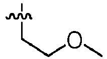 |
| 4 | 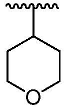 | CH | CH | NH | O | 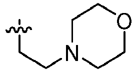 |
| 5 | 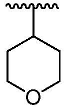 | CH | CH | NH | O | 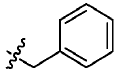 |
| 6 | 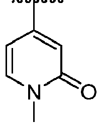 | CH | CH | NH | O | 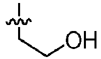 |
| 7 | 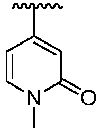 | CH | CH | NH | O | 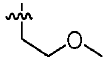 |
| 8 | 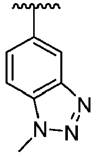 | CH | CH | NH | O | 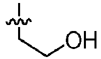 |
| 9 | 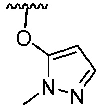 | CH | CH | NH | O | 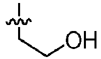 |
| 10 | 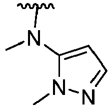 | CH | CH | NH | O | 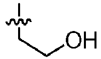 |
| 11 | 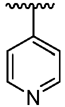 | CH | CH | NH | O | 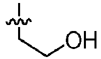 |
| 12 | 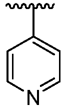 | N | CH | NH | O | 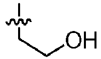 |
| 13 | 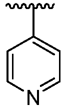 | CH | N | NH | O | 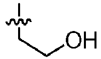 |
| 14 | 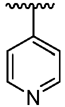 | CH | CH | O | NH | 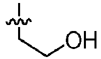 |
| 15 | 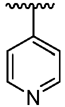 | CH | CH | O | NBn | H |
| 16 | 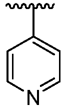 | CH | CH | O | CHBn | H |
| 17 | 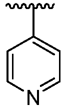 | CH | CH | NH | O | 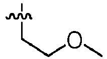 |
| 18 | 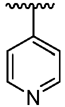 | CH | CH | NH | O | 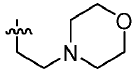 |
| 19 | 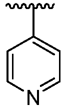 | CH | CH | NH | O | 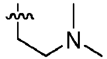 |
| 20 | 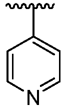 | CH | CH | NH | O | 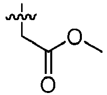 |
| 21 | 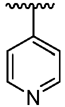 | CH | CH | NH | O | 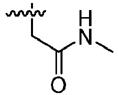 |
| 22 | 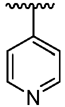 | CH | CH | NH | O | 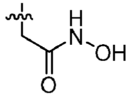 |
| 23 | 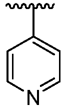 | CH | CH | NH | O | 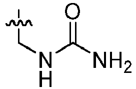 |
| 24 | 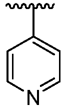 | CH | CH | NH | O | 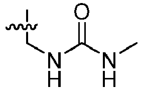 |
| 25 | 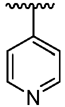 | CH | CH | NH | O | 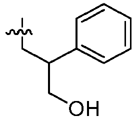 |
| 26 | 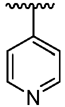 | CH | CH | NH | O | 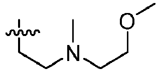 |
| 27 | 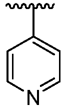 | CH | CH | NH | O | 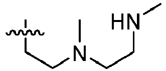 |
| 28 | 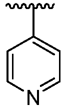 | CH | CH | NH | O | 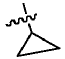 |
| 29 | 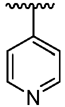 | CH | CH | NH | O | 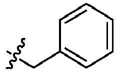 |
| 30 | 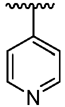 | CH | CH | NH | O | 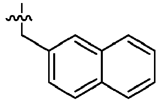 |
| 31 | 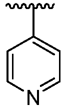 | CH | CH | NH | O | 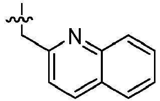 |
| 32 | 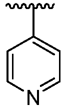 | CH | CH | NH | O | 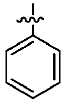 |
| 33 | 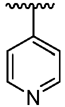 | CH | CH | NH | O | 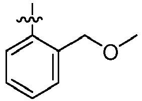 |
| 34 | 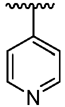 | CH | CH | NH | O | 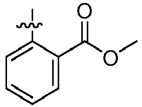 |
| 35 | 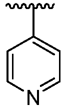 | CH | CH | NH | O | 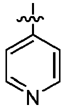 |
| 36 | 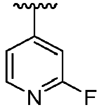 | CH | CH | NH | O | 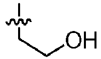 |
| 37 | 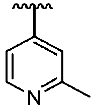 | CH | CH | NH | O | 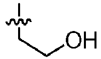 |
| 38 | 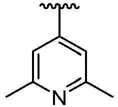 | CH | CH | NH | O | 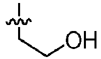 |
| 39 | 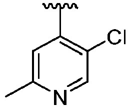 | CH | CH | NH | O | 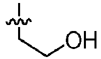 |
| 40 | 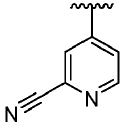 | CH | CH | NH | O | 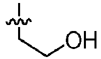 |
| 41 | 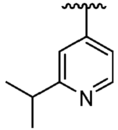 | CH | CH | NH | O | 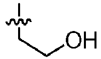 |
| 42 | 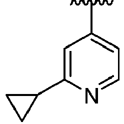 | CH | CH | NH | O | 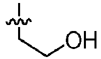 |
| 43 | 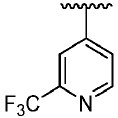 | CH | CH | NH | O | 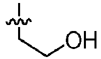 |
| 44 | 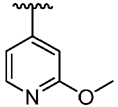 | CH | CH | NH | O | 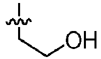 |

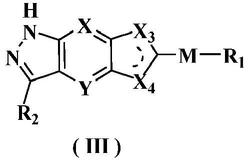

| Compound No. | R₂ | X | Y | X₃ | X₄ | M | Ri |
|---|---|---|---|---|---|---|---|
| 45 | 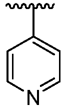 | CH | CH | NH | N | C=O | 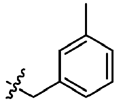 |
| 46 | 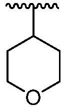 | CH | CH | NH | N | C=O | 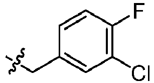 |
| 47 | 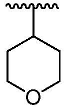 | CH | CH | NH | N | C=O | 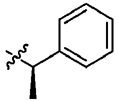 |
| 48 | 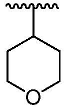 | CH | CH | NH | N | C=O | 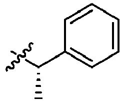 |
| 49 | 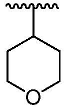 | CH | CH | NH | N | C=O | 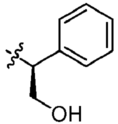 |
| 50 | 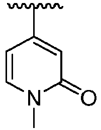 | CH | CH | NH | N | C=O | 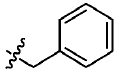 |
| 51 | 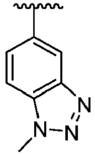 | CH | CH | NH | N | C=O | 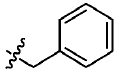 |
| 52 | 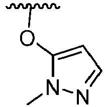 | CH | CH | NH | N | C=O | 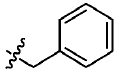 |
| 53 | 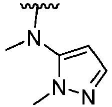 | CH | CH | NH | N | C=O | 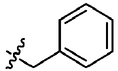 |
| 54 | 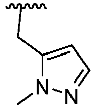 | CH | CH | NH | N | C=O | 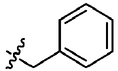 |
| 55 | 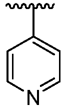 | CH | CH | NH | N | C=O | 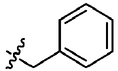 |
| 56 | 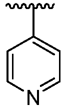 | CH | CH | N | NH | C=O | 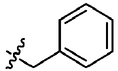 |
| 57 | 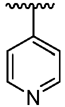 | CH | CH | NH | N | C=O | 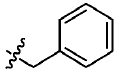 |
| 58 | 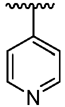 | CH | CH | NH | N | O=C-O | 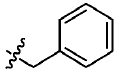 |
| 59 | 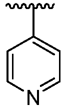 | CH | CH | NH | N | O=C-N | 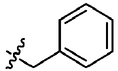 |
| 60 | 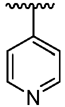 | CH | CH | NH | N | C=N(OH) | 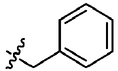 |
| 61 | 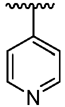 | CH | CH | NH | N | O=S=O | 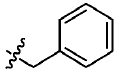 |
| 62 | 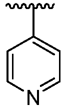 | CH | CH | NH | N | O=PH-O | 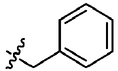 |
| 63 | 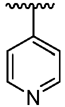 | N | CH | NH | N | C=O | 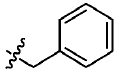 |
| 64 | 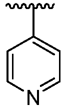 | CH | N | NH | N | C=O | 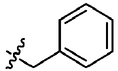 |
| 65 | 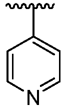 | CH | CH | NH | N | C=O | 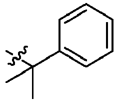 |
| 66 | 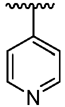 | CH | CH | NH | N | C=O | 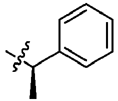 |
| 67 | 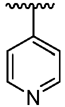 | CH | CH | NH | N | C=O | 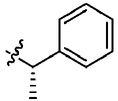 |
| 68 | 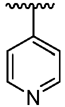 | CH | CH | NH | N | C=O | 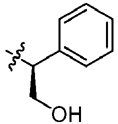 |
| 69 | 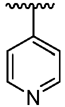 | CH | CH | NH | N | C=O | 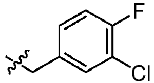 |
| 70 | 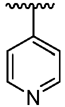 | CH | CH | NH | N | C=O | 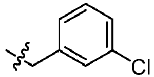 |
| 71 | 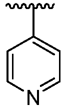 | CH | CH | NH | N | C=O | 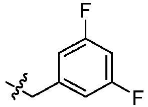 |
| 72 | 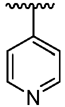 | CH | CH | NH | N | C=O | 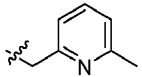 |
| 73 | 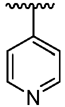 | CH | CH | NH | N | C=O | 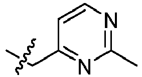 |
| 74 | 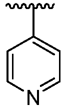 | CH | CH | NH | N | C=O | 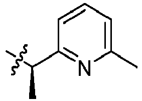 |
| 75 | 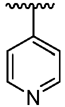 | CH | CH | NH | N | C=O | 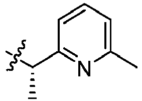 |
| 76 | 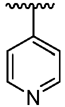 | CH | CH | NH | N | C=O | 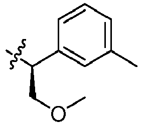 |
| 77 | 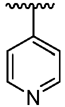 | CH | CH | NH | N | C=O | 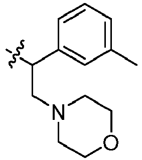 |
| 78 | 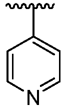 | CH | CH | NH | N | C=O | 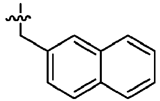 |
| 79 | 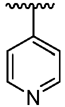 | CH | CH | NH | N | C=O | 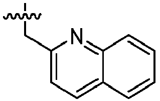 |
| 80 | 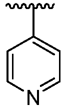 | CH | CH | NH | N | C=O | 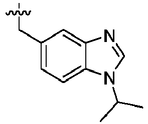 |
| 81 | 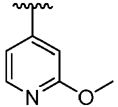 | CH | CH | NH | N | C=O | 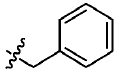 |
| 82 | 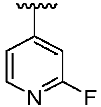 | CH | CH | NH | N | C=O | 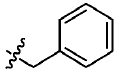 |
| 83 | 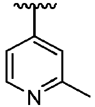 | CH | CH | NH | N | C=O | 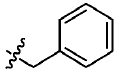 |
| 84 | 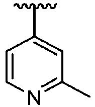 | CH | CH | NH | N | C=O | 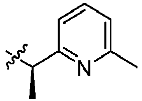 |
| 85 | 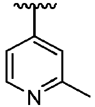 | CH | CH | NH | N | C=O | 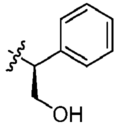 |
| 86 | 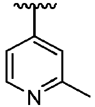 | CH | CH | NH | N | C=O | 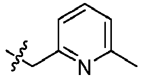 |
| 87 | 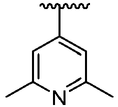 | CH | CH | NH | N | C=O | 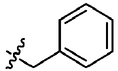 |
| 88 | 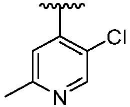 | CH | CH | NH | N | C=O | 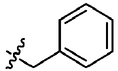 |
| 89 | 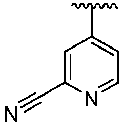 | CH | CH | NH | N | C=O | 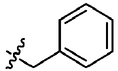 |
| 90 | 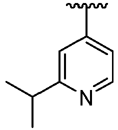 | CH | CH | NH | N | C=O | 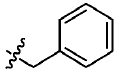 |
| 91 | 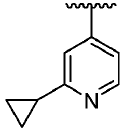 | CH | CH | NH | N | C=O | 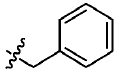 |
| 92 | 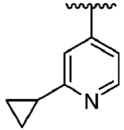 | CH | CH | NH | N | C=O | 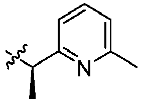 |
| 93 | 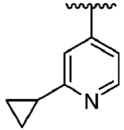 | CH | CH | NH | N | C=O | 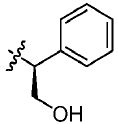 |
| 94 | 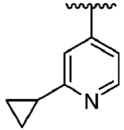 | CH | CH | NH | N | C=O | 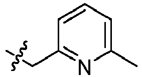 |
| 95 | 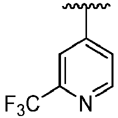 | CH | CH | NH | N | C=O | 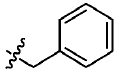 |
| 96 | 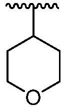 | CH | CH | NH | CH | C=O | 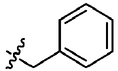 |
| 97 | 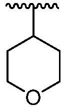 | CH | CH | NH | CH | C=O | 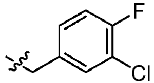 |
| 98 | 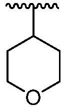 | CH | CH | NH | CH | C=O | 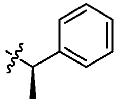 |
| 99 | 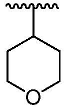 | CH | CH | NH | CH | C=O | 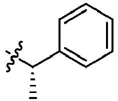 |
| 100 | 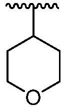 | CH | CH | NH | CH | C=O | 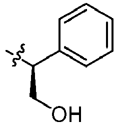 |
| 101 | 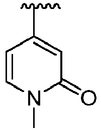 | CH | CH | NH | CH | C=O | 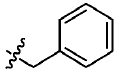 |
| 102 | 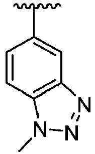 | CH | CH | NH | CH | C=O | 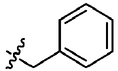 |
| 103 | 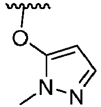 | CH | CH | NH | CH | C=O | 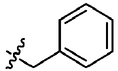 |
| 104 | 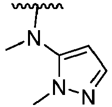 | CH | CH | NH | CH | C=O | 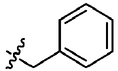 |
| 105 | 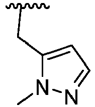 | CH | CH | NH | CH | C=O | 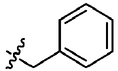 |
| 106 | 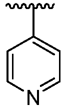 | CH | CH | NH | CH | C=O | 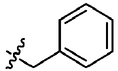 |
| 107 | 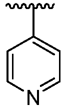 | CH | CH | NH | CH | C=S | 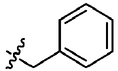 |
| 108 | 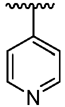 | CH | CH | NH | CH | O=C-O | 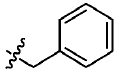 |
| 109 | 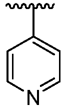 | CH | CH | NH | CH | O=C-NH | 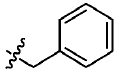 |
| 110 | 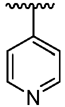 | CH | CH | NH | CH | C=N(OH) | 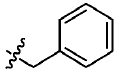 |
| 111 | 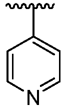 | CH | CH | NH | CH | O=S=O | 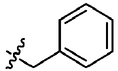 |
| 112 | 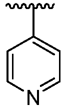 | CH | CH | NH | CH | O=PH-O | 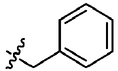 |
| 113 | 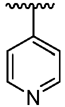 | N | CH | NH | CH | C=O | 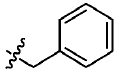 |
| 114 | 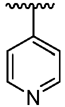 | CH | N | NH | CH | C=O | 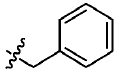 |
| 115 | 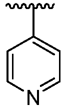 | CH | CH | NH | CH | C=O | 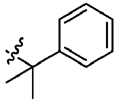 |
| 116 | 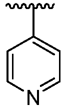 | CH | CH | NH | CH | C=O | 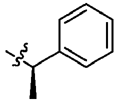 |
| 117 | 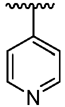 | CH | CH | NH | CH | C=O | 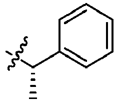 |
| 118 | 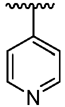 | CH | CH | NH | CH | C=O | 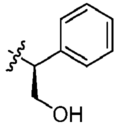 |
| 119 | 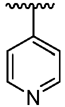 | CH | CH | NH | CH | C=O | 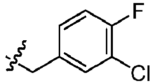 |
| 120 | 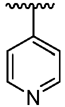 | CH | CH | NH | CH | C=O | 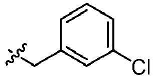 |
| 121 | 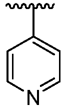 | CH | CH | NH | CH | C=O | 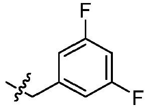 |
| 122 | 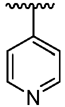 | CH | CH | NH | CH | C=O | 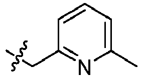 |
| 123 | 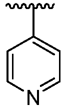 | CH | CH | NH | CH | C=O | 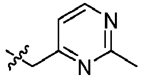 |
| 124 | 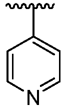 | CH | CH | NH | CH | C=O | 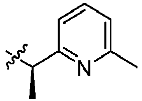 |
| 125 | 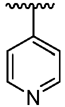 | CH | CH | NH | CH | C=O | 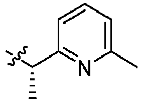 |
| 126 | 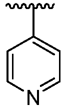 | CH | CH | NH | CH | C=O | 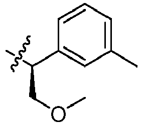 |
| 127 | 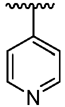 | CH | CH | NH | CH | C=O | 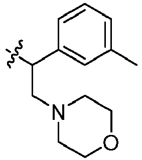 |
| 128 | 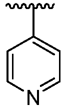 | CH | CH | NH | CH | C=O | 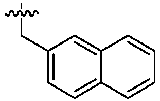 |
| 129 | 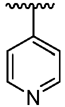 | CH | CH | NH | CH | C=O | 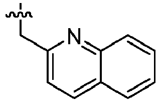 |
| 130 | 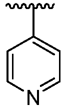 | CH | CH | NH | CH | C=O | 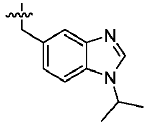 |
| 131 | 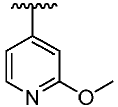 | CH | CH | NH | CH | C=O | 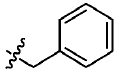 |
| 132 | 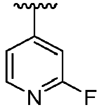 | CH | CH | NH | CH | C=O | 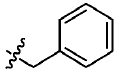 |
| 133 | 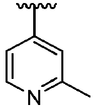 | CH | CH | NH | CH | C=O | 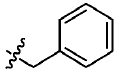 |
| 134 | 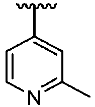 | CH | CH | NH | CH | C=O | 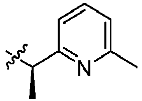 |
| 135 | 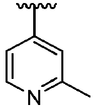 | CH | CH | NH | CH | C=O | 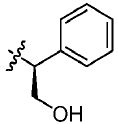 |
| 136 | 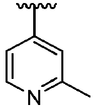 | CH | CH | NH | CH | C=O | 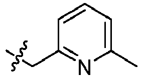 |
| 137 | 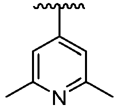 | CH | CH | NH | CH | C=O | 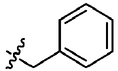 |
| 138 | 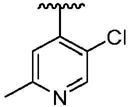 | CH | CH | NH | CH | C=O | 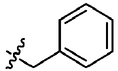 |
| 139 | 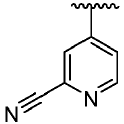 | CH | CH | NH | CH | C=O | 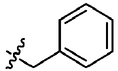 |
| 140 | 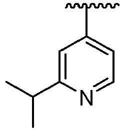 | CH | CH | NH | CH | C=O | 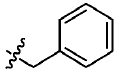 |
| 141 | 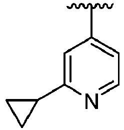 | CH | CH | NH | CH | C=O | 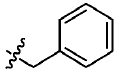 |
| 142 | 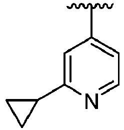 | CH | CH | NH | CH | C=O | 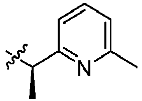 |
| 143 | 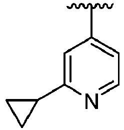 | CH | CH | NH | CH | C=O | 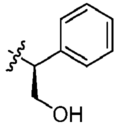 |
| 144 | 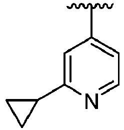 | CH | CH | NH | CH | C=O | 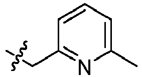 |
| 145 | 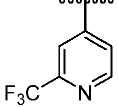 | CH | CH | NH | CH | C=O | 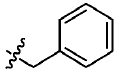 |

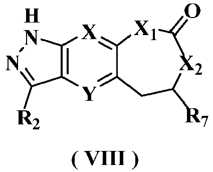

| Compound No. | R₂ | X | Y | X₁ | X₂ | R₇ |
|---|---|---|---|---|---|---|
| 146 | 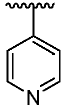 | CH | CH | NH | O | Me |
| 147 | 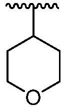 | CH | CH | NH | O | 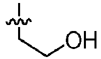 |
| 148 | 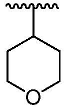 | CH | CH | NH | O | 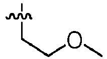 |
| 149 | 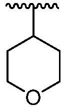 | CH | CH | NH | O | 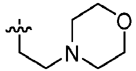 |
| 150 | 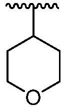 | CH | CH | NH | O | 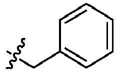 |
| 151 | 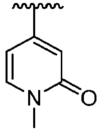 | CH | CH | NH | O | 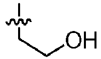 |
| 152 | 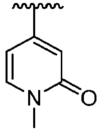 | CH | CH | NH | O | 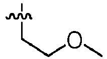 |
| 153 | 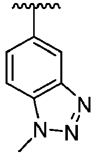 | CH | CH | NH | O | 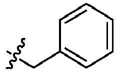 |
| 154 | 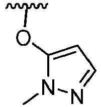 | CH | CH | NH | O | 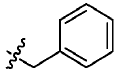 |
| 155 | 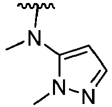 | CH | CH | NH | O | 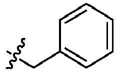 |
| 156 | 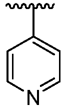 | CH | CH | NH | O | 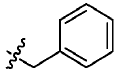 |
| 157 | 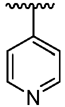 | N | CH | NH | O | 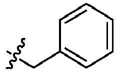 |
| 158 | 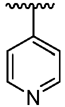 | CH | N | NH | O | 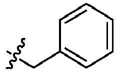 |
| 159 | 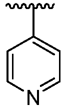 | CH | CH | O | NH | 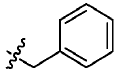 |
| 160 | 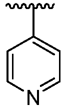 | CH | CH | O | NBn | H |
| 161 | 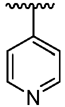 | CH | CH | O | CHBn | H |
| 162 | 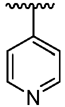 | CH | CH | NH | O | 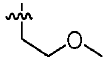 |
| 163 | 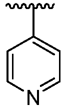 | CH | CH | NH | O | 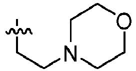 |
| 164 | 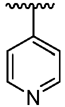 | CH | CH | NH | O | 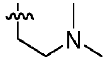 |
| 165 | 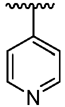 | CH | CH | NH | O | 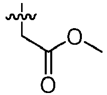 |
| 166 | 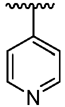 | CH | CH | NH | O | 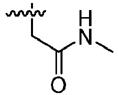 |
| 167 | 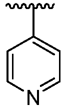 | CH | CH | NH | O | 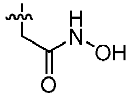 |
| 168 | 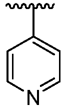 | CH | CH | NH | O | 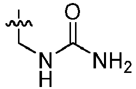 |
| 169 | 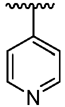 | CH | CH | NH | O | 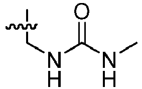 |
| 170 | 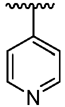 | CH | CH | NH | O | 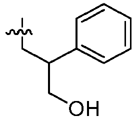 |
| 171 | 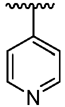 | CH | CH | NH | O | 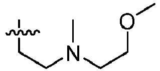 |
| 172 | 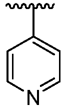 | CH | CH | NH | O | 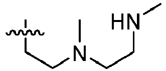 |
| 173 | 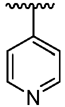 | CH | CH | NH | O | 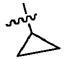 |
| 174 | 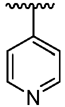 | CH | CH | NH | O | 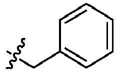 |
| 175 | 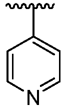 | CH | CH | NH | O | 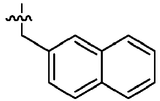 |
| 176 | 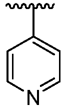 | CH | CH | NH | O | 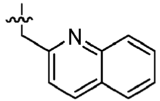 |
| 177 | 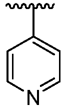 | CH | CH | NH | O | 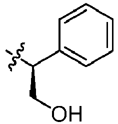 |
| 178 | 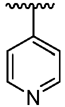 | CH | CH | NH | O | 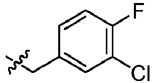 |
| 179 | 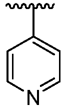 | CH | CH | NH | O | 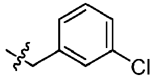 |
| 180 | 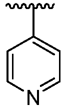 | CH | CH | NH | O | 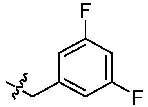 |
| 181 | 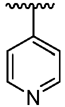 | CH | CH | NH | O | 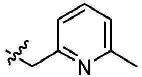 |
| 182 | 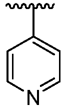 | CH | CH | NH | O | 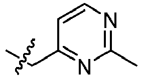 |
| 183 | 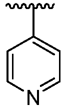 | CH | CH | NH | O | 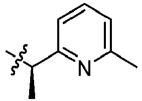 |
| 184 | 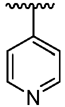 | CH | CH | NH | O | 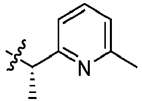 |
| 185 | 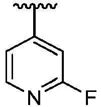 | CH | CH | NH | O | 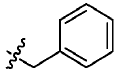 |
| 186 | 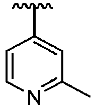 | CH | CH | NH | O | 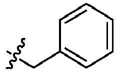 |
| 187 | 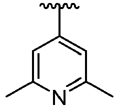 | CH | CH | NH | O | 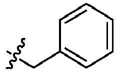 |
| 188 | 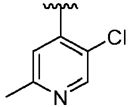 | CH | CH | NH | O | 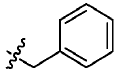 |
| 189 | 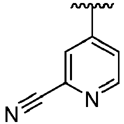 | CH | CH | NH | O | 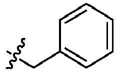 |
| 190 | 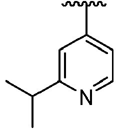 | CH | CH | NH | O | 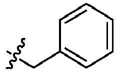 |
| 191 | 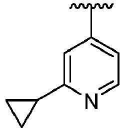 | CH | CH | NH | O | 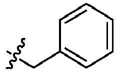 |
| 192 | 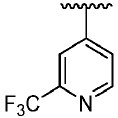 | CH | CH | NH | O | 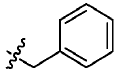 |
| 193 | 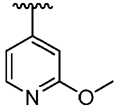 | CH | CH | NH | O | 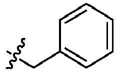 |

### Example 1

### Preparation of (R)-N-(1-phenylethyl)-3-(pyridin-4-yl)-1,7-dihydropyrrolo[3,2-f]indazole-6-carbox amide

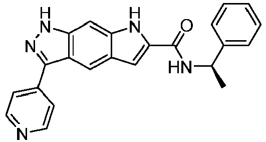

### Step 1: Preparation of methyl 3-(6-nitro-3-(pyridin-4-yl)-1-trityl-1H-indazol-5-yl)acrylate

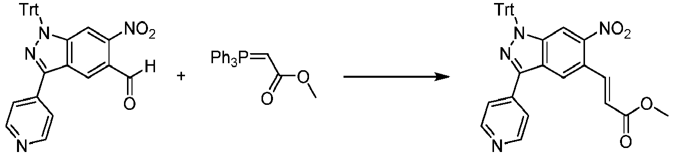

6-Nitro-3-(pyridin-4-yl)-1-trityl-1H-indazole-5-carbaldehyde (350 mg, 0.68 mmol) and methoxyformylmethylenetriphenylphosphine (344 mg, 1.03 mmol) were dissolved in toluene (20 mL). The reaction solution was heated to 105°C, and stirred for 2 hours. After completion of the reaction, the reaction solution was concentrated and purified by column chromatography [eluent: petroleum ether ~ petroleum ether/ethyl acetate (60:40)] to obtain a light cyan solid product methyl 3-(6-nitro-3-(pyridin-4-yl)-1-trityl-1H-indazol-5-yl)acrylate (200 mg, yield: 51%).
MS m/z (ESI): 567.1 [M+H]⁺.

### Step 2: Preparation of 3-(6-nitro-3-(pyridin-4-yl)-1-trityl-1H-indazol-5-yl)acrylic acid

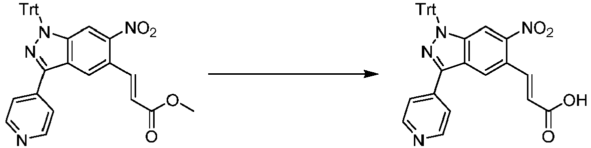

Methyl 3-(6-nitro-3-(pyridin-4-yl)-1-trityl-1H-indazol-5-yl)acrylate (150 mg, 0.26 mmol) and lithium hydroxide monohydrate (22 mg, 0.53 mmol) were dissolved in tetrahydrofuran (8 mL) and water (2 mL). The reaction solution was stirred at room temperature for 4 hours. After completion of the reaction, 0.5 N aqueous acetic acid solution (30 mL) was added to the reaction solution to quench the reaction, and the resulting mixture was extracted with ethyl acetate (30 mL*2). The organic phase was washed with aqueous sodium chloride solution (20 mL), dried over sodium sulfate, and concentrated to obtain a orange solid product 3-(6-nitro-3-(pyridin-4-yl)-1-trityl-1H-indazol-5-yl)acrylic acid (150 mg, yield: 100%).
MS m/z (ESI): 553.1 [M+H]⁺.

### Step 3: Preparation of (R)-3-(6-nitro-3-(pyridin-4-yl)-1-trityl-1H-indazol-5-yl)-N-(1-phenylethyl)acrylami de

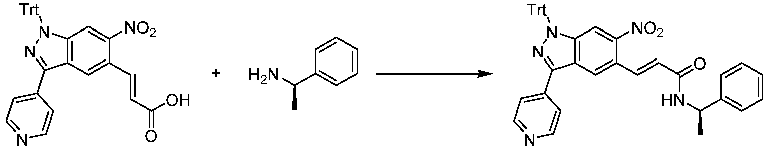

Triethylamine (816 mg, 0.81 mmol) was added to a solution of 3-(6-nitro-3-(pyridin-4-yl)-1-trityl-1H-indazol-5-yl)acrylic acid (150 mg, 0.27 mmol), (R)-1-phenylethane-1-amine (49 mg, 0.40 mmol) and O-(7-azabenzotriazol-1-yl)-N,N,N',N'-tetramethyluronium hexafluorophosphate (207 mg, 0.54 mmol) in tetrahydrofuran (20 mL). After completion of the addition, the reaction solution was stirred at room temperature for 16 hours. Ethyl acetate (60 mL) was added to the reaction solution. The solution was washed successively with 0.5 N acetic acid (30 mL*2), aqueous sodium bicarbonate solution (20 mL) and aqueous sodium chloride solution (20 mL), dried over sodium sulfate, and concentrated to obtain a yellow solid product (R)-3-(6-nitro-3-(pyridin-4-yl)-1-trityl-1H-indazol-5-yl)-N-(1-phenylethyl)acrylamide (150 mg, yield: 84%).
MS m/z (ESI): 656.2 [M+H]⁺.

### Step 4: Preparation of (R)-N-(1-phenylethyl)-3-(pyridin-4-yl)-1-trityl-1,7-dihydropyrrolo[3,2-f]indazole-6-carboxamide

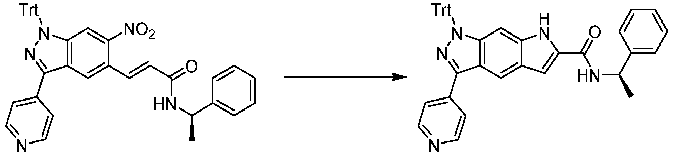

(R)-3-(6-Nitro-3-(pyridin-4-yl)-1-trityl-1H-indazol-5-yl)-N-(1-phenylethyl)acryla mide (130 mg, 0.19 mmol) was reacted with triethyl phosphite (3 mL) under a microwave condition at 140°C for 45 minutes. The reaction solution was concentrated and purified by column chromatography [eluent: petroleum ether ∼ petroleum ether/ethyl acetate (40:60)] to obtain a yellow oily product (R)-N-(1-phenylethyl)-3-(pyridin-4-yl)-1-trityl-1,7-dihydropyrrolo[3,2-f]indazole-6-car boxamide (50 mg, yield: 40%).
MS m/z (ESI): 624.2 [M+H]⁺.

### Step 5: Preparation of (R)-N-(1-phenylethyl)-3-(pyridin-4-yl)-1,7-dihydropyrrolo[3,2-f]indazole-6-carbox amide

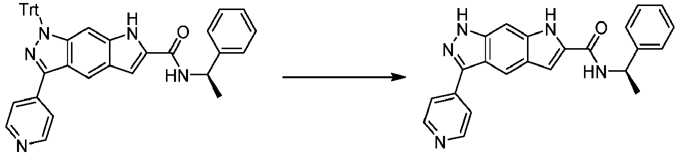

Trifluoroacetic acid (4 mL) was added to a solution of (R)-N-(1-phenylethyl)-3-(pyiidin-4-yl)-1-trityl-1,7-dihydropyrrolo[3,2-f]indazole-6-car boxamide (50 mg, 0.08 mmol) and triethylsilane (19 mg, 0.16 mmol) in dichloromethane (2 mL). The reaction solution was stirred at room temperature for 2 hours. The reaction solution was concentrated to dryness, and methanol was added to dissolve the residues. The resulting solution was adjusted to alkaline by adding aqua ammonia, and purified by thin layer chromatography (developing solvent: CH₂Cl₂/MeOH=10/1) to obtain a yellow solid product (R)-N-(1-phenylethyl)-3-(pyridin-4-yl)-1,7-dihydropyrrolo[3,2-f]indazole-6-carboxami de (6 mg, yield: 20%).
¹H NMR (400 MHz, MeOD) δ 8.66 (d, *J* = 4 Hz, 2H), 8.41 (s, 1H), 8.12 (d, *J* = 4 Hz, 2H), 7.53 (s, 1H), 7.44 (d, *J* = 8 Hz, 2H), 7.39 - 7.30 (m, 3H), 7.26 - 7.22 (m, 1H), 5.29 (q, *J* = 8 Hz, 1H), 1.61 (d, *J* = 8 Hz, 3H).
MS m/z (ESI): 382.1 [M+H]⁺.

### Example 2

### Preparation of (R)-N-(1-phenylethyl)-3-(pyridin-4-yl)-1,7-dihydroimidazo[4,5-f]indazole-6-carbox amide

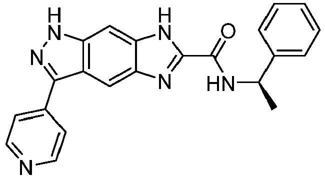

### Step 1: Preparation of 3-bromo-5,6-dinitro-1-trityl-1H-indazole

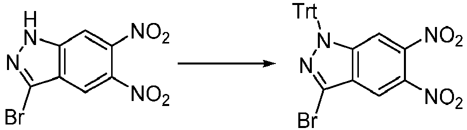

3-Bromo-5,6-dinitro-1H-indazole (5.0 g, 17.42 mmol), cesium carbonate (8.51 g, 26.12 mmol) and DMF (50 mL) were stirred, and added with triphenylchloromethane (5.83 g, 20.91 mmol) in batches. The reaction solution was stirred at room temperature overnight. The reaction solution was added with 250 mL of water, stirred for 1 hour and filtered. The resulting solid was washed with water to obtain a solid product 3-bromo-5,6-dinitro-1-trityl-1H-indazole (11.47 g, wet weight). The product was used directly in the next step without drying.

### Step 2: Preparation of 5,6-dinitro-3-(pyridin-4-yl)-1-trityl-1H-indazole

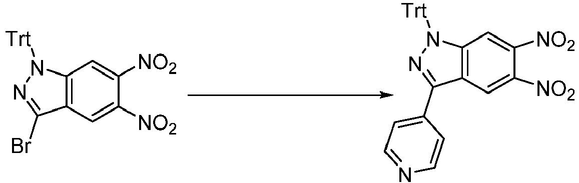

3-Bromo-5,6-dinitro-1-trityl-1H-indazole (11.47 g, 21.66 mmol), 4-pyridineboronic acid (5.33 g, 43.36 mmol), Pd(dppf)Cl₂ (1.59 g, 4.33 mmol), potassium carbonate (6.0 g, 14 mmol) and dioxane/water (125 mL/25 mL) were stirred under a nitrogen atmosphere at 80°C for 5 hours. The reaction solution was cooled to room temperature, added with water, extracted with ethyl acetate, and purified by column chromatography to obtain a solid product 5,6-dinitro-3-(pyridin-4-yl)-1-trityl-1H-indazole (7.50 g, yield in two steps: 81%).
MS m/z (ESI): 528.1 [M+H]⁺.

### Step 3: Preparation of 3-(pyridin-4-yl)-1-trityl-1H-indazole-5,6-diamine

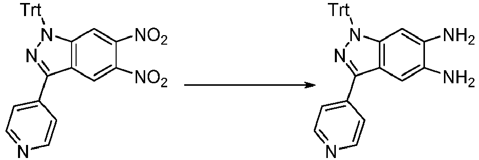

5,6-Dinitro-3-(pyridin-4-yl)-1-trityl-1H-indazole (2.5 g, 4.74 mmol), 10% Pd/C (200 mg) and THF (150 mL) were subjected to a hydrogenation reaction at room temperature overnight. The reaction solution was filtered, and concentrated to dryness to obtain a brown solid product 3-(pyridin-4-yl)-1-trityl-1H-indazole-5,6-diamine, which was used directly in the next step.
MS m/z (ESI): 468.2 [M+H]⁺.

### Step 4: Preparation of 3-(pyridin-4-yl)-6-(trichloromethyl)-1-trityl-1,7-dihydroimidazo[4,5-f]indazole

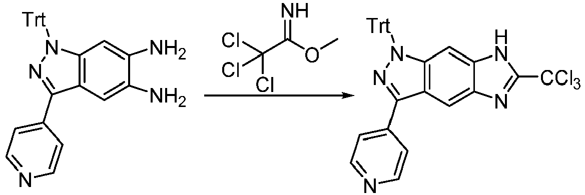

3-(Pyridin-4-yl)-1-trityl-1H-indazole-5,6-diamine (obtained in the previous step), methyl 2,2,2-trichloroacetimidate (920 mg, 5.22 mmol) and acetic acid (25 mL) were stirred at room temperature overnight. The reaction solution was added with 100 ml of water, and stirred for 1 hour. The reaction solution was filtered, and the resulting solid was washed with water to obtain a brown solid product 3-(pyridin-4-yl)-6-(trichloromethyl)-1-trityl-1,7-dihydroimidazo[4,5-f]indazole (4.32 g, wet weight). The product was used directly in the next step.
MS m/z (ESI): 594.1 596.1 [M+H]⁺.

### Step 5: Preparation of methyl 3-(pyridin-4-yl)-1-trityl-1,7-dihydroimidazo[4,5-f]indazole-6-carboxylate

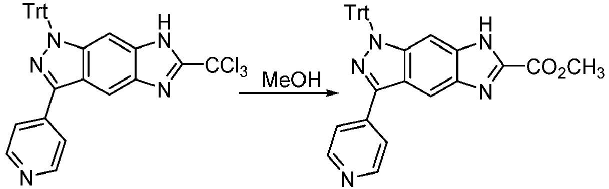

3-(Pyridin-4-yl)-6-(trichloromethyl)-1-trityl-1,7-dihydroimidazo[4,5-f]indazole (4.32 g) and methanol (50 mL) were refluxed overnight, followed by concentrating to dryness. The reaction solution was added with isopropyl ether, stirred for 30 min and filtered. The resulting solid was washed with isopropyl ether to obtain a brown solid crude product methyl 3-(pyridin-4-yl)-1-trityl-1,7-dihydroimidazo[4,5-f]indazole-6-carboxylate (2.44 g).
MS m/z (ESI): 536.2 [M+H]⁺.

### Step 6: Preparation of (R)-N-(1-phenylethyl)-3-(pyridin-4-yl)-1-trityl-1,7-dihydroimidazo[4,5-f]indazole-6 -carboxamide

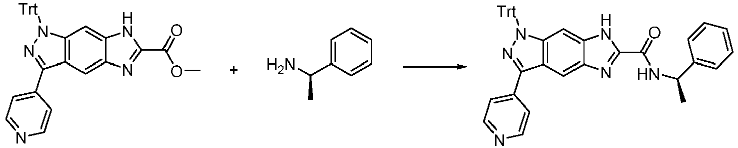

Methyl 3-(pyridin-4-yl)-1-trityl-1,7-dihydroimidazo[4,5-f]indazole-6-carboxylate (100 mg, 0.19 mmol) and (*R*)-1-phenylethane-1-amine (1.0 mL) were added to a microwave reaction tube. The reaction solution was heated to 150°C for 60 min. After cooling to room temperature, 20 mL of ethyl acetate was added to the reaction solution. The ethyl acetate layer was washed with saturated ammonium chloride solution and saturated sodium chloride solution, dried over anhydrous sodium sulfate, and purified by column chromatography to obtain a crude product (80 mg), which was used directly in the next step.
MS m/z (ESI): 625.1 [M+H]⁺.

### Step 7: Preparation of (R)-N-(1-phenylethyl)-3-(pyridin-4-yl)-1,7-dihydroimidazo[4,5-f]indazole-6-carbox amide

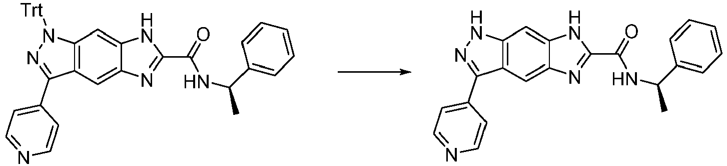

The crude (R)-N-(1-phenylethyl)-3-(pyridin-4-yl)-1,7-dihydroimidazo[4,5-f]indazole-6-carboxami de (80 mg) obtained in the previous step was dissolved in 4 mL of dichloromethane, and added with 4 mL of trifluoroacetic acid. The reaction solution was reacted at room temperature for 3 hours, followed by concentrating to dryness. The resulting crude product was dissolved in a mixed solution of ethyl acetate and tetrahydrofuran. The solution was washed with saturated sodium bicarbonate solution and saturated sodium chloride solution, and dried over anhydrous sodium sulfate. The resulting organic solution was concentrated to dryness, and purified by preparative chromatography to obtain a yellow solid product (*R*)-N-(1-phenylethyl)-3-(pyridin-4-yl)-1,7-dihydroimidazo[4,5-f]indazole-6-carboxami de (15 mg, yield in two steps: 21%).
¹H NMR (400 MHz, DMSO-*d*₆) δ: 13.45-13.39 (m, 1H), 13.21-13.08 (m, 1H), 9.43 - 9.33 (m, 1H), 8.72 (s, 2H), 8.44 - 8.04 (m, 3H), 7.99-7.87(m,1H), 7.58(m, 2H), 7.36 - 7.32 (m, 2H), 7.26-7.22(m, 1H), 5.25 - 5.21 (m, 1H), 1.57 (d, *J=* 7.0 Hz, 3H).
MS m/z (ESI): 383.1 [M+H]⁺.

### Example 3

### Preparation of N-(3,4-dimethoxybenzyl)-3-(pyridin-4-yl)-1,7-dihydroimidazo[4,5-f]indazole-6-car boxamide

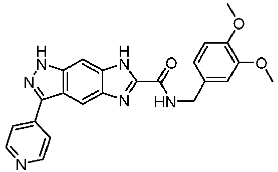

N-(3,4-Dimethoxybenzyl)-3-(pyridin-4-yl)-1,7-dihydroimidazo[4,5-f]indazole-6-ca rboxamide was obtained from methyl 3-(pyridin-4-yl)-1-trityl-1,7-dihydroimidazo[4,5-f]indazole-6-carboxylate and (3,4-dimethoxyphenyl) methylamine as the starting materials in accordance with Steps 6 and 7 of Example 2.
¹H NMR (400 MHz, DMSO-*d*₆) δ 13.48 (s, 1H), 9.37 (d, *J* = 8.6 Hz, 1H), 8.71 - 8.64 (m, 2H), 8.13 (s, 1H), 7.99 (s, 1H), 7.96 - 7.89 (m, 2H), 7.72 (t, *J* = 10.2 Hz, 1H), 6.93 - 6.84 (m, 2H), 6.89 - 6.78 (m, 1H), 4.72 (dt, *J* = 10.0, 1.0 Hz, 2H), 3.82 (d, *J* = 8.5 Hz, 6H).
MS m/z (ESI): 429.1 [M+H]⁺.

### Example 4

### Preparation of N-(3-chloro-4-fluorobenzyl)-3-(pyridin-4-yl)-1,7-dihydroimidazo[4,5-f]indazole-6-c arboxamide

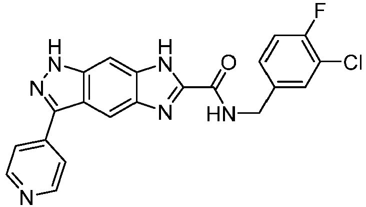

N-(3-Chloro-4-fluorobenzyl)-3-(pyridin-4-yl)-1,7-dihydroimidazo[4,5-f]indazole-6 -carboxamide was obtained from methyl 3-(pyridin-4-yl)-1-trityl-1,7-dihydroimidazo[4,5-f]indazole-6-carboxylate and (3-chloro-4-fluorophenyl) methylamine as the starting materials in accordance with Steps 6 and 7 of Example 2.
¹H NMR (400 MHz, DMSO-*d*₆) δ 13.47(s, 1H), 9.38 (d, *J* = 8.6 Hz, 1H), 8.13 (s, 1H), 7.99 (s, 1H), 7.96 - 7.89 (m, 2H), 7.72 (t, *J* = 10.2 Hz, 1H), 7.38 (dd, *J* = 7.4, 4.9 Hz, 1H), 7.14 (ddq, *J* = 9.1, 7.9, 1.2 Hz, 2H), 4.73 (dt, *J* = 10.1, 1.0 Hz, 2H).
MS m/z (ESI): 421.2[M+H]⁺.

### Example 5

### Preparation of (R)-N-methyl-N-(1-phenylethyl)-3-(pyridin-4-yl)-1,7-dihydroimidazo[4,5-f]indazole -6-carboxamide

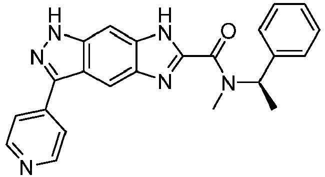

(R)-N-Methyl-N-(1-phenyl ethyl)-3-(pyridin-4-yl)-1,7-dihydroimidazo[4,5-f]indazo le-6-carboxamide was obtained from methyl 3-(pyridin-4-yl)-1-trityl-1,7-dihydroimidazo[4,5-f]indazole-6-carboxylate and (R)-N-methyl-1-phenylethan-1-amine as the starting materials in accordance with Steps 6 and 7 of Example 2.
¹H NMR (400 MHz, DMSO-*d*₆) δ 13.47 (s, 1H), 9.39 (d, *J* = 8.6 Hz, 1H), 8.71 - 8.64 (m, 2H), 8.05 (s, 1H), 7.96 - 7.89 (m, 3H), 7.37 - 7.23 (m, 5H), 5.27 - 5.17 (m, 1H), 3.23 (s, 3H), 1.47 (d, *J* = 6.6 Hz, 3H).
MS m/z (ESI): 397.1 [M+H]⁺.

### Example 6

### Preparation of (R)-3-(pyridin-4-yl)-N-(1-(p-tolyl)ethyl)-1,7-dihydroimidazo[4,5-f]indazole-6-carbo xamide

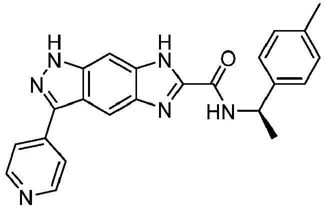

### Step 1: Preparation of (R)-3-(pyridin-4-yl)-N-(1-(p-tolyl)ethyl)-1-trityl-1,7-dihydroimidazo[4,5-f]indazole-6-carboxamide

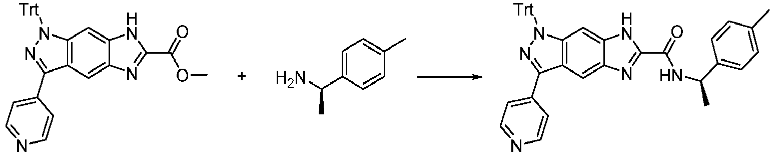

Methyl 3-(pyridin-4-yl)-1-trityl-1,7-dihydroimidazo[4,5-f]indazole-6-carboxylate (100 mg, 0.19 mmol) and (R)-1-(p-tolyl)ethan-1-amine (1.0 mL) were added to a microwave reaction tube. The reaction solution was heated to 150°C for 60 min. After cooling to room temperature, 20 mL of ethyl acetate was added to the reaction solution. The ethyl acetate layer was washed with saturated ammonium chloride solution and saturated sodium chloride solution, dried over anhydrous sodium sulfate, and purified by column chromatography to obtain a crude product (80 mg, yield: 67%), which was used directly in the next step.
MS m/z (ESI): 639.3 [M+H]⁺.

### Step 2: Preparation of (R)-3-(pyridin-4-yl)-N-(1-(p-tolyl)ethyl)-1,7-dihydroimidazo[4,5-f]indazole-6-carbo xamide

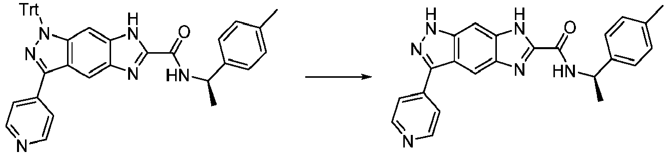

The crude (R)-3-(pyridin-4-yl)-N-(1-(p-tolyl)ethyl)-1-trityl-1,7-dihydroimidazo[4,5-f]indazole-6-c arboxamide (80 mg) was dissolved in 4 mL of dichloromethane, and added with 4 mL of trifluoroacetic acid. The reaction solution was reacted at room temperature for 3 hours, followed by concentrating to dryness. The resulting crude product was dissolved in a mixed solution of ethyl acetate and tetrahydrofuran. The solution was washed with saturated sodium bicarbonate solution and saturated sodium chloride solution, and dried over anhydrous sodium sulfate. The resulting organic solution was concentrated to dryness, and purified by preparative chromatography to obtain a yellow solid product (R)-3-(pyridin-4-yl)-N-(1-(p-tolyl)ethyl)-1,7-dihydroimidazo[4,5-f]indazole-6-carboxa mide (10 mg, yield: 20%).
¹H NMR (400 MHz, MeOD) δ 8.57 (d, *J* = 4 Hz, 2H), 8.37 (s, 0.6H), 8.15 (s, 0.4H), 8.02 (d, *J* = 4 Hz, 2H), 7.79 (s, 0.4H), 7.56 (s, 0.6H), 7.25 (d, *J* = 8 Hz, 2H), 7.08 (d, *J* = 8 Hz, 2H), 5.12-5.16 (m, 1H), 2.22 (s, 3H), 1.52 (d, *J* = 8 Hz, 3H).
MS m/z (ESI): 397.1[M+H]⁺.

### Example 7

### Preparation of (R)-N-(1-(4-fluorophenyl)ethyl)-3-(pyridin-4-yl)-1,7-dihydroimidazo[4,5-f]indazole -6-carboxamide

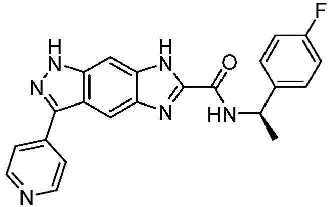

### Step 1: Preparation of (R)-N-(1-(4-fluorophenyl)ethyl)-3-(pyridin-4-yl)-1-trityl-1,7-dihydroimidazo[4,5-f]i ndazole-6-carboxamide

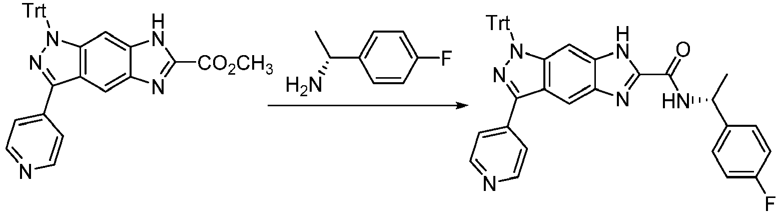

Methyl 3-(pyridin-4-yl)-1-trityl-1,7-dihydroimidazo[4,5-f]indazole-6-carboxylate (1.5 g, 2.80 mmol) and (R)-1-(4-fluorophenyl)ethan-1-amine (2.5 mL) were stirred under a microwave condition at 150°C for 1 h. The reaction solution was cooled, concentrated to dryness, and purified by column chromatography to obtain a thick crude product, which was used directly in the next step.
MS m/z (ESI): 643.2[M+H]⁺.

### Step 2: Preparation of (R)-N-(1-(4-fluorophenyl)ethyl)-3-(pyridin-4-yl)-1,7-dihydroimidazo[4,5-f]indazole -6-carboxamide

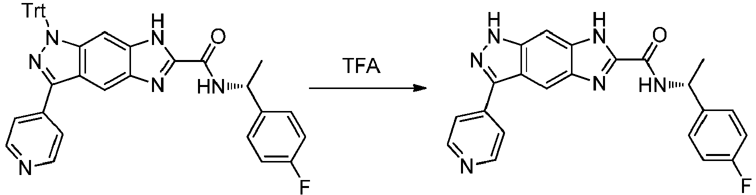

The product obtained in the previous step was dissolved in dichloromethane (5 mL). The solution was added with trifluoroacetic acid (5 mL), and stirred at room temperature for 2 h. The reaction solution was concentrated to dryness, and purified by column chromatography to obtain a yellow solid (R)-N-(1-(4-fluorophenyl)ethyl)-3-(pyridin-4-yl)-1, 7-dihydroimidazo[4,5-f]indazole-6-c arboxamide (603 mg, yield in two steps: 54%).
¹H NMR (400 MHz, MeOD) δ 8.89 (s, 1H), 8.55 (s, 1H), 8.18 (s, 1H), 7.85(s, 1H), 7.28 (dd, *J* = 15.0, 8.3 Hz, 1H), 6.80 (ddd, *J* = 20.8, 17.5, 10.7 Hz, 3H), 6.28 (dd, *J*= 16.7, 1.8 Hz, 1H), 5.80 (dd, *J* = 10.6, 1.7 Hz, 1H), 4.56 (m, 1H), 3.98 - 3.81 (m, 3H).
MS m/z (ESI): 401.1 [M+H]⁺.

### Example 8

### Preparation of (R)-N-(1-(naphthalen-1-yl)ethyl)-3-(pyridin-4-yl)-1,7-dihydroimidazo[4,5-f]indazol e-6-carboxamide

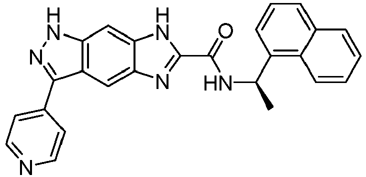

(R)-N-(1-(Naphthalen-1-yl)ethyl)-3-(pyridin-4-yl)-1,7-dihydroimidazo[4,5-f]indaz ole-6-carboxamide was obtained from methyl 3-(pyridin-4-yl)-1-trityl-1,7-dihydroimidazo[4,5-f]indazole-6-carboxylate and (R)-1-(naphthalen-1-yl)ethan-1-amine as the starting materials in accordance with Steps 6 and 7 of Example 2.
¹H NMR (400 MHz, DMSO-*d*₆) δ 13.43-13.41 (m, 1H), 13.18-13.14 (m, 1H), 9.62-9.52 (m, 1H), 8.72-8.70 (m, 2H), 8.43 (s, 0.6H), 8.28 (d, *J* = 8 Hz, 1H), 8.12 (s, 0.4H), 8.04 (d, *J* = 8 Hz, 1.3H), 7.98-7.96 (m, 1.7H), 7.86 (d, *J* = 8 Hz, 1H), 7.74 (d, *J* = 8 Hz, 1H), 7.64 - 7.48 (m, 3H), 6.03-6.01 (m, 1H), 1.56 (d, *J* = 8 Hz, 3H).
MS m/z (ESI): 433.2 [M+H]⁺.

### Example 9

### Preparation of (R)-N-(1-(pyridin-2-yl)ethyl)-3-(pyridin-4-yl)-1,7-dihydroimidazo[4,5-f]indazole-6-carboxamide

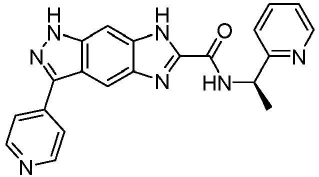

(R)-N-(1-(pyridin-2-yl)ethyl)-3-(pyridin-4-yl)-1,7-dihydroimidazo[4,5-f]indazole-6-carboxamide was obtained from methyl 3-(pyridin-4-yl)-1-trityl-1,7-dihydroimidazo[4,5-f]indazole-6-carboxylate and (R)-1-(pyridin-2-yl)ethan-1-amine as the starting materials in accordance with Steps 6 and 7 of Example 2.
¹H NMR (400 MHz, DMSO-*d*₆) δ13.47 - 13.11 (m, 2H), 9.50 (d, *J* = 8 Hz, 1H), 8.72 (d, *J* = 8 Hz, 2H), 8.51-8.47 (m, 1H), 8.02 (d, *J* = 4 Hz, 2H), 7.77 - 7.73 (m, 1H), 7.42 - 7.30 (m, 3H), 7.24 - 7.22 (m, 1H), 5.27 - 5.19 (m, 1H), 1.56 (d, *J* = 8 Hz, 3H).
MS m/z (ESI): 384.1 [M+H]⁺.

### Example 10

### Preparation of (S)-N-(1-phenylethyl)-3-(pyridin-4-yl)-1,7-dihydroimidazo[4,5-f]indazole-6-carbox amide

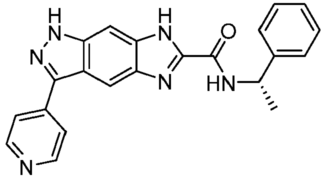

(S)-N-(1-phenylethyl)-3-(pyridin-4-yl)-1,7-dihydroimidazo[4,5-f]indazole-6-carbo xamide was obtained from methyl 3-(pyridin-4-yl)-1-trityl-1,7-dihydroimidazo[4,5-f]indazole-6-carboxylate and (*S*)-1-phenylethane-1-amine as the starting materials in accordance with Steps 6 and 7 of Example 2.
¹H NMR (400 MHz, DMSO-*d*₆) δ 13.45 (s, 0.4H), 13.39 (s, 0.6H), 13.21 (s, 0.4H), 13.08 (s, 0.6H), 9.44 (d, *J* = 7.2 Hz, 0.4H), 9.35 (d, *J* = 7.2 Hz, 0.6H), 8.72 (s, 2H), 8.45 (s, 0.6H), 8.13 (s, 0.4H), 8.09 - 7.93 (m, 2H), 7.88 (s, 0.4H), 7.59 (s, 0.6H), 7.55 - 7.42 (m, 2H), 7.40 - 7.29 (m, 2H), 7.29 - 7.15 (m, 1H), 5.25 - 5.22 (m, 1H), 1.57 (d, *J* = 6.4 Hz, 3H).
MS m/z (ESI): 383.1 [M+H]⁺.

### Example 11

### Preparation of N-(1-phenylethyl)-3-(pyridin-4-yl)-1,7-dihydroimidazo[4,5-f]indazole-6-carboxami de

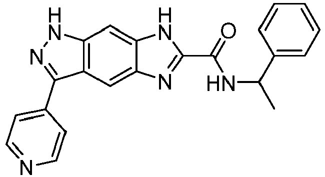

### Step 1: Preparation of N-(1-phenylethyl)-3-(pyridin-4-yl)-1-trityl-1,7-dihydroimidazo[4,5-f]indazole-6-car boxamide

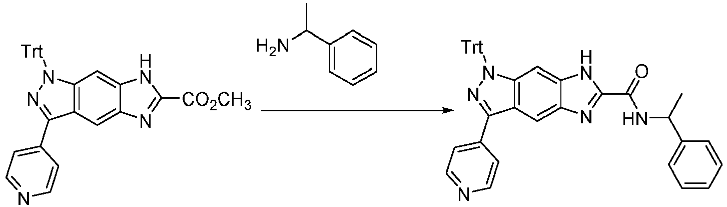

Methyl 3-(pyridin-4-yl)-1-trityl-1,7-dihydroimidazo[4,5-f]indazole-6-carboxylate (100 mg, 0.18 mmol) and 1-phenylethane-1-amine (1.5 mL) were stirred under a microwave condition at 150°C for 1 h. The reaction solution was cooled, concentrated to dryness, and purified by column chromatography to obtain a crude product N-(1-phenylethyl)-3-(pyridin-4-yl)-1-trityl-1,7-dihydroimidazo[4,5-f]indazole-6-carbox amide (27 mg).
MS m/z (ESI): 625.2[M+H]⁺.

### Step 2: Preparation of N-(1-phenylethyl)-3-(pyridin-4-yl)-1,7-dihydroimidazo[4,5-f]indazole-6-carboxami de

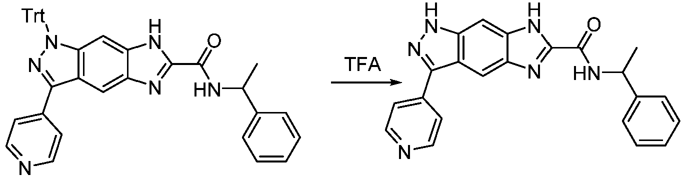

The crude N-(1-phenylethyl)-3-(pyridin-4-yl)-1-trityl-1,7-dihydroimidazo[4,5-f]indazole-6-carbox amide (27 mg) was dissolved in dichloromethane (2 mL). The solution was added with trifluoroacetic acid (2 mL), and stirred at room temperature for 2 h. The reaction solution was concentrated to dryness, and purified by column chromatography to obtain a yellow solid N-(1-phenylethyl)-3-(pyridin-4-yl)-1,7-dihydroimidazo[4,5-f]indazole-6-carboxamide (5 mg, yield in two steps: 7%).
¹H NMR (400 MHz, DMSO-*d*₆) δ 13.48 (s, 1H), 9.37 (d, *J* = 8.6 Hz, 1H), 8.71 - 8.64 (m, 2H), 8.13 (s, 1H), 7.99 (s, 1H), 7.96 - 7.89 (m, 2H), 7.36 - 7.27 (m, 2H), 7.31 - 7.20 (m, 3H), 6.75 (d, *J* = 9.3 Hz, 1H), 5.20 (dqt, *J* = 8.7, 6.8, 0.9 Hz, 1H), 1.57 (d, *J* = 6.7 Hz, 3H).
MS m/z (ESI): 383.1 [M+H]⁺.

### Example 12

### Preparation of N-(2-phenylpropan-2-yl)-3-(pyridin-4-yl)-1,7-dihydroimidazo[4,5-flindazole-6-carb oxamide

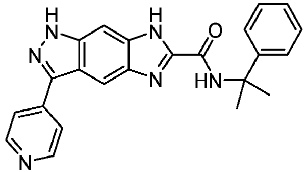

N-(2-Phenylpropan-2-yl)-3-(pyridin-4-yl)-1,7-dihydroimidazo[4,5-f]indazole-6-car boxamide was obtained from methyl 3-(pyridin-4-yl)-1-trityl-1,7-dihydroimidazo[4,5-f]indazole-6-carboxylate and 2-phenylpropan-2-amine as the starting materials in accordance with Steps 6 and 7 of Example 2.
¹H NMR (400 MHz, DMSO-*d*₆) δ 13.51 - 13.00 (m, 2H), 9.26 - 9.17 (m, 1H), 8.72 (d, *J* = 4 Hz, 2H), 8.50 (s, 0.6H), 8.07 - 7.91 (m, 2.7H), 7.59 (s, 0.6H), 7.45 (d, *J* = 8 Hz, 2H), 7.35 (t, *J* = 8 Hz, 2H), 7.28 - 7.23 (m, 1H), 1.56 (s, 6H).
MS m/z (ESI): 397.1 [M+H]⁺.

### Example 13

### Preparation of (S)-N-(2-methoxy-1-phenylethyl)-3-(pyridin-4-yl)-1,7-dihydroimidazo[4,5-f]indazol e-6-carboxamide

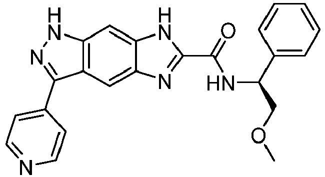

(S)-N-(2-methoxy-1-phenylethyl)-3-(pyridin-4-yl)-1,7-dihydroimidazo[4,5-f]indaz ole-6-carboxamide was obtained from methyl 3-(pyridin-4-yl)-1-trityl-1,7-dihydroimidazo[4,5-f]indazole-6-carboxylate and (S)-2-methoxy-1-phenylethan-1-amine as the starting materials in accordance with Steps 6 and 7 of Example 2.
¹H NMR (400 MHz, DMSO-*d*₆) δ 13.48 (s, 1H), 9.37 (d*, J* = 8.6 Hz, 1H), 8.72 (d, *J* = 5.2 Hz, 2H), 8.34 (s, 2H), 8.04 (d*, J* = 5.1 Hz, 2H), 7.82 - 7.60 (m, 1H), 7.50 (d, *J* = 7.4 Hz, 2H), 7.36 (t, *J* = 7.5 Hz, 2H), 7.28 (t, *J* = 7.3 Hz, 1H), 5.31 (td, *J* = 8.4, 4.8 Hz, 1H), 3.87 (t, *J* = 9.2 Hz, 2H), 3.64 (d, *J* = 5.0 Hz, 3H).
MS m/z (ESI): 413.1 [M+H]⁺.

### Example 14

### Preparation of (R)-N-(1-(4-nitrophenyl)ethyl)-3-(pyridin-4-yl)-1,7-dihydroimidazo[4,5-f]indazole-6-carboxamide

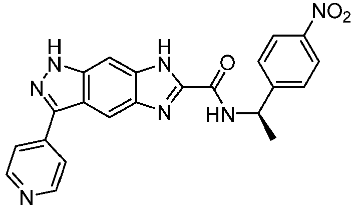

(R)-N-(1-(4-nitrophenyl)ethyl)-3-(pyridin-4-yl)-1,7-dihydroimidazo[4,5-f]indazole -6-carboxamide was obtained from methyl 3-(pyridin-4-yl)-1-trityl-1,7-dihydroimidazo[4,5-f]indazole-6-carboxylate and (R)-1-(4-nitrophenyl)ethan-1-amine as the starting materials in accordance with Steps 6 and 7 of Example 2.
¹H NMR (400 MHz, DMSO-*d*₆) δ13.44-13.39 (m, 1H), 13.20-13.08 (m, 1H), 9.43 - 9.33 (m, 1H), 8.68 - 8.62 (m, 2H), 8.22 - 8.14 (m, 2H), 8.00 - 7.94 (m, 2H), 7.70 (s, 2H), 7.62 - 7.54 (m, 2H), 5.27 - 5.22 (m, 1H), 1.56 (d, *J* = 6.4 Hz, 3H).
MS m/z (ESI): 428.1 [M+H]⁺.

### Example 15

### Preparation of (R)-N-(1-(4-bromophenyl)ethyl)-3-(pyridin-4-yl)-1,7-dihydroimidazo[4,5-f]indazole -6-carboxamide

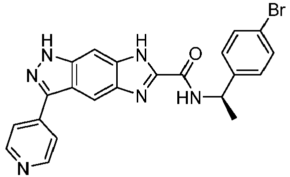

(R)-N-(1-(4-bromophenyl)ethyl)-3-(pyridin-4-yl)-1,7-dihydroimidazo[4,5-f]indazo le-6-carboxamide was obtained from methyl 3-(pyridin-4-yl)-1-trityl-1,7-dihydroimidazo[4,5-f]indazole-6-carboxylate and (R)-1-(4-bromophenyl)ethan-1-amine as the starting materials in accordance with Steps 6 and 7 of Example 2.
¹H NMR (400 MHz, DMSO-*d*₆) δ 13.47 (s, 1H), 9.48 (d*, J* = 7.9 Hz, 1H), 8.71 - 8.64 (m, 2H), 8.13 (s, 1H), 7.99 (s, 1H), 7.96 - 7.89 (m, 2H), 7.50 - 7.42 (m, 2H), 7.26 - 7.19 (m, 2H), 6.75 (d, *J* = 9.3 Hz, 1H), 5.22 (dqt, *J* = 8.8, 6.8, 1.0 Hz, 1H), 1.57 (d, *J* = 6.7 Hz, 3H).
MS m/z (ESI): 461.1, 463.0 [M+H]⁺.

### Example 16

### Preparation of (R)-3-(pyridin-4-yl)-N-(1-(3-(trifluoromethyl)phenyl)ethyl)-1,7-dihydroimidazo[4,5 -f]indazole-6-carboxamide

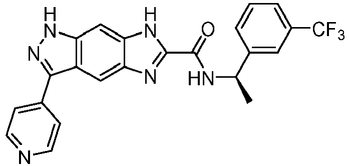

### Step 1: Preparation of (R)-3-(pyridin-4-yl)-N-(1-(4-(trifluoromethyl)phenyl)ethyl)-1-trityl-1,7-dihydroimi dazo[4,5-f]indazole-6-carboxamide

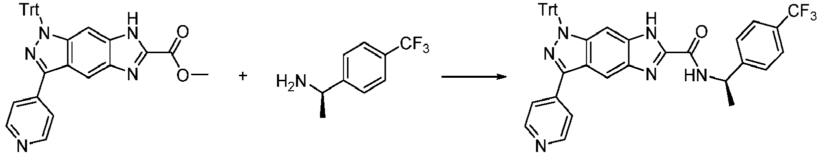

Methyl 3-(pyridin-4-yl)-1-trityl-1,7-dihydroimidazo[4,5-f]indazole-6-carboxylate (100 mg, 0.19 mmol) and (R)-1-(4-(trifluoromethyl)phenyl)ethan-1-amine (1.0 mL) were added to a microwave reaction tube. The reaction solution was heated to 150°C for 60 min. After cooling to room temperature, 20 mL of ethyl acetate was added to the reaction solution. The ethyl acetate layer was washed with saturated ammonium chloride solution and saturated sodium chloride solution, dried over anhydrous sodium sulfate, and purified by column chromatography to obtain a crude product (80 mg, yield: 67%), which was used directly in the next step.
MS m/z (ESI): 693.3 [M+H]⁺.

### Step 2: Preparation of (R)-3-(pyridin-4-yl)-N-(1-(3-(trifluoromethyl)phenyl)ethyl)-1,7-dihydroimidazo[4,5 -f]indazole-6-carboxamide

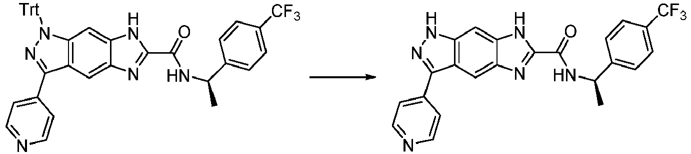

The crude (R)-3-(pyridin-4-yl)-N-(1-(4-(trifluoromethyl)phenyl)ethyl)-1-trityl-1,7-dihydroimidazo [4,5-f]indazole-6-carboxamide (80 mg) was dissolved in 4 mL of dichloromethane, and added with 4 mL of trifluoroacetic acid. The reaction solution was reacted at room temperature for 3 hours, followed by concentrating to dryness. The resulting crude product was dissolved in a mixed solution of ethyl acetate and tetrahydrofuran. The solution was washed with saturated sodium bicarbonate solution and saturated sodium chloride solution, and dried over anhydrous sodium sulfate. The resulting organic solution was concentrated to dryness, and purified by preparative chromatography to obtain a yellow solid product (R)-3-(pyridin-4-yl)-N-(1-(3-(trifluoromethyl)phenyl)ethyl)-1,7-dihydroimidazo[4,5-f]i ndazole-6-carboxamide (10 mg, yield: 19%).
¹H NMR (400 MHz, MeOD) δ 8.59 (d, *J* = 4 Hz, 2H), 8.39 (s, 0.6H), 8.15 (s, 0.4H), 8.06 (d, *J* = 4 Hz, 2H), 7.79 (s, 0.4H), 7.56 (s, 0.6H), 7.49 (d, *J* = 8 Hz, 2H), 7.21 (d, *J* = 8 Hz, 2H), 5.12-5.16 (m, 1H), 1.52 (d, *J* = 8 Hz, 3H).
MS m/z (ESI): 451.1 [M+H]⁺.

### Example 17

### Preparation of (R)-N-(1-phenylpropyl)-3-(pyridin-4-yl)-1,7-dihydroimidazo[4,5-f]indazole-6-carbo xamide

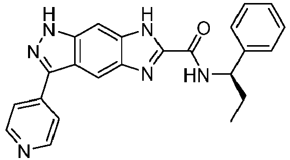

(R)-N-(1-phenylpropyl)-3-(pyridin-4-yl)-1,7-dihydroimidazo[4,5-f]indazole-6-carb oxamide was obtained from methyl 3-(pyridin-4-yl)-1-trityl-1,7-dihydroimidazo[4,5-f]indazole-6-carboxylate and (R)-1-phenylpropan-1-amine as the starting materials in accordance with Steps 6 and 7 of Example 2.
¹H NMR (400 MHz, DMSO-*d*₆) δ 13.56 - 13.03 (m, 2H), 9.25 - 9.14 (m, 1H), 8.72 (d, *J* = 4 Hz, 2H), 8.50 (s, 0.6H), 8.06 - 7.90 (m, 2.7H), 7.57 (s, 0.6H), 7.45 (d, *J* = 8 Hz, 2H), 7.35 (t*, J* = 8 Hz, 2H), 7.28 - 7.23 (m, 1H), 5.14 - 5.09 (m, 1H), 1.75-1.70 (m, 2H), 0.95-0.88 (m, 3H).
MS m/z (ESI): 397.1 [M+H]⁺.

### Example 18

### Preparation of (R)-N-(1-(3-bromophenyl)ethyl)-3-(pyridin-4-yl)-1,7-dihydroimidazo[4,5-f]indazole -6-carboxamide

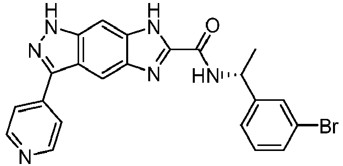

### Step 1: Preparation of (R)-N-(1-(3-bromophenyl)ethyl)-3-(pyridin-4-yl)-1-trityl-1,7-dihydroimidazo[4,5-f]i ndazole-6-carboxamide

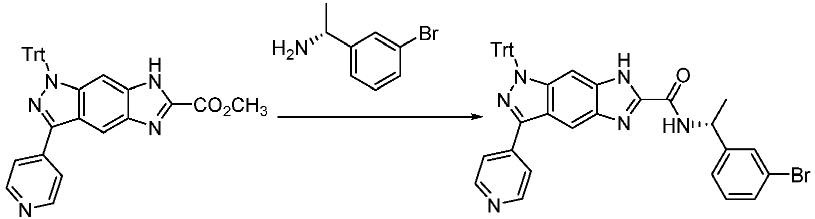

Methyl 3-(pyridin-4-yl)-1-trityl-1,7-dihydroimidazo[4,5-f]indazole-6-carboxylate (100 mg, 0.18 mmol) and (R)-1-(3-bromophenyl)ethan-1-amine (1 mL) were stirred under a microwave condition at 150°C for 1 h. The reaction solution was cooled, concentrated to dryness, and purified by column chromatography to obtain a crude product (R)-N-(1-(3-bromophenyl)ethyl)-3-(pyridin-4-yl)-1-trityl-1,7-dihydroimidazo[4,5-f]inda zole-6-carboxamide (120 mg).
MS m/z (ESI): 703.1 705.2[M+H]⁺.

### Step 2: Preparation of (R)-N-(1-(3-bromophenyl)ethyl)-3-(pyridin-4-yl)-1,7-dihydroimidazo[4,5-f]indazole -6-carboxamide

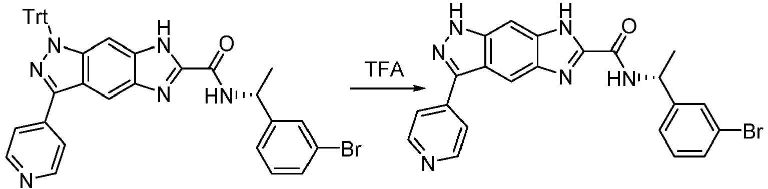

(R)-N-(1-(3-Bromophenyl)ethyl)-3-(pyridin-4-yl)-1-trityl-1,7-dihydroimidazo[4,5-f]indazole-6-carboxamide (120 mg) was dissolved in dichloromethane (2 mL). The solution was added with trifluoroacetic acid (2 mL), and stirred at room temperature for 2 h. The reaction solution was concentrated to dryness, and purified by column chromatography to obtain a yellow solid product (R)-N-(1-(3-bromophenyl)ethyl)-3-(pyridin-4-yl)-1,7-dihydroimidazo[4,5-f]indazole-6-carboxamide (26 mg, yield in two steps: 30%).
¹H NMR (400 MHz, DMSO-*d*₆) δ 13.47 (s, 1H), 9.48 (d, *J* = 7.9 Hz, 1H), 8.71 - 8.64 (m, 2H), 8.13 (s, 1H), 7.99 (s, 1H), 7.96 - 7.89 (m, 2H), 7.56 - 7.47 (m, 2H), 7.31 (dq, *J* = 7.5, 1.4 Hz, 1H), 7.16 (t, *J* = 7.5 Hz, 1H), 6.97 (d, *J* = 9.3 Hz, 1H), 5.20 (dqt, *J* = 8.8, 6.7, 0.9 Hz, 1H), 1.59 (d, *J* = 6.9 Hz, 3H).
MS m/z (ESI): 461.1, 463.0 [M+H]⁺.

### Example 19

### Preparation of (R)-N-(1-(3-methoxyphenyl)ethyl)-3-(pyridin-4-yl)-1,7-dihydroimidazo[4,5-f]indaz ole-6-carboxamide

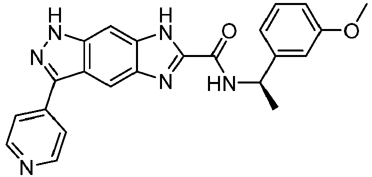

### Step 1: Preparation of (R)-N-(1-(3-methoxyphenyl)ethyl)-3-(pyridin-4-yl)-1-trityl-1,7-dihydroimidazo[4,5-f]indazole-6-carboxamide

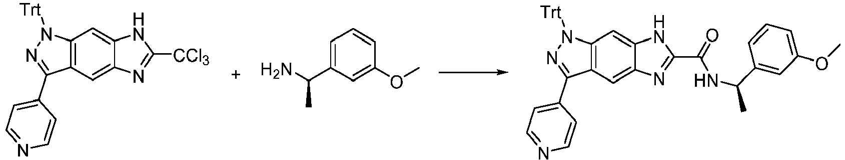

3-(Pyridin-4-yl)-6-(trichloromethyl)-1-trityl-1,7-dihydroimidazo[4,5-f]indazole (100 mg, 0.17 mmol) was dissolved in 12 mL of a mixed solvent of acetonitrile and water (acetonitrile:water = 3:1). The solution was added with sodium bicarbonate (141 mg, 1.70 mmol) and (R)-(+)-1-(3-methoxyphenyl)ethanamine (28 mg, 0.19 mmol), and reacted under a nitrogen atmosphere at 60°C for 2 hours. The reaction solution was concentrated and added with 20 mL of water, the water phase was extracted with ethyl acetate (20 mL*3). The ethyl acetate layer was washed with saturated sodium chloride solution, dried over anhydrous sodium sulfate, and purified by column chromatography (eluent: petroleum ether: ethyl acetate = 1:1 ~ ethyl acetate) to obtain a brown oily crude product (50 mg), which was used directly in the next step.
MS m/z (ESI): 655.1 [M+H]⁺.

### Step 2: Preparation of (R)-N-(1-(3-methoxyphenyl)ethyl)-3-(pyridin-4-yl)-1,7-dihydroimidazo[4,5-f]indaz ole-6-carboxamide

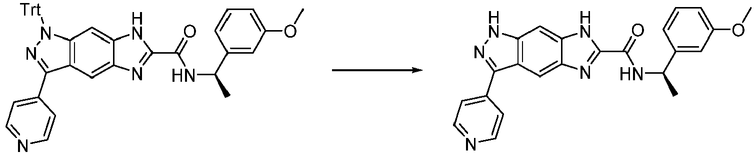

(R)-N-(1-(3-Methoxyphenyl)ethyl)-3-(pyridin-4-yl)-1-trityl-1,7-dihydroimidazo[4, 5-f]indazole-6-carboxamide (50 mg) was dissolved in 4 mL of dichloromethane, and added with 4 mL of trifluoroacetic acid at the same time. The reaction solution was reacted at room temperature for 3 hours, followed by concentrating to dryness. The resulting crude product was dissolved in a mixed solution of ethyl acetate and tetrahydrofuran. The solution was washed with saturated sodium bicarbonate solution and saturated sodium chloride solution, and dried over anhydrous sodium sulfate. The resulting organic solution was concentrated to dryness, and purified by thin layer chromatography (developing solvent: dichloromethane: methanol = 10:1) to obtain a yellow solid product (*R*)-N -(1-(3-methoxyphenyl)ethyl)-3-(pyridin-4-yl)-1,7-dihydroimidazo[4,5-f]indazole-6-carboxamide (7.1 mg, yield: 10%).
¹H NMR (400 MHz, DMSO-*d*₆) δ 13.45-13.39 (m, 1H), 13.21-13.08 (m, 1H), 9.43 - 9.33 (m, 1H), 8.68 - 8.62 (m, 2H), 8.00 - 7.94 (m, 2H), 7.70 (s, 2H), 7.35 - 7.27 (m, 2H), 6.90 - 6.82 (m, 2H), 5.25 - 5.21 (m, 1H), 3.81 (s, 3H), 1.47 (d, *J* = 6.8 Hz, 3H).
MS m/z (ESI): 413.1 [M+H]⁺.

### Example 20

### Preparation of (S)-N-(2-hydroxy-1-phenylethyl)-3-(pyridin-4-yl)-1,7-dihydroimidazo[4,5-f]indazol e-6-carboxamide

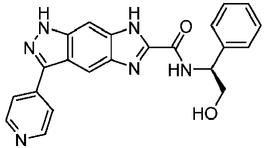

### Step 1: Preparation of (S)-N-(2-hydroxy-1-phenylethyl)-3-(pyridin-4-yl)-1-trityl-1,7-dihydroimidazo[4,5-f] indazole-6-carboxamide

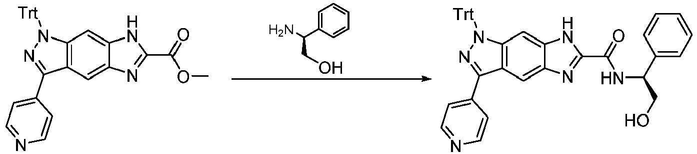

Methyl 3-(pyridin-4-yl)-1-trityl-1,7-dihydroimidazo[4,5-f]indazole-6-carboxylate (100 mg, 0.18 mmol) and (S)-2-amino-2-phenylethan-1-ol(102 mg, 0.75 mmol) were dissolved in dioxane (3 mL). The reaction solution was reacted under a microwave condition at 150°C for 3 hours. The reaction solution was concentrated and purified by column chromatography [eluent: CH₂Cl₂ ∼ CH₂Cl₂/MeOH (97:3)] to obtain a product (S)-N-(2-hydroxy-1-phenylethyl)-3-(pyridin-4-yl)-1-trityl-1,7-dihydroimidazo[4,5-f]ind azole-6-carboxamide (80 mg, yield: 67%).
MS m/z (ESI): 641.2 [M+H]⁺.

### Step 2: Preparation of (S)-N-(2-hydroxy-1-phenylethyl)-3-(pyridin-4-yl)-1,7-dihydroimidazo[4,5-f]indazol e-6-carboxamide

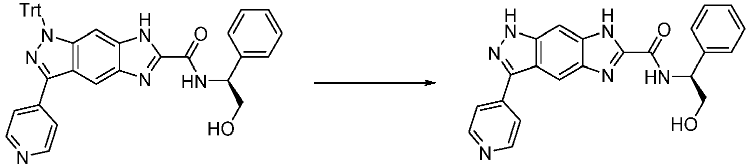

Trifluoroacetic acid (2 mL) was added to a solution of (S)-N-(2-hydroxy-1-phenyl ethyl)-3-(pyridin-4-yl)-1-trityl-1,7-dihydroimidazo[4,5-f]ind azole-6-carboxamide (80 mg, 0.13 mmol) in dichloromethane (2 mL). The reaction solution was stirred at room temperature for 3 hours. The reaction solution was concentrated to dryness, and methanol (5 mL) was added to dissolve the residues. The resulting solution was adjusted to alkaline by aqua ammonia, concentrated, and purified by thin layer chromatography [CH₂Cl₂/MeOH(10:1)∼CH₂Cl₂/MeOH (8:1)] to obtain a product (S)- N-(2-hydroxy-1-phenylethyl)-3-(pyridin-4-yl)-1,7-dihydroimidazo[4,5-f]indazole-6-carboxamide (5.4 mg, yield: 11%).
¹HNMR (400 MHz, DMSO) δ 13.55 - 13.01 (m, 2H), 9.25 - 9.14 (m, 1H), 8.72 (d, *J* = 4 Hz, 2H), 8.50 (s, 0.6H), 8.07 - 7.91 (m, 2.7H), 7.59 (s, 0.6H), 7.45 (d, *J* = 8 Hz, 2H), 7.35 (t, *J* = 8 Hz, 2H), 7.28 - 7.23 (m, 1H), 5.14 - 5.09 (m, 2H), 3.84 - 3.77 (m, 2H).
MS m/z (ESI): 399.1 [M+H]⁺.

### Example 21

### Preparation of (R)-3-(pyridin-4-yl)-N-(1-(m-tolyl)ethyl)-1,7-dihydroimidazo[4,5-f]indazole-6-carb oxamide

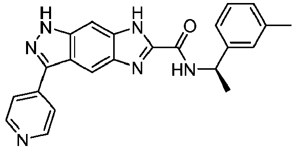

### Step 1: Preparation of (S)-N-(2-hydroxy-1-phenylethyl)-3-(pyridin-4-yl)-1-trityl-1,7-dihydroimidazo[4,5-f] indazole-6-carboxamide

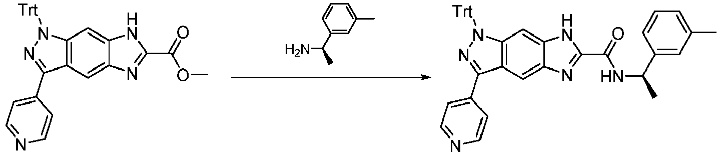

Methyl 3-(pyridin-4-yl)-1-trityl-1,7-dihydroimidazo[4,5-f]indazole-6-carboxylate (100 mg, 0.19 mmol) and (R)-1-(m-tolyl)ethan-1-amine (101 mg, 0.75 mmol) were stirred in N-methylpyrrolidone (3 mL) under a microwave condition at 150°C for 3 hours. The reaction solution was concentrated and purified by column chromatography [eluent: CH₂Cl₂ ∼ CH₂Cl₂/MeOH (97:3)] to obtain a product (R)-3-(pyridin-4-yl)-N-(1-(m-tolyl)ethyl)-1-trityl-1,7-dihydroimidazo[4,5-f]indazole-6-carboxamide (80 mg, yield: 67%).
MS m/z (ESI): 639.4 [M+H]⁺.

### Step 2: Preparation of (R)-3-(pyridin-4-yl)-N-(1-(m-tolyl)ethyl)-1,7-dihydroimidazo[4,5-f]indazole-6-carb oxamide

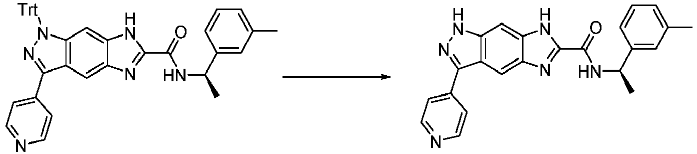

Trifluoroacetic acid (2 mL) was added to a solution of (R)-3-(pyridin-4-yl)-N-(1-(m-tolyl)ethyl)-1-trityl-1,7-dihydroimidazo[4,5-f]indazole-6-carboxamide (80 mg, 0.13 mmol) in dichloromethane (2 mL). The reaction solution was stirred at room temperature for 3 hours. The reaction solution was concentrated to dryness, and methanol (5 mL) was added to dissolve the residues. The resulting solution was adjusted to alkaline by aqua ammonia, concentrated, and purified by thin layer chromatography (CH₂Cl₂/MeOH (8:1)) to obtain a product (R)-3-(pyridin-4-yl)-N-(1-(m-tolyl)ethyl)-1,7-dihydroimidazo[4,5-f]indazole-6-carboxa mide (10 mg, yield: 20%).
¹H NMR (400 MHz, MeOD) δ 8.57 (d, *J* = 4 Hz, 2H), 8.37 (s, 0.6H), 8.15 (s, 0.4H), 8.02 (d, *J* = 4 Hz, 2H), 7.79 (s, 0.4H), 7.56 (s, 0.6H), 7.42-7.30 (m, 3H), 7.12-08 (m, 1H), 5.12-5.16 (m, 1H), 2.22 (s, 3H), 1.52 (d, *J* = 8 Hz, 3H).
MS m/z (ESI): 397.2 [M+H]⁺.

### Example 22

### Preparation of (R)-N-(1-(3-chlorophenyl)ethyl)-3-(pyridin-4-yl)-1,7-dihydroimidazo[4,5-f]indazole -6-carboxamide

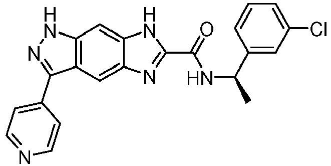

### Step 1: Preparation of (R)-N-(1-(3-chlorophenyl)ethyl)-3-(pyridin-4-yl)-1-trityl-1,7-dihydroimidazo[4,5-f]i ndazole-6-carboxamide

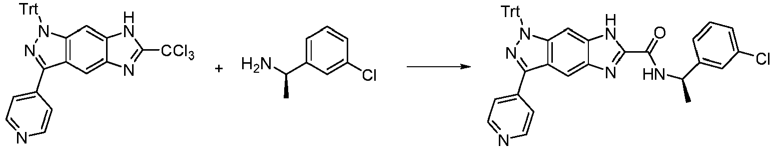

3-(Pyridin-4-yl)-6-(trichloromethyl)-1-trityl-1,7-dihydroimidazo[4,5-f]indazole (2.0 g, 3.36 mmol) was dissolved in 40 mL of a mixed solvent of tetrahydrofuran and water (tetrahydrofuran:water = 3:1). The solution was added with sodium bicarbonate (2.82 g, 33.6 mmol) and (R)-1-(3-chlorophenyl)ethanamine (476 mg, 3.70 mmol), and reacted under a nitrogen atmosphere at room temperature for 4 hours. The reaction solution was concentrated and added with 40 mL of water, the water phase was extracted with ethyl acetate (40 mL*3). The ethyl acetate layer was washed with saturated sodium chloride solution, dried over anhydrous sodium sulfate, and purified by column chromatography (eluent: petroleum ether: ethyl acetate = 1:1 ∼ ethyl acetate) to obtain a brown solid crude product (540 mg), which was used directly in the next step.
MS m/z (ESI): 659.1 [M+H]⁺.

### Step 2: Preparation of (R)-N-(1-(3-chlorophenyl)ethyl)-3-(pyridin-4-yl)-1,7-dihydroimidazo[4,5-f]indazole -6-carboxamide

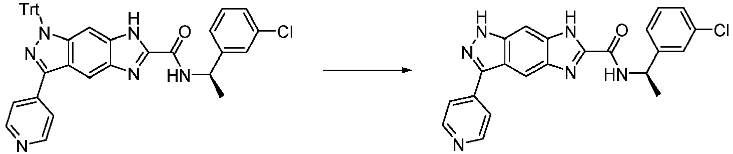

(R)-N-(1-(3-Chlorophenyl)ethyl)-3-(pyridin-4-yl)-1-trityl-1,7-dihydroimidazo[4,5-f]indazole-6-carboxamide (540 mg) was dissolved in 5 mL of dichloromethane, and added with 5 mL of trifluoroacetic acid at the same time. The reaction solution was reacted at room temperature for 3 hours, followed by concentrating to dryness. The resulting crude product was dissolved in a mixed solution of ethyl acetate and tetrahydrofuran. The solution was washed with saturated sodium bicarbonate solution and saturated sodium chloride solution, and dried over anhydrous sodium sulfate. The resulting organic solution was concentrated to dryness, and purified by column chromatography (eluent: dichloromethane: methanol = 10:1) to obtain a light yellow solid product (R)-N-(1-(3-chlorophenyl)ethyl)-3-(pyridin-4-yl)-1,7-dihydroimidazo[4,5-f]indazole-6-carboxamide (300 mg, yield in two steps: 21%).
¹H NMR (400 MHz, DMSO) δ 13.48 (s, 0.4 H), 13.41 (s, 0.6 H), 13.25 (s, 0.4 H), 13.11 (s, 0.6 H), 9.60 (d, *J* = 8.5 Hz, 0.4 H), 9.53 (d, *J* = 8.5 Hz, 0.6 H), 8.78 - 8.67 (m, 2H), 8.46 (s, 0.6 H), 8.13 (s, 0.4 H), 8.06 (d, *J* = 5.9 Hz, 1.2 H), 7.99 (d, *J* = 5.9 Hz, 0.8 H), 7.90 (s, 0.4 H), 7.59 (s, 1.6 H), 7.46 - 7.31 (m, 3H), 5.24 (p, *J* = 7.0 Hz, 1H), 1.57 (d, *J* = 7.0 Hz, 3H).
MS m/z (ESI): 417.1 [M+H]⁺.

### Example 23

### Preparation of N-((1S,2R)-2-hydroxy-2,3-dihydro-1H-inden-1-yl)-3-(pyridin-4-yl)-1,7-dihydroimid azo[4,5-f]indazole-6-carboxamide

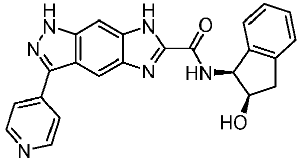

N-((1S,2R)-2-Hydroxy-2,3-dihydro-1H-inden-1-yl)-3-(pyridin-4-yl)-1,7-dihydroim idazo[4,5-f]indazole-6-carboxamide was obtained from methyl 3-(pyridin-4-yl)-1-trityl-1,7-dihydroimidazo[4,5-f]indazole-6-carboxylate and (1S,2R)-1-amino-2,3-dihydro-1H-inden-2-ol as the starting materials in accordance with Steps 6 and 7 of Example 2.
¹HNMR (400 MHz, DMSO-*d*₆) δ 13.42-13.20 (m, 2H), 9.30-9.24 (m, 1H), 8.72 (s, 2H), 8.43 (s, 0.7H), 8.15 (s, 0.3H), 8.04 (s, 2H), 7.85 (s, 0.3H), 7.61 (s, 0.7H), 7.23-7.14 (m, 4H), 5.43 (d, *J* = 4 Hz, 1H), 5.35-5.30 (m, 1H), 4.69 - 4.57 (m, 1H), 3.24-3.18 (m, 1H), 2.79-2.75 (m, 1H).
MS m/z (ESI): 411.1 [M+H]⁺.

### Example 24

### Preparation of N-((1S,2S)-2-hydroxy-2,3-dihydro-1H-inden-1-yl)-3-(pyridin-4-yl)-1,7-dihydroimid azo [4,5-f]indazole-6-carboxamide

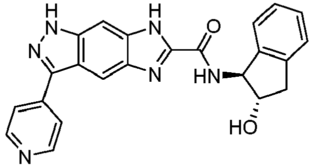

N-((1S,2S)-2-Hydroxy-2,3-dihydro-1H-inden-1-yl)-3-(pyridin-4-yl)-1,7-dihydroim idazo[4,5-f]indazole-6-carboxamide was obtained from methyl 3-(pyridin-4-yl)-1-trityl-1,7-dihydroimidazo[4,5-f]indazole-6-carboxylate and (1S,2S)-1-amino-2,3-dihydro-1H-inden-2-ol as the starting materials in accordance with Steps 6 and 7 of Example 2.
¹HNMR (400 MHz, DMSO-*d*₆) δ 13.41-13.20 (m, 2H), 9.29-9.23 (m, 1H), 8.72 (s, 2H), 8.43 (s, 0.7H), 8.16 (s, 0.3H), 8.04 (s, 2H), 7.86 (s, 0.3H), 7.61 (s, 0.7H), 7.23-7.14 (m, 4H), 5.42 (d, *J* = 4 Hz, 1H), 5.35-5.31 (m, 1H), 4.68 - 4.57 (m, 1H), 3.24-3.19 (m, 1H), 2.80-2.76 (m, 1H).
MS m/z (ESI): 411.1 [M+H]⁺.

### Example 25

### Preparation of (R)-3-(pyridin-4-yl)-N-(1-(thiazol-2-yl)ethyl)-1,7-dihydroimidazo[4,5-f]indazole-6-c arboxamide

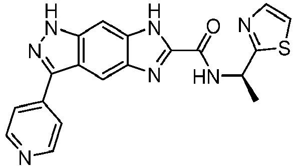

(R)-3-(Pyridin-4-yl)-N-(1-(thiazol-2-yl)ethyl)-1,7-dihydroimidazo[4,5-f]indazole-6 -carboxamide was obtained from methyl 3-(pyridin-4-yl)-1-trityl-1,7-dihydroimidazo[4,5-f]indazole-6-carboxylate and (R)-1-(thiazol-2-yl)ethan-1-amine as the starting materials in accordance with Steps 6 and 7 of Example 2.
¹H NMR (400 MHz, CDCl₃) δ 8.75 (s, 2H), 8.12 (s, 1H), 7.89 (s, 2H), 7.63 (d, *J* = 15.8 Hz, 2H), 7.36 (s, 1H), 7.20 (s, 1H), 5.36 (s, 1H), 1.47 (s, 3H).
MS m/z (ESI): 390.1 [M+H]⁺.

### Example 26

### Preparation of (R)-N-(3,3-dimethylbutan-2-yl)-3-(pyridin-4-yl)-1,7-dihydroimidazo[4,5-f]indazole-6-carboxamide

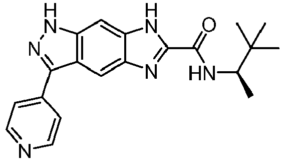

(R)-N-(3,3-Dimethylbutan-2-yl)-3-(pyridin-4-yl)-1,7-dihydroimidazo[4,5-f]indazol e-6-carboxamide was obtained from methyl 3-(pyridin-4-yl)-1-trityl-1,7-dihydroimidazo[4,5-f]indazole-6-carboxylate and (R)-3,3-dimethylbutan-2-amine as the starting materials in accordance with Steps 6 and 7 of Example 2.
¹H NMR (400 MHz, CDCl₃) δ 8.75 (s, 2H), 8.08 (s, 1H), 7.89 (s, 2H), 7.61 (s, 1H), 6.46 (s, 1H), 3.68 (s, 1H), 1.26 (s, 3H), 0.96 (s, 9H).
MS m/z (ESI): 363.2 [M+H]⁺.

### Example 27

### Preparation of (R)-N-(1-cyclohexylethyl)-3-(pyridin-4-yl)-1,7-dihydroimidazo[4,5-f]indazole-6-car boxamide

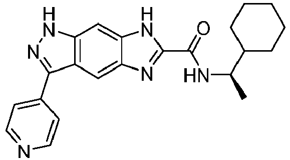

(R)-N-(1-cyclohexylethyl)-3-(pyridin-4-yl)-1,7-dihydroimidazo[4,5-f]indazole-6-c arboxamide was obtained from methyl 3-(pyridin-4-yl)-1-trityl-1,7-dihydroimidazo[4,5-f]indazole-6-carboxylate and (R)-1-cyclohexylethan-1-amine as the starting materials in accordance with Steps 6 and 7 of Example 2.
¹H NMR (400 MHz, DMSO-*d*₆) δ 13.21 (dd, *J* = 11, 4 Hz, 2H), 8.69 - 8.37 (m, 3H), 8.01 - 7.51 (m, 3H), 3.81 (d*, J* = 8.6 Hz, 1H), 1.67 (d, *J* = 2.4 Hz, 2H), 1.57 - 1.39 (m, 2H), 1.24 - 1.06 (m, 9H), 0.89 (d, *J* = 12.3 Hz, 1H), 0.78 (s, 1H).
MS m/z (ESI): 389.1 [M+H]⁺.

### Example 28

### Preparation of (S)-N-(1-(3-chlorophenyl)-2-hydroxyethyl)-3-(pyridin-4-yl)-1,7-dihydroimidazo[4,5 -f]indazole-6-carboxamide

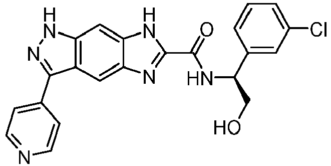

### Step 1: Preparation of (S)-N-(1-(3-chlorophenyl)-2-hydroxyethyl)-3-(pyridin-4-yl)-1-trityl-1,7-dihydroimi dazo[4,5-f]indazole-6-carboxamide

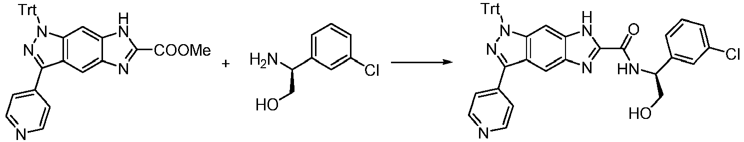

Methyl 3-(pyridin-4-yl)-1-trityl-1,7-dihydroimidazo[4,5-f]indazole-6-carboxylate (100 mg, 0.19 mmol) and (S)-2-amino-2-(3-chlorophenyl)ethanol (2.0 mL) were added to a microwave reaction tube. The reaction solution was heated to 150°C for 60 min. After cooling to room temperature, 20 mL of ethyl acetate was added to the reaction solution. The ethyl acetate layer was washed with saturated ammonium chloride solution and saturated sodium chloride solution, dried over anhydrous sodium sulfate, and purified by column chromatography (eluent: dichloromethane ~ dichloromethane: methanol = 20:1) to obtain a crude product (90 mg), which was used directly in the next step.
MS m/z (ESI): 675.1 [M+H]⁺.

### Step 2: Preparation of (S)-N-(1-(3-chlorophenyl)-2-hydroxyethyl)-3-(pyridin-4-yl)-1,7-dihydroimidazo[4,5 -f]indazole-6-carboxamide

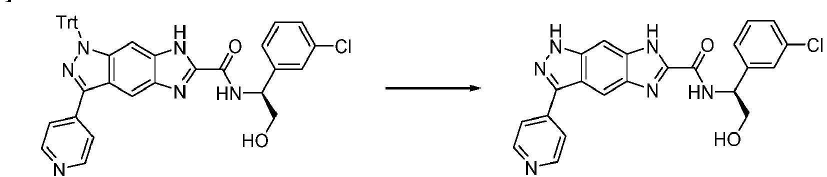

(S)-N-(1-(3-chlorophenyl)-2-hydroxyethyl)-3-(pyridin-4-yl)-1-trityl-1,7-dihydroim idazo[4,5-f]indazole-6-carboxamide was dissolved in 4 mL of dichloromethane, and added with 4 mL of trifluoroacetic acid at the same time. The reaction solution was reacted at room temperature for 3 hours, followed by concentrating to dryness. The resulting crude product was dissolved in a mixed solution of ethyl acetate and tetrahydrofuran. The solution was washed with saturated sodium bicarbonate solution and saturated sodium chloride solution, and dried over anhydrous sodium sulfate. The resulting organic solution was concentrated to dryness, and purified by thin layer chromatography (developing solvent: dichloromethane: methanol = 10:1) to obtain a yellow solid product (S)-N-(1-(3-chlorophenyl)-2-hydroxyethyl)-3-(pyridin-4-yl)-1,7-dihydroimidazo[4,5-f]i ndazole-6-carboxamide (22 mg, yield in two steps: 26%).
¹H NMR (400 MHz, DMSO-*d*₆) δ 13.49 (s, 0.4 H), 13.41 (s, 0.6 H), 13.27 (s, 0.4 H), 13.14 (s, 0.6 H), 9.36 (d, *J* = 8.5 Hz, 0.4 H), 9.27 (d, *J* = 8.5 Hz, 0.6 H), 8.74 - 8.71 (m, 2H), 8.50 (s, 0.6 H), 8.14 (s, 0.4 H), 8.07 (d, *J* = 6.1 Hz, 1.2 H), 7.99 (d, *J* = 6.1 Hz, 0.8 H), 7.91 (s, 0.4 H), 7.60 (s, 0.6 H), 7.56 (s, 1H), 7.47 - 7.28 (m, 3H), 5.19 - 5.07 (m, 2H), 3.89-3.71 (m, 2H).
MS m/z (ESI): 433.1 [M+H]⁺.

### Example 29

### Preparation of (R)-N-(1-(3,5-dimethoxyphenyl)ethyl)-3-(pyridin-4-yl)-1,7-dihydroimidazo[4,5-f]in dazole-6-carboxamide

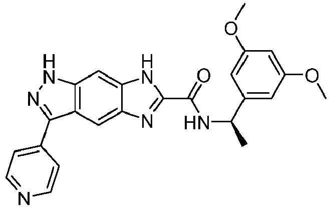

(R)-N-(1-(3,5-dimethoxyphenyl)ethyl)-3-(pyridin-4-yl)-1,7-dihydroimidazo[4,5-f]i ndazole-6-carboxamide was obtained from methyl 3-(pyridin-4-yl)-1-trityl-1,7-dihydroimidazo[4,5-f]indazole-6-carboxylate and (R)-1-(3,5-dimethoxyphenyl)ethan-1-amine as the starting materials in accordance with Steps 6 and 7 of Example 2.
¹H NMR (400 MHz, CDCl₃) δ 8.75 (s, 2H), 8.27 (s, 1H), 7.90 (d, *J* = 8.3 Hz, 3H), 7.65 (s, 1H), 6.65 (s, 2H), 6.36 (s, 1H), 4.97 (s, 1H), 3.79 (s, 6H), 1.48 (s, 3H).
MS m/z (ESI): 443.1 [M+H]⁺.

### Example 30

### Preparation of (S)-N-(2-hydroxy-1-(m-tolyl)ethyl)-3-(pyridin-4-yl)-1,7-dihydroimidazo[4,5-f]indaz ole-6-carboxamide

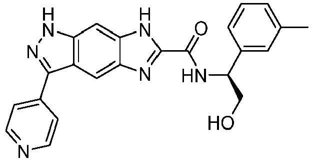

### Step 1: Preparation of (S)-N-(2-hydroxy-1-(m-tolyl)ethyl)-3-(pyridin-4-yl)-1-trityl-1,7-dihydroimidazo[4,5 -f]indazole-6-carboxamide

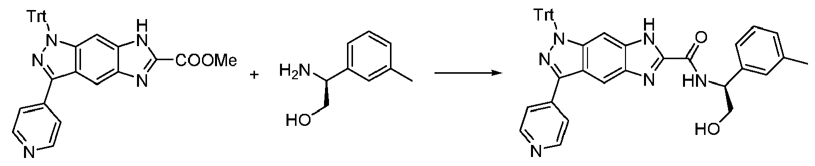

Methyl 3-(pyridin-4-yl)-1-trityl-1,7-dihydroimidazo[4,5-f]indazole-6-carboxylate (100 mg, 0.19 mmol) and (S)-2-amino-2-(m-tolyl)ethanol (2.0 mL) were added to a microwave reaction tube. The reaction solution was heated to 150°C for 60 min. After cooling to room temperature, 20 mL of ethyl acetate was added to the reaction solution. The ethyl acetate layer was washed with saturated ammonium chloride solution and saturated sodium chloride solution, dried over anhydrous sodium sulfate, and purified by column chromatography (eluent: dichloromethane ∼ dichloromethane: methanol = 20:1) to obtain a crude product (75 mg), which was used directly in the next step.
MS m/z (ESI): 655.2 [M+H]⁺.

### Step 2: Preparation of (S)-N-(2-hydroxy-1-(m-tolyl)ethyl)-3-(pyridin-4-yl)-1,7-dihydroimidazo[4,5-f]indaz ole-6-carboxamide

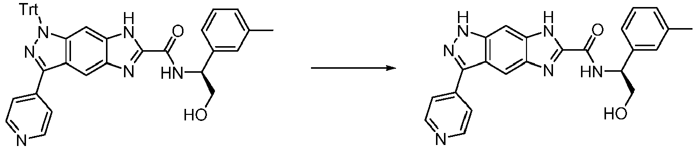

(S)-N-(2-Hydroxy-1-(m-tolyl)ethyl)-3-(pyridin-4-yl)-1-trityl-1,7-dihydroimidazo[4 ,5-f]indazole-6-carboxamide (75 mg) was dissolved in 4 mL of dichloromethane, and added with 4 mL of trifluoroacetic acid at the same time. The reaction solution was reacted at room temperature for 3 hours, followed by concentrating to dryness. The resulting crude product was dissolved in a mixed solution of ethyl acetate and tetrahydrofuran. The solution was washed with saturated sodium bicarbonate solution and saturated sodium chloride solution, and dried over anhydrous sodium sulfate. The resulting organic solution was concentrated to dryness, and purified by thin layer chromatography (developing solvent: dichloromethane: methanol = 10:1) to obtain a yellow solid product (S)-N-(1-(3-chlorophenyl)-2-hydroxyethyl)-3-(pyridin-4-yl)-1,7-dihydroimidazo[4,5-f]i ndazole-6-carboxamide (12 mg, yield in two steps: 15%).
¹H NMR (400 MHz, DMSO) δ 13.49 (s, 0.4 H), 13.42 (s, 0.6 H), 13.26 (s, 0.4 H), 13.13 (s, 0.6 H), 9.26 - 9.08 (m, 1H), 8.73 (d, *J* = 5.5 Hz, 2H), 8.57 - 7.54 (m, 4H), 7.27 - 7.21 (m, 3H), 7.08 (d, *J* = 5.7 Hz, 1H), 5.11 - 5.06 (m, 2H), 3.88 - 3.79 (m, 1H), 3.78 -3.69 (m, 1H), 2.31 (s, 3H).
MS m/z (ESI): 413.1 [M+H]⁺.

### Example 31

### Preparation of (R)-N-(1-(3-cyanophenyl)ethyl)-3-(pyridin-4-yl)-1,7-dihydroimidazo[4,5-f]indazole-6-carboxamide

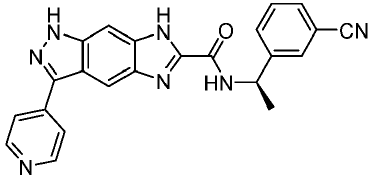

(R)-N-(1-(3-Cyanophenyl)ethyl)-3-(pyridin-4-yl)-1,7-dihydroimidazo[4,5-f]indazo le-6-carboxamide was obtained from methyl 3-(pyridin-4-yl)-1-trityl-1,7-dihydroimidazo[4,5-f]indazole-6-carboxylate and (R)-3-(1-aminoethyl)benzonitrile as the starting materials in accordance with Steps 6 and 7 of Example 2.
¹H NMR (400 MHz, DMSO-*d*₆) δ 13.47 (s, 1H), 9.48 (d, *J* = 7.9 Hz, 1H), 8.71 - 8.64 (m, 2H), 8.13 (s, 1H), 7.99 (s, 1H), 7.96 - 7.89 (m, 2H), 7.78 (q, *J* = 1.4 Hz, 1H), 7.58 (ddq, *J* = 6.8, 3.9, 1.5 Hz, 2H), 7.43 (t, *J* = 7.5 Hz, 1H), 6.97 (d, *J* = 9.3 Hz, 1H), 5.20 (dddd, *J* = 9.3, 7.9, 6.8, 5.8 Hz, 1H), 1.59 (d, *J* = 6.8 Hz, 3H).
MS m/z (ESI): 408.2 [M+H]⁺.

### Example 32

### Preparation of N-(2-hydroxy-1-(3-methoxyphenyl)ethyl)-3-(pyridin-4-yl)-1,7-dihydroimidazo[4,5-f ]indazole-6-carboxamide

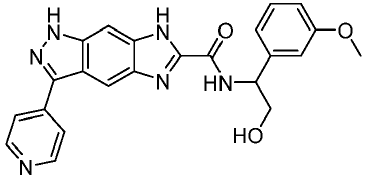

N-(2-Hydroxy-1-(3-methoxyphenyl)ethyl)-3-(pyridin-4-yl)-1,7-dihydroimidazo[4, 5-f]indazole-6-carboxamide was obtained from methyl 3-(pyridin-4-yl)-1-trityl-1,7-dihydroimidazo[4,5-f]indazole-6-carboxylate and 2-amino-2-(3-methoxyphenyl)ethan-1-ol as the starting materials in accordance with Steps 6 and 7 of Example 2.
MS m/z (ESI): 429.1 [M+H]⁺.

### Example 33

### Preparation of (S)-N-(2-amino-2-oxo-1-phenylethyl)-3-(pyridin-4-yl)-1,7-dihydroimidazo[4,5-f]ind azole-6-carboxamide

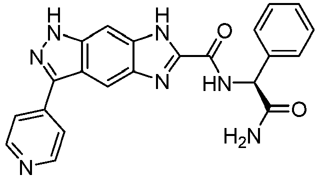

(S)-N-(2-amino-2-oxo-1-phenylethyl)-3-(pyridin-4-yl)-1,7-dihydroimidazo[4,5-f]i ndazole-6-carboxamide was obtained from methyl 3-(pyridin-4-yl)-1-trityl-1,7-dihydroimidazo[4,5-f]indazole-6-carboxylate and (S)-2-amino-2-phenylacetamide as the starting materials in accordance with Steps 6 and 7 of Example 2.
¹H NMR (400 MHz, DMSO-*d*₆) δ 13.48 (s, 1H), 12.72 (s, 1H), 8.71 - 8.64 (m, 2H), 8.13 (s, 1H), 7.99 (s, 1H), 7.96 - 7.89 (m, 2H), 7.67 (d, *J* = 10.8 Hz, 1H), 7.54 - 7.45 (m, 2H), 7.42 - 7.32 (m, 3H), 6.97 (s, 2H), 5.67 (dt, *J* = 11.0, 1.0 Hz, 1H).
MS m/z (ESI): 412.1 [M+H]⁺.

### Example 34

### Preparation of (S)-N-(cyano(phenyl)methyl)-3-(pyridin-4-yl)-1,7-dihydroimidazo[4,5-f]indazole-6-carboxamide

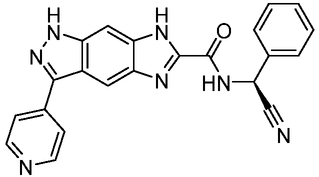

(S)-N-(Cyano(phenyl)methyl)-3-(pyridin-4-yl)-1,7-dihydroimidazo[4,5-f]indazole-6-carboxamide was obtained from methyl 3-(pyridin-4-yl)-1-trityl-1,7-dihydroimidazo[4,5-f]indazole-6-carboxylate and (S)-2-amino-2-phenylacetonitrile as the starting materials in accordance with Steps 6 and 7 of Example 2.
¹H NMR (400 MHz, DMSO-*d*₆) δ 13.47 - 13.11 (m, 2H), 8.85 - 8.71 (m, 3H), 8.45 (s, 0.7H), 8.15 (s, 0.3H), 8.08 - 7.98 (m, 2H), 7.85 (s, 0.3H), 7.51 (s, 0.7H), 7.42 - 7.33 (m, 5H), 5.88 (m, 1H).
MS m/z (ESI): 394.1 [M+H]⁺.

### Example 35

### Preparation of (R)-1-phenylethyl 3-(pyridin-4-yl)-1,7-dihydroimidazo[4,5-f]indazole-6-carboxylate

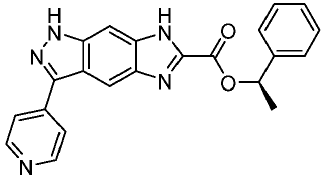

(R)-1-Phenylethyl 3-(pyridin-4-yl)-1,7-dihydroimidazo[4,5-f]indazole -6-carboxylate was obtained from methyl 3-(pyridin-4-yl)-1-trityl-1,7-dihydroimidazo[4,5-f]indazole-6-carboxylate and (R)-1-phenylethan-1-ol as the starting materials in accordance with Steps 6 and 7 of Example 2.
¹H NMR (400 MHz, CDCl₃) δ 8.75 (bs, 2H), 8.30 (s, 1H), 7.89 (d, *J=* 2.9 Hz, 3H), 7.32 (m, 3H), 7.25 (m, 2H), 6.05 (m, 1H), 1.78 (s, 3H).
MS m/z (ESI): 384.1 [M+H]⁺.

### Example 36

### Preparation of (R)-3-(2-methylpyridin-4-yl)-N-(1-phenylethyl)-1,7-dihydroimidazo[4,5-f]indazole-6-carboxamide

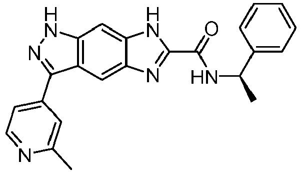

### Step 1: Preparation of 3-(2-methylpyridin-4-yl)-5,6-dinitro-1-trityl-1H-indazole

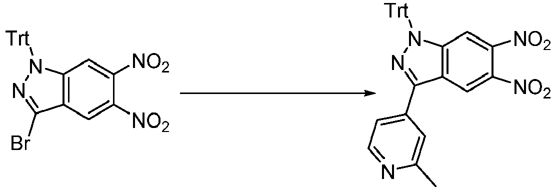

3-Bromo-5,6-dinitro-1-trityl-1H-indazole (1.0 g, 1.89 mmol), (2-methylpyridin-4-yl)boronic acid (517 mg, 3.77 mmol), Pd(dppf)Cl₂(138 mg, 0.19 mmol) and potassium carbonate (522 mg, 3.77 mmol) were stirred in dioxane/water (30 mL/6 mL) under a nitrogen atmosphere at 80°C for 5 hours. The reaction solution was cooled, added with water, extracted with ethyl acetate, concentrated to dryness, and purified by column chromatography to obtain a yellow solid 3-(2-methylpyridin-4-yl)-5,6-dinitro-1-trityl-1H-indazole (886 mg, yield: 86.6%).
MS m/z (ESI): 542.1 [M+H]⁺.

### Step 2: Preparation of 3-(2-methylpyridin-4-yl)-1-trityl-1H -indazole-5,6-diamine

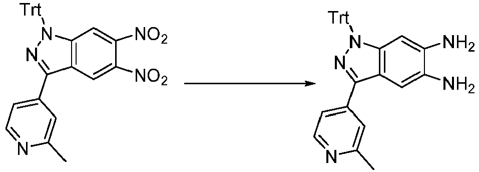

3-(2-Methylpyridin-4-yl)-5,6-dinitro-1-trityl-1H-indazole (886 mg, 1.63 mmol) was dissolved in THF (30 mL), added with 10% Pd/C, and reacted under a hydrogen atmosphere overnight. The reaction solution was filtered, and concentrated to dryness to obtain a grey solid 3-(2-methylpyridin-4-yl)-1-trityl-1H-indazole-5,6-diamine (768 mg, yield: 97%).
MS m/z (ESI): 482.1 [M+H]⁺.

### Step 3: Preparation of 3-(2-methylpyridin-4-yl)-6-(trichloromethyl)-1-trityl-1,7-dihydroimidazo[4,5-f]inda zole

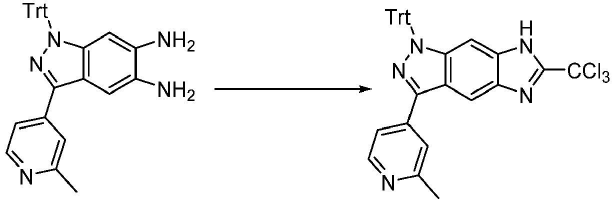

3-(2-Methylpyridin-4-yl)-1-trityl-1H-indazole-5,6-diamine (768 mg, 1.63 mmol), methyl 2,2,2-trichloroacetimidate (318 mg, 1.80 mmol) and acetic acid (10 mL) were stirred at room temperature overnight. The reaction solution was added with 35 ml of water and stirred. The reaction solution was added with water and extracted with dichloromethane. The solution was dried, concentrated to dryness, and purified by column chromatography to obtain 3-(2-methylpyridin-4-yl)-6-(trichloromethyl)-1-trityl-1,7-dihydroimidazo[4,5-f]indazole (560 mg, yield: 57%).
MS m/z (ESI): 610.1 [M+H]⁺.

### Step 4: Preparation of methyl 3-(2-methylpyridin-4-yl)-1-trityl-1,7-dihydroimidazo[4,5-f]indazole-6-carboxylate

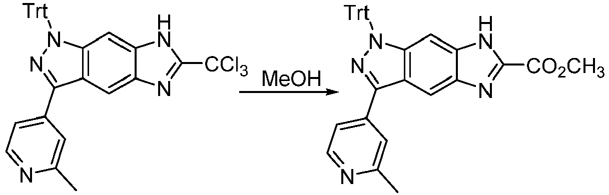

3-(2-Methylpyridin-4-yl)-6-(trichloromethyl)-1-trityl-1,7-dihydroimidazo[4,5-f]ind azole (300 mg, 0.49 mmol) was refluxed in methanol (10 mL) overnight, and concentrated to dryness to obtain a crude product methyl 3-(2-methylpyridin-4-yl)-1-trityl-1,7-dihydroimidazo[4,5-f]indazole-6-carboxylate(304 mg).
MS m/z (ESI): 550.2[M+H]⁺.

### Step 5: Preparation of (R)-3-(2-methylpyridin-4-yl)-N-(1-phenylethyl)-1-trityl-1,7-dihydroimidazo[4,5-f]in dazole-6-carboxamide

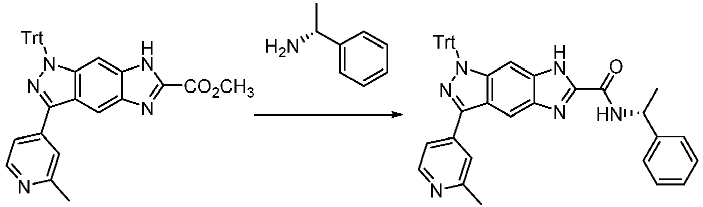

Methyl 3-(2-methylpyridin-4-yl)-1-trityl-1,7-dihydroimidazo[4,5-f]indazole-6-carboxylate (100 mg, 0.18 mmol) and (R)-1-phenylethan-1-amine (1.5 mL) were stirred under a microwave condition at 150°C for 1 h. The reaction solution was cooled, concentrated to dryness, and purified by column chromatography to obtain a thick product (80 mg), which was used directly in the next step.
MS m/z (ESI): 640.1 [M+H]⁺.

### Step 6: Preparation of (R)-3-(2-methylpyridin-4-yl)-N-(1-phenylethyl)-1,7-dihydroimidazo[4,5-f]indazole-6-carboxamide

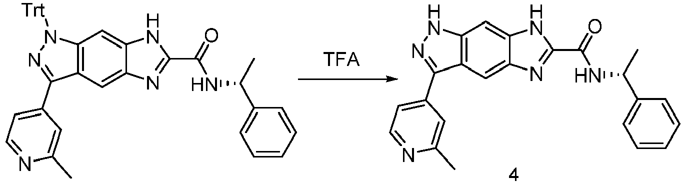

(R)-3 -(2-Methylpyridin-4-yl)-N-(1-phenylethyl)-1-trityl-1,7-dihydroimidazo[4,5-f] indazole-6-carboxamide (80 mg) was dissolved in dichloromethane (2 mL). The solution was added with trifluoroacetic acid (2 mL), and stirred at room temperature for 2 h. The reaction solution was concentrated to dryness, and purified by column chromatography to obtain a yellow solid (R)-3-(2-methylpyridin-4-yl)-N-(1-phenylethyl)-1,7-dihydroimidazo[4,5-f]indazole-6-c arboxamide (37 mg, yield in two steps: 52%).
¹H NMR (400 MHz, DMSO-*d*₆) δ 13.47 (s, 1H), 9.48 (d, *J* = 7.9 Hz, 1H), 8.62 (d, *J* = 7.5 Hz, 1H), 8.11 (s, 1H), 8.01 (s, 1H), 7.86 (dd, *J* = 7.5, 1.5 Hz, 1H), 7.61 (t, *J* = 1.1 Hz, 1H), 7.36 - 7.27 (m, 2H), 7.31 - 7.20 (m, 3H), 6.75 (d, *J* = 9.3 Hz, 1H), 5.20 (dqt, *J* = 8.7, 6.8, 0.9 Hz, 1H), 2.74 (s, 3H), 1.57 (d, *J* = 6.7 Hz, 3H).
MS m/z (ESI): 397.1 [M+H]⁺.

### Example 37

### Preparation of (R)-3-(2-fluoropyridin-4-yl)-N-(l-phenylethyl)-1,7-dihydroimidazo[4,5-flindazole-6 -carboxamide

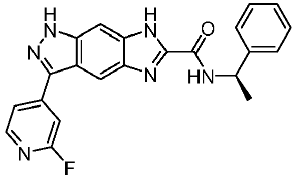

(R)-3-(2-Fluoropyridin-4-yl)-N-(1-phenylethyl)-1,7-dihydroimidazo[4,5-f]indazole -6-carboxamide was obtained from methyl 3-(2-fluoropyridin-4-yl)-1-trityl-1,7-dihydroimidazo[4,5-f]indazole-6-carboxylate and (R)-1-phenylethan-1-amine as the starting materials in accordance with Steps 6 and 7 of Example 2.
¹H NMR (400 MHz, DMSO-*d*₆) δ 13.47 (s, 1H), 9.48 (d, *J* = 7.9 Hz, 1H), 8.66 (d, *J=* 7.5 Hz, 1H), 8.13 (s, 1H), 8.03 - 7.92 (m, 3H), 7.36 - 7.20 (m, 5H), 6.75 (d*, J* = 9.3 Hz, 1H), 5.20 (dqt, *J* = 8.7, 6.7, 0.9 Hz, 1H), 1.57 (d, *J* = 6.7 Hz, 3H).
MS m/z (ESI): 401.1 [M+H]⁺.

### Example 38

### Preparation of (R)-3-(2,6-dimethylpyridin-4-yl)-N-(1-phenylethyl)-1,7-dihydroimidazo[4,5-f]indaz ole-6-carboxamide

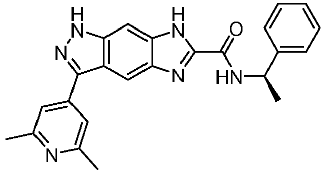

(R)-3-(2,6-dimethylpyridin-4-yl)-N-(1-phenylethyl)-1,7-dihydroimidazo[4,5-f]inda zole-6-carboxamide was obtained from methyl 3-(2,6-dimethylpyridin-4-yl)-1-trityl-1,7-dihydroimidazo[4,5-f]indazole-6-carboxylate and (R)-1-phenylethan-1-amine as the starting materials in accordance with Steps 6 and 7 of Example 2.
¹H NMR (400 MHz, DMSO-*d*₆) δ 13.47 (s, 1H), 9.48 (d*, J* = 7.9 Hz, 1H), 8.09 (s, 1H), 8.02 (s, 1H), 7.46 (s, 2H), 7.31 (ddd, *J* = 7.1, 2.1, 0.9 Hz, 2H), 7.31 - 7.20 (m, 3H), 6.75 (d, *J* = 9.3 Hz, 1H), 5.20 (dqt, *J* = 8.7, 6.8, 0.9 Hz, 1H), 2.61 (s, 6H), 1.57 (d, *J* = 6.7 Hz, 3H).
MS m/z (ESI): 411.1 [M+H]⁺.

### Example 39

### Preparation of (R)-3-(1-methyl-6-oxo-1,6-dihydropyridin-3-yl)-N-(1-phenylethyl)-1,7-dihydroimid azo[4,5-f]indazole-6-carboxamide

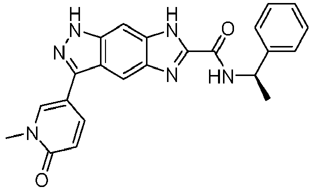

### Step 1: Preparation of (R)-3-(1-methyl-6-oxo-1,6-dihydropyridin-3-yl)-N-(1-phenylethyl)-1-trityl-1,7-dihy droimidazo[4,5-f]indazole-6-carboxamide

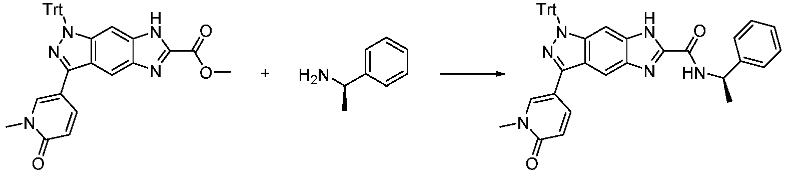

Methyl 3-(1-methyl-6-oxo-1,6-dihydropyridin-3-yl)-1-trityl-1,7-dihydroimidazo[4,5-f]indazole-6-carboxylate (100 mg, 0.19 mmol) and (R)-1-phenylethan-1-amine (1 mL) were added to a microwave reaction tube. The reaction solution was heated to 150°C for 60 min. After cooling to room temperature, 20 mL of ethyl acetate was added to the reaction solution. The ethyl acetate layer was washed with saturated ammonium chloride solution and saturated sodium chloride solution, dried over anhydrous sodium sulfate, and purified by column chromatography to obtain a crude product (80 mg, yield: 68%), which was used directly in the next step.
MS m/z (ESI): 655.3 [M+H]⁺.

### Step 2: Preparation of (R)-3-(1-methyl-6-oxo-1,6-dihydropyridin-3-yl)-N-(1-phenylethyl)-1,7-dihydroimid azo[4,5-f]indazole-6-carboxamide

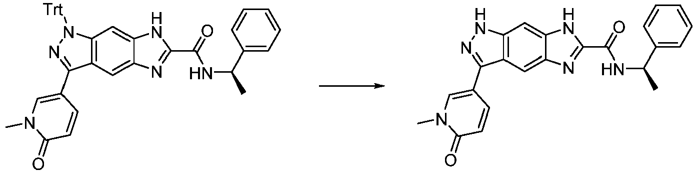

The crude (R)-3-(1-methyl-6-oxo-1,6-dihydropyridin-3-yl)-N-(1-phenylethyl)-1-trityl-1,7-dihydroi midazo[4,5-f]indazole-6-carboxamide (80 mg) was dissolved in 4 mL of dichloromethane, and added with 4 mL of trifluoroacetic acid at the same time. The reaction solution was reacted at room temperature for 3 hours, followed by concentrating to dryness. The resulting crude product was dissolved in a mixed solution of ethyl acetate and tetrahydrofuran. The solution was washed with saturated sodium bicarbonate solution and saturated sodium chloride solution, and dried over anhydrous sodium sulfate. The resulting organic solution was concentrated to dryness, and purified by preparative chromatography to obtain a yellow solid (R)-3-(1-methyl-6-oxo-1,6-dihydropyridin-3-yl)-N-(1-phenylethyl)-1,7-dihydroimidazo [4,5-f]indazole-6-carboxamide (6 mg, yield: 12%).
¹H NMR (400 MHz, DMSO-*d*₆) δ 13.11-12.90 (m, 2H), 9.41-9.21 (m, 1H), 8.39-8.38 (m, 1.7H), 8.12 - 7.99 (m, 1H), 7.97 (s, 0.3H), 7.77 (s, 0.3H), 7.48-7.47 (m, 2.7H), 7.37-7.33 (m, 2H), 7.27-7.25 (m, 1H), 6.60-6.57 (m, 1H), 5.24 - 5.20 (m, 1H), 3.61-3.59 (m, 3H), 1.56 (d, *J* = 8 Hz, 3H).
MS m/z (ESI): 413.2 [M+H]⁺.

### Example 40

### Preparation of (R)-3-(6-cyanopyridin-3-yl)-N-(1-phenylethyl)-1,7-dihydroimidazo[4,5-f]indazole-6 -carboxamide

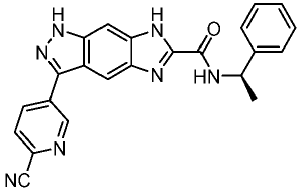

(R)-3-(6-Cyanopyridin-3-yl)-N-(1-phenylethyl)-1,7-dihydroimidazo[4,5-f]indazole -6-carboxamide was obtained from methyl 3-(6-cyanopyridin-3-yl)-1-trityl-1,7-dihydroimidazo[4,5-f]indazole-6-carboxylate and (R)-1-phenylethan-1-amine as the starting materials in accordance with Steps 6 and 7 of Example 2.
¹H NMR (400 MHz, DMSO-*d*₆) δ 13.11 - 12.90 (m, 2H), 9.41 - 9.21 (m, 2H), 8.39 - 8.31 (m, 1.7H), 8.20 - 8.11 (m, 1H), 8.05 (s, 0.3H), 7.87 (s, 0.3H), 7.53 - 7.49 (m, 1.7H), 7.37 - 7.33 (m, 2H), 7.27 - 7.23 (m, 1H), 6.60 - 5.57 (m, 1H), 5.24 - 5.20 (m, 1H), 1.56 (d*, J* = 8 Hz, 3H).
MS m/z (ESI): 408.1 [M+H]⁺.

### Example 41

### Preparation of 3-(6-cyanopyridin-3-yl)-N-cyclopropyl-1,7-dihydroimidazo[4,5-flindazole-6-carbox amide

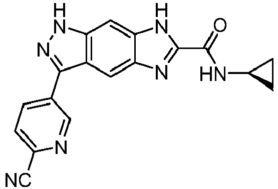

3-(6-Cyanopyridin-3-yl)-N-cyclopropyl-1,7-dihydroimidazo[4,5-f]indazole-6-carb oxamide was obtained from methyl 3-(6-cyanopyridin-3-yl)-1-trityl-1,7-dihydroimidazo[4,5-f]indazole-6-carboxylate and cyclopropylamine as the starting materials in accordance with Steps 6 and 7 of Example 2.
¹H NMR (400 MHz, CDCl₃) δ 8.12 (s, 1H), 7.86 (s, 1H), 6.62 (d, *J* = 7.3 Hz, 2H), 5.61 (s, 1H), 2.75 (s, 1H), 0.62 (dd, *J* = 9, 7.7 Hz, 4H).
MS m/z (ESI): 344.1 [M+H]⁺.

### Example 42

### Preparation of (R)-N-(1-(4-aminophenyl)ethyl)-3-(pyridin-4-yl)-1,7-dihydroimidazo[4,5-f]indazole -6-carboxamide

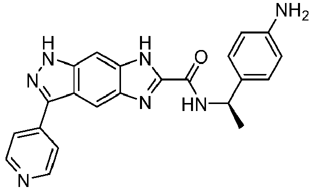

(R)-N-(1-(4-Aminophenyl)ethyl)-3-(pyridin-4-yl)-1,7-dihydroimidazo[4,5-f]indazo le-6-carboxamide was obtained from methyl 3-(pyridin-4-yl)-1-trityl-1,7-dihydroimidazo[4,5-f]indazole-6-carboxylate and (R)-4-(1-aminoethyl)aniline as the starting materials in accordance with Steps 6 and 7 of Example 2.
¹H NMR (400 MHz, DMSO-*d*₆) δ13.43-13.37 (m, 1H), 13.18-13.03 (m, 1H), 9.43 - 9.33 (m, 1H), 8.68 - 8.62 (m, 2H), 8.00 - 7.94 (m, 2H), 7.70 (s, 2H), 7.19-7.11 (m, 2H), 6.53 - 6.48 (m, 2H), 5.25 - 5.21 (m, 1H), 4.33 (s, 2H), 1.55 (d, *J* = 7.0 Hz, 3H).
MS m/z (ESI): 398.1 [M+H]⁺.

### Example 43

### Preparation of (R)-N-(1-(3,5-dichlorophenyl)ethyl)-3-(pyridin-4-yl)-1,7-dihydroimidazo[4,5-f]inda zole-6-carboxamide

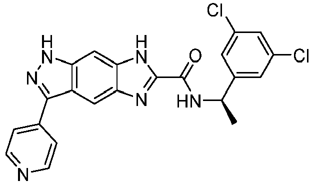

(R)-N-(1-(3,5-Dichlorophenyl)ethyl)-3-(pyridin-4-yl)-1,7-dihydroimidazo[4,5-f]in dazole-6-carboxamide was obtained from methyl 3-(pyridin-4-yl)-1-trityl-1,7-dihydroimidazo[4,5-f]indazole-6-carboxylate and (R)-1-(3,5-dichlorophenyl)ethan-1-amine as the starting materials in accordance with Steps 6 and 7 of Example 2.
¹H NMR (400 MHz, CDCl₃) δ 8.75 (s, 2H), 8.29 (s, 1H), 7.89 (d, *J* = 0.8 Hz, 3H), 7.56 (s, 1H), 7.34 (s, 2H), 7.28 (s, 1H), 4.97 (s, 1H), 1.48 (s, 3H).
MS m/z (ESI): 451.1, 453.1 [M+H]⁺.

### Example 44

### Preparation of N-(1-(6-methylpyridin-2-yl)ethyl)-3-(pyridin-4-yl)-1,7-dihydroimidazo[4,5-f]indazo le-6-carboxamide

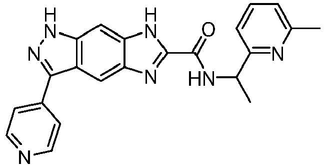

### Step 1: N-(1-(6-Methylpyridin-2-yl)ethyl)-3-(pyridin-4-yl)-1-trityl-1,7-dihydroimidazo[4,5-f]indazole-6-carboxamide

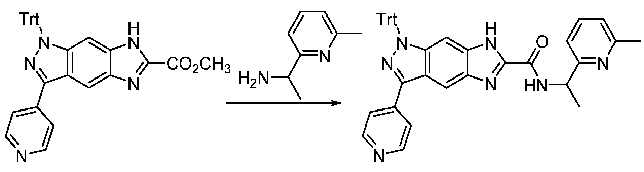

Methyl 3-(pyridin-4-yl)-1-trityl-1,7-dihydroimidazo[4,5-f]indazole-6-carboxylate (450 mg, 0.84 mmol), 1-(6-methylpyridin-2-yl)ethan-1-amine (572 mg, 4.20 mmol) and NMP (6.0 mL) were added to a 30 mL microwave reaction tube. The reaction solution was heated to 120°C and stirred for 2 h. The reaction solution was cooled, concentrated to dryness, and purified by column chromatography to obtain a brown solid product N-(1-(6-methylpyridin-2-yl)ethyl)-3-(pyridin-4-yl)-1-trityl-1,7-dihydroimidazo[4,5-f]in dazole-6-carboxamide (348 mg, yield: 65%).
MS m/z (ESI): 640.3 [M+H]⁺.

### Step 2: Preparation of N-(1-(6-methylpyridin-2-yl)ethyl)-3-(pyridin-4-yl)-1,7-dihydroimidazo[4,5-f]indazo le-6-carboxamide

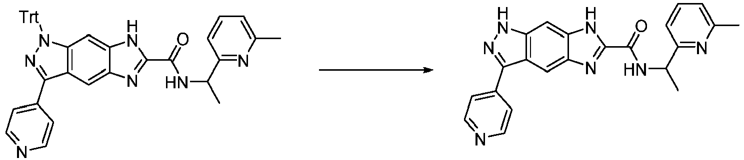

N-(1-(6-methylpyridin-2-yl)ethyl)-3-(pyridin-4-yl)-1-trityl-1,7-dihydroimidazo[4,5 -f]indazole-6-carboxamide (348 mg, 0.84 mmol) was dissolved in dichloromethane (20 mL), and added with trifluoroacetic acid (5 mL). The reaction solution was stirred at room temperature for 2 hours. At the end of the reaction, the reaction solution was concentrated to remove the solvent and obtain a crude product. The crude product was dissolved in 10 mL of methanol, added with ammonia/methanol (5mL, 7M), and stirred for half an hour. The reaction solution was concentrated to dryness, and purified by column chromatography to obtain a white solid product N-(1-(6-methylpyridin-2-yl)ethyl)-3-(pyridin-4-yl)-1,7-dihydroimidazo[4,5-f]indazole-6 -carboxamide (92 mg, yield: 27%).
¹H NMR (400 MHz, DMSO-*d*₆) δ 13.30 (d*, J* = 8.7 Hz, 2H), 9.22 (s, 1H), 8.73 (d, *J* = 4.8 Hz, 2H), 8.53 (s, 1H), 8.06 (d, *J* = 3.1 Hz, 2H), 7.67 (dd, *J* = 3.2, 3.1 Hz, 2H), 7.32 (d, *J* = 7.7 Hz, 1H), 7.18 (d, *J* = 7.6 Hz, 1H), 5.29 - 5.17 (m, 1H), 2.53 (s, 3H), 1.55 (d, *J* = 6.9 Hz, 3H).
MS m/z (ESI): 398.1 [M+H]⁺.

### Example 45

### Preparation of (R)-N-(1-(3-fluorophenyl)ethyl)-3-(pyridin-4-yl)-1,7-dihydroimidazo[4,5-f]indazole -6-carboxamide

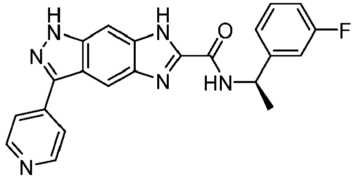

### Step 1: Preparation of (R)-N-(1-(3-fluorophenyl)ethyl)-3-(pyridin-4-yl)-1-trityl-1,7-dihydroimidazo[4,5-f]i ndazole-6-carboxamide

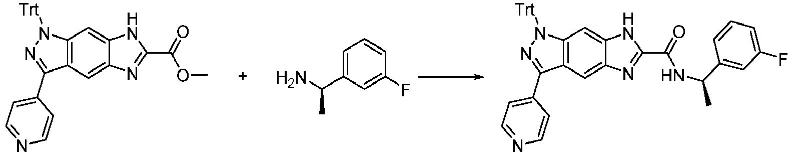

Methyl 3-(pyridin-4-yl)-1-trityl-1,7-dihydroimidazo[4,5-f]indazole-6-carboxylate (200 mg, 0.37 mmol) and (R)-1-(3-fluorophenyl)ethan-1-amine (1.0 mL) were added to a microwave reaction tube. The reaction solution was heated to 150°C for 60 min. After cooling to room temperature, 20 mL of ethyl acetate was added to the reaction solution. The ethyl acetate layer was washed with saturated ammonium chloride solution and saturated sodium chloride solution, dried over anhydrous sodium sulfate, and purified by column chromatography to obtain a crude product (R)-N-(1-(3-fluorophenyl)ethyl)-3-(pyridin-4-yl)-1-trityl-1,7-dihydroimidazo[4,5-f]inda zole-6-carboxamide (160 mg, yield: 67%), which was used directly in the next step.
MS m/z (ESI): 643.3 [M+H]⁺.

### Step 2: Preparation of (R)-N-(1-(3-fluorophenyl)ethyl)-3-(pyridin-4-yl)-1,7-dihydroimidazo[4,5-f]indazole -6-carboxamide

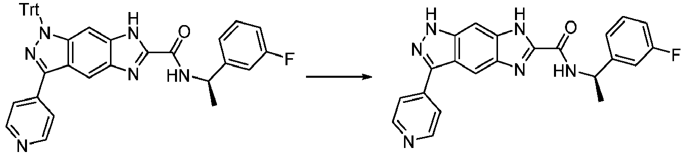

The crude (R)-N-(1-(3-fluorophenyl)ethyl)-3-(pyridin-4-yl)-1-trityl-1,7-dihydroimidazo[4,5-f]inda zole-6-carboxamide (160 mg) was dissolved in 4 mL of dichloromethane, and added with 4 mL of trifluoroacetic acid at the same time. The reaction solution was reacted at room temperature for 3 hours, followed by concentrating to dryness. The resulting crude product was dissolved in a mixed solution of ethyl acetate and tetrahydrofuran. The solution was washed with saturated sodium bicarbonate solution and saturated sodium chloride solution, and dried over anhydrous sodium sulfate. The resulting organic solution was concentrated to dryness, and purified by preparative chromatography to obtain a yellow solid product (R)-N-(1-(3-fluorophenyl)ethyl)-3-(pyridin-4-yl)-1,7-dihydroimidazo[4,5-f]indazole-6-c arboxamide (30 mg, yield: 27%).
¹H NMR (400 MHz, DMSO-*d*₆) δ 13.46 (s, 1H), 9.50 (d, *J* = 8 Hz, 1H), 8.72 (d, *J* = 8 Hz, 2H), 8.36 (s, 2H), 8.02 (d, *J* = 4 Hz, 2H), 7.62 - 7.73 (m, 1H), 7.42 - 7.30 (m, 2H), 7.14 - 7.06 (m, 2H), 5.27 - 5.23 (m, 1H), 1.56 (d, *J=* 8 Hz, 3H).
MS m/z (ESI): 401.2 [M+H]⁺.

### Example 46

### Preparation of (R)-8-chloro-N-(1-phenylethyl)-3-(pyridin-4-yl)-1,7-dihydroimidazo[4,5-f]indazole-6-carboxamide

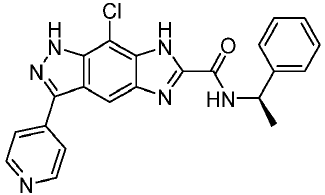

(R)-8-Chloro-N-(1-phenylethyl)-3-(pyridin-4-yl)-1,7-dihydroimidazo[4,5-f]indazol e-6-carboxamide was prepared in accordance with Example 2.
¹H NMR (400 MHz, DMSO-*d*₆) δ 13.56 (s, 1H), 13.32 (s, 1H), 9.39 (d*, J* = 7.5 Hz, 1H), 8.71 (s, 2H), 7.75 (s, 2H), 7.54 - 7.25 (m, 6H), 5.26 - 5.22 (m, 1H), 1.58 (d, *J* = 6.2 Hz, 3H).
MS m/z (ESI): 417.1, 419.1 [M+H]⁺.

### Example 47 and Example 48

### Preparation of (R)-N-(1-(6-methylpyridin-2-yl)ethyl)-3-(pyridin-4-yl)-1,7-dihydroimidazo[4,5-f]in dazole-6-carboxamide and (S)-N-(1-(6-methylpyridin-2-yl)ethyl)-3-(pyridin-4-yl)-1,7-dihydroimidazo[4,5-f]ind azole-6-carboxamide

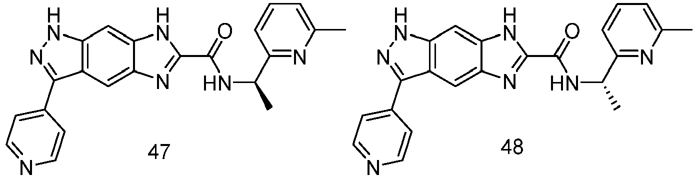

N-(1-(6-Methylpyridin-2-yl)ethyl)-3-(pyridin-4-yl)-1,7-dihydroimidazo[4,5-f]inda zole-6-carboxamide (45 mg, 0.11 mmol) was resolved on a chiral column to obtain (R)-N-(1-(6-methylpyridin-2-yl)ethyl)-3-(pyridin-4-yl)-1,7-dihydroimidazo[4,5-f]indaz ole-6-carboxamide (8.0 mg, 0.02 mmol) and (S)-N-(1-(6-methylpyridin-2-yl)ethyl)-3-(pyridin-4-yl)-1,7-dihydroimidazo[4,5-f]indazo le-6-carboxamide (6.7 mg, 0.017 mmol).

### Example 47

¹H NMR (400 MHz, DMSO-*d*₆) δ 13.30 (d*, J* = 9.7 Hz, 2H), 9.22 (s, 1H), 8.73 (d, *J* = 4.8 Hz, 2H), 8.53 (s, 1H), 8.06 (d, *J* = 3.3 Hz, 2H), 7.67 (dd, *J* = 2.8, 2.1 Hz, 2H), 7.32 (d*, J* = 7.7 Hz, 1H), 7.18 (d, *J* = 7.6 Hz, 1H), 5.29 - 5.17 (m, 1H), 2.53 (s, 3H), 1.55 (d, *J* = 6.9 Hz, 3H).
MS m/z (ESI): 398.1 [M+H]⁺.

### Example 48

¹H NMR (400 MHz, DMSO-*d*₆) δ 13.34 (d*, J* = 9.8 Hz, 2H), 9.17 (s, 1H), 8.72 (d, *J* = 4.8 Hz, 2H), 8.51 (s, 1H), 8.07 (d, *J* = 3.3 Hz, 2H), 7.66 (dd, *J* = 2.8, 2.1 Hz, 2H), 7.34 (d*, J* = 7.8 Hz, 1H), 7.19 (d, *J* = 7.6 Hz, 1H), 5.29 - 5.20 (m, 1H), 2.51 (s, 3H), 1.50 (d, *J* = 6.9 Hz, 3H).
MS m/z (ESI): 398.1 [M+H]⁺.

### Example 49

### Preparation of (R)-N-(1-(2-chlorophenyl)ethyl)-3-(pyridin-4-yl)-1,7-dihydroimidazo[4,5-f)indazole -6-carboxamide

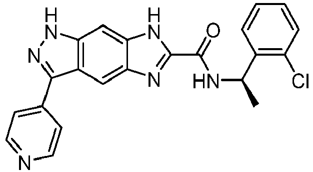

(R)-N-(1-(2-Chlorophenyl)ethyl)-3-(pyridin-4-yl)-1,7-dihydroimidazo[4,5-f]indazo le-6-carboxamide was obtained from methyl 3-(pyridin-4-yl)-1-trityl-1,7-dihydroimidazo[4,5-f]indazole-6-carboxylate and (R)-1-(2-chlorophenyl)ethan-1-amine as the starting materials in accordance with Steps 6 and 7 of Example 2.
¹H NMR (400 MHz, DMSO-*d*₆) δ 13.47 (s, 1H), 9.48 (d, *J* = 7.9 Hz, 1H), 8.71 - 8.64 (m, 2H), 8.13 (s, 1H), 7.99 (s, 1H), 7.96 - 7.89 (m, 2H), 7.44 - 7.36 (m, 1H), 7.36 - 7.28 (m, 1H), 7.24 - 7.13 (m, 2H), 6.83 (d, *J* = 9.5 Hz, 1H), 5.28 (dqd, *J* = 9.5, 6.8, 1.0 Hz, 1H), 1.68 (d, *J=* 6.9 Hz, 3H).
MS m/z (ESI): 417.1, 419.1 [M+H]⁺.

### Example 50

### Preparation of (S)-N-(2-(methylamino)-1-phenylethyl)-3-(pyridin-4-yl)-1,7-dihydroimidazo[4,5-f]i ndazole-6-carboxamide

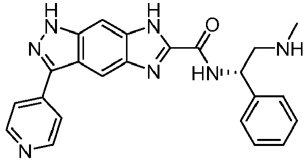

### Step 1: Preparation of (S)-N-(2-hydroxy-1-phenylethyl)-3-(pyridin-4-yl)-1-trityl-1,7-dihydroimidazo[4,5-f] indazole-6-carboxamide

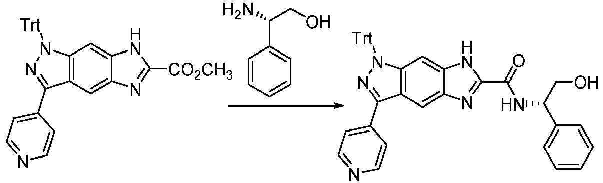

Methyl 3-(pyridin-4-yl)-1-trityl-1,7-dihydroimidazo[4,5-f]indazole-6-carboxylate (50 mg, 0.93 mmol), (S)-2-amino-2-phenylethan-1-ol (640 mg, 4.66 mmol) and NMP (10 mL) were added to an 150 mL microwave reaction tube. The reaction solution was heated and stirred for 2 h. The reaction solution was cooled, concentrated to dryness, and purified by column chromatography to obtain a brown solid product (S)-N-(2-hydroxy-1-phenylethyl)-3-(pyridin-4-yl)-1-trityl-1,7-dihydroimidazo[4,5-f]ind azole-6-carboxamide (340 mg, yield: 56%).
MS m/z (ESI): 641.2 [M-H]⁺.

### Step 2: Preparation of (S)-N-(2-oxo-1-phenylethyl)-3-(pyridin-4-yl)-1-trityl-1,7-dihydroimidazo[4,5-f]inda zole-6-carboxamide

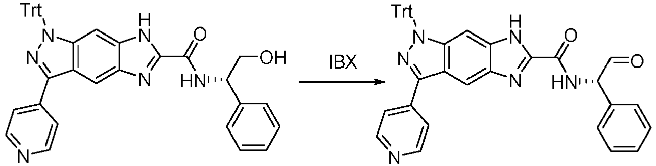

(S)-N-(2-Hydroxy-1 -phenyl ethyl)-3 -(pyridin-4-yl)-1 -trityl-1,7-dihydroimidazo[4,5 -f]indazole-6-carboxamide (220 mg, 0.34 mmol) was dissolved in 20 mL of DMSO. The solution was added with IBX (769 mg, 2.74 mmol), and stirred at room temperature overnight. The reaction solution was concentrated to dryness, and purified by column chromatography to obtain a crude brown solid product (S)-N-(2-oxo-1-phenylethyl)-3-(pyridin-4-yl)-1-trityl-1,7-dihydroimidazo[4,5-f]indazol e-6-carboxamide (300 mg).
MS m/z (ESI): 639.2[M-H]⁺.

### Step 3: Preparation of (S)-N-(2-(methylamino)-1-phenylethyl)-3-(pyridin-4-yl)-1-trityl-1, 7 -dihydroimidaz o[4,5-indazole-6-carboxamide

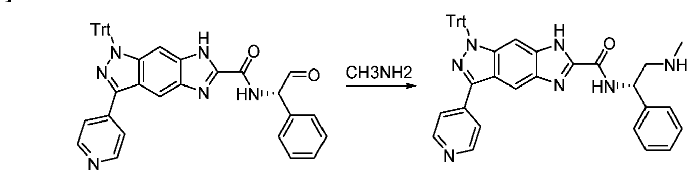

(S)-N-(2-Oxo-1-phenylethyl)-3 -(pyridin-4-yl)-1-trityl-1,7-dihydroimidazo[4,5-f]in dazole-6-carboxamide (100 mg), 30% methylamine/methanol solution (3 mL) and glacial acetic acid (2 mL) were dissolved in dichloromethane/methanol (10 mL/10 mL), and stirred at room temperature for 30 min. The reaction solution was added with sodium cyanoborohydride (49 mg, 0.78 mmol), and stirred at room temperature overnight. The reaction solution was concentrated to dryness, added with water, and extracted with dichloromethane. The solution was dried, filtered, and concentrated to dryness. The residues were purified by column chromatography to obtain a crude thick product (S)-N-(2-(methylamino)-1-phenylethyl)-3-(pyridin-4-yl)-1-trityl-1,7-dihydroimidazo[4, 5-f]indazole-6-carboxamide (100 mg).
MS m/z (ESI): 654.2[M-H]⁺.

### Step 4: Preparation of (S)-N-(2-(methylamino)-1-phenylethyl)-3-(pyridin-4-yl)-1,7-dihydroimidazo[4,5-f]i ndazole-6-carboxamide

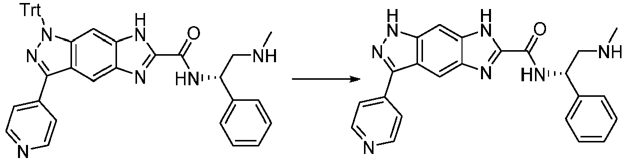

The thick substance (S)-N-(2-(methylamino)-1-phenyl ethyl)-3-(pyridin-4-yl)-1-trityl-1,7-dihydroimidazo[4, 5-f]indazole-6-carboxamide (crude, 100 mg) obtained in the above Step 3 was dissolved in dichloromethane (1 mL), and added with trifluoroacetic acid (2 mL). The reaction solution was stirred at room temperature for 2 h. The reaction solution was concentrated to dryness, and purified by column chromatography to obtain a solid product (S)-N-(2-(methylamino)-1-phenylethyl)-3-(pyridin-4-yl)-1,7-dihydroimidazo[4,5-f]inda zole-6-carboxamide (3.6 mg, yield in three steps: 7.6%).
MS m/z (ESI): 412.1 [M+H]⁺.

### Example 51

### Preparation of N-(1-(pyrazin-2-yl)ethyl)-3-(pyridin-4-yl)-1,7-dihydroimidazo[4,5-f]indazole-6-carb oxamide

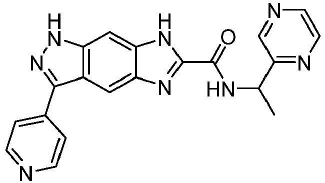

N-(1-(Pyrazin-2-yl)ethyl)-3-(pyridin-4-yl)-1,7-dihydroimidazo[4,5-f]indazole-6-ca rboxamide was obtained from methyl 3-(pyridin-4-yl)-1-trityl-1,7-dihydroimidazo[4,5-f]indazole-6-carboxylate and 1-(pyrazin-2-yl)ethan-1-amine as the starting materials in accordance with Steps 6 and 7 of Example 2.
¹H NMR (400 MHz, CDCl3) δ 8.75 (s, 2H), 8.58 (d, *J=* 2.4 Hz, 3H), 8.30 (s, 1H), 7.89 (d*, J*= 1.8 Hz, 3H), 7.19 (s, 1H), 4.86 (s, 1H), 1.49 (s, 3H).
MS m/z (ESI): 385.1 [M+H]⁺.

### Example 52

### Preparation of N-((2-methylpyridin-4-yl)methyl)-3-(pyridin-4-yl)-1,7-dihydroimidazo[4,5-f]indazo le-6-carboxamide

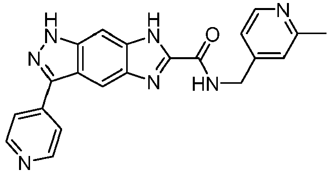

N-((2-Methylpyridin-4-yl)methyl)-3-(pyridin-4-yl)-1,7 -dihydroimidazo[4,5-f]indaz ole-6-carboxamide was obtained from methyl 3-(pyridin-4-yl)-1-trityl-1,7-dihydroimidazo[4,5-f]indazole-6-carboxylate and (2-methyl-pyridin-4-yl)methylamine as the starting materials in accordance with Steps 6 and 7 of Example 2.
¹H NMR (400 MHz, DMSO-*d*₆) δ 13.47 (s, 1H), 9.48 (d, *J* = 7.9 Hz, 1H), 8.71 - 8.64 (m, 2H), 8.38 (d, *J* = 7.6 Hz, 1H), 8.13 (s, 1H), 7.99 (s, 1H), 7.96 - 7.89 (m, 2H), 7.72 (t, *J* = 10.2 Hz, 1H), 7.32 - 7.27 (m, 1H), 7.18 (dd, *J* = 7.5, 1.7 Hz, 1H), 4.93 (d, *J* = 10.1 Hz, 2H), 2.50 (s, 3H).
MS m/z (ESI): 384.1 [M+H]⁺.

### Example 53

### Preparation of N-((5-methylpyridin-3-yl)methyl)-3-(pyridin-4-yl)-1,7-dihydroimidazo[4,5-f]indazo le-6-carboxamide

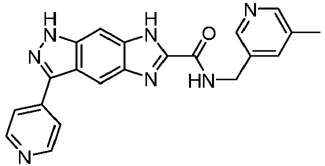

N-((5-Methylpyridin-3-yl)methyl)-3-(pyridin-4-yl)-1,7-dihydroimidazo[4,5-f]indaz ole-6-carboxamide was obtained from methyl 3-(pyridin-4-yl)-1-trityl-1,7-dihydroimidazo[4,5-f]indazole-6-carboxylate and (5-methylpyridin-3-yl)methylamine as the starting materials in accordance with Steps 6 and 7 of Example 2.
¹H NMR (400 MHz, DMSO-*d*₆) δ 13.47-13.10 (m, 2H), 9.28-9.20 (m, 1H), 8.72 (d, *J* = 8 Hz, 2H), 8.57 (s, 1H), 8.44 (s, 1H), 8.02 (d, *J* = 4 Hz, 2H), 7.82 (s, 1H), 7.77 - 7.73 (m, 2H), 4.23 (s, 2H), 2.23 (s, 3H).
MS m/z (ESI): 384.1 [M+H]⁺.

### Example 54

### Preparation of (S)-N-(2-(cyclopropylamino)-1-phenylethyl)-3-(pyridin-4-yl)-1,7-dihydroimidazo[4, 5-f]indazole-6-carboxamide

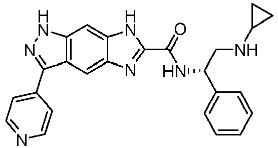

### Step 1: Preparation of (S)-N-(2-hydroxy-1-phenylethyl)-3-(pyridin-4-yl)-1-trityl-1,7-dihydroimidazo[4,5-f] indazole-6-carboxamide

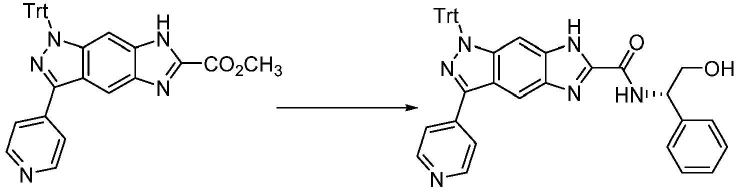

Methyl 3-(pyridin-4-yl)-1-trityl-1,7-dihydroimidazo[4,5-f]indazole-6-carboxylate (2 g, 3.73 mmol) and (S)-2-amino-2-phenylethan-1-ol (2.56 g, 18.66 mmol) were stirred under a microwave condition at 140°C for 1 h. The reaction solution was added with water, and extracted with ethyl acetate. The solution was dried, concentrated to dryness, and purified by column chromatography to obtain (S)-N-(2-hydroxy-1-phenyl ethyl)-3-(pyridin-4-yl)-1-trityl-1,7-dihydroimidazo[4,5-f]ind azole-6-carboxamide (1.46 g, yield: 61%).
MS m/z (ESI): 641.2 [M+H]⁺.

### Step 2: Preparation of (S)-N-(2-oxo-1-phenylethyl)-3-(pyridin-4-yl)-1-trityl-1,7-dihydroimidazo[4,5-f]inda zole-6-carboxamide

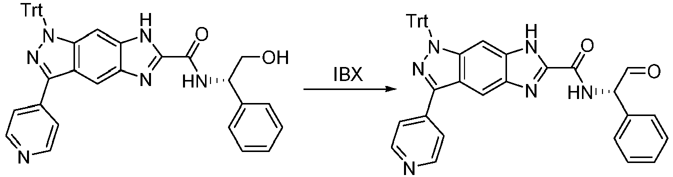

(S)-N-(2-Hydroxy-1-phenylethyl)-3-(pyridin-4-yl)-1-trityl-1,7-dihydroimidazo[4,5 -f]indazole-6-carboxamide (1.46 g, 2.28 mmol) was dissolved in DMSO (10 mL). The solution was added with IBX (5.1 g, 18.21 mmol), and stirred under a nitrogen atmosphere at room temperature for 3 h. The reaction solution was added with dichloromethane, and washed with sodium carbonate solution. The solution was dried, concentrated to dryness, and purified by column chromatography to obtain a oily crude product (2.52 g).
MS m/z (ESI): 639.2 [M+H]⁺.

### Step 3: Preparation of (S)-N-(2-(cyclopropylamino)-1-phenylethyl)-3-(pyridin-4-yl)-1-trityl-1,7-dihydroim idazo[4,5-f]indazole-6-carboxamide

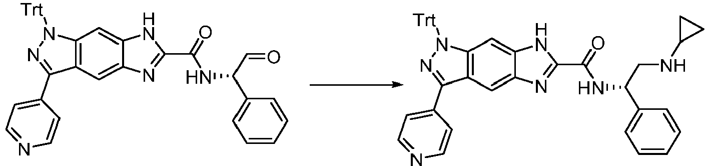

(S)-N-(2-Oxo-1-phenyl ethyl)-3-(pyridin-4-yl)-1-trityl-1,7-dihydroimidazo[4,5-f]in dazole-6-carboxamide (20 mg, 0.031 mmol) and cyclopropylamine (18 mg, 0.31 mmol) were stirred in dichloromethane/methanol (10 mL/10 mL). The reaction solution was added with 1 mL of acetic acid, and stirred for 30 min. The reaction solution was added with sodium cyanoborohydride (10 mg, 0.16 mmol), and stirred overnight. The reaction solution was added with water, and extracted with dichloromethane. The solution was dried, concentrated to dryness, and purified by column chromatography to obtain an oil, which was used directly in the next step.
MS m/z (ESI): 704.2 [M+H]⁺.

### Step 4: Preparation of (S)-N-(2-((2,2-difluoroethyl)amino)-1-phenylethyl)-3-(pyridin-4-yl)-1,7-dihydroimi dazo[4,5-f]indazole-6-carboxamide

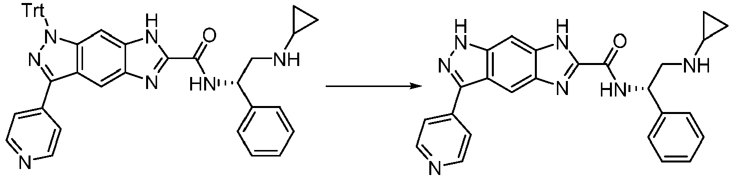

The oil obtained in the previous step was dissolved in dichloromethane (2 mL), and added with trifluoroacetic acid (2 mL). The reaction solution was stirred at room temperature for 2 h. The reaction solution was concentrated to dryness, and purified by column chromatography to obtain an off-white solid (S)-N-(2-((2,2-difluoroethyl)amino)-1-phenylethyl)-3-(pyridin-4-yl)-1,7-dihydroimidaz o[4,5-f]indazole-6-carboxamide (3.5 mg, yield in two steps: 25%).
¹H NMR (400 MHz, DMSO-*d*₆) δ 13.47 (s, 1H), 9.48 (d, *J* = 7.9 Hz, 1H), 8.71 - 8.64 (m, 2H), 8.13 (s, 1H), 7.99 (s, 1H), 7.96 - 7.89 (m, 2H), 7.40 - 7.23 (m, 5H), 7.10 (d, *J* = 10.3 Hz, 1H), 5.12 (dtt, *J* = 10.2, 6.9, 1.0 Hz, 1H), 3.22 (dt, *J* = 7.7, 6.1 Hz, 1H), 3.11 (ddd, *J* = 12.8, 7.0, 6.0 Hz, 1H), 3.00 (ddd, *J* = 12.5, 7.0, 6.1 Hz, 1H), 2.54 - 2.41 (m, 1H), 1.05 - 0.77 (m, 4H).
MS m/z (ESI): 438.2 [M+H]⁺.

### Example 55

### Preparation of N-(2-fluoro-l-phenylethyl)-3-(pyridin-4-yl)-1,7-dihydroimidazo [4,5-f) indazole-6-ca rboxamide

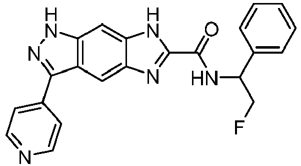

N-(2-Fluoro-1-phenylethyl)-3-(pyridin-4-yl)-1,7-dihydroimidazo[4,5-f]indazole-6-carboxamide was obtained from methyl 3-(pyridin-4-yl)-1-trityl-1,7-dihydroimidazo[4,5-f]indazole-6-carboxylate and 2-fluoro-1-phenylethan-1-amine as the starting materials in accordance with Steps 6 and 7 of Example 2.
¹H NMR (400 MHz, DMSO-*d*₆) δ 13.47 (s, 1H), 9.48 (d, *J* = 7.9 Hz, 1H), 8.71 - 8.64 (m, 2H), 8.13 (s, 1H), 7.99 (s, 1H), 7.96 - 7.89 (m, 2H), 7.65 (d, *J* = 11.0 Hz, 1H), 7.41 - 7.32 (m, 4H), 7.32 - 7.23 (m, 1H), 5.55 - 5.38 (m, 1H), 4.99 (d, *J* = 7.0 Hz, 1H), 4.87 (d, *J* = 7.1 Hz, 1H).
MS m/z (ESI): 401.1[M+H]⁺.

### Example 56

### Preparation of N-((2-methylpyrimidin-4-yl)methyl)-3-(pyridin-4-yl)-1,7-dihydroimidazo[4,5-f]inda zole-6-carboxamide

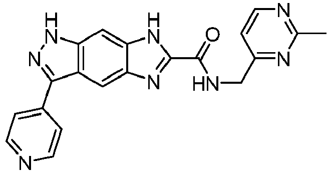

N-((2-Methylpyrimidin-4-yl)methyl)-3-(pyridin-4-yl)-1,7-dihydroimidazo[4,5-f]in dazole-6-carboxamide was obtained from methyl 3-(pyridin-4-yl)-1-trityl-1,7-dihydroimidazo[4,5-f]indazole-6-carboxylate and (2-methylpyrimidin-4-yl)methylamine as the starting materials in accordance with Steps 6 and 7 of Example 2.
¹H NMR (400 MHz, DMSO-*d*₆) δ 13.49 (s, 1H), 9.47 (d*, J* = 7.9 Hz, 1H), 9.01 (d, *J* = 7.4 Hz, 1H), 8.71 - 8.64 (m, 2H), 8.13 (s, 1H), 7.99 (s, 1H), 7.97 - 7.88 (m, 3H), 7.24 (d, *J=* 7.5 Hz, 1H), 4.43 (d, *J=* 10.1 Hz, 2H), 2.46 (s, 3H).
MS m/z (ESI): 385.1 [M+H]⁺.

### Example 57

### Preparation of (R)-N-(1-phenylethyl)-3-(pyrimidin-4-yl)-1,7-dihydroimidazo[4,5-f]indazole-6-carb oxamide

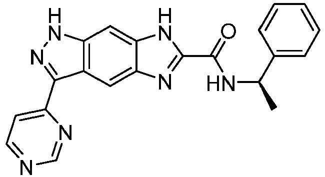

(R)-N-(1-Phenylethyl)-3-(pyrimidin-4-yl)-1,7-dihydroimidazo[4,5-f]indazole-6-car boxamide was prepared in accordance with Example 2.
¹H NMR (400 MHz, DMSO-*d*₆) δ 13.67 (s, 0.6 H), 13.59 (s, 0.4 H), 13.24 (s, 0.6 H), 13.08 (s, 0.4 H), 9.44 - 9.38 (m, 1H), 9.36 - 9.31 (m, 1H), 9.00 - 8.70 (m, 2H), 8.22 - 8.19 (m, 1H), 7.89 (s, 0.6 H), 7.60 (s, 0.4 H), 7.49 (d, *J* = 7.5 Hz, 2H), 7.35 (t, *J* = 7.6 Hz, 2H), 7.25 (t, *J* = 7.3 Hz, 1H), 5.34 - 5.14 (m, 1H), 1.58 (d, *J* = 7.0 Hz, 3H).
MS m/z (ESI): 384.1 [M+H]⁺.

### Example 58

### Preparation of 3-(pyridin-4-yl)-N-(2,2,2-trifluoro-1-phenylethyl)-1,7-dihydroimidazo[4,5-f]indazol e-6-carboxamide

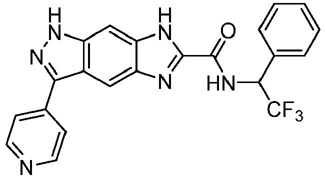

3-(Pyridin-4-yl)-N-(2,2,2-trifluoro-1-phenylethyl)-1,7-dihydroimidazo[4,5-f]indaz ole-6-carboxamide was obtained from methyl 3-(pyridin-4-yl)-1-trityl-1,7-dihydroimidazo[4,5-f]indazole-6-carboxylate and 2,2,2-trifluoro-1-phenylethan-1-amine as the starting materials in accordance with Steps 6 and 7 of Example 2.
¹H NMR (400 MHz, DMSO-*d₆*) δ: 13.56 (s, 1H), 13.32 (s, 1H), 9.39 (d, *J* = 7.5 Hz, 1H), 8.68 - 8.62 (m, 2H), 8.00 - 7.94 (m, 2H), 7.70 (s, 2H), 7.41 - 7.30 (m, 4H), 7.30 - 7.26 (m, 1H), 7.05 - 6.91 (m, 1H).
MS m/z (ESI): 437.1 [M+H]⁺.

### Example 59

### Preparation of (R)-N-(1-phenylethyl)-3-(pyridin-4-yl)-1,7-dihydroimidazo[4,5-b]pyrazolo[4,3-e]py ridine-6-carboxamide

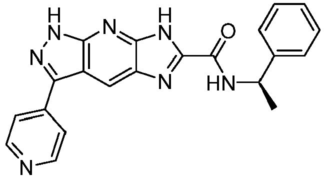

### Step 1: Preparation of (2,6-difluoropyridin-3-yl)(pyridin-4-yl)methanol

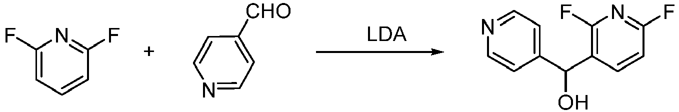

2,6-Difluoropyridine (3.45 g, 30 mmol) was dissolved in 100 mL of anhydrous tetrahydrofuran, and cooled to -78°C. The solution was added dropwise with 2.0 M solution of LDA in THF/n-heptane (15.8 mL, 31.5 mmol) under a nitrogen atmosphere, and then added with 4-pyridinaldehyde (3.86 g, 36 mmol) by injection. The reaction solution was warmed up gradually from -78°C to room temperature. After completion of the reaction, the reaction solution was added with 1.8 mL of acetic acid, and stirred at room temperature for 30 minutes. The reaction solution was added directly with silica gel, concentrated to dryness, and purified by column chromatography to obtain a light yellow solid (R)-N-(1-phenylethyl)-3-(pyridin-4-yl)-1,7-dihydroimidazo[4,5-b]pyrazolo[4,3-e]pyridi ne-6-carboxamide (5.0 g, yield: 75%).
MS m/z (ESI): 223.1 [M+H]⁺.

### Step 2: Preparation of (2,6-difluoropyridin-3-yl)(pyridin-4-yl)methanone

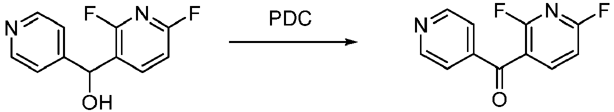

(2,6-Difluoropyridin-3-yl)(pyridin-4-yl)methanol (5.0 g, 22.5 mmol) was dissolved in 250 mL of DMF. The solution was added with PDC (25.4 g, 67.5 mmol) in batches, and reacted at room temperature for 4 hours. The reaction solution was concentrated, added with 200 mL of water, and extracted with ethyl acetate. The ethyl acetate layer was washed with saturated NaCl solution, dried over anhydrous sodium sulfate, and purified by column chromatography to obtain a white solid (2,6-difluoropyridin-3-yl)(pyridin-4-yl)methanone (3.6 g, yield: 73%).
MS m/z (ESI): 221.1 [M+H]⁺.

### Step 3: Preparation of 6-fluoro-3-(pyridin-4-yl)-1H-pyrazolo[3,4-b]pyridine

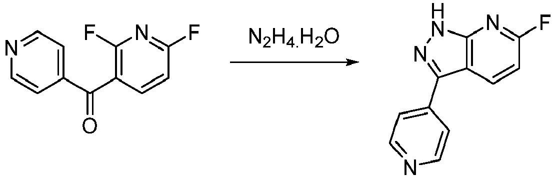

(2,6-Difluoropyridin-3-yl)(pyridin-4-yl)methanone (3.6 g, 16.4 mmol) was dissolved in 30 mL of 1,4-dioxane. The solution was added with 85% hydrazine hydrate solution (1.2 g, 19.7 mmol), and reacted at room temperature for 3 hours. The reaction solution was added with silica gel, concentrated to dryness directly, and purified by column chromatography to obtain a white solid 6-fluoro-3-(pyridin-4-yl)-1H-pyrazolo[3,4-b]pyridine (2.6 g, yield: 74%).
MS m/z (ESI): 215.1 [M+H]⁺.

### Step 4: Preparation of 3-(pyridin-4-yl)-1H-pyrazolo[3,4-b]pyridin-6-amine

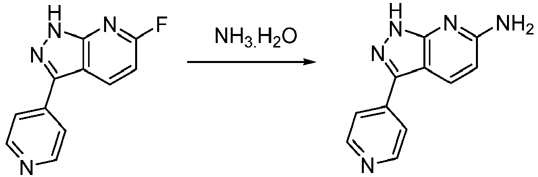

6-Fluoro-3-(pyridin-4-yl)-1H-pyrazolo[3,4-b]pyridine (0.5 g, 2.33 mmol), 4 mL of DMSO and 8 mL of concentrated ammonia were added to a microwave reaction tube. The reaction solution was heated to 110°C by microwave for 2 hours. The reaction solution was cooled, poured into 100 mL of water and stirred. The precipitated solid was filtered, and the filter cake was washed with water and dried under vacuum to obtain a crude product 3-(pyridin-4-yl)-1H-pyrazolo[3,4-b]pyridin-6-amine (450 mg).
MS m/z (ESI): 212.1 [M+H]⁺.

### Step 5: Preparation of 5-nitro-3-(pyridin-4-yl)-1H-pyrazolo [3,4-b] pyridin-6-amine

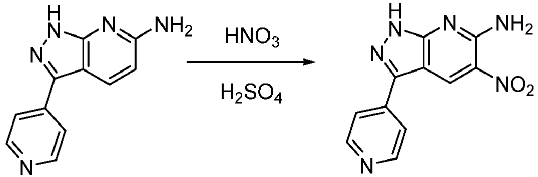

3-(Pyridin-4-yl)-1H-pyrazolo[3,4-b]pyridin-6-amine (450 mg, 2.13 mmol) was dissolved in 7.5 mL of concentrated sulfuric acid. The solution was placed in an ice bath, and added dropwise with concentrated nitric acid (310 mg, 3.20 mmol). The reaction solution was heated to 55°C and reacted for 6 hours. The reaction solution was cooled, poured into 50 mL of ice water and stirred. The pH was adjusted to neutral with 4N NaOH solution. The precipitated solid was filtered, and the filter cake was washed with water and dried under vacuum to obtain a yellow solid 5-nitro-3-(pyridin-4-yl)-1H-pyrazolo[3,4-b]pyridin-6-amine (200 mg, yield: 36%).
MS m/z (ESI): 257.1 [M+H]⁺.

### Step 6: Preparation of 3-(pyridin-4-yl)-1H-pyrazolo[3,4-b]pyridine-5,6-diamine

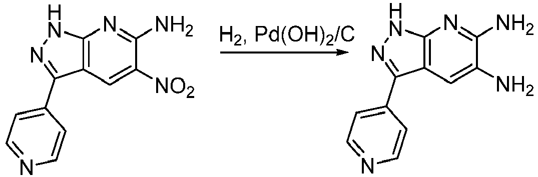

5-Nitro-3-(pyridin-4-yl)-1H-pyrazolo[3,4-b]pyridin-6-amine (200 mg, 0.78 mmol) was dissolved in a mixed solvent of DMF and THF (25 mL: 25 mL). The solution was added with 50 mg of 20% Pd(OH)₂/C (containing about 50% water). The reaction system was purged with hydrogen by a hydrogen balloon several times. The reaction solution was reacted at room temperature overnight. The reaction solution was filtered to remove Pd(OH)₂/C, and concentrated to dryness to obtain a crude product 3-(pyridin-4-yl)-1H-pyrazolo[3,4-b]pyridine-5,6-diamine (200 mg), which was used directly in the next step.
MS m/z (ESI): 227.1 [M+H]⁺.

### Step 7: Preparation of methyl 3-(pyridin-4-yl)-1,7-dihydroimidazo[4,5-b]pyrazolo[4,3-e]pyridine-6-carboxylate

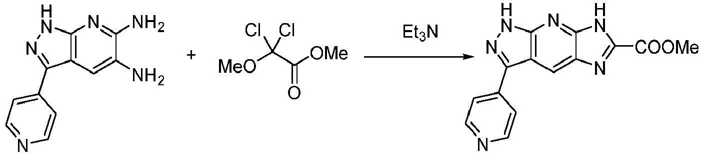

3-(Pyridin-4-yl)-1H-pyrazolo[3,4-b]pyridine-5,6-diamine (150 mg, 0.66 mmol) was dissolved in 10 mL of methanol in a microwave reaction tube. The solution was added with 2 mL of triethylamine and 1.5 mL of methyl dichloromethoxyacetate, and heated to 100°C by microwave for 1.5 hours. The reaction solution was cooled to room temperature, added with silica gel, concentrated to dryness, and purified by column chromatography to obtain a crude product methyl **3-(pyridin-4-yl)-1,7-dihydroimidazo[4,5-b]pyrazolo[4,3-e]pyridine-6-carboxylate** (120 mg).
MS m/z (ESI): 295.1 [M+H]⁺.

### Step 8: Preparation of (R)-N-(1-phenylethyl)-3-(pyridin-4-yl)-1,7-dihydroimidazo[4,5-b]pyrazolo[4,3-e]py ridine-6-carboxamide

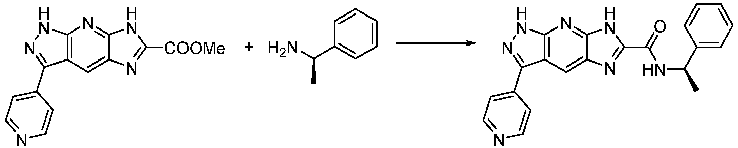

Methyl **3-(pyridin-4-yl)-1,7-dihydroimidazo[4,5-b]pyrazolo[4,3-e]pyridine-6-carboxylate** (130 mg, 0.44 mmol) and 2 mL of (*R*)-(+)-1-phenylethylamine were added to a microwave reaction tube. The reaction solution was heated to 140°C by microwave for one hour. The reaction solution was cooled, and added with 20 mL of 2-methyltetrahydrofuran. The organic phase was washed with saturated NH₄Cl solution and saturated NaCl solution successively, dried over anhydrous sodium sulfate, and purified by preparative HPLC to obtain a light yellow solid (R)-N-(1-phenylethyl)-3-(pyridin-4-yl)-1,7-dihydroimidazo[4,5-b]pyrazolo[4,3-e]pyridi ne-6-carboxamide (6.8 mg, yield: 4%).
¹H NMR (400 MHz, DMSO-*d*₆) δ 13.35-113.13 (m, 2H), 9.45-9.34 (m, 1H), 8.72 (d, *J* = 8 Hz, 2H), 8.02 (d, *J* = 8 Hz, 2H), 7.86 (s, 0.4H), 7.57 (s, 0.6H), 7.46-7.23 (m, 5H), 5.24-5.21 (m, 1H), 1.56 (d, *J=* 8 Hz, 3H).
MS m/z (ESI): 384.1 [M+H]⁺.

### Example 60

### Preparation of (R)-N-(1-methoxypropan-2-yl)-3-(pyridin-4-yl)-1,7-dihydroimidazo[4,5-f]indazole-6-carboxamide

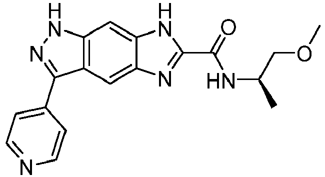

(R)-N-(1-Methoxypropan-2-yl)-3-(pyridin-4-yl)-1,7-dihydroimidazo[4,5-f]indazol e-6-carboxamide was obtained from methyl 3-(pyridin-4-yl)-1-trityl-1,7-dihydroimidazo[4,5-f]indazole-6-carboxylate and (R)-1-methoxypropan-2-amine as the starting materials in accordance with Steps 6 and 7 of Example 2.
¹H NMR (400 MHz, MeOD) δ 8.58 (d, *J* = 4 Hz, 2H), 8.38 (s, 0.6H), 8.16 (s, 0.4H), 8.03 (d, *J* = 4 Hz, 2H), 7.80 (s, 0.4H), 7.58 (s, 0.6H), 4.27-4.22 (m, 1H), 3.52-3.36 (m, 2H), 3.32 (s, 3H), 1.23 (d, *J=* 8 Hz, 3H).
MS m/z (ESI): 351.1 [M+H]⁺.

### Example 61

### Preparation of N-(2-hydroxy-2-methylpropyl)-3-(pyridin-4-yl)-1,7-dihydroimidazo[4,5-f]indazole-6-carboxamide

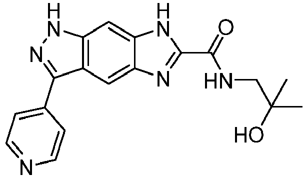

N-(2-Hydroxy-2-methylpropyl)-3-(pyridin-4-yl)-1,7-dihydroimidazo[4,5-f]indazol e-6-carboxamide was obtained from methyl 3-(pyridin-4-yl)-1-trityl-1,7-dihydroimidazo[4,5-f]indazole-6-carboxylate and 1-amino-2-methylpropan-2-ol as the starting materials in accordance with Steps 6 and 7 of Example 2.
¹H NMR (400 MHz, DMSO-*d*₆) δ 13.74 (s, 1H), 10.80 (s, 1H), 8.80 (d*, J* = 5.5 Hz, 1H), 8.48 - 8.41 (m, 2H), 8.26 (d, *J=* 5.5 Hz, 2H), 7.75 (s, 1H), 7.35 (s, 1H), 4.04 (brs, 1H), 3.35 (d, *J* = 6.0 Hz, 2H), 1.16 (s, 6H).
MS m/z (ESI): 351.1 [M+H]⁺.

### Example 62

### Preparation of (R)-N-(1-(2-fluorophenyl)ethyl)-3-(pyridin-4-yl)-1,7-dihydroimidazo[4,5-f]indazole -6-carboxamide

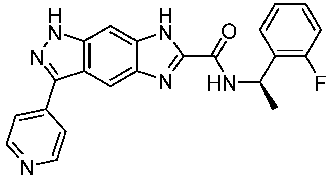

(R)-N-(1-(2-Fluorophenyl)ethyl)-3-(pyridin-4-yl)-1,7-dihydroimidazo[4,5-f]indazo le-6-carboxamide was obtained from methyl 3-(pyridin-4-yl)-1-trityl-1,7-dihydroimidazo[4,5-f]indazole-6-carboxylate and (R)-1-(2-fluorophenyl)ethan-1-amine as the starting materials in accordance with Steps 6 and 7 of Example 2.
¹H NMR (400 MHz, DMSO-*d*₆) δ 13.49 (s, 1H), 9.47 (d*, J* = 7.9 Hz, 1H), 8.72 (d, *J* = 5.2 Hz, 2H), 8.35 (s, 4H), 8.03 (d, *J* = 5.1 Hz, 2H), 7.72 (s, 1H), 7.61 (t, *J* = 7.2 Hz, 1H), 7.32 (dd, *J* = 13.2, 5.8 Hz, 1H), 7.26 - 7.14 (m, 2H), 5.57 - 5.43 (m, 1H), 1.56 (d, *J=* 7.0 Hz, 3H).
MS m/z (ESI): 401.1 [M+H]⁺.

### Example 63

### Preparation of (R)-N-(1-(3-chloro-4-fluorophenyl)ethyl)-3-(pyridin-4-yl)-1,7-dihydroimidazo[4,5-f ]indazole-6-carboxamide

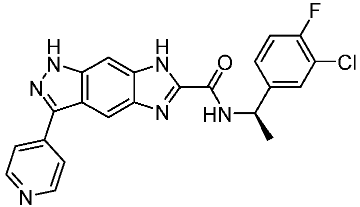

(R)-N-(1-(3-Chloro-4-fluorophenyl)ethyl)-3-(pyridin-4-yl)-1,7-dihydroimidazo[4,5 -f]indazole-6-carboxamide was obtained from methyl 3-(pyridin-4-yl)-1-trityl-1,7-dihydroimidazo[4,5-f]indazole-6-carboxylate and (R)-1-(3-chloro-4-fluorophenyl)ethan-1-amine as the starting materials in accordance with Steps 6 and 7 of Example 2.
¹H NMR (400 MHz, DMSO-*d*₆) δ 13.42 (s, 1H), 9.46 (d*, J* = 8.4 Hz, 1H), 8.65 (d, *J* = 4.9 Hz, 2H), 8.27 (s, 5H), 7.96 (d, *J* = 5.3 Hz, 2H), 7.66 (dd, *J* = 7.2, 1.9 Hz, 2H), 7.48 - 7.38 (m, 1H), 7.29 (ddd, *J* = 25.1, 12.7, 7.0 Hz, 2H), 5.23 - 5.11 (m, 1H), 1.49 (d, *J=* 7.0 Hz, 3H).
MS m/z (ESI): 435.1, 437.1 [M+H]⁺.

### Example 64

### Preparation of (S)-(3-phenylmorpholino)(3-(pyridin-4-yl)-1,7-dihydroimidazo[4,5-f]indazol-6-yl)m ethanone

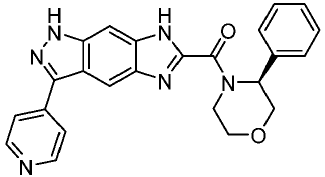

(S)-(3-Phenylmorpholino)(3-(pyridin-4-yl)-1,7-dihydroimidazo[4,5-f]indazol-6-yl) methanone was obtained from methyl 3-(pyridin-4-yl)-1-trityl-1,7-dihydroimidazo[4,5-f]indazole-6-carboxylate and (S)-3-phenylmorpholine as the starting materials in accordance with Steps 6 and 7 of Example 2.
¹H NMR (400 MHz, DMSO-*d*₆) δ 13.51 - 13.00 (m, 2H), 9.26 - 9.17 (m, 1H), 8.72 (d, *J* = 4 Hz, 2H), 8.50 (s, 0.6H), 8.07 - 7.91 (m, 2.7H), 7.59 (s, 0.6H), 7.45 (d, *J=* 8 Hz, 2H), 7.35 (t, *J=* 8 Hz, 2H), 7.28 - 7.23 (m, 1H), 5.21-5.16 (m, 1H), 4.62-4.58 (m, 1H), 3.87 -3.82 (m, 1H), 3.62 -3.40 (m, 4H).
MS m/z (ESI): 425.1 [M+H]⁺.

### Example 65

### (S)-(2-Phenylaziridin-1-yl)(3-(pyridin-4-yl)-1,7-dihydroimidazo [4,5-f]indazol-6-yl) methanone

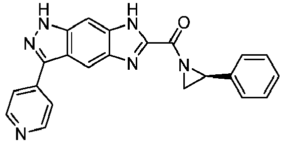

### Step 1: Preparation of (S)-N-(2-hydroxy-1-phenylethyl)-3-(pyridin-4-yl)-1-trityl-1,7-dihydroimidazo[4,5-f] indazole-6-carboxamide

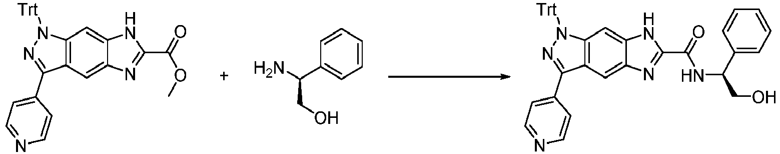

Methyl 3-(pyridin-4-yl)-1-trityl-1,7-dihydroimidazo[4,5-f]indazole-6-carboxylate (200 mg, 0.37 mmol) and (S)-2-amino-2-phenylethan-1-ol (205 mg, 1.49 mmol) were reacted in N-methylpyrrolidone (2 mL) under a microwave condition at 150°C for one hour. The reaction solution was concentrated and purified by column chromatography [eluent: dichloromethane ~ dichloromethane/methanol (97:3)] to obtain 230 mg of crude product, which was further purified by thin layer chromatography (developing solvent: CH₂Cl₂/MeOH=12/1) to obtain a yellow solid product (S)-N-(2-hydroxy-1-phenyl ethyl)-3-(pyridin-4-yl)-1-trityl-1,7-dihydroimidazo[4,5-f]ind azole-6-carboxamide (120 mg, yield: 50%).
MS m/z (ESI): 641.2 [M+H]+.

### Step 2: Preparation of (S)-(2-phenylaziridin-1-yl)(3-(pyridin-4-yl)-1-trityl-1,7-dihydroimidazo [4,5-f]indaz ol-6-yl)methanone

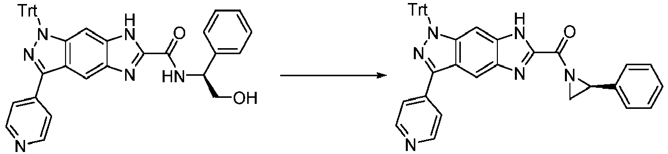

The solution of (S)-N-(2-hydroxy-1-phenyl ethyl)-3-(pyridin-4-yl)-1-trityl-1,7-dihydroimidazo[4,5-f]ind azole-6-carboxamide (70 mg, 0.11 mmoL) and triphenylphosphine (57 mg, 0.22 mmol) in tetrahydrofuran (5 mL) was added with the solution of diisopropyl azodicarboxylate (44 mg, 0.22 mmol) in tetrahydrofuran (1 mL) at 0°C. After completion of the addition, the reaction solution was stirred at room temperature for 18 hours. The reaction was quenched by water (20 mL), and then extracted with ethyl acetate (20 mL*2). The organic phases were combined, concentrated and purified by thin layer chromatography (developing solvent: CH₂Cl₂/MeOH=10/1) to obtain a yellow solid product (S)-(2-phenylaziridin-1-yl)(3-(pyridin-4-yl)-1-trityl-1,7-dihydroimidazo[4,5-f]indazol-6 -yl)methanone (30 mg, yield: 44%).
MS m/z (ESI): 623.2 [M+H]+.

### Step 3: Preparation of (S)-(2-Phenylaziridin-1-yl)(3-(pyridin-4-yl)-1,7-dihydroimidazo[4,5-f]indazol-6-yl) methanone

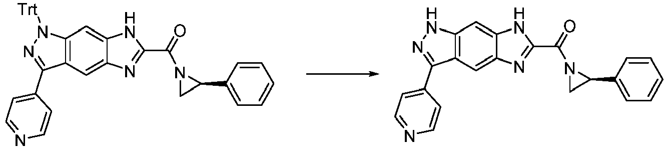

The solution of (S)-(2-phenylaziridin-1-yl)(3-(pyridin-4-yl)-1-trityl-1,7-dihydroimidazo[4,5-f]indazol-6 -yl)methanone (30 mg, 0.048 mmol) in dichloromethane (2 mL) was added with trifluoroacetic acid (2 mL). The reaction solution was stirred at room temperature for one hour. The reaction solution was added dropwise to an aqueous sodium bicarbonate solution (20 mL). After completion of the addition, the solution was extracted with ethyl acetate (20 mL*2). The organic phases were combined, concentrated and purified by thin layer chromatography (developing solvent: CH₂Cl₂/MeOH=8/1) to obtain a yellow solid product (S)-(2-Phenylaziridin-1-yl)(3-(pyridin-4-yl)-1,7-dihydroimidazo[4,5-f]indazol-6-yl)met hanone (6.2 mg, yield: 34%).
¹H NMR (400 MHz, DMSO-*d*₆) δ 13.50 - 13.00 (m, 2H), 8.72 (d, *J* = 4 Hz, 2H), 8.49 (s, 0.6H), 8.08 - 7.93 (m, 2.8H), 7.59 (s, 0.6H), 7.44 (d, *J* = 8 Hz, 2H), 7.37 (t, *J* = 8 Hz, 2H), 7.29 - 7.23 (m, 1H), 2.96-2.84 (m, 1H), 2.02 (d, *J=* 8 Hz, 1H), 1.75 (d, *J=* 8 Hz, 1H).
MS m/z (ESI): 381.1 [M+H]+.

### Example 66

### Preparation of (R)-N-(2-phenylpropyl)-3-(pyridin-4-yl)-1,7-dihydroimidazo[4,5-flindazole-6-carbo xamide

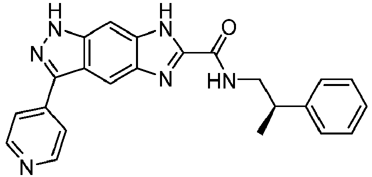

### Step 1: (R)-N-(2-Phenylpropyl)-3-(pyridin-4-yl)-1-trityl-1,7-dihydroimidazo[4,5-f]indazole -6-carboxamide

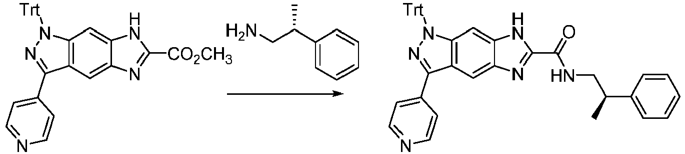

Methyl 3-(pyridin-4-yl)-1-trityl-1,7-dihydroimidazo[4,5-f]indazole-6-carboxylate (240 mg, 0.45 mmol) was dissolved in (R)-2-phenylpropan-1-amine (5 mL). The solution was added to a microwave reaction tube (30 mL), and stirred under a microwave condition at 150°C for 2 h. The reaction solution was cooled, concentrated to dryness, and purified by column chromatography to obtain a brown solid product (R)-N-(2-phenylpropyl)-3-(pyridin-4-yl)-1-trityl-1, 7-dihydroimidazo[4,5-f]indazole-6-c arboxamide (234 mg, yield: 81%).
MS m/z (ESI): 639.2 [M+H]⁺.

### Step 2: Preparation of (R)-N-(2-phenylpropyl)-3-(pyridin-4-yl)-1,7-dihydroimidazo[4,5-flindazole-6-carbo xamide

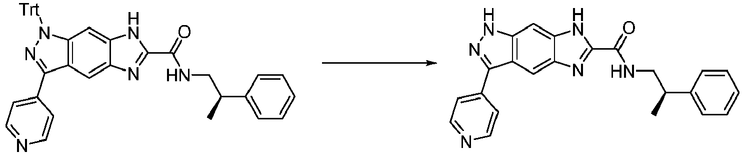

(R)-N-(2-Phenylpropyl)-3-(pyridin-4-yl)-1-trityl-1,7-dihydroimidazo[4,5-f]indazol e-6-carboxamide (234 mg, 0.37 mmol) was dissolved in dichloromethane (20 mL), and added with trifluoroacetic acid (5 mL). The reaction solution was stirred at room temperature for 2 hours. At the end of the reaction, the reaction solution was concentrated to remove the solvent and obtain a crude product. The crude product was dissolved in 10 mL of methanol, added with ammonia/methanol (5mL, 7M), and stirred for half an hour. The reaction solution was concentrated to dryness, and purified by column chromatography to obtain a white solid product (R)-N-(2-phenylpropyl)-3-(pyridin-4-yl)-1,7-dihydroimidazo[4,5-f]indazole-6-carboxa mide (38 mg, yield: 26%).
¹H NMR (400 MHz, DMSO) δ 13.26 (d, *J* = 13.4 Hz, 2H), 8.71 (d, *J* = 4.6 Hz, 2H), 8.34 (d*, J* = 5.5 Hz, 1H), 8.03 (s, 2H), 7.60 (s, 1H), 7.36 - 7.19 (m, 5H), 3.44 - 3.40 (m, 2H), 3.16 (dt, *J=* 14.0, 7.0 Hz, 1H), 1.25 (d, *J=* 7.0 Hz, 3H).
MS m/z (ESI): 397.2 [M+H]⁺.

### Example 67

### Preparation of (S)-N-(2-phenylpropyl)-3-(pyridin-4-yl)-1,7-dihydroimidazo[4,5-f]indazole-6-carbo xamide

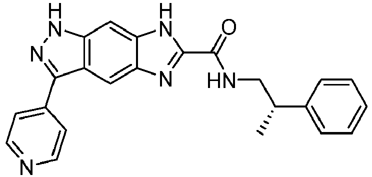

### Step 1: (S)-N-(2-Phenylpropyl)-3-(pyridin-4-yl)-1-trityl-1,7-dihydroimidazo[4,5-f]indazole-6-carboxamide

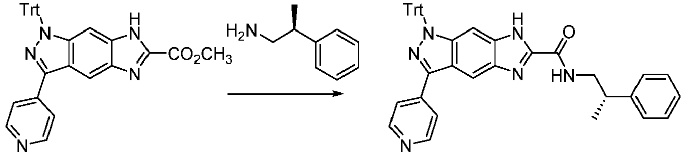

Methyl 3-(pyridin-4-yl)-1-trityl-1,7-dihydroimidazo[4,5-f]indazole-6-carboxylate (240 mg, 0.45 mmol) was dissolved in (R)-2-phenylpropan-1-amine (5 mL). The solution was added to a microwave reaction tube (30 mL), and stirred under a microwave condition at 150°C for 2 h. The reaction solution was cooled, concentrated to dryness, and purified by column chromatography to obtain a brown solid product (S)-N-(2-Phenylpropyl)-3-(pyridin-4-yl)-1-trityl-1, 7 -dihydroimidazo[4,5-f]indazole-6-c arboxamide (205 mg, yield: 71%).
MS m/z (ESI): 639.2 [M+H]⁺.

### Step 2: Preparation of (S)-N-(2-phenylpropyl)-3-(pyridin-4-yl)-1,7-dihydroimidazo[4,5-flindazole-6-carbo xamide

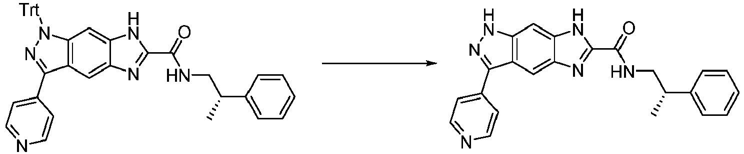

(R)-N-(2-Phenylpropyl)-3-(pyridin-4-yl)-1-trityl-1,7-dihydroimidazo[4,5-f]indazol e-6-carboxamide (205 mg, 0.32 mmol) was dissolved in dichloromethane (20 mL), and added with trifluoroacetic acid (5 mL). The reaction solution was stirred at room temperature for 2 hours. At the end of the reaction, the reaction solution was concentrated to remove the solvent and obtain a crude product. The crude product was dissolved in 10 mL of methanol, added with ammonia/methanol (5mL, 7M), and stirred for half an hour. The reaction solution was concentrated to dryness, and purified by column chromatography to obtain a white solid product (R)-N-(2-phenylpropyl)-3-(pyridin-4-yl)-1,7-dihydroimidazo[4,5-f]indazole-6-carboxa mide (24 mg, yield: 19%).
¹H NMR (400 MHz, DMSO-*d*₆) δ 13.36 (s, 2H), 8.65 (d, *J* = 4.6 Hz, 2H), 8.28 (d, *J* = 5.5 Hz, 1H), 7.97 (s, 2H), 7.54 (s, 1H), 7.28 - 7.20 (m, 4H), 7.17 - 7.11 (m, 1H), 3.54 - 3.46 (m, 2H), 3.09 (dd, *J=* 14.2, 7.0 Hz, 1H), 1.19 (d, *J=* 7.0 Hz, 3H).
MS m/z (ESI): 397.3 [M+H]⁺.

### Example 68

### Preparation of (R)-N-(1-morpholinopropan-2-yl)-3-(pyridin-4-yl)-1,7-dihydroimidazo[4,5-f]indazo le-6-carboxamide

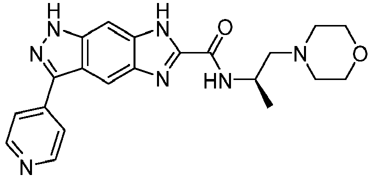

(R)-N-(1-Morpholinopropan-2-yl)-3-(pyridin-4-yl)-1,7-dihydroimidazo[4,5-f]indaz ole-6-carboxamide was obtained from methyl 3-(pyridin-4-yl)-1-trityl-1,7-dihydroimidazo[4,5-f]indazole-6-carboxylate and (R)-1-morpholinopropan-2-amine as the starting materials in accordance with Steps 6 and 7 of Example 2.
¹HNMR (400 MHz, CDCl3) δ 8.75 (s, 2H), 8.10 (s, 1H), 7.89 (s, 2H), 7.72 (s, 1H), 6.50 (s, 1H), 4.01 (s, 1H), 3.56 (s, 3H), 2.92 - 2.43 (m, 2H), 2.58 (s, 2H), 2.58 (s, 2H), 1.31 (s, 3H).
MS m/z (ESI): 406.2 [M+H]⁺.

### Example 69

### Preparation of 3-(1-phenylethyl)-5-(3-(pyridin-4-yl)-1-trityl-1,7-dihydroimidazo[4,5-f]indazol-6-yl) -1,2,4-oxadiazole

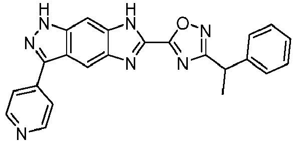

### Step 1: Preparation of (Z)-N'-hydroxy-2-phenylpropanimidamide

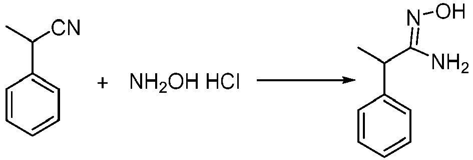

2-Phenylpropanenitrile (1.0 g, 7.6 mmol) was dissolved in ethanol (15 mL)/water (5 mL). The solution was added with sodium carbonate (1.62 g, 15.28 mmol) and hydroxylamine hydrochloride (1.06 g, 15.25 mmol), and stirred at 80°C for 4 h. The reaction solution was cooled, added with 50 mL of water, and extracted with dichloromethane. The organic phases were combined, dried over anhydrous sodium sulfate and filtered. The solution was concentrated to dryness to obtain a white solid product (Z)-N'-hydroxy-2-phenylpropanimidamide (1.10 g, yield: 88%).
MS m/z (ESI): 165.1 [M-H]⁺.

### Step 2: Preparation of 3-(1-phenylethyl)-5-(3-(pyridin-4-yl)-1-trityl-1,7-dihydroimidazo[4,5-f]indazol-6-yl) -1,2,4-oxadiazole

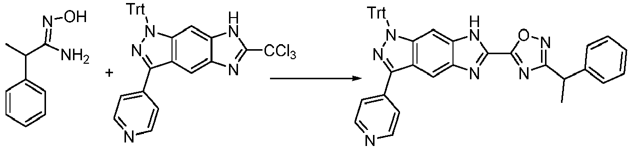

3-(Pyridin-4-yl)-6-(trichloromethyl)-1-trityl-1,7-dihydroimidazo[4,5-f]indazole (100 mg, 0.16 mmol), (Z)-N'-hydroxy-2-phenylpropanimidamide (28 mg, 0.17 mmol), sodium carbonate solution (2 mL) and acetonitrile (10 mL) were added successively to a 50 mL flask. The reaction solution was heated to 60°C and stirred for 2 h. The reaction solution was cooled, added with water (50 mL), and extracted with dichloromethane. The organic phases were combined, dried over anhydrous sodium sulfate, filtered, and concentrated to dryness. The resulting residues were purified by column chromatography to obtain a crude thick product (45 mg).
MS m/z (ESI): 650.2[M-H]⁺.

### Step 3: Preparation of 3-(1-phenylethyl)-5-(3-(pyridin-4-yl)-1-trityl-1,7-dihydroimidazo[4,5-f]indazol-6-yl) -1,2,4-oxadiazole

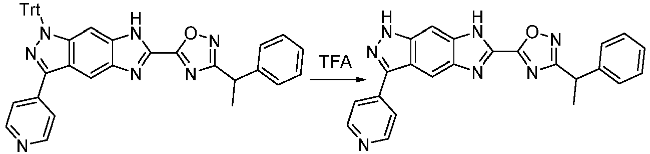

The thick substance 3-(1-phenylethyl)-5-(3-(pyridin-4-yl)-1-trityl-1,7-dihydroimidazo[4,5-f]indazol-6-yl)-1, 2,4-oxadiazole (45 mg) obtained in the previous step was dissolved in dichloromethane (1 mL), and added with trifluoroacetic acid (2 mL). The reaction solution was stirred at room temperature for 1 h. The reaction solution was concentrated to dryness, and purified by column chromatography to obtain a solid product 3-(1-phenylethyl)-5-(3-(pyridin-4-yl)-1-trityl-1,7-dihydroimidazo[4,5-f]indazol-6-yl)-1, 2,4-oxadiazole (4.3 mg, yield in two steps: 6.3%).
¹H NMR (400 MHz, DMSO-*d*₆) δ: 13.43-13.37 (m, 1H), 13.20-13.05 (m, 1H), 8.72 (s, 2H), 8.45 - 8.04 (m, 3H), 7.99-7.87(m,1H), 7.58(m, 2H), 7.36 - 7.32 (m, 2H), 7.26-7.22(m, 1H), 5.25-5.21 (m, 1H), 1.47 (d, *J =* 6.0 Hz, 3H).
MS m/z (ESI): 408.1[M-H]⁺.

### Example 70

### Preparation of (R)-N-(1-(cyclopropylamino)propan-2-yl)-3-(pyridin-4-yl)-1,7-dihydroimidazo[4,5-f]indazole-6-carboxamide

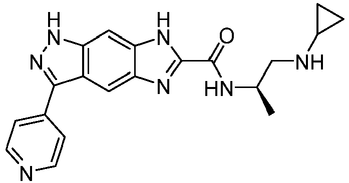

(R)-N-(1-(Cyclopropylamino)propan-2-yl)-3-(pyridin-4-yl)-1,7-dihydroimidazo[4, 5-f]indazole-6-carboxamide was obtained from methyl 3-(pyridin-4-yl)-1-trityl-1,7-dihydroimidazo[4,5-f]indazole-6-carboxylate and (R)-N¹-cyclopropylpropane-1,2-diamine as the starting materials in accordance with Steps 6 and 7 of Example 2.
¹H NMR (400 MHz, CDCl3) δ 13.18 (s, 1H), 8.64 - 7.12 (m, 7H), 3.94 (s, 1H), 2.52 (d, *J=* 3.9 Hz, 2H), 2.27 (s, 3H), 1.89 (s, 1H), 0.96 (d, *J=* 4.7 Hz, 3H).
MS m/z (ESI): 376.1 [M+H]⁺.

### Example 71

### Preparation of N-(3-hydroxy-1-phenylpropyl)-3-(pyridin-4-yl)-1,7-dihydroimidazo [4,5-f]indazole-6-carboxamide

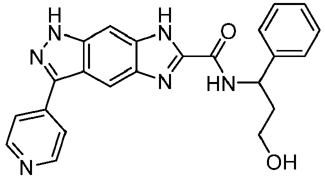

N-(3-Hydroxy-1-phenylpropyl)-3-(pyridin-4-yl)-1,7-dihydroimidazo[4,5-f]indazol e-6-carboxamide was obtained from methyl 3-(pyridin-4-yl)-1-trityl-1,7-dihydroimidazo[4,5-f]indazole-6-carboxylate and 3-amino-3-phenylpropan-1-ol as the starting materials in accordance with Steps 6 and 7 of Example 2.
¹H NMR (400 MHz, DMSO-*d*₆) δ 13.56 - 13.03 (m, 2H), 9.25 - 9.14 (m, 1H), 8.70 (d, *J* = 4 Hz, 2H), 8.49 (s, 0.6H), 8.06 - 7.90 (m, 2.7H), 7.57 (s, 0.6H), 7.46 (d, *J* = 8 Hz, 2H), 7.35 (t, *J* = 8 Hz, 2H), 7.28 - 7.23 (m, 1H), 5.14 - 5.09 (m, 1H), 4.50 (s, 1H), 3.85-3.80 (m, 2H), 2.14-2.03 (m, 2H).
MS m/z (ESI): 413.1 [M+H]⁺.

### Example 72

### Preparation of N-(2-oxo-2-phenylethyl)-3-(pyridin-4-yl)-1,7-dihydroimidazo[4,5-f]indazole-6-carb oxamide

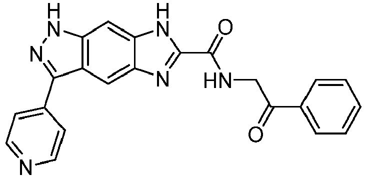

N-(2-Oxo-2-phenylethyl)-3-(pyridin-4-yl)-1,7-dihydroimidazo[4,5-f]indazole-6-ca rboxamide was obtained from methyl 3-(pyridin-4-yl)-1-trityl-1,7-dihydroimidazo[4,5-f]indazole-6-carboxylate and 2-amino-1-phenylethan-1-one as the starting materials in accordance with Steps 6 and 7 of Example 2.
¹H NMR (400 MHz, CDCl3) δ 8.75 (s, 2H), 8.33 (s, 1H), 8.09 (s, 2H), 7.90 (d, *J*= 7.3 Hz, 3H), 7.68 (s, 1H), 7.57 (s, 2H), 7.34 (s, 1H), 4.36 (s, 2H).
MS m/z (ESI): 397.1 [M+H]⁺.

### Example 73

### Preparation of N'-benzyl-3-(pyridin-4-yl)-1,7-dihydroimidazo [4,5-f]indazole-6-carbohydrazide

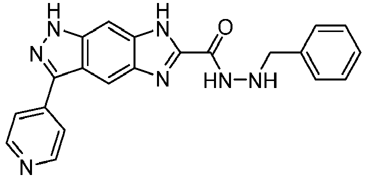

N'-Benzyl-3-(pyridin-4-yl)-1,7-dihydroimidazo[4,5-f]indazole-6-carbohydrazide was obtained from methyl 3-(pyridin-4-yl)-1-trityl-1,7-dihydroimidazo[4,5-f]indazole-6-carboxylate and benzylhydrazine as the starting materials in accordance with Steps 6 and 7 of Example 2.
¹H NMR (400 MHz, DMSO-*d*₆) δ 13.42-13.22 (m, 2H), 10.09 (s, 1H), 8.74 (d, *J* = 4 Hz, 2H), 8.49 (s, 1H), 8.07 (s, 2H), 7.62 (s, 1H), 7.29-7.18 (m, 2H), 6.88 (d, *J* = 8.0 Hz, 2H), 6.80 (t, *J=* 8 Hz, 1H), 5.09 (s, 1H), 3.91 (s, 2H).
MS m/z (ESI): 384.1 [M+H]⁺.

### Example 74

### Preparation of N-((1-methyl-1H-pyrazol-4-yl)methyl)-3-(pyridin-4-yl)-1,7-dihydroimidazo[4,5-f]in dazole-6-carboxamide

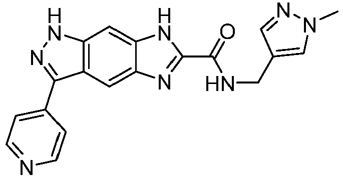

N-((1-Methyl-1H-pyrazol-4-yl)methyl)-3-(pyridin-4-yl)-1,7-dihydroimidazo[4,5-f] indazole-6-carboxamide was obtained from methyl 3-(pyridin-4-yl)-1-trityl-1,7-dihydroimidazo[4,5-f]indazole-6-carboxylate and (1-methyl-1H-pyrazol-4-yl)methylamine as the starting materials in accordance with Steps 6 and 7 of Example 2.
¹H NMR (400 MHz, DMSO-*d*₆) δ 13.58 (s, 1H), 9.31 (d*, J* = 7.8 Hz, 1H), 8.77 (d, *J* = 5.4 Hz, 2H), 8.37 (s, 1H), 8.18 (d, *J* = 5.4 Hz, 2H), 7.72 (s, 1H), 7.64 (s, 1H), 7.41 (s, 1H), 4.37 (d, *J* = 6.2 Hz, 2H), 3.80 (s, 3H).
MS m/z (ESI): 373.1 [M+H]⁺.

### Example 75

### Preparation of (3-phenylpiperazin-1-yl)(3-(pyridin-4-yl)-1,7-dihydroimidazo[4,5-f]indazol-6-yl)me thanone

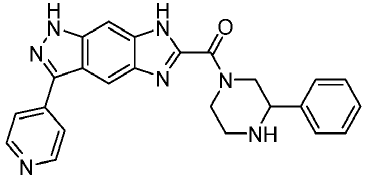

(3-Phenylpiperazin-1-yl)(3-(pyridin-4-yl)-1,7-dihydroimidazo[4,5-f]indazol-6-yl) methanone was obtained from methyl 3-(pyridin-4-yl)-1-trityl-1,7-dihydroimidazo[4,5-f]indazole-6-carboxylate and 2-phenylpiperazine as the starting materials in accordance with Steps 6 and 7 of Example 2.
¹H NMR (400 MHz, CDCl3) δ 8.74 (s, 2H), 8.28 (s, 1H), 7.89 (d, *J* = 5.5 Hz, 3H), 7.45-7.14 (m, 5H), 4.04 (s, 1H), 3.86 (s, 1H), 3.58 - 3.24 (m, 3H), 2.83 (d*, J* = 5.0 Hz, 2H).
MS m/z (ESI): 424.2 [M+H]⁺.

### Example 76

### Preparation of N'-phenyl-3-(pyridin-4-yl)-1,7-dihydroimidazo[4,5-f]indazole-6-carbohydrazide

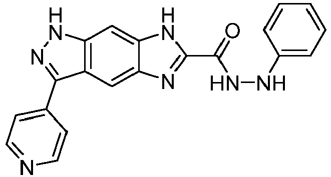

### Step 1: Preparation of N'-phenyl-3-(pyridin-4-yl)-1-trityl-1,7-dihydroimidazo[4,5-f]indazole-6-carbohydr azide

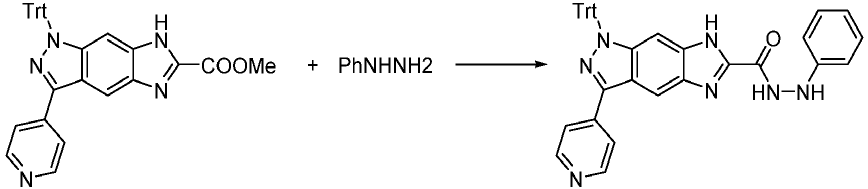

Methyl 3-(pyridin-4-yl)-1-trityl-1,7-dihydroimidazo[4,5-f]indazole-6-carboxylate (400 mg, 0.74 mmol) and 4 mL of phenylhydrazine were added to a microwave reaction tube. The reaction solution was heated to 140°C by microwave for one hour. 50 mL of ethyl acetate was added to the reaction solution. The ethyl acetate layer was washed with saturated NH₄Cl solution and saturated NaCl solution successively, dried over anhydrous sodium sulfate, and purified by column chromatography to obtain a crude product N'-phenyl-3-(pyridin-4-yl)-1-trityl-1,7-dihydroimidazo[4,5-f]indazole-6-carbohydrazide (290 mg), which was used directly in the next step.
MS m/z (ESI): 612.1 [M+H]⁺.

### Step 2: Preparation of N'-phenyl-3-(pyridin-4-yl)-1,7-dihydroimidazo[4,5-f]indazole-6-carbohydrazide

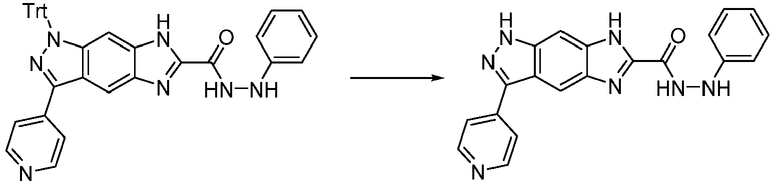

N'-Phenyl-3-(pyridin-4-yl)-1-trityl-1,7-dihydroimidazo[4,5-f]indazole-6-carbohydr azide (290 mg, 0.47 mmol) obtained in the above step was dissolved in a mixed solvent of dichloromethane and trifluoroacetic acid (4 mL:4 mL). The reaction solution was reacted at room temperature for 2 hours. The reaction solution was concentrated to dryness, added with 10 mL of methanol, and placed in an ice bath. The residual trifluoroacetic acid was neutralized with a 7.0 M solution of NH₃ in methanol. The resulting solution was concentrated to dryness, and purified by column chromatography to obtain a crude product, which was pulped in ethyl acetate to obtain a yellow solid N'-phenyl-3-(pyiidin-4-yl)-1,7-dihydroimidazo[4,5-f]indazole-6-carbohydrazide (80 mg, yield in two steps: 29%).
¹H NMR (400 MHz, DMSO-*d*₆) δ 13.52 (s, 1H), 13.22 (s, 1H), 10.87 (s, 1H), 8.75 (d, *J* = 5.7 Hz, 2H), 8.49 (s, 1H), 8.15 - 8.12 (m, 2H), 7.72 (s, 1H), 7.18 (t, *J* = 7.9 Hz, 2H), 6.82 - 6.73 (m, 3H), 3.41 (s, 1H).
MS m/z (ESI): 370.1 [M+H]⁺.

### Example 77

### Preparation of N'-methyl-N'-phenyl-3-(pyridin-4-yl)-1,7-dihydroimidazo[4,5-f]indazole-6-carbohy drazide

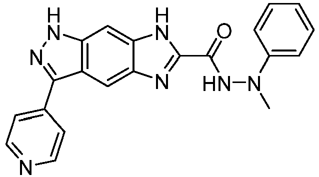

N'-Methyl-N'-phenyl-3-(pyridin-4-yl)-1,7-dihydroimidazo[4,5-f]indazole-6-carboh ydrazide was obtained from methyl 3-(pyridin-4-yl)-1-trityl-1,7-dihydroimidazo[4,5-f]indazole-6-carboxylate and 1-methyl-1-phenylhydrazine as the starting materials in accordance with Example 76.
¹H NMR (400 MHz, DMSO-*d*₆) δ 13.42-13.22 (m, 2H), 11.09 (s, 1H), 8.72 (d, *J* = 4 Hz, 2H), 8.49 (s, 1H), 8.05 (s, 2H), 7.62 (s, 1H), 7.29 -7.17 (m, 2H), 6.87 (d, *J* = 8.0 Hz, 2H), 6.79 (t, *J* = 8 Hz, 1H), 3.23 (s, 3H).
MS m/z (ESI): 384.1 [M+H]⁺.

### Example 78

### Preparation of N-(2-methoxy-2-phenylethyl)-3-(pyridin-4-yl)-1,7-dihydroimidazo[4,5-f]indazole-6-carboxamide

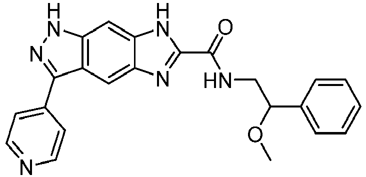

### Step 1: Preparation of N-(2-methoxy-2-phenylethyl)-3-(pyridin-4-yl)-1-trityl-1,7-dihydroimidazo[4,5-f]ind azole-6-carboxamide

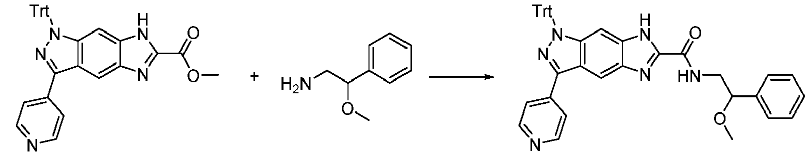

Methyl 3-(pyridin-4-yl)-1-trityl-1,7-dihydroimidazo[4,5-f]indazole-6-carboxylate (100 mg, 0.19 mmol) and 2-methoxy-2-phenylethan-1-amine (1 mL) were added to a microwave reaction tube. The reaction solution was heated to 150°C for 60 min. After cooling to room temperature, 20 mL of ethyl acetate was added to the reaction solution. The ethyl acetate layer was washed with saturated ammonium chloride solution and saturated sodium chloride solution, dried over anhydrous sodium sulfate, and purified by column chromatography to obtain a crude product N-(2-methoxy-2-phenylethyl)-3-(pyridin-4-yl)-1-trityl-1,7-dihydroimidazo[4,5-f]indazo le-6-carboxamide (80 mg, yield: 67%), which was used directly in the next step.
MS m/z (ESI): 655.3 [M+H]⁺.

### Step 2: Preparation of N-(2-methoxy-2-phenylethyl)-3-(pyridin-4-yl)-1,7-dihydroimidazo[4,5-f]indazole-6-carboxamide

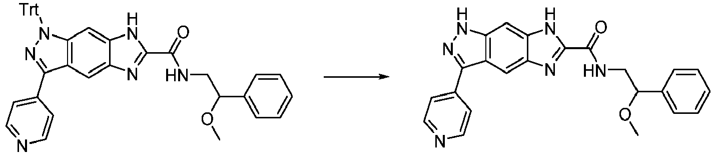

The crude N-(2-methoxy-2-phenylethyl)-3-(pyridin-4-yl)-1-trityl-1,7-dihydroimidazo[4,5-f]indazo le-6-carboxamide (80 mg) was dissolved in 4 mL of dichloromethane, and added with 4 mL of trifluoroacetic acid at the same time. The reaction solution was reacted at room temperature for 3 hours, followed by concentrating to dryness. The resulting crude product was dissolved in a mixed solution of ethyl acetate and tetrahydrofuran. The solution was washed with saturated sodium bicarbonate solution and saturated sodium chloride solution, and dried over anhydrous sodium sulfate. The resulting organic solution was concentrated to dryness, and purified by preparative chromatography to obtain a yellow solid product N-(2-methoxy-2-phenylethyl)-3-(pyridin-4-yl)-1,7-dihydroimidazo[4,5-f]indazole-6-car boxamide (10 mg, yield: 20%).
¹H NMR (400 MHz, DMSO-*d*₆) δ 13.47 - 13.11 (m, 2H), 8.86 - 8.70 (m, 3H), 8.49 (s, 0.7H), 8.12 (s, 0.3H), 8.08 - 7.98 (m, 2H), 7.88 (s, 0.3H), 7.58 (s, 0.7H), 7.42 - 7.33 (m, 5H), 4.54 - 4.51 (m, 1H), 3.63 - 3.53 (m, 2H), 3.20 (d, *J=* 4 Hz, 3H).
MS m/z (ESI): 413.2 [M+H]⁺.

### Example 79

### Preparation of N'-(3-fluorophenyl)-3-(pyridin-4-yl)-1,7-dihydroimidazo[4,5-f]indazole-6-carbohyd razide

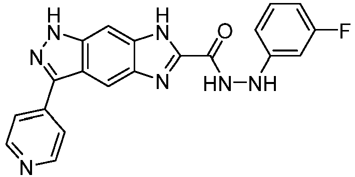

N'-(3-Fluorophenyl)-3-(pyridin-4-yl)-1,7-dihydroimidazo[4,5-f]indazole-6-carbohy drazide was obtained from methyl 3-(pyridin-4-yl)-1-trityl-1,7-dihydroimidazo[4,5-f]indazole-6-carboxylate and (3-fluorophenyl)hydrazine as the starting materials in accordance with Example 76.
¹H NMR (400 MHz, CDCl₃) δ 8.75 (s, 2H), 8.32 (s, 1H), 7.90 (d, *J=* 11.4 Hz, 3H), 7.38 (s, 1H), 7.22 (s, 1H), 7.00 (s, 1H), 6.83 (s, 1H), 6.55 (s, 1H), 3.17 (s, 1H). MS m/z (ESI): 388.1 [M+H]⁺.

### Example 80

### Preparation of N'-(4-fluorophenyl)-3-(pyridin-4-yl)-1,7-dihydroimidazo[4,5-f]indazole-6-carbohyd razide

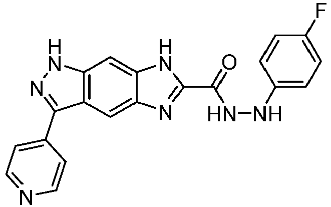

N'-(4-Fluorophenyl)-3-(pyridin-4-yl)-1,7-dihydroimidazo[4,5-f]indazole-6-carbohy drazide was obtained from methyl 3-(pyridin-4-yl)-1-trityl-1,7-dihydroimidazo[4,5-f]indazole-6-carboxylate and (4-fluorophenyl)hydrazine as the starting materials in accordance with Example 76.
¹H NMR (400 MHz, DMSO-*d*₆) 813.40 (s, 1H), 13.10 (s, 1H), 9.48 - 9.38 (m, 2H), 8.71 - 8.64 (m, 2H), 8.13 (s, 1H), 7.99 (s, 1H), 7.96 - 7.89 (m, 2H), 7.09 - 7.00 (m, 2H), 6.83 (ddt, *J* = 6.4, 5.0, 1.5 Hz, 2H).
MS m/z (ESI): 388.1 [M+H]⁺.

### Example 81

### Preparation of N'-(3-chloro-4-fluorophenyl)-3-(pyridin-4-yl)-1,7-dihydroimidazo[4,5-f]indazole-6-carbohydrazide

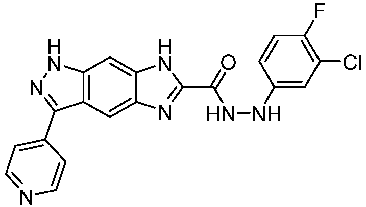

N'-(3-Chloro-4-Fluorophenyl)-3-(pyridin-4-yl)-1,7-dihydroimidazo[4,5-f]indazole-6-carbohydrazide was obtained from methyl 3-(pyridin-4-yl)-1-trityl-1,7-dihydroimidazo[4,5-f]indazole-6-carboxylate and (3-chloro-4-fluorophenyl)hydrazine as the starting materials in accordance with Example 76.
¹H NMR (400 MHz, DMSO-*d*₆) δ 13.52 (s, 1H), 13.22 (s, 1H), 10.87 (s, 1H), 8.75 (d, *J=* 5.7 Hz, 2H), 8.49 (s, 1H), 8.15 - 8.12 (m, 2H), 7.72 (s, 1H), 7.25 - 7.17 (m, 1H), 7.05 - 6.99 (m, 1H), 6.77 - 6.69 (m, 1H).
MS m/z (ESI): 422.1 424.1 [M+H]⁺.

### Example 82

### Preparation of (R)-3-morpholino-N-(1-phenylethyl)-1,7-dihydroimidazo [4,5-f]indazole-6-carboxa mide

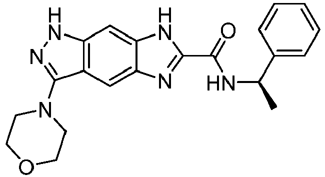

### Step 1: Preparation of 4-(5,6-dinitro-1-trityl-1H-indazol-3-yl)morpholine

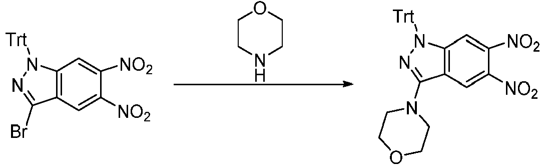

Morpholine (383.8 mg, 4.1 mmol, 1.1 eq.), Pd(OAc)₂ (179.61mg, 0.8 mmol, 0.2 eq) and X-antPhos (555 mg, 0.96 mmol, 0.24 eq) were dissolved in anhydrous dioxane (6.0 mL). The reaction system was purged with nitrogen for 2 minutes. The reaction solution was added to a 15 mL sealed tube, heated to 100°C and stirred for 10 min. The reaction solution was cooled to room temperature, added with the compound 3-bromo-5,6-dinitro-1-trityl-1H-indazole (2.12 g, 4.0 mmol, 1.0 eq) and cesium carbonate (3.91 g, 12 mmol, 3.0 eq), and stirred at room temperature for 5 min. The reaction solution was then heated to 100°C and stirred for 90 min (the reaction process was monitored by TLC and LCMS frequently to avoid side reactions). After completion of the reaction, the solvent was removed, and the residues were extracted with ethyl acetate. The solution was dried, concentrated to remove the solvent and obtain a crude product, which was purified by flash column chromatography to obtain a compound 4-(5,6-dinitro-1-trityl-1H-indazol-3-yl)morpholine (1.2 g, yield: 56%).
MS m/z (ESI): 536.2 [M+H]⁺.

### Step 2: Preparation of 3-morpholino-1-trityl-1H-indazole-5,6-diamine

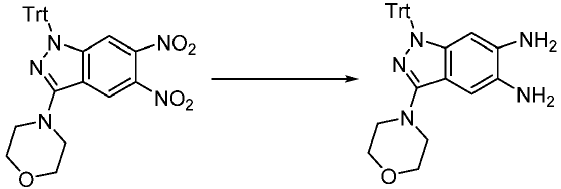

The compound 4-(5,6-dinitro-1-trityl-1H -indazol-3-yl)morpholine (crude, 1.2 g) was dissolved in tetrahydrofuran (120 mL), and added with palladium hydroxide (300 mg). The reaction system was purged with hydrogen three times. The reaction solution was stirred overnight (12 hours) under the pressure of a hydrogen balloon. After completion of the reaction, the reaction solution was filtered, concentrated to remove the solvent and obtain a crude product, which was purified by flash column chromatography to obtain a compound 3-morpholino-1-trityl-1H-indazole-5,6-diamine (400 mg, yield: 38%).
MS m/z (ESI): 476.3 [M+H]⁺.

### Step 3: Preparation of methyl 3-morpholino-1-trityl-1,7-dihydroimidazo[4,5-f]indazole-6-carboxylate

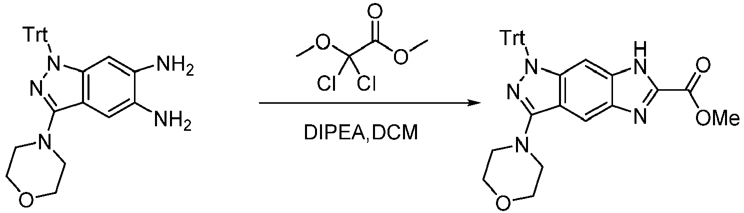

The compound 3-morpholino-1-trityl-1H-indazole-5,6-diamine (400 mg, 0.84 mmol, 1.0 eq) was dissolved in dichloromethane (40 mL). The solution was cooled to 0°C, added with DIPEA (2.1 mL, 12.6 mmol, 15 eq, d=0.782 g/mL) and methyl 2,2-dichloro-2-methoxyacetate (1.45 g, 8.4 mmol, 10 eq), and stirred at room temperature overnight. After completion of the reaction, the reaction solution was added slowly to 100 mL of saturated NaHCO3 solution. The solution was extracted with dichloromethane, dried and purified by column chromatography to obtain a target compound methyl 3-morpholino-1-trityl-1,7-dihydroimidazo[4,5-f]indazole-6-carboxylate (106 mg, yield: 23%).
MS m/z (ESI): 544.23 [M+H]⁺.

### Step 4: Preparation of (R)-3-morpholino-N-(1-phenylethyl)-1-trityl-1,7-dihydroimidazo[4,5-f]indazole-6-c arboxamide

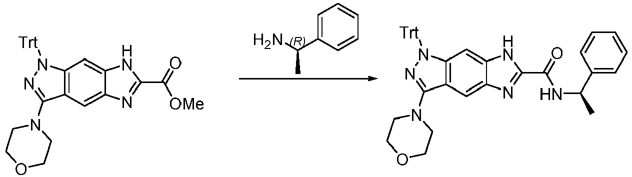

The compound methyl 3-morpholino-1-trityl-1,7-dihydroimidazo[4,5-f]indazole-6-carboxylate (106 mg, 0.19 mmol) was dissolved in (R)-1-phenylethan-1-amine (2 mL). The reaction solution was added to a 30 mL microwave reaction tube, and reacted at 150°C for 3 hours. After completion of the reaction, the reaction solution was dissolved in ethyl acetate (200 mL), and washed with water three times. The solution was dried, concentrated to dryness, and purified by column chromatography to obtain a compound (R)-3-morpholino-N-(1-phenylethyl)-1-trityl-1,7-dihydroimidazo[4,5-f]indazole-6-carb oxamide (100 mg, yield: 81%).
MS m/z (ESI): 633.4 [M+H]⁺.

### Step 5: Preparation of (R)-3-morpholino-N-(1-phenylethyl)-1,7-dihydroimidazo[4,5-f]indazole-6-carboxa mide

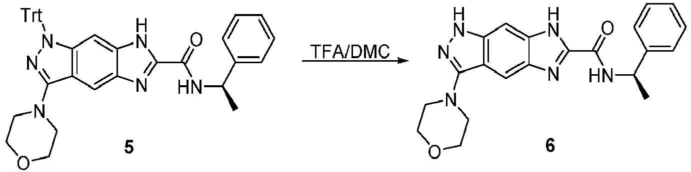

The compound (R)-3-morpholino-N-(1-phenylethyl)-1-trityl-1,7-dihydroimidazo[4,5-f]indazole-6-carb oxamide (100 mg, 0.16 mmol) was dissolved in dichloromethane (10 mL), and added with trifluoroacetic acid (2 mL). The reaction solution was stirred at room temperature for 3 hours. After completion of the reaction, the reaction solution was concentrated to dryness, the resulting residues were dissolved in NH3/MeOH solution (10 mL) and stirred for 30 min. The reaction solution was concentrated to dryness to obtain a crude product, which was purified by preparative HPLC to obtain a target compound (R)-3-morpholino-N-(1-phenylethyl)-1,7-dihydroimidazo[4,5-f]indazole-6-carboxamide (11 mg, yield: 18%).
¹H NMR (400 MHz, CDCl3) δ 7.89 (s, 1H), 7.66 (s, 1H), 7.34 - 7.25 (m, 5H), 7.17 (s, 1H), 4.97 (d, *J=* 12.0 Hz, 1H), 3.70 (t, *J=* 9.2 Hz, 4H), 3.22 (t, *J=* 9.3 Hz, 4H), 1.48 (d*, J* = 11.9 Hz, 3H).
MS m/z (ESI): 391.2 [M+H]⁺.

### Example 83

### Preparation of (R)-3-(methyl(pyridin-3-yl)amino)-N-(1-phenylethyl)-1,7-dihydroimidazo[4,5-f]ind azole-6-carboxamide

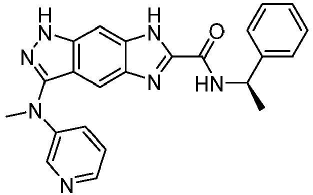

(R)-3-(Methyl(pyridin-3-yl)amino)-N-(1-phenylethyl)-1,7-dihydroimidazo[4,5-f]in dazole-6-carboxamide was prepared in accordance with Example 82.
¹H NMR (400 MHz, DMSO-*d*₆) δ 12.93 (s, 1H), 12.48-12.32 (m, 1H), 9.38-9.27 (m, 1H), 8.29-8.06 (m, 2H), 7.72 (s, 0.3H), 7.45-7.40 (m, 2H), 7.35-7.18 (m, 6.7H), 5.26 -5.12 (m, 1H), 3.49 (d, *J* = 8.0 Hz, 3H), 1.56-1.51 (m, 3H).
MS m/z (ESI): 412.1 [M+H]⁺.

### Example 84

### Preparation of (R)-3-(6-methoxypyridin-3-yl)-N-(1-phenylethyl)-1,7-dihydroimidazo[4,5-f]indazol e-6-carboxamide

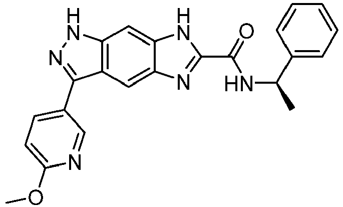

(R)-3-(6-Methoxypyridin-3-yl)-N-(1-phenylethyl)-1,7-dihydroimidazo[4,5-f]indaz ole-6-carboxamide was prepared in accordance with Example 2.
¹H NMR (400 MHz, DMSO-*d*₆) δ 13.15-13.00 (m, 2H), 9.43-9.34 (m, 1H), 8.82-8.76 (m, 1H), 8.33-8.26 (m, 1.6H), 7.96 (s, 0.4H), 7.81 (s, 0.4H), 7.55-7.44 (m, 2.6H), 7.34 (t, *J=* 8 Hz, 2H), 7.26-7.24 (m, 1H), 7.07-7.00 (m, 1H), 5.34-5.11 (m, 1H), 3.95 (s, 3H), 1.57 (d, *J=* 8 Hz, 3H).
MS m/z (ESI): 413.1 [M+H]⁺.

### Example 85

### Preparation of (R)-3-(6-isopropoxypyridin-3-yl)-N-(1-phenylethyl)-1,7-dihydroimidazo[4,5-f]indaz ole-6-carboxamide

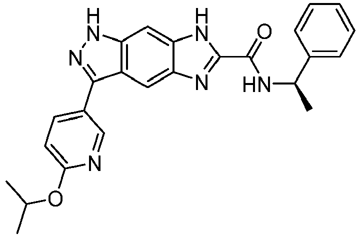

(R)-3-(6-Isopropoxypyridin-3-yl)-N-(1-phenylethyl)-1,7-dihydroimidazo[4,5-f]ind azole-6-carboxamide was prepared in accordance with Example 2.
¹H NMR (400 MHz, DMSO-*d*₆) δ 13.14-13.00 (m, 2H), 9.43-9.34 (m, 1H), 8.78-8.74 (m, 1H), 8.33-8.18 (m, 1.6H), 7.96 (s, 0.4H), 7.80 (s, 0.4H), 7.54-7.42 (m, 2.6H), 7.34 (t, *J=* 8Hz, 2H), 7.26-7.24 (m, 1H), 6.98 - 6.87 (m, 1H), 5.39-5.33 (m, 1H), 5.24-5.21 (m, 1H), 1.56 (d, *J* = 8 Hz, 3H), 1.35 (d, *J* = 8 Hz, 6H).
MS m/z (ESI): 441.2 [M+H]⁺.

### Example 86

### Preparation of (R)-3-(2-methoxypyridin-4-yl)-N-(1-phenylethyl)-1,7-dihydroimidazo[4,5-f]indazol e-6-carboxamide

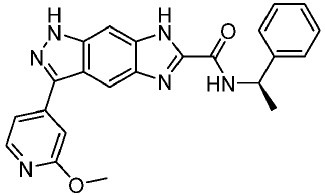

(R)-3-(2-Methoxypyridin-4-yl)-N-(1-phenylethyl)-1,7-dihydroimidazo[4,5-f]indaz ole-6-carboxamide was prepared in accordance with Example 2.
¹H NMR (400 MHz, DMSO-*d*₆) δ 13.42 - 13.07 (m, 2H), 9.46 - 9.34 (m, 1H), 8.35 (s, 0.5H), 8.32 (d, *J* = 4 Hz, 1H), 8.07 (s, 0.4H), 7.86 (s, 0.4H), 7.66 -7.57 (m, 1.6H), 7.48 (d, *J* = 8 Hz, 2H), 7.36 - 7.33 (m, 3H), 7.26 - 7.23 (m, 1H), 5.27 - 5.19 (m, 1H), 3.94 (s, 3H), 1.57 (d, *J* = 8 Hz, 3H).
MS m/z (ESI): 413.2 [M+H]⁺.

### Example 87

### Preparation of (R)-3-(4-methoxypiperidin-1-yl)-N-(1-phenylethyl)-1, 7-dihydroimidazo[4,5-f]indaz ole-6-carboxamide

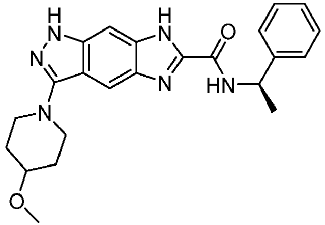

(R)-3-(4-Methoxypiperidin-1-yl)-N-(1-phenylethyl)-1,7-dihydroimidazo[4,5-f]inda zole-6-carboxamide was prepared in accordance with Example 82.
¹H NMR (400 MHz, DMSO-*d*₆) δ 13.65 (s, 1H), 10.80 (s, 1H), 9.41 (d*, J* = 9.3 Hz, 1H), 7.70 (s, 2H), 7.36 - 7.22 (m, 5H), 5.19 - 5.07 (m, 1H), 3.77 - 3.64 (m, 1H), 3.44 - 3.32 (m, 2H), 3.31 (s, 3H), 3.28 - 3.16 (m, 2H), 2.06 - 1.92 (m, 2H), 1.79 - 1.65 (m, 2H), 1.47 (d, *J* = 6.4 Hz, 3H).
MS m/z (ESI): 419.2 [M+H]⁺.

### Example 88 (R)-N-(1-(3-Cyclopropylphenyl)ethyl)-3-(pyridin-4-yl)-1,7-dihydroimidazo[4,5-f]in dazole-6-carboxamide

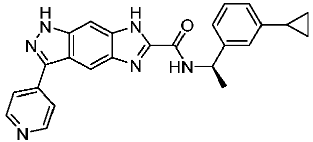

### Step 1: Preparation of tert-butyl (R)-(1-(3-bromophenyl)ethyl)carbamate

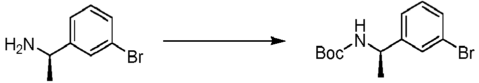

Triethylamine (500 mg, 5 mmol) was added to a solution of (R)-1-(3-bromophenyl)ethanamine (500 mg, 2.5 mmol) in dichloromethane (20 mL). The reaction solution was added dropwise with a solution of di-tert-butyl dicarbonate (648 mg, 3 mmol) in dichloromethane (5 mL) at 0°C, followed by stirring for 3 hours. The reaction solution was added with dichloromethane (50 mL), washed with saturated aqueous citric acid solution (30 mL*2) and saturated aqueous sodium bicarbonate solution (30 mL) successively. The organic phase was concentrated to obtain a colorless transparent oily product tert-butyl (R)-(1-(3-bromophenyl)ethyl)carbamate (750 mg, yield: 100%).

### Step 2: Preparation of tert-butyl (R)-(1-(3-cyclopropylphenyl)ethyl)carbamate

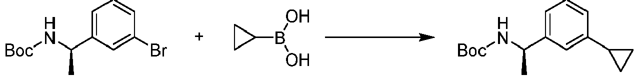

Tert-butyl (R)-(1-(3-bromophenyl)ethyl)carbamate (720 mg, 2.4 mmol), cyclopropylboronic acid (413 mg, 4.8 mmol), [1,1'-bis (diphenylphosphine)ferrocene] palladium dichloride dichloromethane complex (98 mg, 0.12 mmol) and potassium carbonate (994 mg, 7.2 mmol) were reacted in dioxane (10 mL) and water (1 mL) under a microwave condition at 120°C for 1.5 hours. After completion of the reaction, the reaction solution was concentrated and purified by column chromatography [eluent: petroleum ether ~ petroleum ether/ethyl acetate (95/5)] to obtain a colorless oily product tert-butyl (R)-(1-(3-cyclopropylphenyl)ethyl)carbamate (430 mg, yield: 69%).

### Step 3: Preparation of (R)-1-(3-cyclopropylphenyl)ethan-1-amine hydrochloride

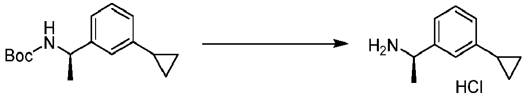

Tert-butyl (R)-(1-(3-cyclopropylphenyl)ethyl)carbamate (430 mg, 1.64 mmol) was stirred in hydrochloric acid ethyl acetate (10 mL, 4 mol/L) for 16 hours. The reaction solution was concentrated to obtain a white solid product (R)-1-(3-cyclopropylphenyl)ethan-1-aminehydrochloride (300 mg, yield: 92%).
MS m/z (ESI): 162.2 [M+H]⁺.

### Step 4: Preparation of (R)-N-(1-(3-cyclopropylphenyl)ethyl)-3-(pyridin-4-yl)-1-trityl-1,7-dihydroimidazo[ 4,5-f]indazole-6-carboxamide

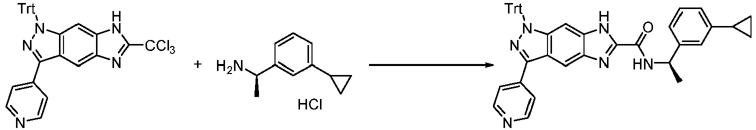

3-(Pyridin-4-yl)-6-(trichloromethyl)-1-trityl-1,7-dihydroimidazo[4,5-f]indazole (100 mg, 0.17 mmol), (R)-1-(3-cyclopropylphenyl)ethan-1-aminehydrochloride (37 mg, 0.18 mmol) and sodium carbonate (212 mg, 2.52 mmol) were stirred in acetonitrile (10 mL) and water (5 mL) at 60°C for 1 hour. The reaction solution was added with tetrahydrofuran (10 mL), and stirred at 60°C for 2 hours. After completion of the reaction, the reaction solution was added with water (30 mL), and extracted with ethyl acetate (30 mL*2). The organic phase was concentrated and purified by column chromatography [eluent: petroleum ether ~ petroleum ether/ethyl acetate (30/70)] to obtain a crude product (75 mg), which was further purified by thin layer chromatography (developing solvent: petroleum ether/ethyl acetate=1/1) to obtain a yellow solid product (R)-N-(1-(3-cyclopropylphenyl)ethyl)-3-(pyridin-4-yl)-1-trityl-1,7-dihydroimidazo[4,5-f]indazole-6-carboxamide (40 mg, yield: 36%).
MS m/z (ESI): 665.3 [M+H]⁺.

### Step 5: Preparation of (R)-N-(1-(3-cyclopropylphenyl)ethyl)-3-(pyridin-4-yl)-1,7-dihydroimidazo[4,5-f]ind azole-6-carboxamide

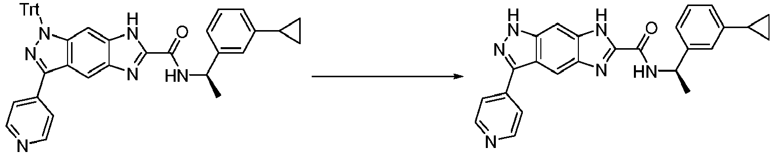

Triethylsilane (28 mg, 0.24 mmol) and trifluoroacetic acid (4 mL) were added to a solution of (R)-N-(1-(3-cyclopropylphenyl)ethyl)-3-(pyridin-4-yl)-1-trityl-1,7-dihydroimidazo[4,5-f]indazole-6-carboxamide (80 mg, 0.12 mmol) in dichloromethane (2 mL). The reaction solution was stirred at room temperature for 1 hour. The reaction solution was concentrated to dryness, and methanol was added to dissolve the residue. The resulting solution was adjusted to alkaline by ammonia, concentrated and purified by thin layer chromatography (developing solvent: CH2Ch/MeOH=10/1) to obtain a yellow solid product (R)-N-(1-(3-cyclopropylphenyl)ethyl)-3-(pyridin-4-yl)-1,7-dihydroimidazo[4,5-f]indazo le-6-carboxamide (26 mg, yield: 51%).
¹H NMR (400 MHz, DMSO-*d*₆) δ 13.46 - 13.39 (m, 1H), 13.12 (s, 1H), 9.40 - 9.31 (m, 1H), 8.72 (s, 2H), 8.45 (s, 0.6H), 8.12 - 7.87 (m, 3H), 7.58 (s, 0.6H), 7.23 - 7.18 (m, 3H), 6.93 (d, *J =* 8 Hz, 1H), 5.20 - 5.16 (m, 1H), 1.90 - 1.89 (m, 1H), 1.54 (d, *J =* 8 Hz, 3H), 0.94 - 0.93 (m, 2H), 0.67 - 0.66 (m, 2H).
MS m/z (ESI): 423.1 [M+H]⁺.

### Example 89

### Preparation of (S)-N-(2-((2,2-difluoroethyl)amino)-1-phenylethyl)-3-(pyridin-4-yl)-1,7-dihydroimi dazo[4,5-f]indazole-6-carboxamide

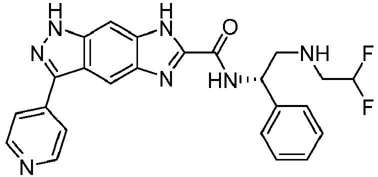

### Step 1: Preparation of (S)-N-(2-((2,2-difluoroethyl)amino)-1-phenylethyl)-3-(pyridin-4-yl)-1-trityl-1,7-dih ydroimidazo [4,5-f]indazole-6-carboxamide

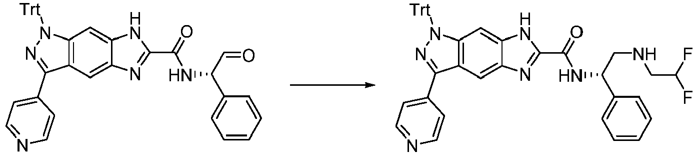

(S)-N-(2-Oxo-1-phenylethyl)-3-(pyridin-4-yl)-1-trityl-1,7-dihydroimidazo[4,5-f]in dazole-6-carboxamide (200 mg, 0.31 mmol) and 2,2-difluoroethylamine (127 mg, 1.56 mmol) were stirred in dichloromethane/methanol (10 mL/5 mL). The reaction solution was added with 3 mL of acetic acid, and stirred for 30 min. The reaction solution was added with sodium cyanoborohydride (98 mg, 1.56 mmol), and stirred overnight. The reaction solution was added with water, extracted with dichloromethane and dried. The solution was concentrated to dryness, and purified by column chromatography to obtain a crude product (S)-N-(2-((2,2-difluoroethyl)amino)-1-phenylethyl)-3-(pyridin-4-yl)-1-trityl-1,7-dihydr oimidazo[4,5-f]indazole-6-carboxamide (38 mg).
MS m/z (ESI): 704.2 [M+H]⁺.

### Step 2: Preparation of (S)-N-(2-((2,2-difluoroethyl)amino)-1-phenylethyl)-3-(pyridin-4-yl)-1,7-dihydroimi dazo[4,5-f]indazole-6-carboxamide

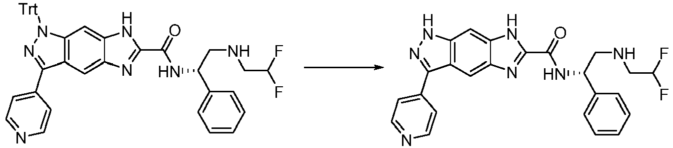

(S)-N-(2-((2,2-Difluoroethyl)amino)-1-phenylethyl)-3-(pyridin-4-yl)-1-trityl-1,7-di hydroimidazo[4,5-f]indazole-6-carboxamide (38 mg) was dissolved in dichloromethane (2 mL), and added with trifluoroacetic acid (2 mL). The reaction solution was stirred at room temperature for 2 h. The reaction solution was concentrated to dryness, and purified by column chromatography to obtain an off-white solid (S)-N-(2-((2,2-difluoroethyl)amino)-1-phenylethyl)-3-(pyridin-4-yl)-1,7-dihydroimidaz o[4,5-f]indazole-6-carboxamide (9 mg, yield in two steps: 6%).
¹H NMR (400 MHz, DMSO-*d*₆) δ 13.40 (s, 1H), 13.10 (s, 1H), 9.18 (s, 1H), 8.71 - 8.64 (m, 2H), 8.13 (s, 1H), 7.99 (s, 1H), 7.96 - 7.89 (m, 2H), 7.40 - 7.23 (m, 5H), 7.03 (d*, J* = 10.1 Hz, 1H), 5.71 (t, *J* = 7.0 Hz, 1H), 5.12 (dtt, *J* = 10.4, 7.1, 1.0 Hz, 1H), 3.28 (dt, *J=* 12.5, 7.3 Hz, 1H), 3.17 (dt, *J=* 12.2, 7.2 Hz, 1H), 3.12 - 2.81 (m, 2H), 1.51 (p, *J* = 7.2 Hz, 1H).
MS m/z (ESI): 462.2 [M+H]⁺.

### Example 90

### Preparation of (S)-N-(2-(dimethylamino)-1-phenylethyl)-3-(pyridin-4-yl)-1,7-dihydroimidazo[4,5-f ]indazole-6-carboxamide

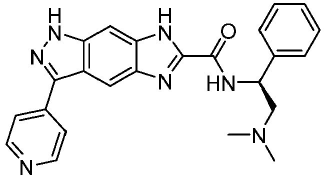

### Step 1: Preparation of (S)-N-(2-hydroxy-1-phenylethyl)-3-(pyridin-4-yl)-1-trityl-1,7-dihydroimidazo[4,5-f] indazole-6-carboxamide

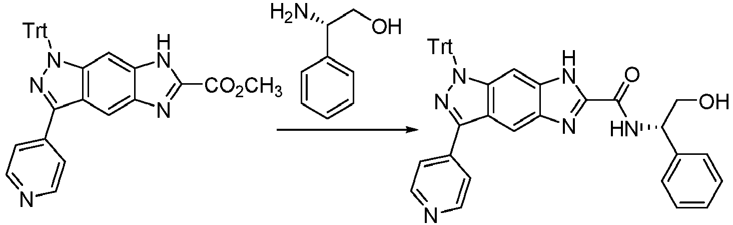

Methyl 3-(pyridin-4-yl)-1-trityl-1,7-dihydroimidazo[4,5-f]indazole-6-carboxylate (2.0 g, 3.73 mmol) and (S)-2-amino-2-phenylethan-1-ol (2.56 g, 18.66 mmol) were stirred under a microwave condition at 140°C for 1 h. The reaction solution was added with water, and extracted with ethyl acetate. The solution was dried, concentrated to dryness, and purified by column chromatography to obtain (S)-N-(2-hydroxy-1-phenylethyl)-3-(pyridin-4-yl)-1-trityl-1,7-dihydroimidazo[4,5-f]ind azole-6-carboxamide (1.46 g, yield: 61%).
MS m/z (ESI): 641.2 [M+H]⁺.

### Step 2: Preparation of (S)-N-(2-oxo-1-phenylethyl)-3-(pyridin-4-yl)-1-trityl-1,7-dihydroimidazo[4,5-f]inda zole-6-carboxamide

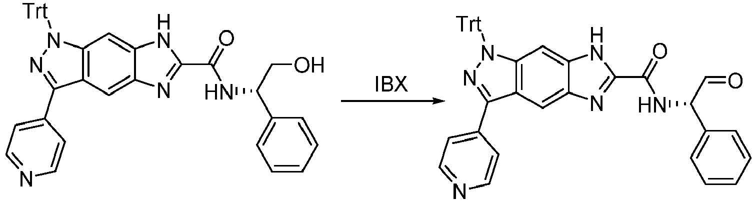

(S)-N-(2-Hydroxy-1-phenylethyl)-3-(pyridin-4-yl)-1-trityl-1,7-dihydroimidazo[4,5 -f]indazole-6-carboxamide (1.46 g, 2.28 mmol) was dissolved in DMSO (10 mL). The solution was added with IBX (5.1 g, 18.21 mmol), and stirred under a nitrogen atmosphere at room temperature for 3 h. The reaction solution was added with dichloromethane, and washed with sodium carbonate solution. The solution was dried, concentrated to dryness, and purified by column chromatography to obtain 2.52 g of oily crude product, which was used directly in the next step.
MS m/z (ESI): 639.2 [M+H]⁺.

### Step 3: Preparation of (S)-N-(2-(dimethylamino)-1-phenylethyl)-3-(pyridin-4-yl)-1-trityl-1,7-dihydroimida zo[4,5-f]indazole-6-carboxamide

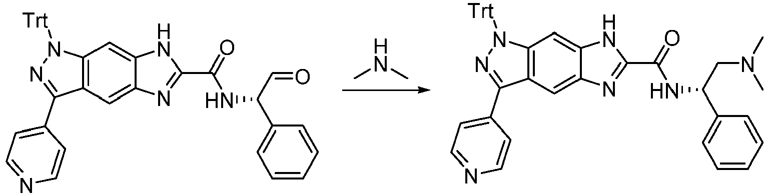

(S)-N-(2-Oxo-1-phenyl ethyl)-3-(pyridin-4-yl)-1-trityl-1,7-dihydroimidazo[4,5-f]in dazole-6-carboxamide (200 mg, 0.31 mmol) and a 2M solution of dimethylamine in methanol (1.57 mL, 3.13 mmol) were stirred in dichloromethane/methanol (10 mL/5 mL). The reaction solution was added with 0.5 mL of acetic acid, and stirred for 30 min. The reaction solution was added with sodium cyanoborohydride (98 mg, 1.56 mmol), and stirred overnight. The reaction solution was added with water, and extracted with dichloromethane. The solution was dried, concentrated to dryness, and purified by column chromatography to obtain an oil (66 mg), which was used directly in the next step.
MS m/z (ESI): 668.3 [M+H]⁺.

### Step 4: Preparation of (S)-N-(2-(dimethylamino)-1-phenylethyl)-3-(pyridin-4-yl)-1,7-dihydroimidazo[4,5-f ]indazole-6-carboxamide

(S)-N-(2-(Dimethylamino)-1-phenylethyl)-3-(pyridin-4-yl)-1-trityl-1,7-dihydroimi dazo[4,5-f]indazole-6-carboxamide (66 mg) was dissolved in dichloromethane (5 mL), and added with trifluoroacetic acid (2 mL). The reaction solution was stirred at room temperature for 1 h. The reaction solution was concentrated to dryness, and purified by column chromatography to obtain an off-white solid (10 mg, yield in two steps: 8%).
¹H NMR (400 MHz, DMSO-*d*₆) δ 13.40 (s, 1H), 13.10 (s, 1H), 9.18 (s, 1H), 8.72 (d, *J =* 5.7 Hz, 2H), 8.50 (s, 0.5H), 8.29 - 7.81 (m, 3H), 7.58 (s, 0.5H), 7.48 (d, *J =* 7.4 Hz, 2H), 7.34 (t, *J* = 7.5 Hz, 2H), 7.26 (t, *J* = 7.3 Hz, 1H), 5.17 (s, 1H), 2.96 (s, 1H), 2.43 (dd, *J* = 12.6, 5.2 Hz, 1H), 2.24 (s, 6H).
MS m/z (ESI): 426.2 [M+H]⁺.

### Example 91

### Preparation of (R)-3-(4-methoxycyclohexyl)-N-(1-phenylethyl)-1,7-dihydroimidazo[4,5-f]indazole-6-carboxamide

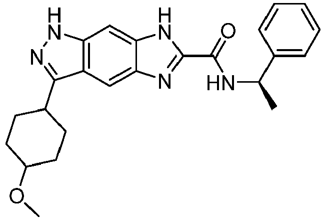

(R)-3-(4-Methoxycyclohexyl)-N-(1-phenyl ethyl)-1,7-dihydroimidazo[4,5-f]indazol e-6-carboxamide was prepared in accordance with Example 2.
¹H NMR (400 MHz, DMSO-*d*₆) δ 12.74 (s, 1H), 10.80 (s, 1H), 9.41 (d*, J* = 9.3 Hz, 1H), 7.70 (s, 2H), 7.36 - 7.22 (m, 5H), 5.13 (dq, *J* = 9.3, 6.2 Hz, 1H), 3.57 - 3.43 (m, 1H), 3.31 (s, 2H), 2.72 (p, *J* = 8.0 Hz, 1H), 2.35 - 2.21 (m, 2H), 1.83 - 1.69 (m, 2H), 1.58 - 1.48 (m, 2H), 1.47 (d, *J =* 6.4 Hz, 3H), 1.21 - 1.07 (m, 2H).
MS m/z (ESI): 418.1 [M+H]⁺.

### Example 92

### Preparation of 3-(4-methoxycyclohex-l-en-1-yl)-N-((R)-1-phenylethyl)-1,7-dihydroimidazo[4,5-f]i ndazole-6-carboxamide

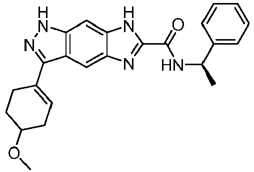

3-(4-Methoxycyclohex-1-en-1-yl)-N-((R)-1-phenylethyl)-1,7-dihydroimidazo[4,5-f ]indazole-6-carboxamide was prepared in accordance with Example 2.
¹H NMR (400 MHz, DMSO-*d*₆) δ13.07 (s, 0.4 H), 12.95 (s, 0.6 H), 12.75 (s, 0.4 H), 12.69 (s, 0.6 H), 9.40 (d*, J* = 8.5 Hz, 0.4 H), 9.30 (d*, J* = 8.5 Hz, 0.6 H), 8.21 (s, 0.6 H), 7.93 (s, 0.4 H), 7.72 (s, 0.4 H), 7.48 - 7.44 (m, 2.6 H), 7.36 - 7.32 (m, 2H), 7.26 - 7.22 (m, 1H), 6.51 (s, 0.6 H), 6.40 (s, 0.4 H), 5.22 (p, *J =* 7.0 Hz, 1H)., 3.64 - 3.53 (m, 1H), 3.33 (s, 3H), 2.82 - 2.80 (m, 1H), 2.69 - 2.63 (m, 2H), 2.26 - 2.22 (m, 1H), 2.06 - 2.03 (m, 1H), 1.79 - 1.64 (m, 1H), 1.56 (d, *J =* 7.0 Hz, 3H).
MS m/z (ESI): 416.1 [M+H]⁺.

### Example 93

### Preparation of (R)-3-(4-oxocyclohexyl)-N-(1-phenylethyl)-1,7-dihydroimidazo[4,5-f]indazole-6-car boxamide

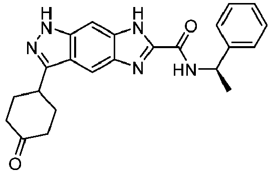

### Step 1: Preparation of 3-bromo-1-trityl-1H-indazole-5,6-diamine

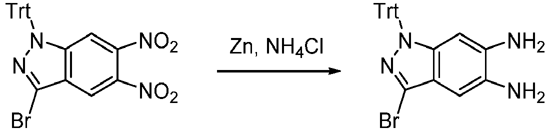

3-Bromo-5,6-dinitro-1-trityl-1H-indazole (7.0 g, 13.2 mmol) was dissolved in 100 mL of tetrahydrofuran. The solution was added with 25 mL of saturated NH₄Cl solution and zinc powder (17.2 g, 264.7 mmol), and heated to 70°C under a nitrogen atmosphere overnight. After completion of the reaction by detection, the reaction solution was filtered to remove the insoluble solid. The filtrate was concentrated to dryness, and purified by column chromatography to obtain a crude product 3-bromo-1-trityl-1H-indazole-5,6-diamine (4.3 g), which was used directly in the next step.
MS m/z (ESI): 469.1 [M+H]⁺.

### Step 2: Preparation of 3-bromo-6-(trichloromethyl)-1-trityl-1,7-dihydroimidazo[4,5-f]indazole

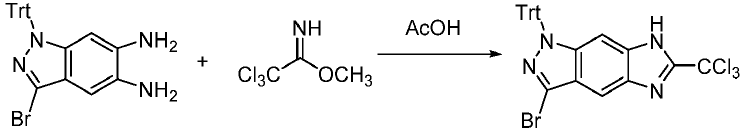

3-Bromo-1-trityl-1H-indazole-5,6-diamine (4.3 g, 9.2 mmol) was dissolved in 20 mL of glacial acetic acid. The solution was added dropwise with methyl 2,2,2-trichloroacetimidate (2.1 g, 12.0 mmol), and reacted at room temperature for 2 hours. The reaction solution was added dropwise to 200 mL of water, and stirred at room temperature for 30 minutes. The precipitated solid was filtered. The filter cake was washed with water, and dried under vacuum to obtain a brown solid 3-bromo-6-(trichloromethyl)-1-trityl-1,7-dihydroimidazo[4,5-f]indazole (4.9 g), which was used directly in the next step.

### Step 3: Preparation of methyl 3-bromo-1-trityl-1,7-dihydroimidazo[4,5-f]indazole-6-carboxylate

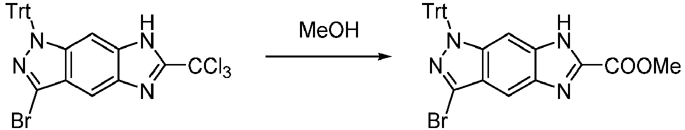

3-Bromo-6-(trichloromethyl)-1-trityl-1,7-dihydroimidazo[4,5-f]indazole (4.9 g, 9.26 mmol) was dissolved in 200 mL of methanol, and heated to reflux for 24 hours. The reaction solution was added with silica gel, concentrated to dryness and purified by column chromatography to obtain a crude product methyl 3-bromo-1-trityl-1,7-dihydroimidazo[4,5-f]indazole-6-carboxylate (2.4 g), which was used directly in the next step.
MS m/z (ESI): 537.1 [M+H]⁺.

### Step 4: Preparation of (R)-3-bromo-N-(1-phenylethyl)-1-trityl-1,7-dihydroimidazo[4,5-f]indazole-6-carbo xamide

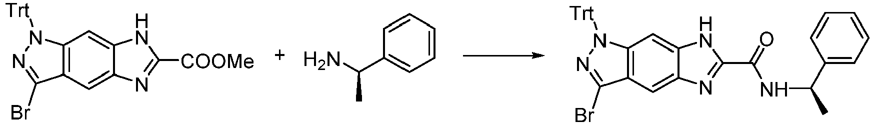

Methyl 3-bromo-1-trityl-1,7-dihydroimidazo[4,5-f]indazole-6-carboxylate (1.5 g, 2.79 mmol) and 7 mL of (*R*)-(+)-1-phenylethanamine were added to a microwave reaction tube. The reaction solution was heated to 140°C by microwave for one hour. The reaction solution was cooled, and added with 50 mL of ethyl acetate. The ethyl acetate layer was washed with saturated NH₄Cl solution and saturated NaCl solution successively, dried over anhydrous sodium sulfate, and purified by column chromatography to obtain a solid (R)-3-bromo-N-(1-phenylethyl)-1-trityl-1,7-dihydroimidazo[4,5-f]indazole-6-carboxam ide (1.05 g, yield in four steps: 20%).
MS m/z (ESI): 626.1 [M+H]⁺.

### Step 5: Preparation of (R)-N-(1-phenylethyl)-3-(1,4-dioxaspiro[4.5]dec-7-en-8-yl)-1-trityl-1,7-dihydroimid azo[4,5-f]indazole-6-carboxamide

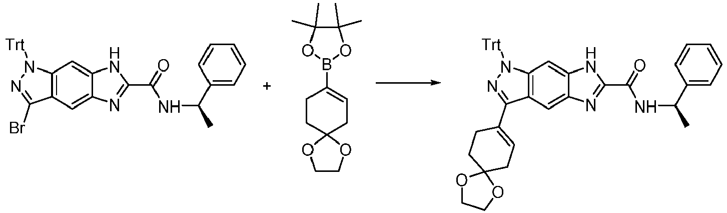

(R)-3-Bromo-N-(1-phenylethyl)-1-trityl-1,7-dihydroimidazo[4,5-f]indazole-6-carb oxamide (100 mg, 0.16 mmol), 1,4-dioxa-spiro[4.5]dec-7-en-8-yl pinacol borate (85 mg, 0.32 mmol), potassium carbonate (66 mg, 0.48 mmol), [1,1'-bis (diphenylphosphine)ferrocene] palladium dichloride (12 mg, 0.016 mmol), 5 mL of 1,4-dioxane and 1 mL of water were added to a microwave reaction tube. After purging nitrogen for 5 minutes, the reaction solution was heated to 120°C by microwave for one hour. The reaction solution was poured into 20 mL of water, and extracted with ethyl acetate. The ethyl acetate layer was washed with saturated NaCl solution, dried over anhydrous sodium sulfate, and purified by column chromatography to obtain a solid (R)-N-(1-phenylethyl)-3-(1,4-dioxaspiro[4.5]dec-7-en-8-yl)-1-trityl-1,7-dihydroimidazo [4,5-f]indazole-6-carboxamide (110 mg, yield: 100%).
MS m/z (ESI): 686.1 [M+H]⁺.

### Step 6: Preparation of (R)-N-(1-phenylethyl)-3-(1,4-dioxaspiro[4.5]decan-8-yl)-1-trityl-1,7-dihydroimidaz o[4,5-f]indazole-6-carboxamide

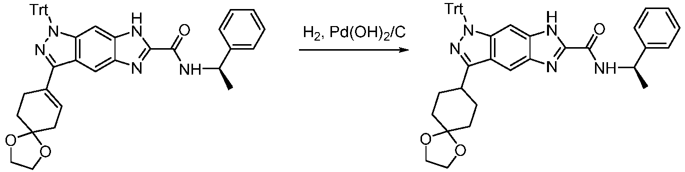

(R)-N-(1-Phenylethyl)-3-(1,4-dioxaspiro[4.5]dec-7-en-8-yl)-1-trityl-1,7-dihydroim idazo[4,5-f]indazole-6-carboxamide (80 mg, 0.12 mmol) was dissolved in 5 mL of ethyl acetate. The solution was added with a catalytic amount of 20% Pd(OH)₂/C (containing about 50% water), purged with hydrogen, and reacted at room temperature for 5 hours. The reaction solution was filtered to remove Pd(OH)₂/C, and concentrated to dryness to obtain a white solid (R)-N-(1-phenylethyl)-3-(1,4-dioxaspiro[4.5]decan-8-yl)-1-trityl-1,7-dihydroimidazo[4, 5-f]indazole-6-carboxamide (70 mg, yield: 85%).
MS m/z (ESI): 688.1 [M+H]⁺.

### Step 7: Preparation of (R)-3-(4-oxocyclohexyl)-N-(1-phenylethyl)-1,7-dihydroimidazo[4,5-f]indazole-6-car boxamide

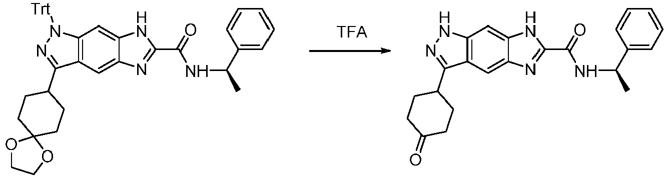

(R)-N-(1-Phenylethyl)-3-(1,4-dioxaspiro[4.5]decan-8-yl)-1-trityl-1,7-dihydroimida zo[4,5-f]indazole-6-carboxamide (70 mg, 0.10 mmol) was dissolved in 5 mL of trifluoroacetic acid, and reacted at room temperature for one hour. The reaction solution was heated to 50°C for one hour. The reaction solution was concentrated to dryness, and added with 20 mL of 2-methyltetrahydrofuran. The organic phase was washed with saturated NaHCO3 solution until neutral, dried over anhydrous sodium sulfate, concentrated to dryness, and purified by column chromatography to obtain a light yellow solid (R)-3-(4-oxocyclohexyl)-N-(1-phenylethyl)-1,7-dihydroimidazo[4,5-f]indazole-6-carbo xamide (18 mg, yield: 45%).
¹H NMR (400 MHz, DMSO-*d*₆) δ 13.14 - 12.90 (m, 1H), 12.90 - 12.75 (m, 1H), 9.44 - 9.19 (m, 1H), 7.70 (s, 2H), 7.36 - 7.25 (m, 5H), 5.22 (p, *J =* 7.0 Hz, 1H), 3.15 - 3.02 (m, 1H), 2.38 - 2.14 (m, 6H), 2.03 - 1.88 (m, 2H), 1.56 (d, *J =* 7.0 Hz, 3H).
MS m/z (ESI): 402.1 [M+H]⁺.

### Example 94

### Preparation of (R)-3-(4-oxocyclohex-1-en-1-yl)-N-(1-phenylethyl)-1,7-dihydroimidazo[4,5-f]indazo le-6-carboxamide

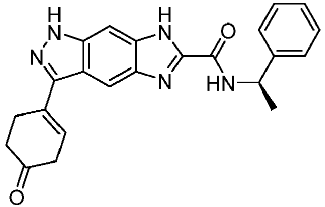

(R)-3-(4-Oxocyclohex-1-en-1-yl)-N-(1-phenyl ethyl)-1,1,7-dihydroimidazo[4,5-f]ind azole-6-carboxamide was prepared in accordance with Example 93.
¹H NMR (400 MHz, DMSO-*d*₆) δ 13.17 - 12.92 (m, 1H), 12.92 - 12.78 (m, 1H), 9.44-9.19 (m, 1H), 8.31 - 7.65 (m, 1H), 7.53 -7.43 (m, 2H), 7.39-7.29 (m, 2H), 7.28 - 7.16 (m, 1H), 6.71 - 6.49 (m, 1H), 5.22 (p, *J =* 7.0 Hz, 1H), 3.93 (s, 1H), 3.25 - 3.05 (m, 3H), 2.69 - 2.55 (m, 2H), 1.56 (d, *J =* 7.0 Hz, 3H).
MS m/z (ESI): 400.1 [M+H]⁺.

### Example 95

### Preparation of (R)-3-(4-methoxyphenyl)-N-(1-phenylethyl)-1,7-dihydroimidazo[4,5-f]indazole-6-ca rboxamide

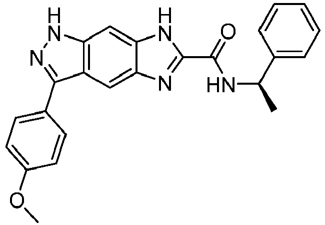

(R)-3-(4-Methoxyphenyl)-N-(1-phenylethyl)-1,7-dihydroimidazo[4,5-f]indazole-6-carboxamide was prepared in accordance with Example 2.
¹H NMR (400 MHz, DMSO-*d*₆) δ 13.26 - 12.76 (m, 2H), 9.48 - 9.21 (m, 1H), 8.28 (s, 0.6H), 7.99 (s, 0.4H), 7.96 (d, *J* = 8.1 Hz, 1H), 7.90 (d, *J* = 8.2 Hz, 1H), 7.79 (s, 0.4H), 7.57 - 7.43 (m, 2.6H), 7.34 (t, *J* = 7.5 Hz, 2H), 7.26 - 7.18 (m, 1H), 7.20 - 7.03 (m, 2H), 5.23 (p, *J =* 7.0 Hz, 1H), 3.84 (s, 3H), 1.57 (d, *J =* 7.0 Hz, 3H).
MS m/z (ESI): 412.1 [M+H]⁺.

### Example 96

### Preparation of (R)-3-(4-isopropoxyphenyl)-N-(1-phenylethyl)-1,7-dihydroimidazo[4,5-f]indazole-6 -carboxamide

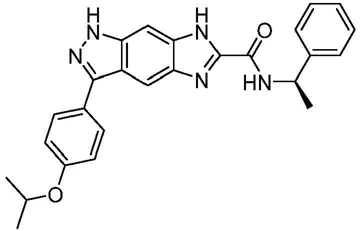

(R)-3-(4-Isopropoxyphenyl)-N-(1-phenylethyl)-1,7-dihydroimidazo[4,5-f]indazole -6-carboxamide was prepared in accordance with Example 2.
¹H NMR (400 MHz, DMSO-*d*₆) δ 13.04-12.85 (m, 2H), 9.41-9.31 (m, 1H), 8.26 (s, 0.6H), 8.02 - 7.70 (m, 2.8H), 7.48-7.48 (m, 2.6H), 7.34 (t, *J =* 8 Hz, 2H), 7.24-7.22 (m, 1H), 7.11-7.08 (m, 2H), 5.29-5.15 (m, 1H), 4.73-4.67 (m, 1H), 1.56 (d, *J* = 8 Hz, 3H), 1.32 (d, *J* = 4 Hz, 6H).
MS m/z (ESI): 440.1 [M+H]⁺.

### Example 97

### Preparation of (R)-4-(6-((1-phenylethyl)carbamoyl)-1,7-dihydroimidazo[4,5-f]indazol-3-yl)pyridin e-N-oxide

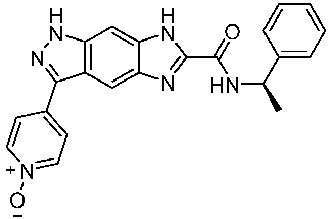

### Step 1: Preparation of (R)-4-(6-((1-phenylethyl)carbamoyl)-1,7-dihydroimidazo[4,5-f]indazol-3-yl)pyridin e-N-oxide

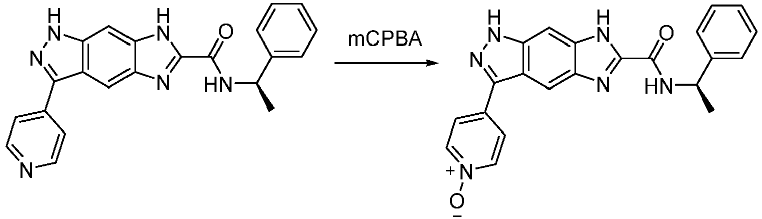

(R)-N-(1-Phenylethyl)-3-(pyridin-4-yl)-1,7-dihydroimidazo[4,5-f]indazole-6-carbo xamide (50 mg, 0.13 mmol) was dispersed in 5 mL of tetrahydrofuran. The solution was added with mCPBA (85%, 53 mg, 0.26 mmol), and reacted at room temperature for 2 hours. The reaction solution was concentrated to dryness, and purified by column chromatography to obtain a solid (R)-4-(6-((1-phenylethyl)carbamoyl)-1,7-dihydroimidazo[4,5-f]indazol-3-yl)pyridine-N -oxide (33 mg, yield: 63%).
¹H NMR (400 MHz, DMSO) δ 13.40 (s, 1H), 13.11 (s, 1H), 9.37 (s, 1H), 8.43 (s, 1H), 8.38 - 8.34 (m, 2H), 8.12 - 8.08 (m, 2H), 7.59 (s, 1H), 7.50 - 7.24 (m, 5H), 5.31 - 5.18 (m, 1H), 1.58 (d, *J* = 7.0Hz, 3H).
MS m/z (ESI): 399.1 [M+H]⁺.

### Example 98

### (R)-N-(1-(3-Isopropylphenyl)ethyl)-3-(pyridin-4-yl)-1,7-dihydroimidazo[4,5-f]indaz ole-6-carboxamide

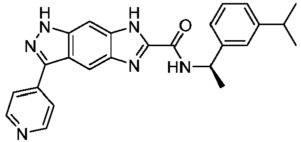

### Step 1: Preparation of (R)-N-(1-(3-bromophenyl)ethyl)-3-(pyridin-4-yl)-1-trityl-1,7-dihydroimidazo[4,5-f]i ndazole-6-carboxamide

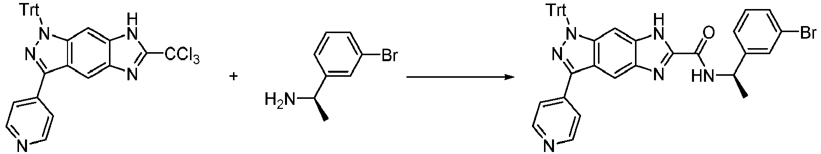

3-(Pyridin-4-yl)-6-(trichloromethyl)-1-trityl-1,7-dihydroimidazo[4,5-f]indazole (2 g, 3.35 mmol), (R)-1-(3-bromophenyl)ethanamine (737 mg, 3.69 mmol) and sodium bicarbonate (4.22 g, 50.25 mmol) were stirred in acetonitrile (50 mL) and water (25 mL) at 60°C for one hour. The reaction solution was added with tetrahydrofuran (50 mL), and stirred at 60°C for 2 hours. After completion of the reaction, the reaction solution was added with water (60 mL), and extracted with ethyl acetate (50 mL*2). The organic phase was concentrated and purified by column chromatography [eluent: petroleum ether ∼ petroleum ether/ethyl acetate (30/70)] to obtain a brown solid product (R)-N-(1-(3-bromophenyl)ethyl)-3-(pyridin-4-yl)-1-trityl-1,7 -dihydroimidazo[4,5-f]inda zole-6-carboxamide (700 mg, yield: 30%).
MS m/z (ESI): 703.1 [M+H]⁺.

### Step 2: Preparation of (R)-N-(1-(3-(prop-1-en-2-yl)phenyl)ethyl)-3-(pyridin-4-yl)-1-trityl-1, 7-dihydroimid azo[4,5-f]indazole-6-carboxamide

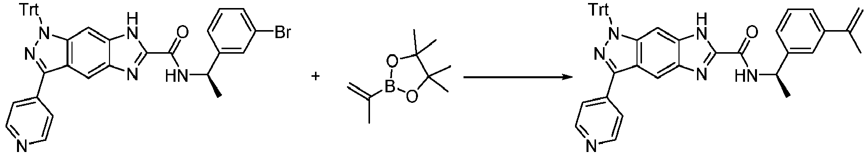

(R)-N-(1-(3-Bromophenyl)ethyl)-3-(pyridin-4-yl)-1-trityl-1,7-dihydroimidazo[4,5-f]indazole-6-carboxamide (200 mg, 0.28 mmol), 4,4,5,5-tetramethyl-2-(prop-l-en-2-yl)-1,3,2-dioxaborolane (191 mg, 1.14 mmol), [1,1'-bis(diphenylphosphine)ferrocene] palladium dichloride dichloromethane complex (23 mg, 0.028 mmol) and potassium carbonate (118 mg, 0.85 mmol) were reacted in dioxane (6 mL) and water (0.6 mL) under a microwave condition at 130°C for 1.5 hours. The reaction solution was concentrated and purified by column chromatography [eluent: petroleum ether ∼ petroleum ether/ethyl acetate (30/70)] to obtain a brown oily product (R)-N-(1-(3-(prop-1-en-2-yl)phenyl)ethyl)-3-(pyridin-4-yl)-1-trityl-1,7-dihydroimidazo[ 4,5-f]indazole-6-carboxamide (100 mg, yield: 53%).
MS m/z (ESI): 665.2 [M+H]⁺.

### Step 3: Preparation of (R)-N-(1-(3-isopropylphenyl)ethyl)-3-(pyridin-4-yl)-1-trityl-1,7-dihydroimidazo[4,5 -f]indazole-6-carboxamide

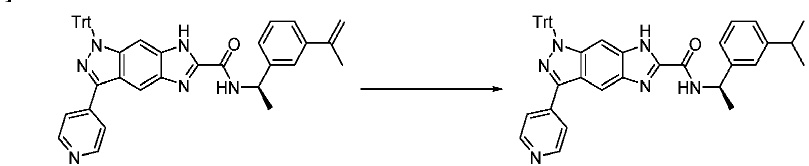

(R)-N-(1-(3-(Prop-1-en-2-yl)phenyl)ethyl)-3-(pyridin-4-yl)-1-trityl-1,7-dihydroimi dazo[4,5-f]indazole-6-carboxamide (100 mg, 0.15 mmol) and Pd(OH)₂/C (100 mg) were stirred in tetrahydrofuran (6 mL) at room temperature under hydrogen atmosphere for 16 hours. The reaction solution was filtered, and the filtrate was concentrated to obtain a yellow-black solid product (R)-N-(1-(3-isopropylphenyl)ethyl)-3-(pyridin-4-yl)-1-trityl-1,7-dihydroimidazo[4,5-f]i ndazole-6-carboxamide (100 mg, yield: 100%).
MS m/z (ESI): 667.3 [M+H]+.

### Step 4: Preparation of (R)-N-(1-(3-isopropylphenyl)ethyl)-3-(pyridin-4-yl)-1,7-dihydroimidazo[4,5-f]indaz ole-6-carboxamide

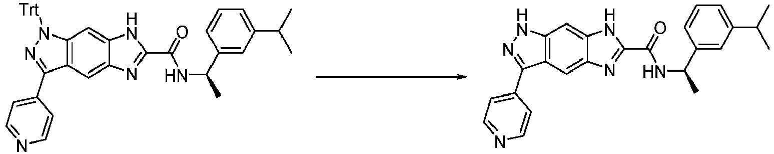

Triethylsilane (35 mg, 0.30 mmol) and trifluoroacetic acid (4 mL) were added to a solution of (R)-N-(1-(3-isopropylphenyl)ethyl)-3-(pyridin-4-yl)-1-trityl-1,7-dihydroimidazo[4,5-f]i ndazole-6-carboxamide (100 mg, 0.15 mmol) in dichloromethane (2 mL). The reaction solution was stirred at room temperature for 1 hour. The reaction solution was concentrated to dryness, and methanol was added to dissolve the residues. The resulting solution was adjusted to alkaline by aqua ammonia, concentrated and purified by thin layer chromatography (developing solvent: CH₂Cl₂/MeOH=10/1) to obtain a yellow solid product (R)-N-(1-(3-isopropylphenyl)ethyl)-3-(pyridin-4-yl)-1,7-dihydroimidazo[4,5-f]indazole -6-carboxamide (28 mg, yield: 44%).
¹H NMR (400 MHz, DMSO-*d*₆) δ 13.41 (d, *J =* 28 Hz, 1H), 13.21 - 13.07 (m, 1H), 9.40 - 9.29 (m, 1H), 8.73 - 8.70 (m, 2H), 8.44 (s, 0.6H), 8.12 (s, 0.4H), 8.05 - 7.97 (m, 2H), 7.87 (s, 0.4H), 7.58 (s, 0.6H), 7.35 (s, 1H), 7.30 - 7.24 (m, 2H), 7.14 - 7.12 (m, 1H), 5.23 - 5.20 (m, 1H), 2.91 - 2.84 (m, 1H), 1.56 (d, *J* = 8 Hz, 3H), 1.20 (d, *J* = 4 Hz, 6H).
MS m/z (ESI): 425.2 [M+H]⁺.

### Example 99

### Preparation of (R)-8-chloro-3-(4-methoxypiperidin-1-yl)-N-(1-phenylethyl)-1,7-dihydroimidazo[4, 5-f]indazole-6-carboxamide

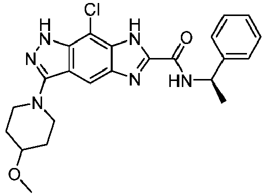

### Step 1: Preparation of (R)-8-chloro-3-(4-methoxypiperidin-1-yl)-N-(l-phenylethyl)-1,7-dihydroimidazo[4, 5-f]indazole-6-carboxamide

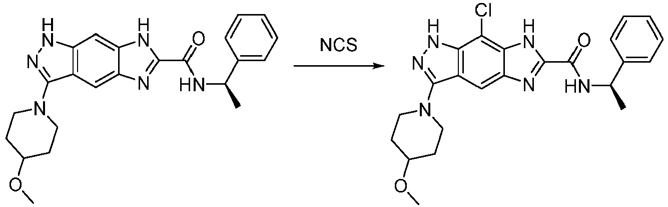

(R)-3-(4-Methoxypiperidin-1-yl)-N-(1-phenylethyl)-1,7-dihydroimidazo[4,5-f]inda zole-6-carboxamide (28 mg, 0.067 mmol) was dissolved in 5 mL of DMF. The solution was added with N-chlorosuccinimide (8.9 mg, 0.067 mmol), and heated to 60°C for 2 hours. The reaction solution was added with 10 mL of water, and extracted with 20 mL of 2-methyltetrahydrofuran. The organic layer was washed with saturated NaCl solution, dried over anhydrous sodium sulfate, concentrated to dryness, and purified by column chromatography to obtain a light yellow solid (R)-8-chloro-3-(4-methoxypiperidin-1-yl)-N-(1-phenylethyl)-1,7-dihydroimidazo[4,5-f] indazole-6-carboxamide (17 mg, yield: 57%).
¹H NMR (400 MHz, DMSO-*d*₆) δ 13.32 (s, 1H), 12.17 (s, 1H), 9.32 (d, *J* = 8.5 Hz, 1H), 7.66 (s, 1H), 7.52 - 7.42 (m, 2H), 7.39 - 7.30 (m, 2H), 7.29 - 7.20 (m, 1H), 5.28 - 5.14 (m, 1H), 3.68 - 3.61 (m, 2H), 3.39 - 3.36 (m, 1H), 3.29 (s, 3H), 3.14 - 2.98 (m, 2H), 2.10 - 1.93 (m, 2H), 1.73 - 1.61 (m, 2H), 1.57 (d, *J =* 7.0 Hz, 3H).
MS m/z (ESI): 453.1 [M+H]⁺.

### Examples 100 and 101

### Preparation of 6-((1-phenylethyl)sulfinyl)-3-(pyridin-4-yl)-1,7-dihydroimidazo[4,5-f]indazole and 6-((1-phenylethyl)sulfonyl)-3-(pyridin-4-yl)-1,7-dihydroimidazo[4,5-f]indazole

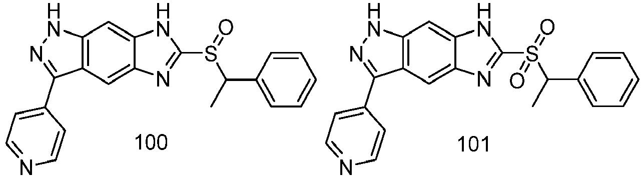

### Step 1: Preparation of 3-(pyridin-4-yl)-1-trityl-1,7-dihydroimidazo[4,5-f]indazole-6-thiol

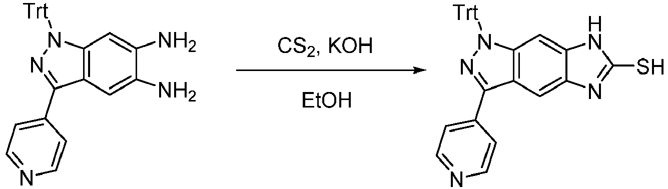

3-(Pyridin-4-yl)-1-trityl-1H-indazole-5,6-diamine (1.0 g, 2.14 mmol) was dissolved in 25 mL of ethanol. The solution was added with carbon disulfide (163 mg, 2.14 mmol) and potassium hydroxide (120 mg, 2.14 mmol), and heated to 60°C for 6 hours. The reaction solution was cooled, and added with 10 mL of water. The pH was adjusted to 4-5 with 4N HCl solution. The precipitated solid was filtered, washed with water, and dried under vacuum to obtain an off-white solid product 3-(pyridin-4-yl)-1-trityl-1,7-dihydroimidazo[4,5-f]indazole-6-thiol (1.1 g, yield: 100%).
MS m/z (ESI): 510.1 [M+H]⁺.

### Step 2: Preparation of 6-((1-phenylethyl)thio)-3-(pyridin-4-yl)-1-trityl-1,7-dihydroimidazo[4,5-f]indazole

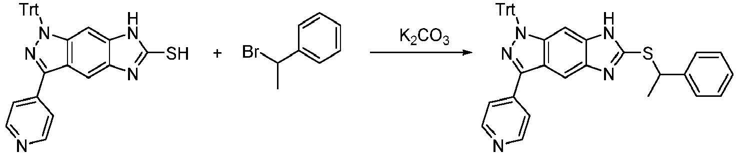

3-(Pyridin-4-yl)-1-trityl-1,7-dihydroimidazo[4,5-f]indazole-6-thiol (600 mg, 1.2 mmol), potassium carbonate (326 mg, 2.4 mmol) and (1-bromoethyl)benzene (218 mg, 1.2 mmol) were dissolved in a mixed solvent of acetone, tetrahydrofuran and water (10 mL:10 mL:4 mL). The reaction solution was heated to 50°C for 2 hours. The reaction solution was concentrated, and extracted with dichloromethane (50 ml*3). The resulting dichloromethane solution was washed with saturated NaCl solution, dried over anhydrous MgSO₄, and purified by column chromatography to obtain a yellow solid 6-((1-phenylethyl)thio)-3-(pyridin-4-yl)-1-trityl-1,7-dihydroimidazo[4,5-f]indazole (270 mg, yield: 37%).
MS m/z (ESI): 614.1 [M+H]⁺.

### Step 3: Preparation of 6-((1-phenylethyl)thio)-3-(pyridin-4-yl)-1,7-dihydroimidazo[4,5-f]indazole

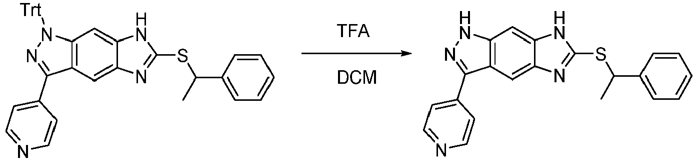

6-((1-Phenylethyl)thio)-3-(pyridin-4-yl)-1-trityl-1,7-dihydroimidazo[4,5-f]indazole (150 mg, 0.16 mmol) was dissolved in a mixed solvent of dichloromethane and trifluoroacetic acid (4 mL:4 mL). The reaction solution was reacted at room temperature for 2 hours. The reaction solution was concentrated to dryness, added with 10 mL of methanol, and placed in an ice bath. The residual trifluoroacetic acid was neutralized with a 7.0 M solution of NH₃ in methanol. The resulting solution was concentrated to dryness, and purified by column chromatography to obtain a yellow solid 6-((1-phenylethyl)thio)-3-(pyridin-4-yl)-1,7-dihydroimidazo[4,5-f]indazole (90 mg, yield: 99%).
MS m/z (ESI): 372.1 [M+H]⁺.

### Step 4: Preparation of 6-((1-phenylethyl)sulfinyl)-3-(pyridin-4-yl)-1,7-dihydroimidazo[4,5-f]indazole and 6-((1-phenylethyl)sulfonyl)-3-(pyridin-4-yl)-1,7-dihydroimidazo[4,5-f]indazole

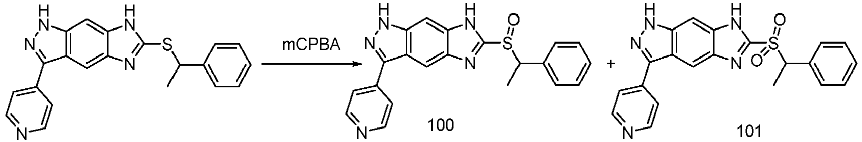

6-((1-Phenylethyl)thio)-3-(pyridin-4-yl)-1,7-dihydroimidazo[4,5-f]indazole (90 mg, 0.24 mmol) was dissolved in a mixed solvent of chloroform and methanol (15 mL:1 mL). The solution was placed in an ice bath, added with mCPBA (42 mg, 0.21 mmol), and reacted at 0°C for 30 minutes. The reaction solution was concentrated to dryness, and purified by column chromatography. The resulting crude product was purified by preparative HPLC to obtain a light yellow solid 6-((1-phenylethyl)sulfinyl)-3-(pyridin-4-yl)-1,7-dihydroimidazo[4,5-f]indazole (5.0 mg, yield: 7%) and 6-((1-phenylethyl)sulfonyl)-3-(pyridin-4-yl)-1,7-dihydroimidazo[4,5-f]indazole (4.8 mg, yield: 6%).

### Example 100:

¹H NMR (400 MHz, DMSO-*d*₆) δ 13.38 (s, 1H), 8.65 (d*, J* = 5.9 Hz, 2H), 8.42 (s, 1H), 8.00 (d, *J =* 5.6 Hz, 2H), 7.64 (s, 1H), 7.27 (d, *J =* 5.8 Hz, 5H), 4.98 (q, *J =* 6.7 Hz, 1H), 1.65 (d, *J* = 7.1 Hz, 3H).
MS m/z (ESI): 388.1 [M+H]⁺.

### Example 101:

¹H NMR (400 MHz, DMSO-*d*₆) δ 13.38 (s, 1H), 8.65 (d*, J* = 5.9 Hz, 2H), 8.42 (s, 1H), 8.00 (d, *J =* 5.8 Hz, 2H), 7.64 (s, 1H), 7.27 (d, *J =* 5.8 Hz, 5H), 4.98 (q, *J =* 7.1 Hz, 1H), 1.65 (d, *J* = 7.1 Hz, 3H).
MS m/z (ESI): 404.1 [M+H]⁺.

### Example 102

### Preparation of 6-benzyl-3-(pyridin-4-yl)-5,6-dihydro-[1,3[oxazino[5,6-f]indazol-7(1H)-one

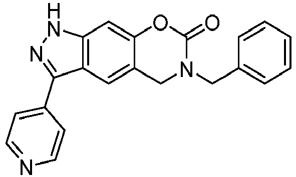

### Step 1: Preparation of 5-((benzylamino)methyl)-3-(pyridin-4-yl)-1H-indazol-6-ol

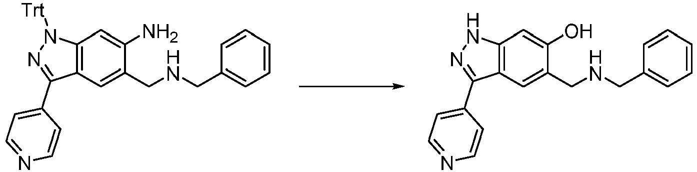

The compound 5-((benzylamino)methyl)-3-(pyridin-4-yl)-1-trityl-1H-indazol-6-amine (60 mg, 0.105 mmol, 1.0 eq) was dissolved in sulfuric acid/water (3 mL/3 mL). The reaction solution was cooled to 0°C, added dropwise with a solution of sodium nitrite (8.0 mg, 0.116 mmol, 1.1 eq) in water (0.5 mL), and reacted at 0°C for 2 hours. The reaction solution was added with 1 mL of water, and heated to 120°C for 2 hours. After completion of the reaction, the reaction solution was slowly added dropwise with saturated sodium bicarbonate solution until pH 7. The solution was extracted with dichloromethane (20 mL*3), dried over anhydrous sodium sulfate, and concentrated to obtain a crude product 5-((benzylamino)methyl)-3-(pyridin-4-yl)-1H-indazol-6-ol, which was used directly in the next step.
MS m/z (ESI): 331.2 [M+H]⁺.

### Step 2: Preparation of 6-benzyl-3-(pyridin-4-yl)-5,6-dihydro-[1,3] oxazino[5,6-f]indazol-7(1H)-one

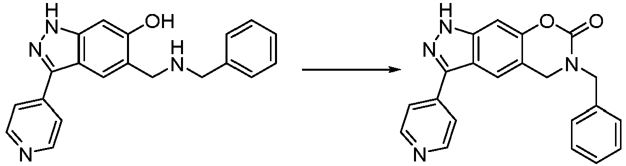

The compound 5-((benzylamino)methyl)-3-(pyridin-4-yl)-1H-indazol-6-ol (34.6 mg, 0.105 mmol, 1.0 eq), N,N'-carbonyldiimidazole (25.5 mg, 0.158 mmol, 1.5 eq) and N,N-diisopropylethylamine (27.1 mg, 0.21 mmol, 2.0 eq) were dissolved in dichloromethane (10 mL). The reaction solution was reacted at room temperature for one hour. After completion of the reaction, the reaction solution was concentrated, and purified by preparative HPLC to obtain a target compound 6-benzyl-3-(pyridin-4-yl)-5,6-dihydro-[1,3]oxazino[5,6-f]indazol-7(1H)-one (7.2 mg, 19%).
MS m/z (ESI): 357.1 [M+H]⁺.
¹H NMR (400 MHz, MeOD-*d*₆) δ 8.65 (d, *J* = 5.1 Hz, 2H), 8.07 (d, *J* = 6.2 Hz, 2H), 7.98 (s, 1H), 7.44-7.38 (m, 4H), 7.36-7.30 (m, 1H), 7.27 (s, 1H), 4.75 (s, 2H), 4.60 (s, 2H).

### Example 103

### Preparation of 3-(pyridin-4-yl)-5,8-dihydro-[1,3]oxazino[5,4-f]indazol-7(1H)-one

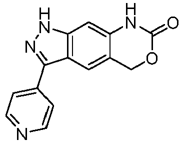

### Step 1: Preparation of 3-bromo-5-methyl-6-nitro-1H-indazole

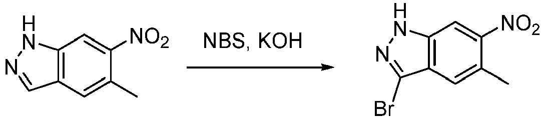

5-Methyl-6-nitro-1H-indazole (10.0 g, 56.44 mmol) was dissolved in 60 mL of DMF. The solution was added with NBS (12.1 g, 67.98 mmol) and KOH (6.3 g, 0.11 mol) successively, and stirred at room temperature for 3 h. The reaction solution was added with 200 mL of water, and stirred for 30 min. The solution was filtered, washed with water and dried to obtain a solid (7.79 g). The filtrate was extracted with dichloromethane and dried to obtain a solid 3-bromo-5-methyl-6-nitro-1H-indazole (8.33 g, yield: 57%) together with the solid above.
MS m/z (ESI): 253.9 255.9 [M-H]⁺.

### Step 2: Preparation of 3-bromo-5-methyl-6-nitro-1-trityl-1H-indazole

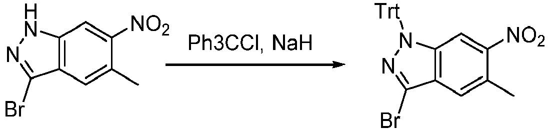

3-Bromo-5-methyl-6-nitro-1H-indazole (16.0 g, 62.48 mmol) and THF (120 mL) were stirred and cooled to 0°C. The solution was added slowly with 60% NaH (5.0 g, 0.12 mol), and stirred for 30 min. The reaction solution was added with triphenylchloromethane (26.1 g, 93.62 mmol) in batches, and stirred at room temperature for 6 h. In an ice bath, the reaction solution was added with water, extracted with ethyl acetate, dried and concentrated to dryness. The residues were added with 30 mL of methanol, and stirred for 1 h. The solution was filtered, washed with methanol and dried to obtain a solid 3-bromo-5-methyl-6-nitro-1-trityl-1H-indazole (35.72 g, yield: 100%).
MS m/z (ESI): 243 [Trt]⁺.

### Step 3: Preparation of 5-methyl-6-nitro-3-(pyridin-4-yl)-1-trityl-1H-indazole

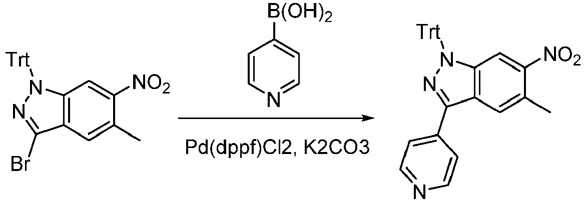

3-Bromo-5-methyl-6-nitro-1-trityl-1H-indazole (35.7 g, 71.67 mmol), pyridin-4-yl boronic acid (13.2 g, 0.11 mol), Pd(dppf)Cl₂ (2.6 g, 3.55 mmol), potassium carbonate (19.8 g, 0.14 mol) and dioxane/water (150 mL/30 mL) were stirred under a nitrogen atmosphere at 80°C overnight. The reaction solution was cooled to room temperature, added with water and ethyl acetate, filtered and dried to obtain 20.22 g of an off-white solid. The filtrate was washed with water, and concentrated to dryness. The residues were added with 30 mL of methanol and stirred. The solution was filtered, washed with methanol and dried to obtain a solid 5-methyl-6-nitro-3-(pyridin-4-yl)-1-trityl-1H-indazole(11.57 g, yield: 32%).
MS m/z (ESI): 497.2 [M+H]⁺.

### Step 4: Preparation of 5-(dibromomethyl)-6-nitro-3-(pyridin-4-yl)-1-trityl-1H-indazole

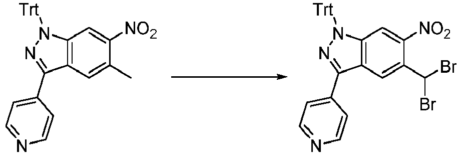

A solution of 5-methyl-6-nitro-3-(pyridin-4-yl)-1-trityl-1H-indazole(1.0 g, 2 mmol) in carbon tetrachloride (100 mL) was added with NBS (1.78 g, 10 mmol) and AIBN (164 mg, 1 mmol) at room temperature, and stirred at 80°C for 11 hours. The reaction solution was cooled to room temperature and filtered. The filter cake was washed with carbon tetrachloride (20 mL) twice, and the filtrate was concentrated to obtain a crude product 5-(dibromomethyl)-6-nitro-3-(pyridin-4-yl)-1-trityl-1H-indazole (1.3 g, yield: 99%).
MS m/z (ESI): 653.0, 655.0, 657.0 [M+H]⁺.

### Step 5: Preparation of 6-nitro-3-(pyridin-4-yl)-1H-indazole-5-carbaldehyde

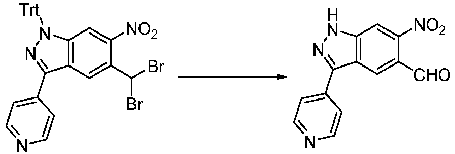

A solution of 5-(dibromomethyl)-6-nitro-3-(pyridin-4-yl)-1-trityl-1H-indazole (1.3 g, 2 mmol) in dioxane (10 mL) was added with H₂SO₄ (20 mL, 5N) at room temperature, and stirred at 100°C for 1 hour. The reaction solution was further added with H₂SO₄ (2.5 mL, 5N), and stirred at 100°C for 1 hour. The reaction solution was cooled to room temperature, added with water (100 mL), and washed with ethyl acetate (30 mL) twice. The water phase was added with solid Na₂CO₃ until pH~9 to precipitate a solid. The mixture was extracted with ethyl acetate (60 mL) twice, and most of the solids were dispersed in the organic phase. The organic phase was concentrated to obtain a crude product 6-nitro-3-(pyridin-4-yl)-1H-indazole-5-carbaldehyde (400 mg, yield: 75%).
MS m/z (ESI): 269.0 [M+H]⁺.

### Step 6: Preparation of (6-nitro-3-(pyridin-4-yl)-1H-indazol-5-yl)methanol

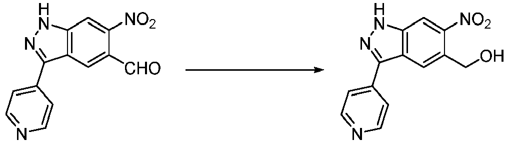

A solution of crude 6-nitro-3-(pyridin-4-yl)-1H-indazole-5-carbaldehyde (50 mg, 0.187 mmol) in ethanol (20 mL) and tetrahydrofuran (5 mL) was added with NaBH₄ (21 mg, 0.56 mmol), and stirred at room temperature for 2 hours. The reaction solution was concentrated to dryness, added with water (20 mL), and extracted with ethyl acetate (20 mL) twice. The organic phases were combined, dried over anhydrous sodium sulfate, and concentrated to obtain (6-nitro-3-(pyridin-4-yl)-1H-indazol-5-yl)methanol (28 mg, yield: 56%).
MS m/z (ESI): 271.1 [M+H]⁺.

### Step 7: Preparation of (6-amino-3-(pyridin-4-yl)-1H-indazol-5-yl)methanol

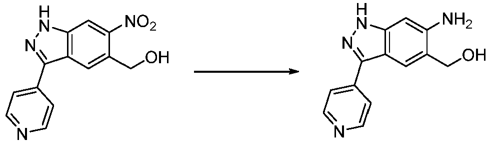

(6-Nitro-3-(pyridin-4-yl)-1H-indazol-5-yl)methanol (28 mg, 0.1 mmol) and dry Pd/C (20 mg) were stirred in tetrahydrofuran (20 mL) under hydrogen atmosphere at room temperature for 2 hours. The reaction solution was filtered, and the filtrate was concentrated to obtain (6-amino-3-(pyridin-4-yl)-1H-indazol-5-yl)methanol (20 mg, yield: 80%).
MS m/z (ESI): 241.1 [M+H]⁺.

### Step 8: Preparation of 3-(pyridin-4-yl)-5,8-dihydro-[1,3[oxazino[5,4-f]indazol-7(1H)-one

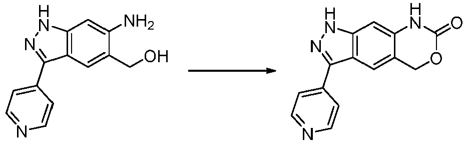

A solution of (6-amino-3-(pyridin-4-yl)-1H-indazol-5-yl)methanol (20 mg, 0.083 mmol) in dichloromethane (10 mL) and tetrahydrofuran (10 mL) was added with triphosgene (40 mg, 0.135 mmol), and stirred at room temperature for 5 minutes. The reaction solution was added dropwise with a solution of DIPEA (75 mg, 0.581 mmol) in dichloromethane (1.5 mL) slowly, and stirred at room temperature for 16 hours after completion of the addition. The reaction solution was added with methanol (5 mL), and stirred for 10 minutes. The reaction solution was further added with a solution of methylamine in methanol (2 M, 3 mL), and stirred for 2 hours. The reaction solution was concentrated, and purified by thin layer chromatography (developing solvent: CH₂Cl₂/MeOH=8/1) to obtain 3-(pyridin-4-yl)-5,8-dihydro-[1,3]oxazino[5,4-f]indazol-7(1H)-one (2 mg, yield: 9%).
¹H NMR (400 MHz, DMSO-*d*₆) δ 13.44 (s, 1H), 13.05 (s, 1H), 8.68 - 8.62 (m, 2H), 8.02 - 7.94 (m, 3H), 7.90 (s, 1H), 5.34 (s, 2H).
MS m/z (ESI): 267.1 [M+H]⁺.

### Example 104

### Preparation of ethyl 2-(7-oxo-3-(pyridin-4-yl)-1,5,7,8-tetrahydro-[1,3]oxazino[5,4-f]indazol-5-yl)acetate

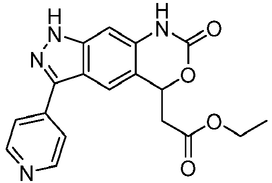

Ethyl 2-(7-oxo-3-(pyridin-4-yl)-1,5,7,8-tetrahydro-[1,3]oxazino[5,4-f]indazol-5-yl)acetate was prepared in accordance with Example 103.
¹H NMR (400 MHz, MeOD) δ 8.51 (d, *J* = 4 Hz, 2H), 7.83 (d, *J* = 4 Hz, 2H), 7.66 (s, 1H), 7.33 (s, 1H), 6.59-6.50 (m, 1H), 6.43-6.39 (m, 1H), 4.06-4.01 (q, *J =* 8 Hz, 2H), 2.94 (d, *J =* 4 Hz, 1H), 2.69 (d, *J =* 4 Hz, 1H), 1.07 (t, *J =* 8 Hz, 3H).
MS m/z (ESI): 353.1 [M+H]⁺.

### Example 105

### Preparation of 2-phenyl-1-(3-(pyridin-4-yl)-1,7-dihydroimidazo[4,5-f]indazol-6-yl)ethan-1-one

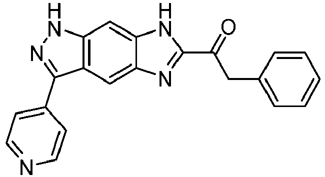

### Step 1: Preparation of 3-bromo-5,6-dinitro-1H-indazole

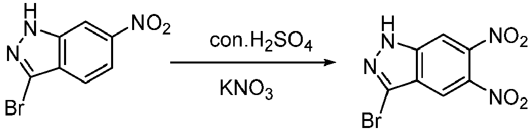

3-Bromo-6-nitro-1H-indazole (3.0 g, 12.4 mmol) was dissolved in 30 mL of concentrated sulfuric acid, and cooled to 0°C. The solution was added dropwise to a solution of potassium nitrate (1.38 g, 13.64 mmol) in concentrated sulfuric acid (30 mL) after cooling to 0°C, and stirred at 0°C for 30 minutes. The ice bath was removed, and the reaction solution was stirred at room temperature overnight. The reaction solution was added dropwise to 360 g of ice water. The precipitate was filtered, washed with water and dried to obtain a target product 3-bromo-5,6-dinitro-1H-indazole (3.2 g, yield: 90%).
¹H NMR (400 MHz, DMSO) δ: 14.72 (s, 1H), 8.59 (s, 1H), 8.54 (s, 1H).

### Step 2: Preparation of 3-bromo-1H-indazole-5,6-diamine

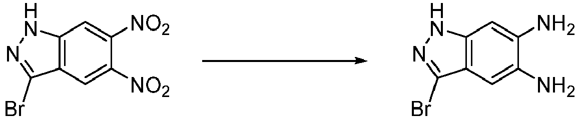

3-Bromo-5,6-dinitro-1H-indazole (3.2 g, 11.15 mmol) was dissolved in 150 mL of tetrahydrofuran and 100 mL of saturated ammonium chloride solution. The solution was added with zinc powder (7.25 g, 111.5 mmol) at 0°C under stirring, and stirred at room temperature until the reduction reaction was completed. The reaction solution was filtered through celite. The filter cake was washed with ethyl acetate, and concentrated to dryness to remove tetrahydrofuran. The residues were added with water, and extracted with ethyl acetate. The solution was washed with water, dried and concentrated to obtain a product 3-bromo-1H-indazole-5,6-diamine (2.203 g, yield: 80%), which was used directly in the next step.
¹H NMR (400 MHz, DMSO-*d*₆) δ: 12.31 (s, 1H), 6.54 (s, 1H), 6.52 (s, 1H), 5.05 (s, 2H), 4.55 (s, 2H).
MS m/z (ESI): 227 [M+H]⁺ ,229 [M+2H]⁺.

### Step 3: Preparation of 1-(3-bromo-1,7-dihydroimidazo[4,5-f]indazol-6-yl)-2-phenylethan-1-one

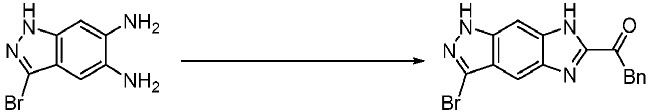

3-Bromo-1H-indazole-5,6-diamine (700 mg, 3.08 mmol) and phenylpyruvic acid (1.01 g, 6.16 mmol) were dissolved in 100 mL of dioxane, and stirred at room temperature for 10 hours. The reaction solution was concentrated to dryness to remove the solvent, and the residues were dissolved in water. The resulting solution was adjusted to alkaline by saturated sodium carbonate solution, and extracted with ethyl acetate. The solution was dried and concentrated to obtain a crude product, which was purified to obtain a target product 1-(3-bromo-1,7-dihydroimidazo[4,5-f]indazol-6-yl)-2-phenylethan-1-one (875 mg, yield: 80%), which was used directly in the next step.
MS m/z (ESI): 355 [M+H]⁺ and 357 [M+2H]⁺.

### Step 4: Preparation of di-tert-butyl 3-bromo-6-(2-phenylacetyl)imidazo[4,5-f]indazole-1,7-dicarboxylate

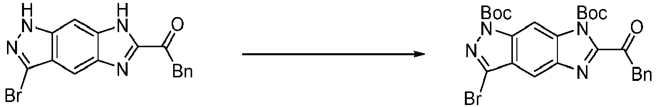

1-(3-Bromo-1,7-dihydroimidazo[4,5-f]indazol-6-yl)-2-phenylethan-1 -one (875 mg, 2.46 mmol) was dissolved in 100 mL of dichloromethane. The reaction solution was added with triethylamine (3.4 mL), DMAP (30 mg, 0.25 mmol) and B_{OC2}O (2.68 g, 12.3 mmol), and stirred at room temperature overnight. The reaction solution was concentrated and purified to obtain a product di-tert-butyl 3-bromo-6-(2-phenylacetyl)imidazo[4,5-f]indazole-1,7-dicarboxylate (128 mg, yield: 15%).
MS m/z (ESI): 555 [M+H]⁺ , 357 [M+2H]⁺.

### Step 5: Preparation of di-tert-butyl 6-(2-phenylacetyl)-3-(pyridin-4-yl)imidazo[4,5-f]indazole-1,7-dicarboxylate

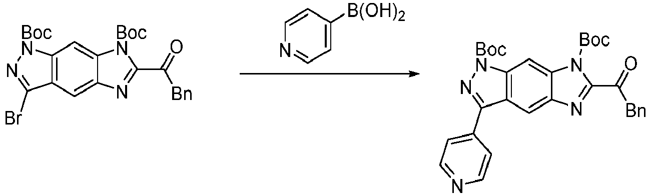

Di-tert-butyl 3-bromo-6-(2-phenylacetyl)imidazo[4,5-f]indazole-1,7-dicarboxylate (300 mg, 0.54 mmol), pyridin-4-yl boronic acid (132 mg, 1.08 mmol) and potassium carbonate (224 mg, 1.62 mmol) were dissolved in 10 ml of tetrahydrofuran and 2 mL of water. The reaction solution was purged with nitrogen to remove air, and added with Pd(dppf)Cl₂ (80 mg, 0.108 mmol). The reaction solution was stirred under a microwave condition at 90°C for 120 minutes. After completion of the reaction, the reaction solution was concentrated to remove the solvent, and extracted to obtain a crude product, which was purified by preparative TLC to obtain a target product di-tert-butyl 6-(2-phenylacetyl)-3-(pyridin-4-yl)imidazo[4,5-f]indazole-1,7-dicarboxylate (30 mg, yield: 10%).
MS m/z (ESI): 554 [M+H]⁺.

### Step 6: Preparation of 2-phenyl-1-(3-(pyridin-4-yl)-1,7-dihydroimidazo[4,5-f]indazol-6-yl)ethan-1-one

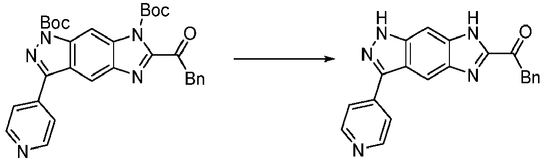

Di-tert-butyl 6-(2-phenylacetyl)-3-(pyridin-4-yl)imidazo[4,5-f]indazole-1,7-dicarboxylate (30 mg, 0.05 mmol) was dissolved in TFA/DCM (0.5 mL/2.0 mL), and stirred at room temperature for 2 hours. The reaction solution was concentrated to obtain a crude product, which was purified by preparative HPLC to obtain a target product 2-phenyl-1-(3-(pyridin-4-yl)-1,7-dihydroimidazo[4,5-f]indazol-6-yl)ethan-1-one (3.2 mg, yield: 16.8%).
¹H NMR (400 MHz, MeOD) δ 8.58-8.48 (m, 2.5H), 8.14-7.91 (m, 2.7H), 7.54 (s, 0.8H), 7.30-7.15 (m, 4.6H), 6.94-6.88 (m, 0.4H), 4.46-4.42 (m, 2H).
MS m/z (ESI): 354.1 [M+H]⁺.

### Example 107

### Preparation of 2-methyl-2-phenyl-1-(3-(pyridin-4-yl)-1,7-dihydroimidazo[4,5-f]indazol-6-yl)propa n-1-one

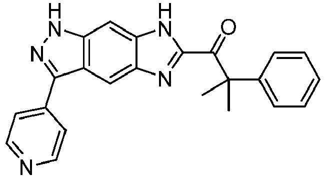

2-Methyl-2-phenyl-1-(3-(pyridin-4-yl)-1,7-dihydroimidazo[4,5-f]indazol-6-yl)prop an-1-one was prepared in accordance with Example 105.
¹H NMR (400 MHz, CDCl₃) δ 8.75 (s, 2H), 8.22 (s, 1H), 7.88 (d, *J* = 5.8 Hz, 3H), 7.50 (s, 2H), 7.31 (d*, J* = 15.0 Hz, 3H), 1.76 (s, 6H).
MS m/z (ESI): 382.1 [M+H]⁺.

### Example 108

### Preparation of (S)-3-hydroxy-2-phenyl-1-(3-(pyridin-4-yl)-1,7-dihydroimidazo [4,5-f]indazol-6-yl)p ropan-1-one

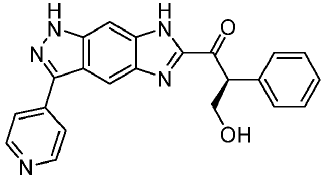

(S)-3-Hydroxy-2-phenyl-1-(3-(pyridin-4-yl)-1,7-dihydroimidazo[4,5-f]indazol-6-yl )propan-1-one was prepared in accordance with Example 105.
¹H NMR (400 MHz, CDC1₃) δ 8.75 (s, 2H), 8.31 (s, 1H), 7.89 (d, *J=* 0.5 Hz, 3H), 7.35 (s, 4H), 7.29 (s, 1H), 4.85 (s, 1H), 4.40 (s, 1H), 4.13 (s, 1H).
MS m/z (ESI): 384.1 [M+H]⁺.

### Example 109

### Preparation of (R)-2-phenyl-1-(3-(pyridin-4-yl)-1,7-dihydroimidazo[4,5-f]indazol-6-yl)propan-1-o ne

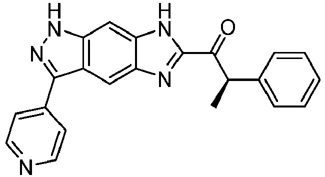

(R)-2-Phenyl-1-(3-(pyridin-4-yl)-1,7-dihydroimidazo[4,5-f]indazol-6-yl)propan-1-one was prepared in accordance with Example 105.
¹H NMR (400 MHz, CDCl₃) δ 8.75 (s, 2H), 8.32 (s, 1H), 7.89 (d, *J* = 2.3 Hz, 3H), 7.35 (s, 4H), 7.29 (s, 1H), 5.00 (s, 1H), 1.55 (s, 3H).
MS m/z (ESI): 368.1 [M+H]⁺.

### Example 110

### Preparation of 1-(3-(2-methylpyridin-4-yl)-1,7-dihydroimidazo[4,5-f]indazol-6-yl)-2-phenylethan-1-one

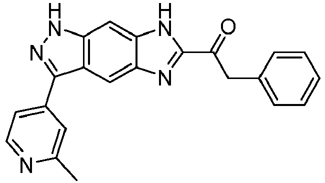

1-(3-(2-Methylpyridin-4-yl)-1,7-dihydroimidazo[4,5-f]indazol-6-yl)-2-phenylethan -1-one was prepared in accordance with Example 105.
¹H NMR (400 MHz, DMSO-*d*₆) δ 13.96 (s, 1H), 10.80 (s, 1H), 8.50 (d*, J* = 5.9 Hz, 1H), 8.00 (dd, *J* = 6.0, 2.3 Hz, 1H), 7.70 (s, 1H), 7.51 (d, *J* = 2.3 Hz, 1H), 7.34 - 7.16 (m, 5H), 4.46 - 4.41 (m, 2H), 2.69 (s, 3H).
MS m/z (ESI): 368.1 [M+H]⁺.

### Example 111

### Preparation of methyl 3-(pyridin-4-yl)-1,7-dihydroimidazo[4,5-f]indazole-6-carboxylate

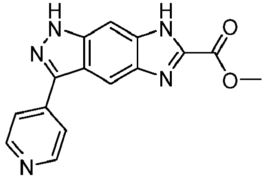

Methyl 3-(pyridin-4-yl)-1,7-dihydroimidazo[4,5-f]indazole-6-carboxylate was prepared in accordance with Example 2.
¹H NMR (400 MHz, DMSO-*d*₆) δ 13.96 (s, 1H), 10.80 (s, 1H), 8.68 - 8.62 (m, 2H), 8.00 - 7.94 (m, 2H), 7.70 (s, 2H), 3.98 (s, 3H).
MS m/z (ESI): 294.1 [M+H]⁺.

### Example 112

### Preparation of 2-methyl-2-phenyl-1-(3-(pyridin-4-yl)-1,7-dihydroimidazo[4,5-f]indazol-6-yl)propa n-1-one

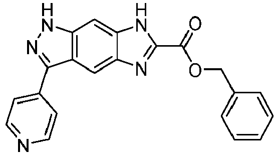

2-Methyl-2-phenyl-1-(3-(pyridin-4-yl)-1,7-dihydroimidazo[4,5-f]indazol-6-yl)prop an-1-one was prepared in accordance with Example 105.
¹H NMR (400 MHz, DMSO-*d*₆) δ 13.40-13.35 (m, 1H), 13.20-13.08 (m, 1H), 8.71 - 8.64 (m, 2H), 8.07 (s, 1H), 7.96 - 7.89 (m, 3H), 7.41 - 7.24 (m, 5H), 5.53 (d*, J* = 1.0 Hz, 2H).
MS m/z (ESI): 382.1 [M+H]⁺.

### Example 113

### Preparation of N-phenyl-3-(pyridin-4-yl)-1,7-dihydroimidazo[4,5-f]indazole-6-carboxamide

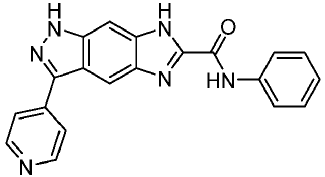

N-Phenyl-3-(pyridin-4-yl)-1,7-dihydroimidazo[4,5-f]indazole-6-carboxamide was obtained from methyl 3-(pyridin-4-yl)-1-trityl-1,7-dihydroimidazo[4,5-f]indazole-6-carboxylate and aniline as the starting materials in accordance with Steps 6 and 7 of Example 2.
¹H NMR (400 MHz, DMSO-*d*₆) δ 13.40-13.35 (m, 1H), 13.20-13.08 (m, 1H), 8.71 - 8.64 (m, 2H), 8.33 (s, 1H), 8.13 (s, 1H), 7.99 (s, 1H), 7.96 - 7.89 (m, 2H), 7.72 - 7.64 (m, 2H), 7.38 - 7.28 (m, 2H), 7.04 (tt, *J =* 7.5, 1.5 Hz, 1H).
MS m/z (ESI): 355.1 [M+H]⁺.

### Example 114

### Preparation of N-benzyl-3-(pyridin-4-yl)-1,7-dihydroimidazo[4,5-flindazole-6-carboxamide

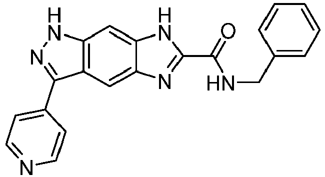

Methyl 3-(pyridin-4-yl)-1,7-dihydroimidazo[4,5-f]indazole-6-carboxylate (7.0 mg, 0.0239 mmol) and benzylamine (1mL) were stirred under a microwave condition at 100°C for 1 h. The reaction solution was concentrated to dryness, and purified by column chromatography to obtain N-benzyl-3-(pyridin-4-yl)-1,7-dihydroimidazo[4,5-f]indazole-6-carboxamide (0.8 mg, yield: 9%).
¹H NMR (400 MHz, DMSO-*d*₆) δ 13.40-13.35 (m, 1H), 13.20-13.08 (m, 1H), 8.71 - 8.64 (m, 2H), 8.13 (s, 1H), 7.99 (s, 1H), 7.96 - 7.89 (m, 2H), 7.51 (s, 1H), 7.37 - 7.23 (m, 5H), 4.64 (dd, *J* = 10.0, 1.0 Hz, 2H).
MS m/z (ESI): 369.1 [M+H]⁺.

### Example 115

### Preparation of 3-(pyridin-4-yl)-1,5,6,9-tetrahydro-8H-[1,3[oxazepino[5,4-f]indazol-8-one

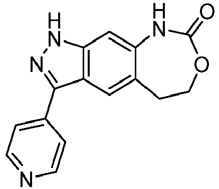

### Step 1: Preparation of 6-nitro-3-(pyridin-4-yl)-1-trityl-1H-indazole-5-carbaldehyde

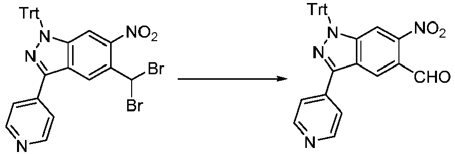

A solution of 5-(dibromomethyl)-6-nitro-3-(pyridin-4-yl)-1-trityl-1H-indazole (1.5 g, 2.29 mmol) in dioxane (80 mL) was added with K₂CO₃ (3.16 g, 22.9 mmol) and H₂O (50 mL) at room temperature, and stirred at 100°C for 18 hours. The reaction solution was cooled to room temperature, concentrated to remove dioxane, and extracted with ethyl acetate (80 mL) twice. The organic phase was concentrated and purified by column chromatography to obtain a product 6-nitro-3-(pyridin-4-yl)-1-trityl-1H-indazole-5-carbaldehyde (360 mg, yield: 31%).
MS m/z (ESI): 511.1 [M+H]⁺.

### Step 2: Preparation of 5-(2-methoxyvinyl)-6-nitro-3-(pyridin-4-yl)-1-trityl-1H -indazole

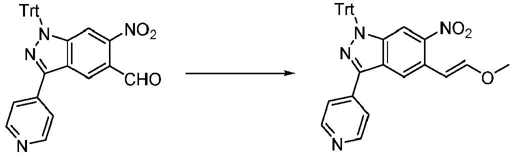

The phosphorus ylide Ph₃PCH₂OMeCl (3.45g, 10 mmol) was added to 100 mL of anhydrous THF at room temperature. The solution was cooled to 0°C under a nitrogen atmosphere, added dropwise with LiHMDS (10 mL, 10 mmol), and stirred for half an hour. The solution was added dropwise with a solution of 6-nitro-3-(pyridin-4-yl)-1-trityl-1H-indazole-5-carbaldehyde (1.0 g, 2 mmol) in THF (50 mL). The reaction solution was stirred for 2 hours, and gradually warmed to room temperature. The reaction solution was added with aqueous NH₄Cl solution (100 mL) to quench the reaction, and extracted with ethyl acetate (30 mL) three times. The organic phase was dried over anhydrous Na₂SO₄, filtered and concentrated to obtain a crude product, which was purified by column chromatography to obtain a product 5-(2-methoxyvinyl)-6-nitro-3-(pyridin-4-yl)-1-trityl-1H-indazole (650 mg, yield: 61%).
MS m/z (ESI): 539.2 [M+H]⁺.

### Step 3: Preparation of 2-(6-nitro-3-(pyridin-4-yl)-1H -indazol-5-yl)acetaldehyde

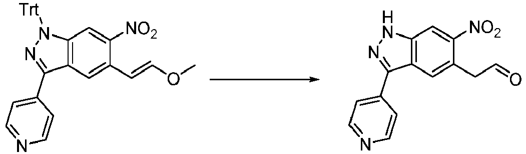

5-(2-Methoxyvinyl)-6-nitro-3-(pyridin-4-yl)-1-trityl-1H-indazole (600 mg, 1.1 mmol) was dissolved in 50 mL of anhydrous THF. The solution was added with 1.5 N HC1 (8 mL, 12 mmol), and heated to 60°C overnight. The reaction solution was added with aqueous NaHCO₃ solution until pH=7, and extracted with ethyl acetate (30 mL) three times. The organic phase was dried over anhydrous Na₂SO₄, filtered and concentrated to obtain a crude product, which was purified by column chromatography to obtain a product 2-(6-nitro-3-(pyridin-4-yl)-1H-indazol-5-yl)acetaldehyde (180 mg, yield: 58%).
MS m/z (ESI): 283.2 [M+H]⁺.

### Step 4: Preparation of 2-(6-amino-3-(pyridin-4-yl)-1H-indazol-5-yl)ethan-1-ol

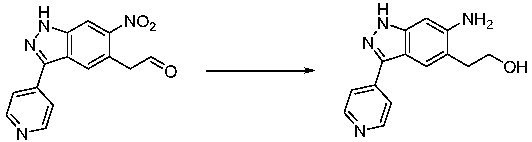

2-(6-Nitro-3-(pyridin-4-yl)-1H-indazol-5-yl)acetaldehyde (160 mg, 0.56 mmol) and wet Pd/C (50 mg) were added to tetrahydrofuran (30 mL), and stirred under hydrogen atmosphere at room temperature for 2 hours. The reaction solution was filtered, and the filtrate was concentrated to obtain a product 2-(6-amino-3-(pyridin-4-yl)-1H-indazol-5-yl)ethan-1-ol (130 mg, yield: 90%).
MS m/z (ESI): 255.2 [M+H]⁺.

### Step 5: Preparation of 3-(pyridin-4-yl)-1,5,6,9-tetrahydro-8H-[1,3]oxazepino[5,4-f]indazol-8-one

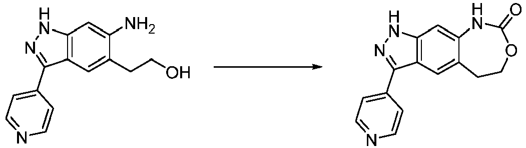

A solution of 2-(6-amino-3-(pyridin-4-yl)-1H-indazol-5-yl)ethan-1-ol (120 mg, 0.47 mmol) in dichloromethane (20 mL) and tetrahydrofuran (20 mL) was added with triphosgene (45 mg, 0.15 mmol) in an ice bath, and stirred at room temperature for 5 minutes. The reaction solution was added dropwise with a solution of DIPEA (97 mg, 0.75 mmol) in dichloromethane (5 mL) slowly, and stirred at room temperature for 16 hours after completion of the addition. The reaction solution was added with methanol (5 mL), and stirred for 10 minutes. The reaction solution was further added with a solution of methylamine in methanol (2 M, 3 mL), and stirred for 2 hours. The reaction solution was concentrated, and purified by column chromatography to obtain a product 3-(pyridin-4-yl)-1,5,6,9-tetrahydro-8H-[1,3]oxazepino[5,4-f]indazol-8-one (15 mg, yield: 11%).
¹H NMR (400 MHz, MeOD) δ: 8.73 (d, *J* = 8 Hz, 2H), 8.51 (d, *J* = 8 Hz, 2H), 8.15 (s, 1H), 7.79 (s, 1H), 4.54 (t, *J =* 6.8 Hz, 2H), 2.88 (t, *J =* 6.8 Hz, 2H).
MS m/z (ESI): 281.2 [M+H]⁺.

### Biological Assay and Evaluation

The present invention is further described below in combination with the following test examples, which do not limit the scope of the present invention.

### I. Enzyme experiment

### 1.1 Determination of inhibition effect of the present compound on ERK-1 kinase activity

The object of this test example is to determine the inhibition effect of the compound on ERK-1 kinase activity. In vitro ERK-1 kinase analysis was carried out by using LANCE Ultra (Perkin Elmer) method. The reaction (reaction system 10 µL) was conducted by adding 2.5 µL of test compound/DMSO (final 4%, V/V, diluted to 10 concentrations (400 nM to 0.02 nM) using a 1:3 dilution scheme), 5 µL of a mixed solution of ERK1 enzyme (Invitrogen, #PV3311, final concentration: 0.4 nM) and substrate Ulight-MBP peptide (Perkin Elmer, #TRF0109-M, final concentration: 0.5 µM) formulated with a kinase buffer (50 mM Hepes pH 7.4, 10 mM MgC1₂, 1 mM EGTA, 0.01% Triton X-100, 2 mM DTT) and 2.5 µL of ATP solution (Invitrogen, #PV3227, final concentration: 38.15 µM) formulated with the same buffer successively to a 384-well plate (Perkin Elmer OPTIPLATE™) and mixing well. The reaction mixture was incubated at room temperature for 60 minutes. The reaction was stopped by adding IX LANCE Detection buffer (PerkinElmer#CR97-100) diluted with ultra-pure water, 10 mM EDTA (Invitrogen#15575038) and 1 nM Eu-anti-p-MBP (PerkinElmer#TRF0201-M) antibody in 10 µL/well. The plate was incubated at room temperature for 60 minutes. Excitation reading was performed on Synergy HI Hybird Reader, H1MFD (Biotek) with an excitation wavelength of 320 nm. TR-FRET rate was calculated by dividing the acceptor emission signal (signal value at 665 nM) by the Eu donor emission signal (signal value at 615 nM). The inhibition TR-FRET rate of the well treated by the compound was calculated relative to the Max signal (DMSO control) and Min signal (no enzyme added) control wells on the plate {% inhibition rate=100-[(test compound-Min average)] / (Max average -Min average)×100}. The 10 concentrations of the compound after 4-fold dilution of the reaction system were 100 nM to 0.005 nM. Abs_IC₅₀ value was calculated by using GraphPad prism fits percentage inhibition rate and ten-point concentration data in a 4-parameter non-linear logic formula.

### 1.2 Determination of inhibition effect of the present compound on ERK2 kinase activity

The object of this test example is to determine the inhibition effect of the compound on ERK-2 kinase activity. In vitro ERK-2 kinase analysis was carried out by using LANCE Ultra (Perkin Elmer) method. The reaction (reaction system 10 µL) was conducted by adding 2.5 µL of test compound/DMSO (final 4%, V/V, diluted to 10 concentrations (400 nM to 0.02 nM) using a 1:3 dilution scheme), 5 µL of a mixed solution of ERK-2 enzyme (Invitrogen, #PV3313, final concentration: 0.08 nM) and substrate Ulight-MBP peptide (Perkin Elmer, #TRF0109-M, final concentration: 0.5 µM) formulated with a kinase buffer (50 mM Hepes pH 7.4, 10 mM MgC1₂, 1 mM EGTA, 0.01% Triton X-100, 2 mM DTT) and 2.5 µL of ATP solution (Invitrogen, #PV3227, final concentration: 38.15 µM) formulated with the same buffer successively to a 384-well plate (Perkin Elmer OPTIPLATE™) and mixing well. The reaction mixture was incubated at room temperature for 60 minutes. The reaction was stopped by adding IX LANCE Detection buffer (PerkinElmer#CR97-100) diluted with ultra-pure water, 10 mM EDTA (Invitrogen#15575038) and 1 nM Eu-anti-p-MBP (PerkinElmer#TRF0201-M) antibody in 10 µL/well. The plate was incubated at room temperature for 60 minutes. Excitation reading was performed on Synergy HI Hybird Reader, H1MFD (Biotek) with an excitation wavelength of 320 nm. TR-FRET rate was calculated by dividing the acceptor emission signal (signal value at 665 nM) by the Eu donor emission signal (signal value at 615 nM). The inhibition TR-FRET rate of the well treated by the compound was calculated relative to the Max signal (DMSO control) and Min signal (no enzyme added) control wells on the plate {% inhibition rate=100-[(test compound-Min average)] / (Max average -Min average)×100}. The 10 concentrations of the compound after 4-fold dilution of the reaction system were 100 nM to 0.005 nM. Abs_IC₅₀ value was calculated by using GraphPad prism fits percentage inhibition rate and ten-point concentration data in a 4-parameter non-linear logic formula.

It can be seen from the above experiments that: the present compound shows a biological activity of about 0.01 nM to 100 nM (IC₅₀) in the ERK inhibition test.

In some embodiments, the IC₅₀ of the present compound on ERK-1 and/or ERK-2 is less than about 100 nM, preferably less than about 10 nM, further preferably less than about 5 nM, more preferably less than about 1 nM, and most preferably less than 0.1 nM. In some embodiments, the IC₅₀ of the present compound on ERK is less than about 100 nM, preferably less than about 10 nM, further preferably less than about 5 nM, more preferably less than about 1 nM, and most preferably less than 0.1 nM. In some other embodiments, the present compound shows a double binding specificity, and can inhibit ERK kinases (for example ERK-1 kinase, ERK-2 kinase and the like) and protein kinases (for example Ras, Raf, Her-2, MEK1 and the like) with an IC₅₀ value of less than about 100 nM, less than about 10 nM, less than about 5 nM, less than about 1 nM and less than 0.1 nM.

The test data of specific Examples obtained by the above test method is shown in Table 1.

**Table 1: Relative IC₅₀ value of the compound on inhibiting ERK-1 and ERK-2 kinase activity**

| Example No. | ERK-1 IC₅₀ (nM) | ERK-2 IC₅₀ (nM) |
|---|---|---|
| Example 1 | 0.29 | 0.17 |
| Example 2 | 0.42 | 0.19 |
| Example 3 | 5.48 | 3.95 |
| Example 6 | 2.09 | 0.84 |
| Example 7 | 0.41 | 0.18 |
| Example 9 | 8.60 | 3.13 |
| Example 11 | 0.64 | 0.29 |
| Example 13 | 3.01 | 1.39 |
| Example 14 | 9.48 | 5.02 |
| Example 15 | 6.41 | 2.72 |
| Example 16 | 1.00 | 0.73 |
| Example 17 | 2.34 | 1.15 |
| Example 18 | 0.45 | 0.51 |
| Example 19 | 0.35 | 0.20 |
| Example 20 | 0.22 | 0.06 |
| Example 21 | 0.30 | 0.17 |
| Example 22 | 0.26 | 0.17 |
| Example 28 | 0.10 | 0.10 |
| Example 30 | 0.19 | 0.12 |
| Example 34 | 0.79 | 0.34 |
| Example 35 | 1.54 | 0.75 |
| Example 36 | 1.08 | 1.23 |
| Example 37 | 0.52 | 0.18 |
| Example 44 | 0.95 | 0.66 |
| Example 45 | 0.54 | 0.50 |
| Example 46 | 3.30 | 1.70 |
| Example 47 | 0.49 | 0.17 |
| Example 50 | 0.29 | 0.17 |
| Example 51 | 2.42 | 0.91 |
| Example 54 | 0.52 | 0.32 |
| Example 57 | 3.51 | 1.43 |
| Example 63 | 2.93 | 2.88 |
| Example 65 | 3.73 | 1.58 |
| Example 66 | 1.52 | 0.83 |
| Example 67 | 1.44 | 0.69 |
| Example 71 | 2.18 | 0.80 |
| Example 72 | 2.50 | 0.24 |
| Example 76 | 0.25 | 0.08 |
| Example 77 | 2.18 | 0.85 |
| Example 78 | 0.65 | 0.21 |
| Example 84 | 2.61 | 0.93 |
| Example 85 | 3.43 | 3.08 |
| Example 86 | 2.40 | 1.33 |
| Example 88 | 2.27 | 0.99 |
| Example 89 | 1.31 | 0.87 |
| Example 90 | 1.77 | 1.67 |
| Example 94 | 2.24 | 5.85 |
| Example 95 | 4.42 | 2.17 |
| Example 97 | 0.75 | 0.30 |
| Example 98 | 3.32 | 1.02 |
| Example 114 | 2.86 | 1.07 |

### 1.3 Determination of the inhibition activity of the present compound on tumor cell proliferation

The object of this test example is to determine the inhibition effect of the present compound on tumor cell proliferation activity. The inhibition activity of the compound on tumor cell proliferation was determined by CellTiter-Glo method, and the half inhibitory concentration IC₅₀ of the compound for inhibiting cell proliferation activity was obtained. 50~100 µL of tumor cell suspension was inoculated in a 96-well cell culture plate with a density of 1∼5*10⁴ cells/ml. The plate was incubated in an incubator for 16~24 hours (37°C, 5% CO₂). Solutions of test compounds at different concentrations obtained by gradient dilution were added to the cells in the plate. The plate was incubated in an incubator for 3-7 days (37°C, 5% CO₂). 50∼100 µL of CellTiter-Glo reagent was added to each well. The plate was shaked for 10 minutes, and left to stand at room temperature for 10 minutes. The value of the chemiluminescence signal was determined by a microplate reader. The inhibition rate was calculated from the value of the chemiluminescence signal. IC₅₀ of the compound was obtained by curve fitting based on the inhibition rate at different concentrations.

The inhibition effect of the present compound on pancreatic cancer tumor cell Mia Paca 2 proliferation activity was determined by the test, and the obtained IC₅₀ value is shown in Table 2.

**Table 2: Relative IC₅₀ value of the compound on inhibiting pancreatic cancer tumor cell Mia Paca 2 proliferation activity**

| Example No. | IC₅₀ on cell proliferation (nM) Miapaca-2 cell |
|---|---|
| Example 1 | 122.9 |
| Example 2 | 69.06 |
| Example 18 | 109.5 |
| Example 19 | 159.2 |
| Example 21 | 59.06 |
| Example 22 | 99.38 |
| Example 28 | 202.1 |
| Example 30 | 61.63 |
| Example 36 | 244.4 |
| Example 45 | 110.0 |
| Example 50 | 55.54 |
| Example 54 | 309.2 |

Conclusion: the present compound has an obvious inhibition effect on tumor cell proliferation activity.

### II. Pharmacokinetics assay in mice

### 2.1 Research object:

Balb/c Mouse was used as the test animal. The pharmacokinetic behavior of compounds of Example 1, 2 and 7 administrated orally was studied in mice (plasma).

### 2.2 Test protocol

### 2.2.1 Test compounds:

Compounds of Example 1, 2 and 7 prepared by the present invention.

### 2.2.2 Test animals

Male Balb/c Mouse, purchased from Shanghai Jiesijie Laboratory Animal Co., LTD, with Certificate No.: SCXK (Shanghai) 2013-0006 N0.311620400001794.

### 2.2.3 Preparation of the test compounds:

170 g of PEG400 and 20 g of Solutol HS-15 were added to a 250 mL glass flask, and then 10 mL of NMP was added. The solution was mixed well by ultrasound for 10 minutes to obtain a clear solution.

11.2 mg of the compound of Example 1, 10.8 mg of the compound of Example 2 and 9.8 mg of the compound of Example 7 were weighted to a 4 mL glass flasks respectively. 1.940 mL, 2.160 mL and 1.525 mL of the above solution were treated by ultrasound for 10 minutes to obtain a colorless clear solution with a concentration of 5 mg/mL.

### 2.2.4 Administration:

After fasting overnight, the male Balb/c mice were administrated with the compounds (PO), the dose was 50 mg/kg, and the administration volume was 10 mL/kg.

### 2.2.5 Sampling:

Blood was taken before administration and at 0.25, 0.5, 1, 2, 4, 6, 8 and 24 hours after administration. The blood samples were stored in EDTA-2K tubes, and centrifuged for 6 minutes to separate the blood plasma. The plasma samples were stored at -80°C. The mice were fed 4 h after administration.

### 2.2.5 Test results:

The test results obtaind by LCMS/MS method are shown in Table 3.

**Table 3: Pharmacokinetic parameters of the compound in mouse**

| Example No. | Tmax (hr) | Cmax (ng/mL) | AUC₀₋₂₄ (ng/mL*hr) | T_{1/2} (hr) | MRT (hr) |
|---|---|---|---|---|---|
| Example 1 | 0.5 | 1310.0 | 14740.0 | 6.59 | 8.99 |
| Example 2 | 2 | 15866.7 | 50393.4 | 0.82 | 2.44 |
| Example 7 | 1 | 8256.7 | 27324.9 | 0.83 | 2.33 |

The compounds of Example 1, 2 and 7 have a good exposure in mouse plasma at a dose of 50 mg/kg.

### III. Tumor inhibtion experiment in MiaPaca 2 xenograft model

### 3.1 Experiment object:

BALB/c nude mice were used as test animals. In vivo pharmacodynamic test was carried out in human pancreatic cancer cell MiaPaca 2 xenograft (CDX) model to evaluate the antitumor effect of the test compound.

### 3.2 Experimental instruments and reagents:

### 3.2.1 Instruments:

Clean bench (BSC-1300II A2, Medical Equipment Factory of Shanghai Boxun Industrial Co., Ltd.)
CO₂ incubator (Thermo-311, Thermo)
Centrifuge (Centrifuge 5720R, Eppendorf)
Automatic cell counter (Countess II, Life Technologies)
Pipette (10-20 µL, Eppendorf)
Microscope (Ts 2, Nikon)
Vernier caliper (CD-6"AX, Mitutoyo, Japan)
Cell culture flask (T25/T75/T225, Corning)
Constant temperature water tank (HWS12, Shanghai Yiheng Scientific)

### 3.2.2 Reagents:

DMEM (11995-065, Gibco)
Fetal bovine serum (FBS) (10091-148, Gibco)
0.25% trypsin (25200-056, Gibco)
Streptomycin-penicillin (P/S) (SV30010, GE)
Phosphate buffered saline (PBS) (10010-023, Gibco)
Matrigel (356234, Corning)
Gln (25030-081, Gibco)

### 3.3 Experimental process:

MiaPaca 2 cells were obtained from the cell bank. After recovery, the cells were added to a DMEM medium (containing 10% FBS, 1% Glu and 1% P/S), and incubated in the CO₂ incubator (the incubator temperature was 37°C, and the CO₂ concentration was 5%). The cells were subjected to passage after spreading 80-90% of the bottom of the culture flask. After passage, the cells were incubated in the CO₂ incubator. This process was repeated until the number of cells met the required by *in vivo* pharmacological inoculation. Cells in log phase were collected, and counted by the automatic cell counter. The cells were re-suspended with PBS and Matrigel (1:1 by volume) based on the counting results to obtain a cell suspension (cell density: 8 cells/ml), wich was placed in an ice box.

Female BALB/c nude mice (6-8 weeks old, body weight: 18-22 grams) were used as test animals. The mice were maintained in individually ventilated cages (5 mice per cage) in a special pathogen-free environment. All cages, padding and water were disinfected before use. All animals had free access to standard certified commercial laboratory diets. The nude mice were labeled with disposable ear tags before the experiment. The skin of the inoculated area was sterilized with 75% medical alcohol before inoculation. Each mouse was subcutaneously inoculated with 0.1 ml (containing 8*10⁶ cells) of MiaPaca 2 tumor cells on the right back. Administration was started when the average tumor volume reached 100-200 mm³. The test compound was administrated intragastrically daily. The administration dose, frequency and efficacy of each group at the end of the experiment are shown in Tables 4 and 5. The tumor volume (mm³) was measured with vernier caliper twice a week. Calculation formula: V=0.5*D*d*d, wherein D and d refer to the major and minor diameter of the tumor, respectively. The anti-tumor efficacy was determined by dividing the average tumor increase volume of compound-treated animals by the average tumor increase volume of untreated animals. Calculation formula of tumor inhibition rate: TGI(%)=1-[(Vt-V0)administration group/(Vt-V0)solvent control group]*100%. All animals were euthanized after the experiment.

**Tables 4: Pharmacodynamic parameters of the compound in xenograft mouse**

| **Groups** | **Tumor volume (mm³, Mean ± SD)** | | **ΔT/ΔC(%)** | **TGI (%)** |
|---|---|---|---|---|
| | **Day 0** | **Day 21** | **Day 21** | **Day 21** |
| Vehicle QD x 3w | 156±27 | 805±246 | - | - |
| Example 2 | 156±24 | 214±104 | 8.86 | 91.14 |
| 100 mg/kg QD x3w | | | | |
| Example 2 50 mg/kg BID x 3w | 156±27 | 253±32 | 14.93 | 85.07 |
| Example 7 100 mg/kg QD x 3w | 156±33 | 426±205 | 41.63 | 58.3 |

After continuous oral administration for 21 days, the compound of Example 2 can significantly inhibit the growth of xenograft in MiaPaca 2 nude mouse under the two administration conditions of 100 mg/kg QD and 50 mg/kg BID, and shows a good efficacy; the compound of Example 7 can also significantly inhibit the growth of tumor under the administration condition of 100 mg/kg QD.

**Tables 5: Pharmacodynamic parameters of the compound in xenograft mouse**

| **Groups** | **Tumor volume (mm³, Mean ± SD)** | | **ΔT/ΔC(%)** | **TGI(%)** |
|---|---|---|---|---|
| | **Day 0** | **Day 16** | **Day 16** | **Day 16** |
| Vehicle QD x 16d | 155±21 | 606±143 | - | - |
| Example 1 50 mg/kg QD x 16d | 154±29 | 214±97 | 13.37 | 86.63 |

After continuous oral administration for 16 days, the compound of Example 1 can significantly inhibit the growth of xenograft in MiaPaca 2 nude mouse under the administration condition of 50 mg/kg QD.

### IV. hERG potassium channel inhibition activity test

### 4.1 Cell preparation

4.1.1 CHO-hERG cells were cultured in a 175 cm2 culture flask. After the cell density reached 60∼80%, the culture solution was removed. The cells were washed with 7 mL of PBS once, and dissociated with 3 mL of Detachin.
4.1.2 After completion of dissociation, the cells were neutralized with 7 mL of culture solution. The solution was centrifuged, and the supernate was removed. The cells were resuspended in 5 mL of culture solution. The cell indensity is ensured as 2-5 resuspension.

### 4.2 Solution formulation

**Tables 6: Components of intracellular and extracellular fluids**

| Reagents | Extracellular fluid (mM) | Intracellular fluid (mM) |
|---|---|---|
| CaCl₂ | 2 | 5.374 |
| MgCl₂ | 1 | 1.75 |
| KCl | 4 | 120 |
| NaCl | 145 | - |
| Glucose | 10 | - |
| HEPES | 10 | 10 |
| EGTA | - | 5 |
| Na-ATP | - | 4 |
| pH | 7.40 (adjusted with NaOH), Osmolarity∼305 mOsm | 7.25 (adjusted with KOH), Osmolarity∼290 mOsm |

### 4.3 Electrophysiological recording process

Single cell sealing impedance and formation of whole-cell mode were automatically performed by Qpatch instrument. After obtaining the whole-cell recording mode, the cell was clamped at -80 mV. The cell first underwent pre-voltage of -50 mV for 50 msec, then underwent depolarization stimulation at +40 mV for 5 sec, and then underwent repolarization at -50 mV for 5 sec, and then the voltage returned to -80 mV. The cell underwent the stimulation at the voltage every 15 sec, and the data were recorded for 2 min, then extracellular fluid was administrated, and then the data were recorded for 5 min. Then, the administration process begun. The concentration of the test compound started from the lowest concentration, each test concentration was administrated for 2.5 min. At least three cells were tested for each concentration.

### 4.4 Compound formulation

4.4.1 20 mM mother liquor of the compound was diluted with extracellular fluid. 5 uL of 20 mM mother liquor of the compound was added with 2495 uL of extracellular fluid to obtain a concentration of 40 uM (500-fold dilution). The solution was subjected to a 3-fold serial dilution with extracellular fluid containing 0.2% DMSO to obtain a required final concentration.
4.4.2 The highest test concentration was 40 uM. The 6 concentrations were 40, 13.33, 4.44, 1.48, 0.49 and 0.16 uM.
4.4.3 The DMSO content in the final test concentration did not exceed 0.2%. This concentration of DMSO had no effect on hERG potassium channel.

### 4.5 Data analysis

The experimental data was analyzed by XLFit software.

### 4.6 Quality control

Environment: humidity 20∼50%, temperature 22∼25%
Reagents: the reagents used were purchased from Sigma, with a purity of> 98%
The experimental data in the report must meet the following criteria:
Whole cell sealing impedance > 100 M
Tail current amplitude > 400 pA
Pharmacological parameters:
The inhibition effect of Cisapride at multiple concentrations on hERG channel was used as the positive control.

### 4.7 Experimental results

Results of inhibition effect of the Examples at multiple concentrations on hERG current:

**Tables 7: Results of inhibition effect of Examples at multiple concentrations on hERG current**

| Example No. | hERG (uM) |
|---|---|
| Example 2 | >40 |
| Example 105 | >40 |

Inhibition of cardiac hERG potassium channel by drug is the main cause of drug-induced QT prolongation syndrome. It can be seen from the experimental results that the compounds of Examples 2 and 105 have no inhibition effect on cardiac hERG potassium channel. Cardiotoxic effects at high doses can thus be avoided.

In summary, the present invention provides a series of highly active, highly selective ERK1/2 kinase inhibitors with novel structures. The compound shows an excellent pharmacokinetic property in rat and mouse, and exhibits a good efficacy in Miapaca tumor-bearing mouse model. The compound has a great potential to be developed as a drug against oncological diseases.

## Claims

1. A compound of formula (I), a stereoisomer thereof or a pharmaceutically acceptable salt thereof: 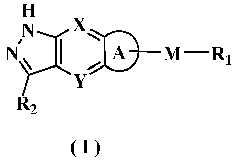 wherein:
X and Y are each independently selected from the group consisting of N and -CR₃;
M is selected from the group consisting of a bond, 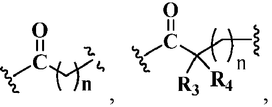 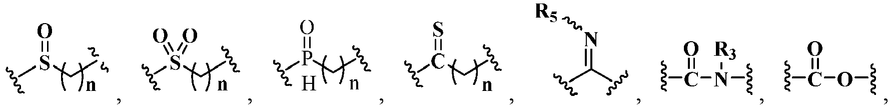 -(CH₂)ₙ- and -CR₃R₄;
ring A is selected from the group consisting of cycloalkyl, heterocyclyl, aryl and heteroaryl, wherein the cycloalkyl, heterocyclyl, aryl and heteroaryl are optionally further substituted by one or more substituents selected from the group consisting of deuterium, oxo, alkyl, deuterated alkyl, halogen, amino, nitro, hydroxy, cyano, alkoxy, hydroxyalkyl, cycloalkyl, heterocyclyl, aryl, heteroaryl, -(CH₂)ₙR₃, -(CH₂)ₙOR₃, -(CH₂)ₙSR₃, -(CH₂)ₙC(O)R₃, -(CH₂)ₙC(O)OR₃, -(CH₂)ₙS(O)ₘR₃, -(CH₂)ₙNR₃R₄, -(CH₂)ₙC(O)NR₃R₄, -(CH₂)ₙNR₃C(O)R₄ and -(CH₂)ₙNR₃S(O)ₘR₄;
R₁ is selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl, heteroaryl, -(CH₂)ₙR₃, -(CH₂)ₙOR₃, -(CH₂)ₙSR₃, -(CH₂)ₙC(O)R₃, -(CH₂)ₙC(O)OR₃, -(CH₂)ₙS(O)ₘR₃, -(CH₂)ₙNR₃R₄, -(CH₂)ₙC(O)NR₃R₄, -(CH₂)ₙNR₃C(O)R₄ and -(CH₂),NR₃S(O)ₘR₄, wherein the alkyl, haloalkyl, cycloalkyl, heterocyclyl, aryl and heteroaryl are optionally further substituted by one or more substituents selected from the group consisting of deuterium, alkyl, haloalkyl, halogen, amino, nitro, cyano, hydroxy, alkoxy, haloalkoxy, hydroxyalkyl, cycloalkyl, heterocyclyl, aryl, heteroaryl, -(CH₂)ₙR₅, -(CH₂)ₙOR₅, -(CH₂)ₙSR₅, -(CH₂)ₙC(O)R₅, -(CH₂)ₙC(O)OR₅, -(CH₂)ₙS(O)ₘR₅, -(CH₂)ₙNR₅R₆, -(CH₂)ₙC(O)NR₅R₆, -(CH₂)ₙNR₅C(O)R₆ and -(CH₂)ₙNR₅S(O)ₘR₆;
R₂ is selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl, heteroaryl, -(CH₂)ₙR₃, -(CH₂)ₙOR₃, -(CH₂)ₙSR₃, -(CH₂)ₙC(O)R₃, -(CH₂)ₙC(O)OR₃, -(CH₂)ₙS(O)ₘR₃, -(CH₂)ₙNR₃R₄, -(CH₂)ₙC(O)NR₃R₄, -(CH₂)ₙNR₃C(O)R₄ and -(CH₂)ₙNR₃S(O)ₘR₄, wherein the alkyl, haloalkyl, cycloalkyl, heterocyclyl, aryl and heteroaryl are optionally further substituted by one or more substituents selected from the group consisting of deuterium, oxo, alkyl, haloalkyl, halogen, amino, nitro, cyano, hydroxy, alkoxy, haloalkoxy, hydroxyalkyl, cycloalkyl, heterocyclyl, aryl, heteroaryl, -(CH₂)ₙR₅, -(CH₂)ₙORs, -(CH₂)ₙSR₅, -(CH₂)ₙC(O)R₅, -(CH₂)ₙC(O)OR₅, -(CH₂)ₙS(O)ₘR₅, -(CH₂)ₙNR₅R₆, -(CH₂)ₙC(O)NR₅R₆, -(CH₂)ₙC(O)NHR₅, -(CH₂)ₙNR₅C(O)R₆ and -(CH₂)ₙNR₅S(O)ₘR₆;
R₃ and R₄ are identical or different and are each independently selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl, heteroaryl, -(CH₂)ₙR₅, -(CH₂)ₙOR₅, -(CH₂)ₙSR₅, -(CH₂)ₙC(O)R₅, -(CH₂)ₙC(O)OR₅, -(CH₂)ₙS(O)ₘR₅, -(CH₂)ₙNR₅R₆, -(CH₂)ₙC(O)NR₅R₆, -(CH₂)ₙC(O)NHR₅, -(CH₂)ₙNR₅C(O)R₆ and -(CH₂)ₙNR₅S(O)ₘR₆, wherein the alkyl, haloalkyl, cycloalkyl, heterocyclyl, aryl and heteroaryl are optionally further substituted by one or more substituents selected from the group consisting of deuterium, alkyl, halogen, hydroxy, amino, nitro, cyano, ester group, alkoxy, hydroxyalkyl, substituted or unsubstituted cycloalkyl, substituted or unsubstituted heterocyclyl, substituted or unsubstituted aryl and substituted or unsubstituted heteroaryl;
R₅ and R₆ are identical or different and are each independently selected from the group consisting of hydrogen, deuterium, alkyl, deuterated alkyl, haloalkyl, hydroxy, amino, cycloalkyl, heterocyclyl, aryl and heteroaryl, wherein the alkyl, cycloalkyl, heterocyclyl, aryl and heteroaryl are optionally further substituted by one or more substituents selected from the group consisting of deuterium, alkyl, halogen, hydroxy, amino, nitro, cyano, alkoxy, hydroxyalkyl, cycloalkyl, heterocyclyl, aryl and heteroaryl;
m is an integer of 0, 1 or 2; and
n is an integer of 0, 1, 2, 3, 4 or 5.

2. The compound of formula (I), a stereoisomer thereof or a pharmaceutically acceptable salt thereof according to claim 1, which is a compound of formula (II), a stereoisomer thereof or a pharmaceutically acceptable salt thereof: 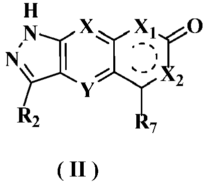 wherein:
X and Y are each independently selected from the group consisting of N and -CR₃, and preferably CH;
X₁ and X₂ are each independently selected from the group consisting of O, -NR₃ and -CR₃;
R₂ is selected from the group consisting of -(CH₂)ₙR₃, -(CH₂)ₙOR₃, -(CH₂)ₙNR₃R₄, cycloalkyl, heterocyclyl, aryl and heteroaryl, wherein the cycloalkyl, heterocyclyl, aryl and heteroaryl are optionally further substituted by one or more substituents selected from the group consisting of deuterium, oxo, alkyl, deuterated alkyl, halogen, amino, nitro, hydroxy, cyano, alkoxy, hydroxyalkyl, cycloalkyl, heterocyclyl, aryl, heteroaryl, -(CH₂)ₙR₅, -(CH₂)ₙOR₅, -(CH₂)ₙSR₅, -(CH₂)ₙC(O)R₅, -(CH₂)ₙC(O)OR₅, -(CH₂)ₙS(O)ₘR₅, -(CH₂)ₙNR₅R₆, -(CH₂)ₙC(O)NR₅R₆, -(CH₂)ₙC(O)NHR₅, -(CH₂)ₙNR₅C(O)R₆ and -(CH₂)ₙNR₅S(O)ₘR₆;
R₇ is selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl, heteroaryl, -(CH₂)ₙR₃, -(CH₂)ₙOR₃, -(CH₂)ₙSR₃, -(CH₂)ₙC(O)R₃, -(CH₂)ₙC(O)OR₃, -(CH₂)ₙS(O)ₘR₃, -(CH₂)ₙNR₃R₄, -(CH₂)ₙC(O)NR₃R₄, -(CH₂)ₙNR₃C(O)R₄ and -(CH₂)ₙNR₃S(O)ₘR₄, wherein the alkyl, haloalkyl, cycloalkyl, heterocyclyl, aryl and heteroaryl are optionally further substituted by one or more substituents selected from the group consisting of deuterium, alkyl, haloalkyl, halogen, amino, nitro, cyano, hydroxy, alkoxy, haloalkoxy, hydroxyalkyl, cycloalkyl, heterocyclyl, aryl, heteroaryl, -(CH₂)ₙR₅, -(CH₂)ₙOR₅, -(CH₂)ₙSR₅, -(CH₂)ₙC(O)R₅, -(CH₂)ₙC(O)OR₅, -(CH₂)ₙS(O)ₘR₅, -(CH₂)ₙNR₅R₆, -(CH₂)ₙC(O)NR₅R₆, -(CH₂)ₙNR₅C(O)R₆ and -(CH₂)ₙNR₅S(O)ₘR₆;
R₃∼R₆, m and n are as defined in claim 1.

3. The compound of formula (I), a stereoisomer thereof or a pharmaceutically acceptable salt thereof according to claim 1, which is a compound of formula (III), a stereoisomer thereof or a pharmaceutically acceptable salt thereof: 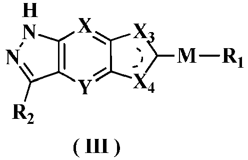 wherein:
X and Y are each independently selected from the group consisting of N and -CR₃, and preferably CH;
X₃ and X₄ are each independently selected from the group consisting of N, NR₃ and -CR₃;
R₂ is selected from the group consisting of -(CH₂)ₙR₃, -(CH₂)ₙOR₃, -(CH₂)ₙNR₃R₄, cycloalkyl, heterocyclyl, aryl and heteroaryl, wherein the cycloalkyl, heterocyclyl, aryl and heteroaryl are optionally further substituted by one or more substituents selected from the group consisting of deuterium, oxo, alkyl, deuterated alkyl, halogen, amino, nitro, hydroxy, cyano, alkoxy, hydroxyalkyl, cycloalkyl, heterocyclyl, aryl, heteroaryl, -(CH₂)ₙR₅, -(CH₂)ₙOR₅, -(CH₂)ₙSR₅, -(CH₂)ₙC(O)R₅, -(CH₂)ₙC(O)OR₅, -(CH₂)ₙS(O)ₘR₅, -(CH₂)ₙNR₅R₆, -(CH₂)ₙC(O)NR₅R₆, -(CH₂)ₙC(O)NHR₅, -(CH₂)ₙNR₅C(O)R₆ and -(CH₂)ₙNR₅S(O)ₘR₆;
M, R₁, R₃∼R₆, m and n are as defined in claim 1.

4. The compound of formula (I), a stereoisomer thereof or a pharmaceutically acceptable salt thereof according to claim 1, which is a compound of formula (IV), a stereoisomer thereof or a pharmaceutically acceptable salt thereof: 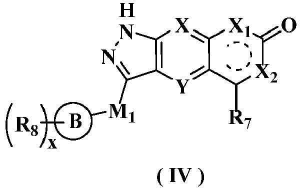 wherein:
M₁ is selected from the group consisting of a bond, O, NR₃, 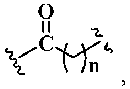 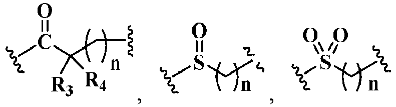 and -(CH₂)ₙ-, and preferably selected from the group consisting of a bond, O, NR₃, 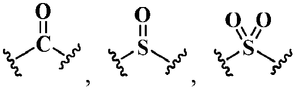 and -(CH₂)ₙ-;
ring B is selected from the group consisting of cycloalkyl, heterocyclyl, aryl and heteroaryl, wherein the cycloalkyl, heterocyclyl, aryl and heteroaryl are optionally further substituted by one or more substituents selected from the group consisting of deuterium, oxo, alkyl, deuterated alkyl, halogen, amino, nitro, hydroxy, cyano, alkoxy, hydroxyalkyl, cycloalkyl, heterocyclyl, aryl and heteroaryl;
R₈ is selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl and heteroaryl, wherein the alkyl, haloalkyl, cycloalkyl, heterocyclyl, aryl and heteroaryl are optionally further substituted by one or more substituents selected from the group consisting of deuterium, alkyl, haloalkyl, halogen, amino, nitro, cyano, hydroxy, alkoxy, haloalkoxy, hydroxyalkyl, cycloalkyl, heterocyclyl, aryl and heteroaryl;
x is an integer of 0, 1, 2, 3 or 4; and
X, Y, X₁, X₂, R₃∼R₇, m and n are as defined in claim 2.

5. The compound of formula (I), a stereoisomer thereof or a pharmaceutically acceptable salt thereof according to claim 3, which is a compound of formula (V), a stereoisomer thereof or a pharmaceutically acceptable salt thereof: 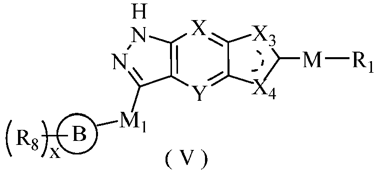 wherein:
X₃ and X₄ are each independently selected from the group consisting of N, NR₃ and -CR₃;
M₁ is selected from the group consisting of a bond, O, NR₃, 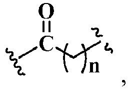 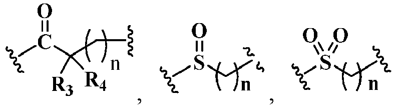 and -(CH₂)ₙ-, and preferably selected from the group consisting of a bond, O, NR₃, 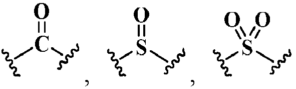 and -(CH₂)ₙ-;
ring B is selected from the group consisting of cycloalkyl, heterocyclyl, aryl and heteroaryl, wherein the cycloalkyl, heterocyclyl, aryl and heteroaryl are optionally further substituted by one or more substituents selected from the group consisting of deuterium, oxo, alkyl, deuterated alkyl, halogen, amino, nitro, hydroxy, cyano, alkoxy, hydroxyalkyl, cycloalkyl, heterocyclyl, aryl and heteroaryl;
R₈ is selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl and heteroaryl, wherein the alkyl, haloalkyl, cycloalkyl, heterocyclyl, aryl and heteroaryl are optionally further substituted by one or more substituents selected from the group consisting of deuterium, alkyl, haloalkyl, halogen, amino, nitro, cyano, hydroxy, alkoxy, haloalkoxy, hydroxyalkyl, cycloalkyl, heterocyclyl, aryl and heteroaryl;
M, X, Y, R₁, m, n and x are as defined in claim 3.

6. The compound of formula (I), a stereoisomer thereof or a pharmaceutically acceptable salt thereof according to claim 5, which is a compound of formula (V-A), (V-B) or (V-C), a stereoisomer thereof or a pharmaceutically acceptable salt thereof: 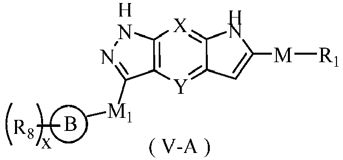 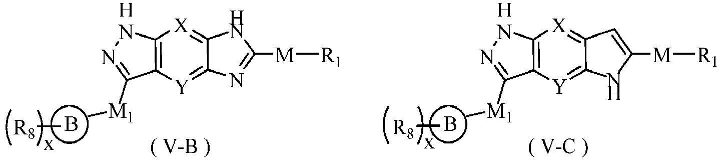 wherein:
ring B, M, M₁, X, Y, R₁, R₈ and x are as defined in claim 5.

7. The compound of formula (I), a stereoisomer thereof or a pharmaceutically acceptable salt thereof according to claim 5, which is a compound of formula (V-D), a stereoisomer thereof or a pharmaceutically acceptable salt thereof: 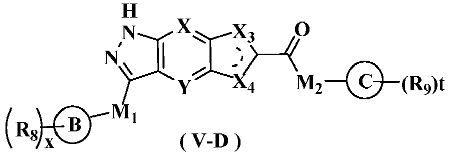 wherein:
M₂ is selected from the group consisting of a bond, -(CH₂)ₙCR₃R₄-, -O(CH₂)ₙCR₃R₄-, -(CH₂)ₙNR₃-, -(CH₂)ₙN(R₃)NR₄-, -(CH₂)ₙNHCR₃R₄- and -CR₃R₄-;
ring C is selected from the group consisting of cycloalkyl, heterocyclyl, aryl and heteroaryl, wherein the cycloalkyl, heterocyclyl, aryl and heteroaryl are optionally further substituted by one or more substituents selected from the group consisting of deuterium, oxo, alkyl, deuterated alkyl, halogen, amino, nitro, hydroxy, cyano, alkoxy, hydroxyalkyl, cycloalkyl, heterocyclyl, aryl and heteroaryl;
ring C is preferably a structure selected from the group consisting of: 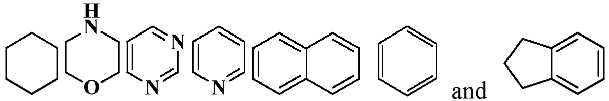
R₉ is selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl, heteroaryl, -(CH₂)ₙR₃, -(CH₂)ₙOR₃, -(CH₂)ₙSR₃, -(CH₂)ₙC(O)R₃, -(CH₂)ₙC(O)OR₃, -(CH₂)ₙS(O)ₘR₃, -(CH₂)ₙNR₃R₄, -(CH₂)ₙC(O)NR₃R₄, -(CH₂)ₙNR₃C(O)R₄ and -(CH₂)ₙNR₃S(O)ₘR₄;
t is an integer of 0, 1, 2, 3, 4 or 5;
ring B, M₁, X, Y, R₈ and x are as defined in claim 5.

8. The compound of formula (I), a stereoisomer thereof or a pharmaceutically acceptable salt thereof according to claims 4 to 7,
wherein:
ring B is a structure selected from the group consisting of:

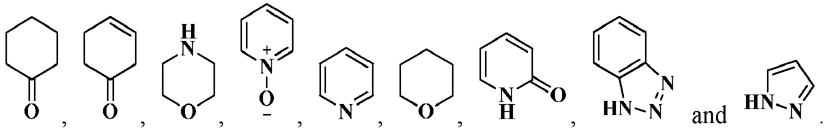

9. The compound of formula (I), a stereoisomer thereof or a pharmaceutically acceptable salt thereof according to claim 4, which is a compound of formula (VI), a stereoisomer thereof or a pharmaceutically acceptable salt thereof: 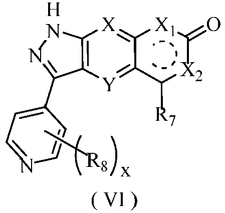 wherein:
X, Y, X₁, X₂, R₇, R₈ and x are as defined in claim 4.

10. The compound of formula (I), a stereoisomer thereof or a pharmaceutically acceptable salt thereof according to claim 5, which is a compound of formula (VII), a stereoisomer thereof or a pharmaceutically acceptable salt thereof: 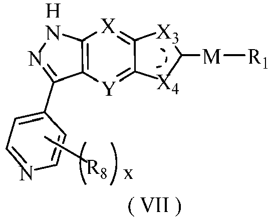 wherein:
M, X, Y, X₃, X₄, R₁, R₈ and x are as defined in claim 5.

11. The compound of formula (I), a stereoisomer thereof or a pharmaceutically acceptable salt thereof according to claim 1, which is a compound of formula (VIII), a stereoisomer thereof or a pharmaceutically acceptable salt thereof: 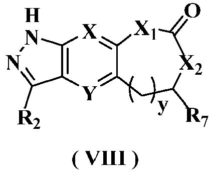 wherein:
X and Y are each independently selected from the group consisting of N and -CR₃, and preferably CH;
X₁ and X₂ are each independently selected from the group consisting of O, -NR₃ and -CR₃;
R₂ is selected from the group consisting of -(CH₂)ₙR₃, -(CH₂)ₙOR₃, -(CH₂)ₙNR₃R₄, cycloalkyl, heterocyclyl, aryl and heteroaryl, wherein the cycloalkyl, heterocyclyl, aryl and heteroaryl are optionally further substituted by one or more substituents selected from the group consisting of deuterium, alkyl, deuterated alkyl, halogen, amino, nitro, hydroxy, cyano, alkoxy, hydroxyalkyl, cycloalkyl, heterocyclyl, aryl, heteroaryl, -(CH₂)ₙR₅, -(CH₂)ₙOR₅, -(CH₂)ₙSR₅, -(CH₂)ₙC(O)R₅, -(CH₂)ₙC(O)OR₅, -(CH₂)ₙS(O)ₘR₅, -(CH₂)ₙNR₅R₆, -(CH₂)ₙC(O)NR₅R₆, -(CH₂)ₙNR₅C(O)R₆ and -(CH₂)ₙNR₅S(O)ₘR₆;
R₇ is selected from the group consisting of hydrogen, deuterium, alkyl, deuterated alkyl, haloalkyl, alkoxy, haloalkoxy, halogen, amino, nitro, hydroxy, cyano, cycloalkyl, heterocyclyl, aryl, heteroaryl, -(CH₂)ₙR₃, -(CH₂)ₙOR₃, -(CH₂)ₙSR₃, -(CH₂)ₙC(O)R₃, -(CH₂)ₙC(O)OR₃, -(CH₂)ₙS(O)ₘR₃, -(CH₂)ₙNR₃R₄, -(CH₂)ₙC(O)NR₃R₄, -(CH₂)nNR₃C(O)R₄ and -(CH₂)ₙNR₃S(O)ₘR₄, wherein the alkyl, haloalkyl, cycloalkyl, heterocyclyl, aryl and heteroaryl are optionally further substituted by one or more substituents selected from the group consisting of deuterium, alkyl, haloalkyl, halogen, amino, nitro, cyano, hydroxy, alkoxy, haloalkoxy, hydroxyalkyl, cycloalkyl, heterocyclyl, aryl, heteroaryl, -(CH₂)ₙR₅, -(CH₂)ₙORs, -(CH₂)ₙSR₅, -(CH₂),C(O)R₅, -(CH₂)ₙC(O)OR₅, -(CH₂)ₙS(O)ₘR₅, -(CH₂)ₙNR₅R₆, -(CH₂)ₙC(O)NR₅R₆, -(CH₂)ₙNR₅C(O)R₆ and -(CH₂)ₙNR₅S(O)ₘR₆; and
y is an integer of 0, 1, 2 or 3, and preferably y is 1; and
R₃∼R₆, m and n are as defined in claim 1.

12. The compound of formula (I), a stereoisomer thereof or a pharmaceutically acceptable salt thereof according to claim 1, which is a compound of formula (VII-A), a stereoisomer thereof or a pharmaceutically acceptable salt thereof: 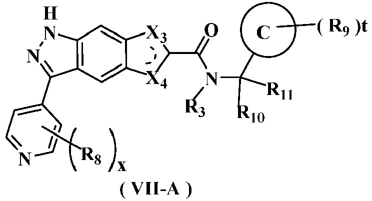 wherein:
X₃ and X₄ are each independently selected from the group consisting of N, NH and CH;
ring C is a structure selected from the group consisting of: 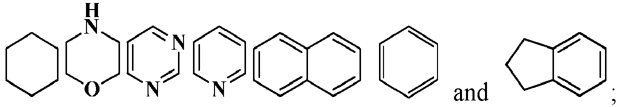
R₃ is selected from the group consisting of hydrogen, deuterium, C₁₋₈ alkyl, C₁₋₈ deuterated alkyl, C₁₋₈ haloalkyl, C₁₋₈ alkoxy and C₁₋₈ haloalkoxy;
R₈ is selected from the group consisting of hydrogen, deuterium, C₁₋₈ alkyl, C₁₋₈ deuterated alkyl, C₁₋₈ haloalkyl, C₁₋₈ alkoxy, C₁₋₈ haloalkoxy, halogen, amino, nitro, hydroxy, cyano, C₃₋₁₀ cycloalkyl, 3 to 10 membered heterocyclyl, 6 to 10 membered aryl and 5 to 12 membered heteroaryl;
R₉ is selected from the group consisting of hydrogen, deuterium, C₁₋₈ alkyl, C₁₋₈ deuterated alkyl, C₁₋₈ haloalkyl, C₁₋₈ alkoxy, C₁₋₈ haloalkoxy, halogen, amino, nitro, hydroxy, cyano, C₃₋₁₀ cycloalkyl, 3 to 10 membered heterocyclyl, 6 to 10 membered aryl and 5 to 12 membered heteroaryl;
R₁₀ and R₁₁ are identical or different and are each independently selected from the group consisting of hydrogen, deuterium, C₁₋₈ alkyl, C₁₋₈ deuterated alkyl, C₁₋₈ haloalkyl, C₁₋₈ alkoxy, C₁₋₈ haloalkoxy, halogen, amino, nitro, hydroxy, cyano, C₃₋₁₀ cycloalkyl, 3 to 10 membered heterocyclyl, 6 to 10 membered aryl, 5 to 12 membered heteroaryl, -(CH₂)ₙR₅, -(CH₂)ₙOR₅, -(CH₂)ₙSR₅, -(CH₂)ₙC(O)R₅, -(CH₂)ₙ,C(O)OR₅, -(CH₂)ₙS(O)ₘR₅, -(CH₂)ₙNR₅R₆, -(CH₂)ₙC(O)NR₅R₆, -(CH₂)ₙC(O)NHR₅, -(CH₂)ₙNR₅C(O)R₆ and -(CH₂)ₙNR₅S(O)ₘR₆-, wherein the C₁₋₈ alkyl, C₁₋₈ deuterated alkyl, C₁₋₈ haloalkyl, C₁₋₈ alkoxy, C₁₋₈ haloalkoxy, C₃₋₁₀ cycloalkyl, 3 to 10 membered heterocyclyl, 6 to 10 membered aryl and 5 to 12 membered heteroaryl are optionally further substituted by one or more substituents selected from the group consisting of deuterium, C₁₋₈ alkyl, halogen, hydroxy, amino, nitro, cyano, ester group, C₁₋₈ alkoxy, C₁₋₈ hydroxyalkyl, C₃₋₁₀ cycloalkyl, 3 to 10 membered heterocyclyl, 6 to 10 membered aryl and 5 to 12 membered heteroaryl;
R₅ and R₆ are identical or different and are each independently selected from the group consisting of hydrogen, deuterium, C₁₋₈ alkyl, C₁₋₈ deuterated alkyl, C₁₋₈ haloalkyl, hydroxy, amino, C₃₋₁₀ cycloalkyl, 3 to 10 membered heterocyclyl, 6 to 10 membered aryl and 5 to 12 membered heteroaryl, wherein the C₁₋₈ alkyl, C₁₋₈ deuterated alkyl, C₁₋₈ haloalkyl, C₃₋₁₀ cycloalkyl, 3 to 10 membered heterocyclyl, 6 to 10 membered aryl and 5 to 12 membered heteroaryl are optionally further substituted by one or more substituents selected from the group consisting of deuterium, C₁₋₈ alkyl, C₁₋₈ deuterated alkyl, C₁₋₈ haloalkyl, halogen, hydroxy, amino, nitro, cyano, C₁₋₈ alkoxy, C₁₋₈ hydroxyalkyl, C₃₋₁₀ cycloalkyl, 3 to 10 membered heterocyclyl, 6 to 10 membered aryl and 5 to 12 membered heteroaryl;
m is an integer of 0, 1 or 2;
n is an integer of 0, 1, 2, 3, 4 or 5;
x is an integer of 0, 1, 2, 3 or 4; and
t is an integer of 0, 1, 2, 3, 4 or 5.

13. The compound of formula (I), a stereoisomer thereof or a pharmaceutically acceptable salt thereof according to any one of claims 1, 3, 5, 6, 8 and 10, **characterized in that** R₁ is selected from the group consisting of hydrogen, C₁₋₈ alkyl, 5 to 10 membered aryl, 5 to 10 membered heteroaryl, -(CH₂)ₙR₃, -(CH₂)ₙOR₃, -(CH₂)ₙC(O)R₃, -(CH₂)ₙC(O)OR₃ and -(CH₂)ₙNR₃R₄, wherein the C₁₋₈ alkyl, 5 to 10 membered aryl and 5 to 10 membered heteroaryl are optionally further substituted by one or more substituents selected from the group consisting of C₁₋₈ alkyl, halogen, 5 to 10 membered aryl, 5 to 10 membered heteroaryl, -(CH₂)ₙR₅, -(CHR₄)ₙR₅ and -(CH₂)ₙOR₅;
preferably selected from the group consisting of hydrogen, C₁₋₆ alkyl, 5 to 6 membered aryl, 5 to 6 membered heteroaryl, -(CH₂)ₙR₃, -(CH₂)ₙOR₃, -(CH₂)ₙC(O)R₃ and -(CH₂)ₙNR₃R₄; and
more preferably selected from the group consisting of hydrogen, C₁₋₃ alkyl, 5 to 6 membered aryl, 5 to 6 membered heteroaryl, -(CH₂)ₙR₃, -(CH₂)ₙOR₃, -(CH₂)ₙC(O)R₃ and -(CH₂)ₙNR₃R₄.

14. The compound of formula (I), a stereoisomer thereof or a pharmaceutically acceptable salt thereof according to any one of claims 4-10, 12 and 13, **characterized in that** R₈ is selected from the group consisting of hydrogen, cyano, C₁₋₈ alkyl, C₁₋₈ alkoxy, halogen, oxo and C₁₋₈ haloalkyl; preferably selected from the group consisting of C₁₋₆ alkyl, C₁₋₆ alkoxy and halogen; and more preferably selected from the group consisting of C₁₋₃ alkyl, C₁₋₃ alkoxy and halogen.

15. The compound of formula (I), a stereoisomer thereof or a pharmaceutically acceptable salt thereof according to any one of claims 1 to 14, selected from the group consisting of:

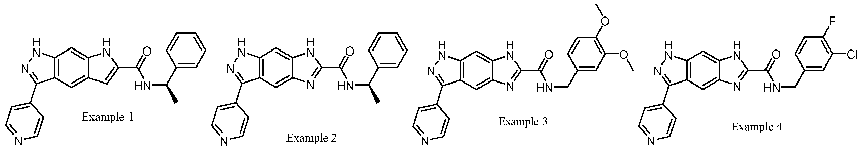

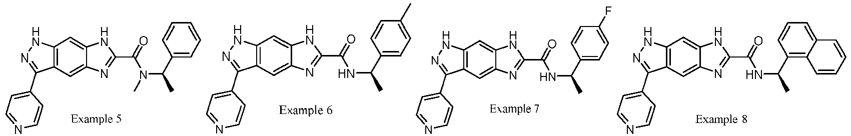

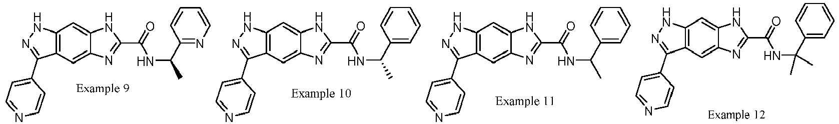

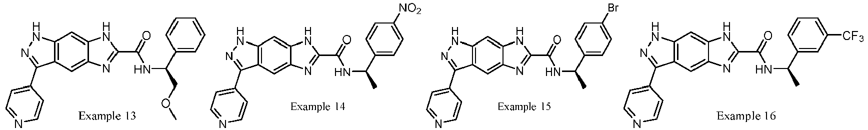

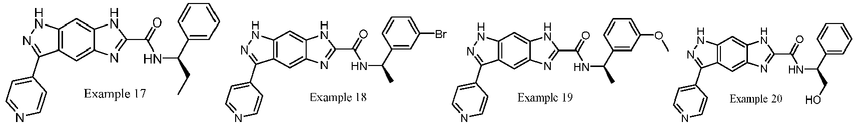

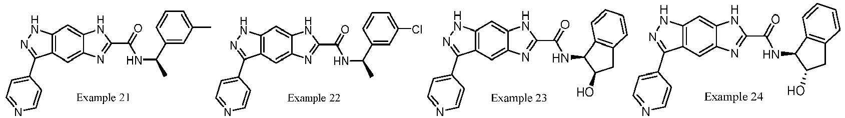

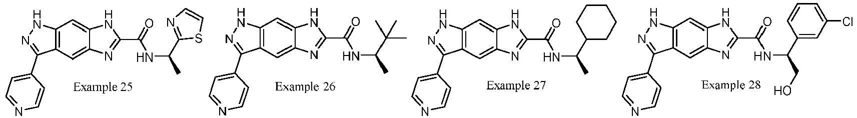

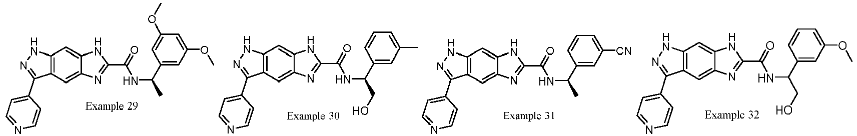

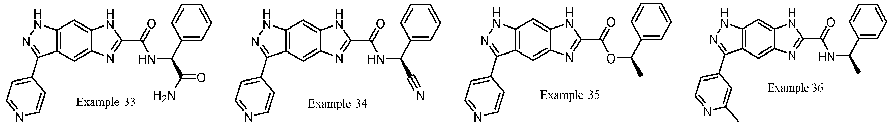

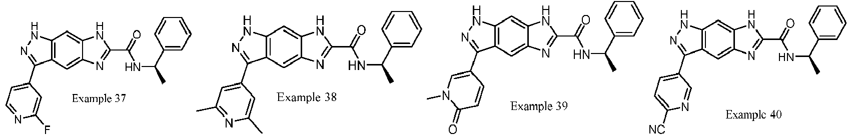

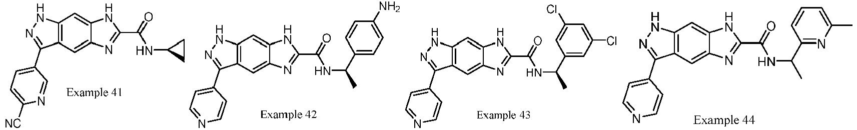

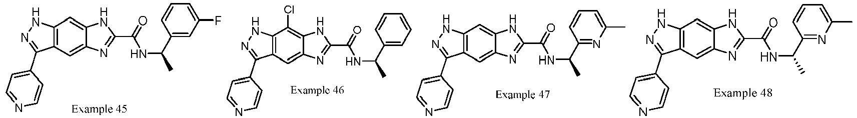

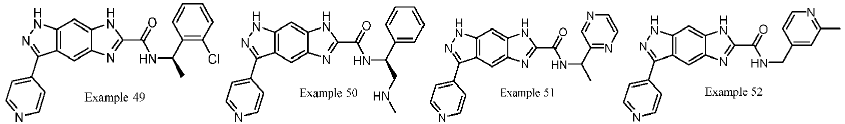

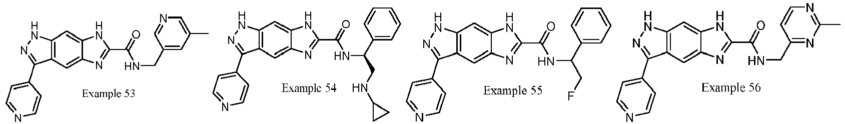

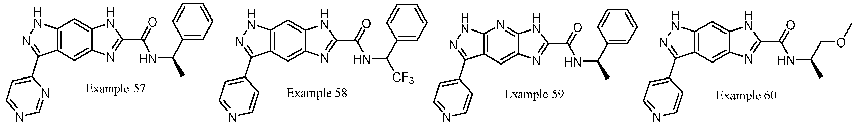

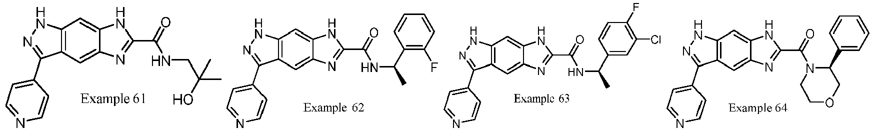

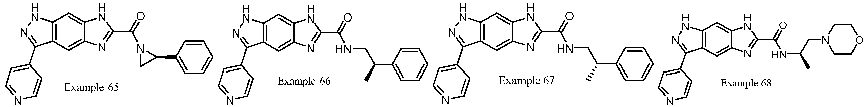

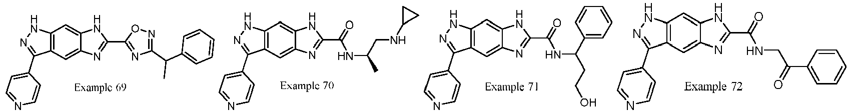

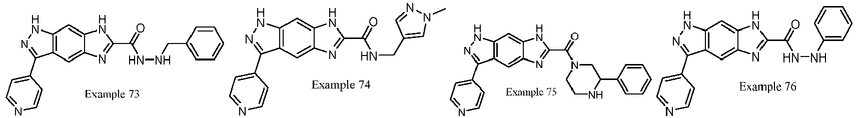

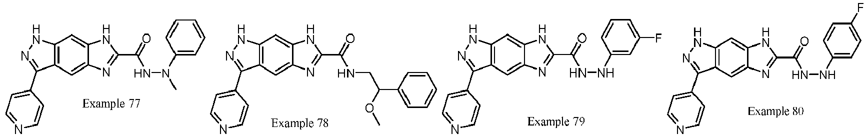

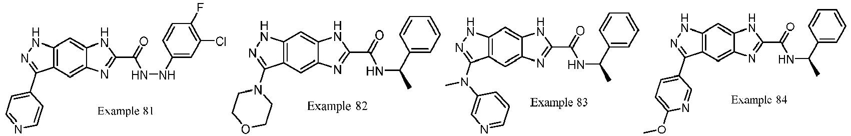

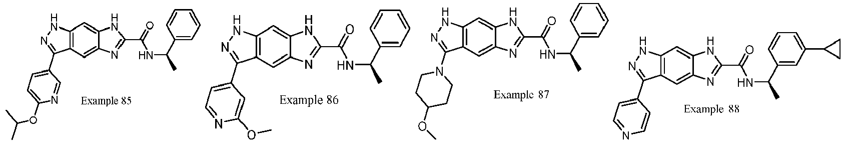

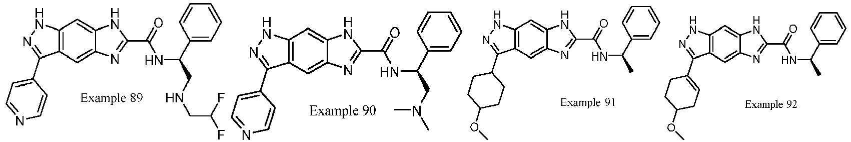

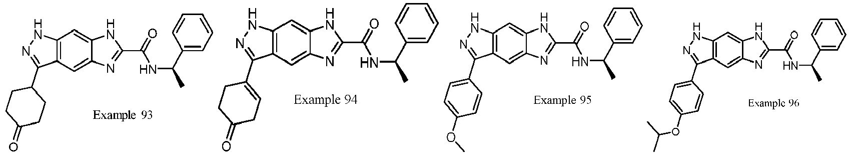

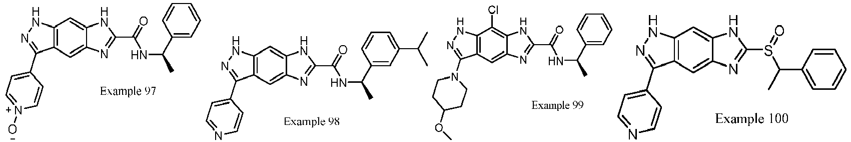

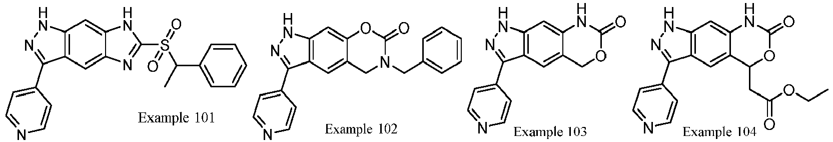

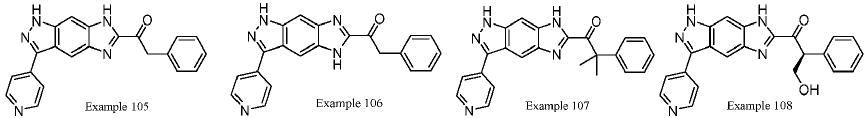

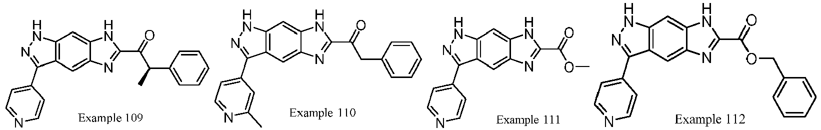

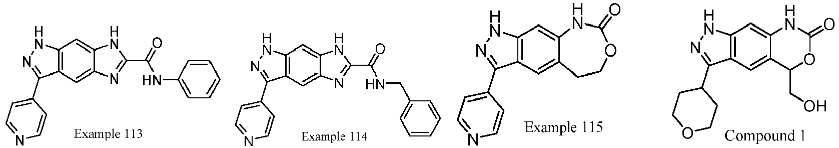

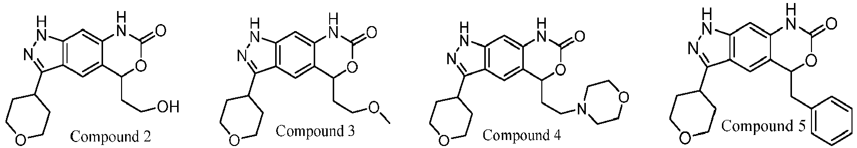

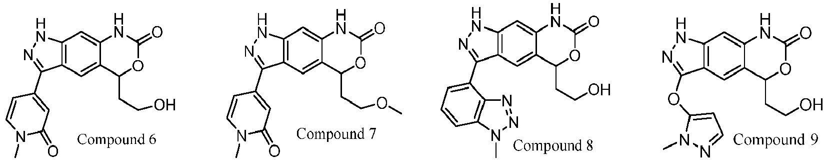

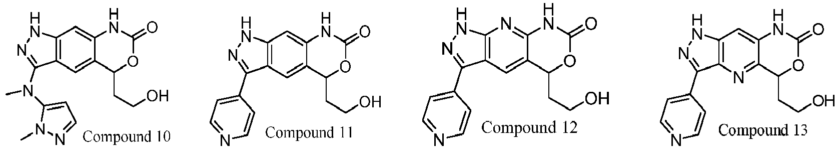

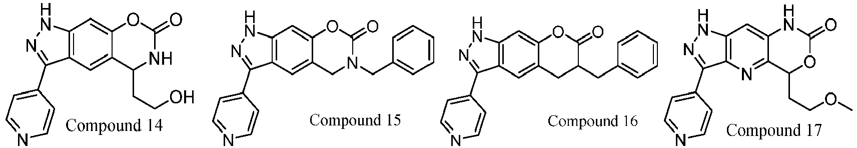

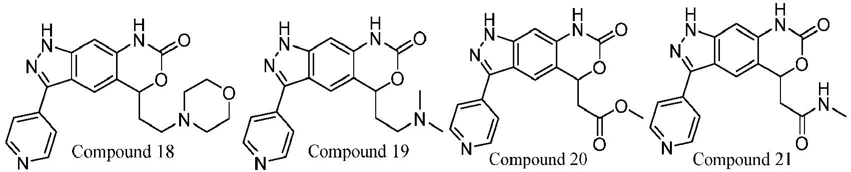

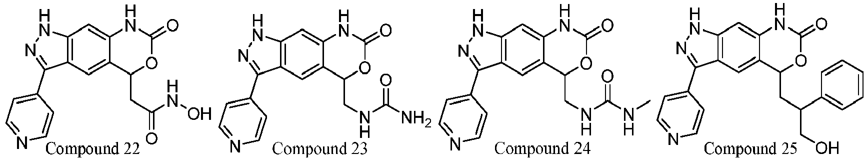

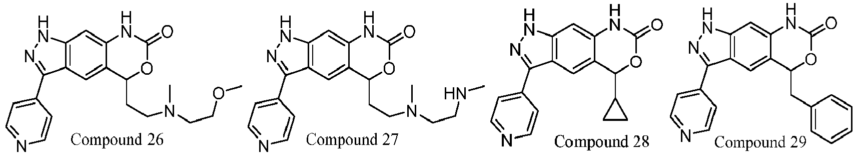

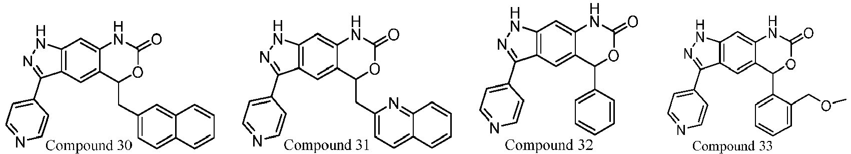

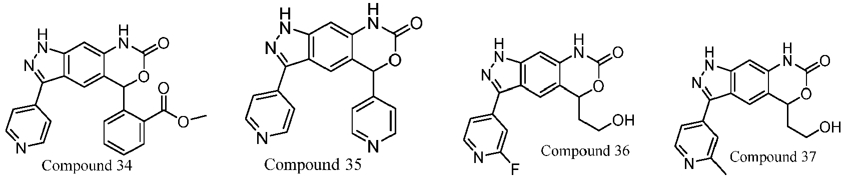

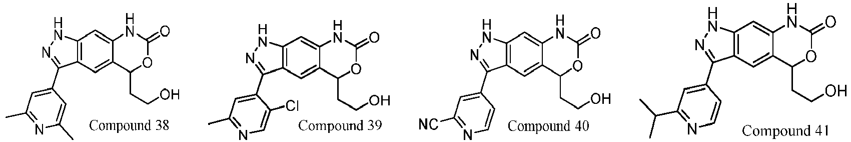

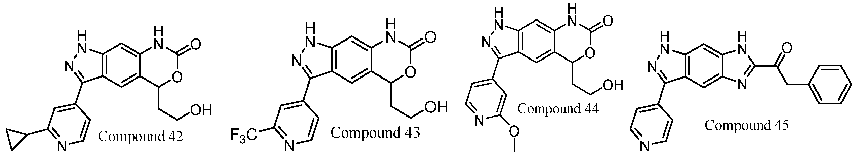

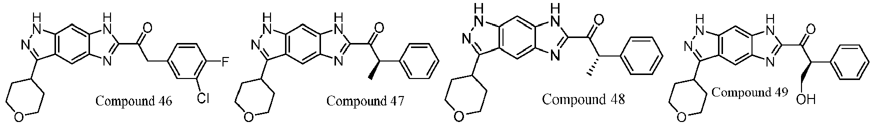

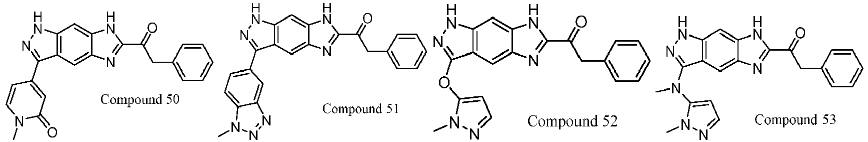

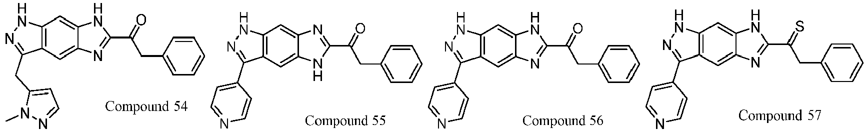

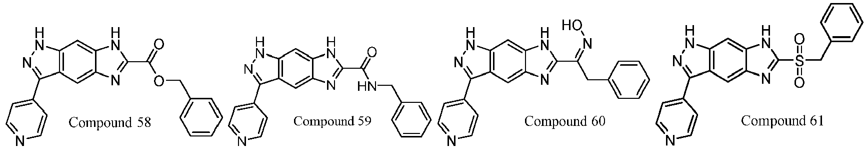

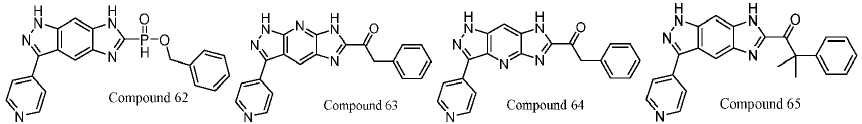

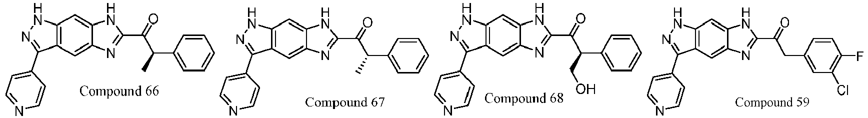

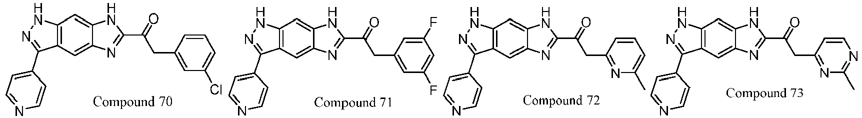

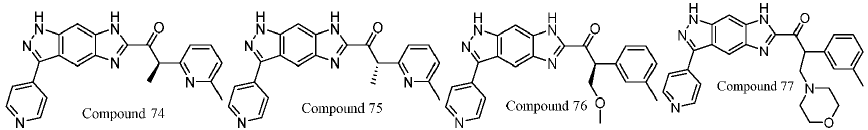

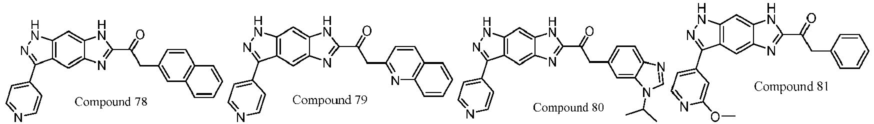

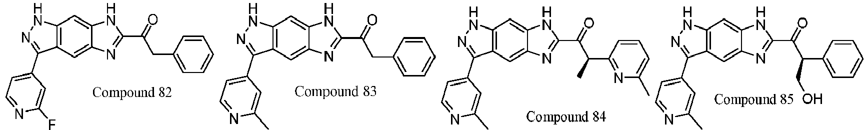

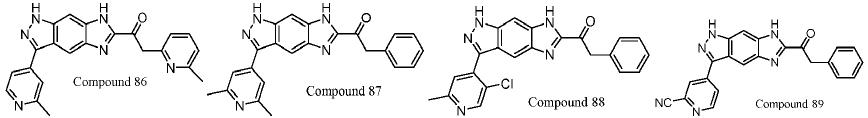

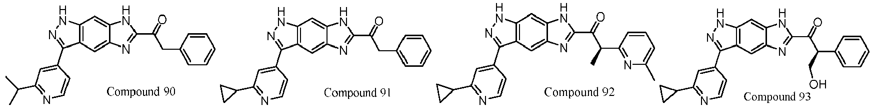

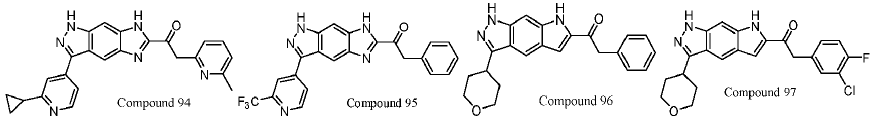

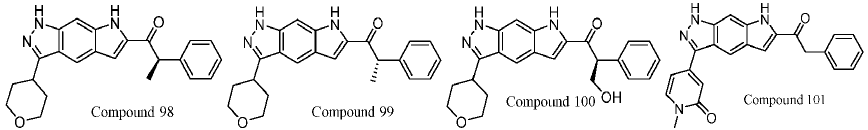

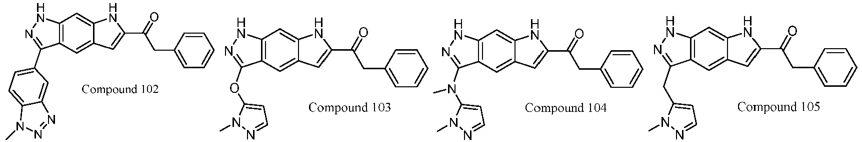

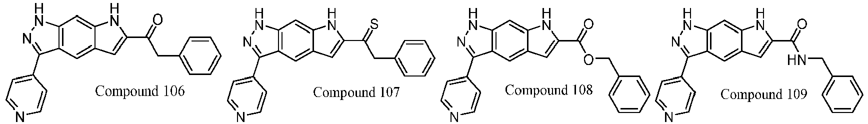

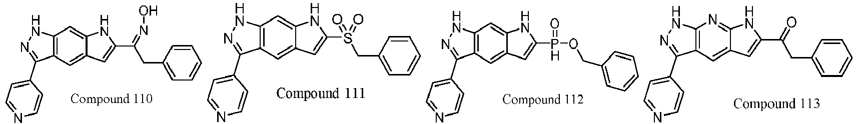

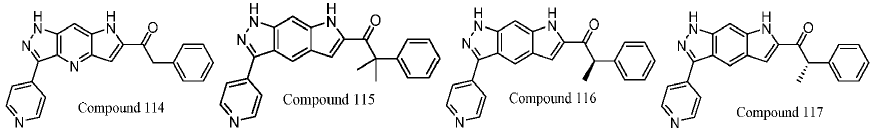

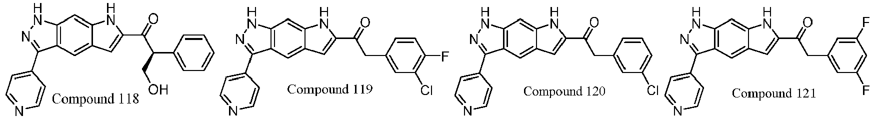

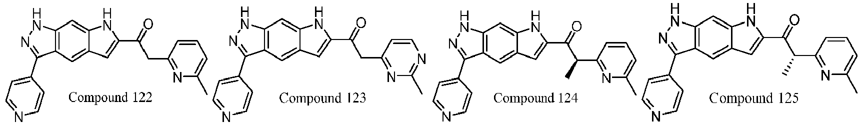

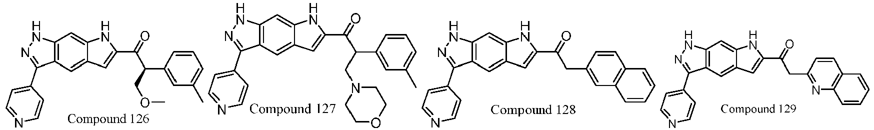

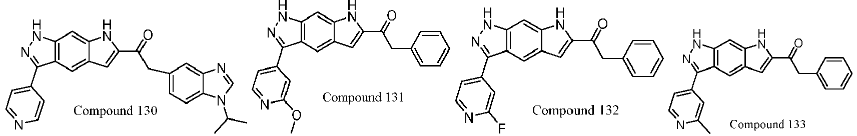

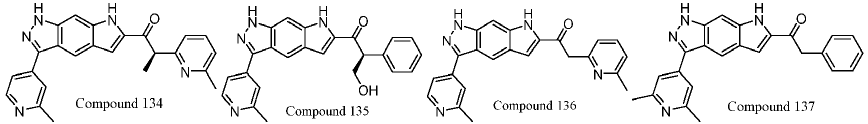

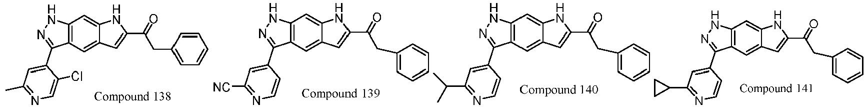

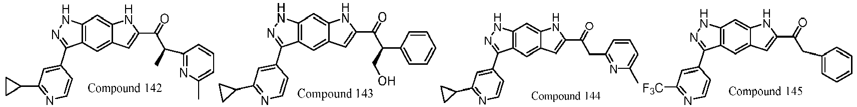

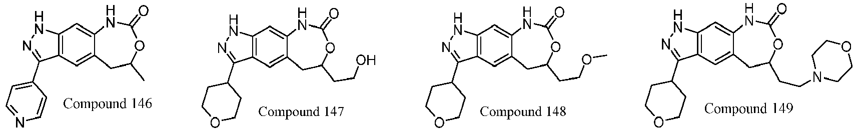

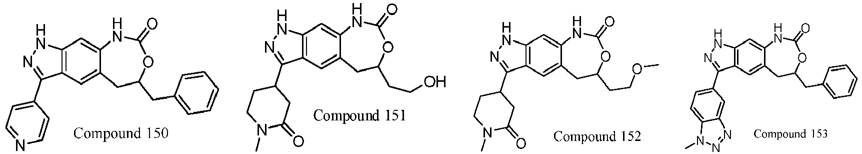

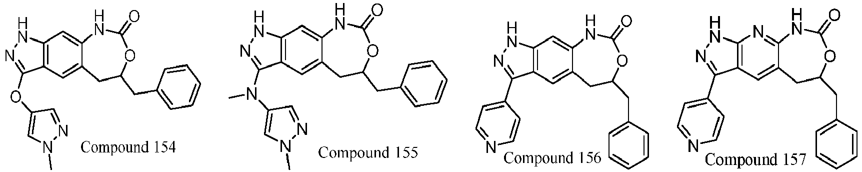

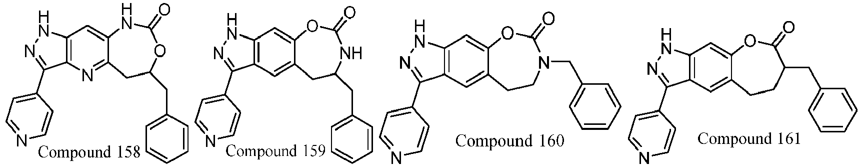

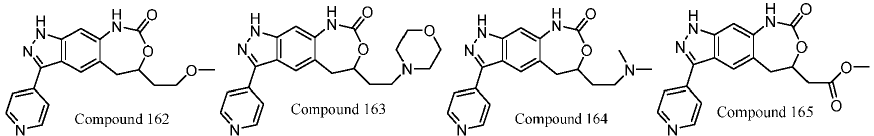

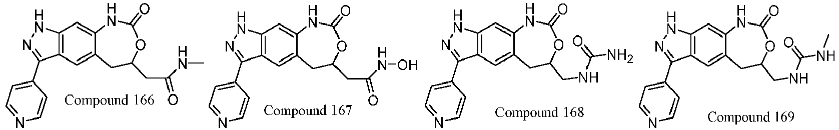

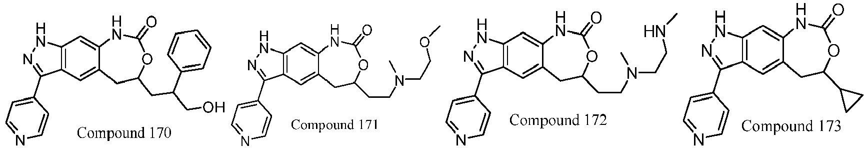

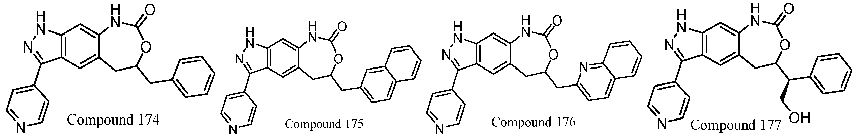

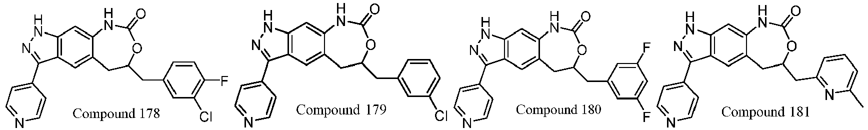

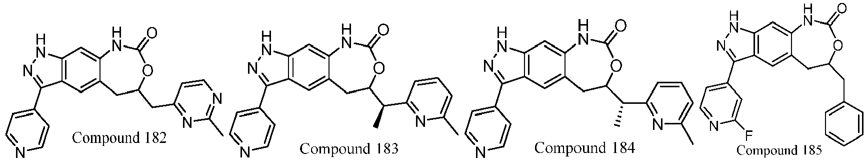

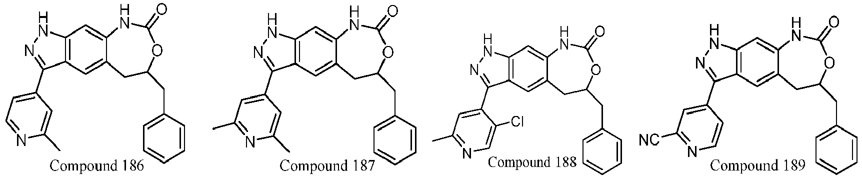

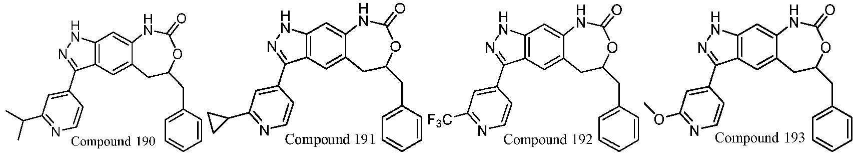

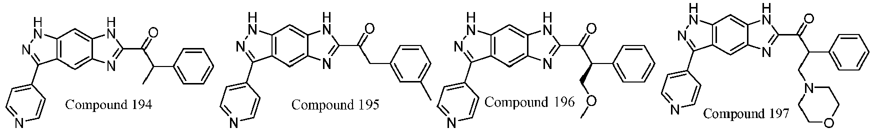

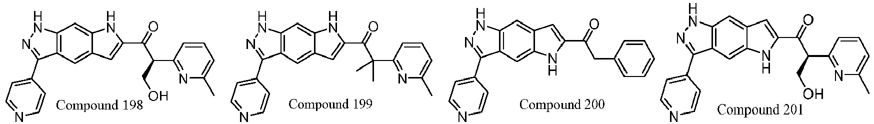

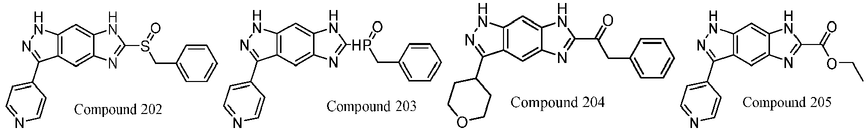

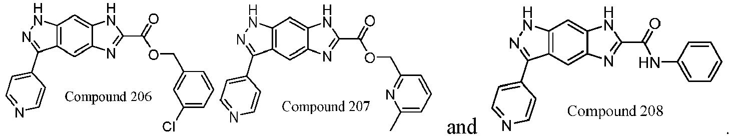

16. An intermediate for preparing the compound of formula (VII-A), a stereoisomer thereof or a pharmaceutically acceptable salt, which is a compound of formula (IX), a stereoisomer thereof or a pharmaceutically acceptable salt thereof: 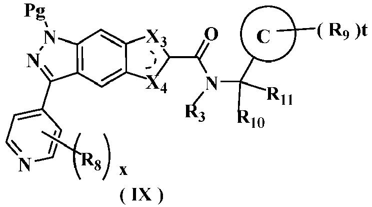 wherein:
Pg is an amino protecting group selected from the group consisting of benzyloxycarbonyl, *tert*-butoxycarbonyl, 9-fluorenylmethoxycarbonyl, benzyl, *p*-methoxybenzyl, allyloxycarbonyl, trityl and phthaloyl, and preferably trityl;
ring C, X₃, X₄, R₃, R₈₋R₁₁, t and x are as defined in claim 12.

17. A method for preparing the compound of formula (VII-A), comprising the following step of: 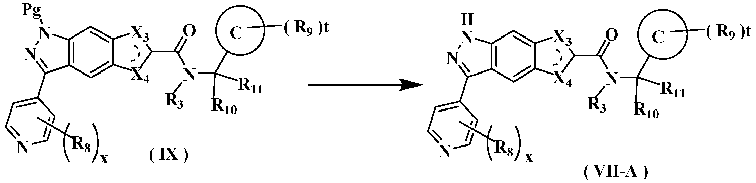 subjecting a compound of formula (IX) to a deprotection reaction under an acidic condition to obain the compound of formula (VII-A);
wherein:
ring C, Pg, X₃, X₄, R₃, R₈-R₁₁, t and x are as defined in claim 16.

18. A pharmaceutical composition, comprising a therapeutically effective amount of the compound of formula (I), a stereoisomer thereof or a pharmaceutically acceptable salt thereof according to any one of claims 1 to 15, and one or more pharmaceutically acceptable carriers, diluents or excipients.

19. Use of the compound of formula (I), a stereoisomer thereof or a pharmaceutically acceptable salt thereof according to any one of claims 1 to 15, or the pharmaceutical composition according to claim 18 in the preparation of an ERK inhibitor medicament.

20. Use of the compound of formula (I), a stereoisomer thereof or a pharmaceutically acceptable salt thereof according to any one of claims 1 to 15, or the pharmaceutical composition according to claim 18 in the preparation of a medicament for treating a cancer, bone disease, inflammatory disease, immunological disease, nervous system disease, metabolic disease, respiratory disease and heart disease, wherein the cancer is selected from the group consisting of breast cancer, pancreatic cancer, non-small cell lung cancer, thyroid cancer, seminoma, melanoma, bladder cancer, liver cancer, kidney cancer, myelodysplastic syndrome, acute myeloid leukemia and colorectal cancer.
